# EUROPEAN PATENT APPLICATION

(11) **EP 3 345 920 A1**
(43) Date of publication of application: **11.07.2018**
(21) Application number: 17209384.1
(22) Date of filing: 26.10.2010
(51) Int. Cl.: C07K 14/47, G01N 33/50, G01N 33/574

(54) **BREAST TUMOR MARKERS AND METHODS OF USE THEREOF**

(30) Priority: 26.10.2009 EP 09174060
(62) Divisional of application: 10787709.4
(71) Applicant: Externautics S.p.A., 53100 Siena (IT)
(72) Inventor: GRIFANTINI, Renata, 53100 SIENA (IT); PILERI, Piero, 53100 SIENA (IT); CAMPAGNOLI, Susanna, 53100 SIENA (IT); GRANDI, Alberto, 53100 SIENA (IT); PARRI, Matteo, 53100 SIENA (IT); PIERLEONI, Andrea, 53100 SIENA (IT); NOGAROTTO, Renzo, 53100 SIENA (IT)
(74) Representative: Bianchetti Bracco Minoja S.r.l.

(57) **Abstract**

Newly identified proteins as markers for the detection of breast tumors, or as therapeutic targets for treatment thereof; affinity ligands capable of selectively interacting with the newly identified markers, as well as methods for tumor diagnosis and therapy using such ligands.

SEQUENCE LISTING

<110>  Externautics S.p.A.

<120>  breast tumor markers and methos of use thereof

<130>  3325EURDIV

<160>  94

<170>  PatentIn version 3.3

<210>  1
<211>  132
<212>  PRT
<213>  Homo sapiens

<400>  1

Met Ser Gln Gly Arg His Leu Leu Glu Phe Leu Pro Leu Tyr Ile Ala
1               5                   10                  15

Phe Met Leu Arg Gly Val Cys Arg Ile Asp Ala Gly Ser Leu Asn Pro
            20                  25                  30

Glu Leu Phe Leu Pro Met Leu His Glu Glu Asp Trp Cys Trp Glu Ile
        35                  40                  45

Ala Gly His Val Asp Ser Gln Glu Leu Phe Val Gly Leu Phe Ser Ser
    50                  55                  60

Thr Ser Thr Gly His Ala Glu Leu Asp Lys Lys Val Asn Gly Leu Tyr
65                  70                  75                  80

Tyr Asp Ser Val Phe Gln Leu Ser Leu Asp Arg Met Arg His Thr Arg
                85                  90                  95

Ser Met Ala Arg Val Glu Arg Leu Arg His Arg Lys Ala Ile Gln Lys
            100                 105                 110

Lys Thr Gln Leu Val His His Leu Leu Phe Lys Gly Trp Ala Ser Asp
        115                 120                 125

Glu Thr Glu Ile
    130

<210>  2
<211>  399
<212>  DNA
<213>  Homo sapiens

<400>  2
atgtcacaag gcaggcatct tcttgagttt cttccattgt acatagcttt catgttacgt      60

ggggtttgta ggatagacgc tggaagcctt aatccagaac tgtttttgcc aatgttacat 120

gaagaggatt ggtgttggga gatagctggc catgtggact cccaagagtt attcgttggt 180

ttgttttcta gtacctctac tgggcatgca gagctggaca aaaaggttaa tggactttat 240

tatgactctg tattccagtt gtctctggac cgtatgcgtc atacaaggag tatggctaga 300

gtagagaggc tgagacacag gaaagcgatc cagaaaaaga ctcagttagt ccatcatctg 360

ctatttaaag gatgggcttc tgatgaaact gaaatttag 399

<210> 3
<211> 147
<212> PRT
<213> Homo sapiens

<400> 3

Met Gly Phe Ile Phe Ser Lys Ser Met Asn Glu Ser Met Lys Asn Gln
1               5                   10                  15

Lys Glu Phe Met Leu Met Asn Ala Arg Leu Gln Leu Glu Arg Gln Leu
            20                  25                  30

Ile Met Gln Ser Glu Met Arg Glu Arg Gln Met Ala Met Gln Ile Ala
        35                  40                  45

Trp Ser Arg Glu Phe Leu Lys Tyr Phe Gly Thr Phe Phe Gly Leu Ala
    50                  55                  60

Ala Ile Ser Leu Thr Ala Gly Ala Ile Lys Lys Lys Lys Pro Ala Phe
65                  70                  75                  80

Leu Val Pro Ile Val Pro Leu Ser Phe Ile Leu Thr Tyr Gln Tyr Asp
                85                  90                  95

Leu Gly Tyr Gly Thr Leu Leu Glu Arg Met Lys Gly Glu Ala Glu Asp
            100                 105                 110

Ile Leu Glu Thr Glu Lys Ser Lys Leu Gln Leu Pro Arg Gly Met Ile
        115                 120                 125

Thr Phe Glu Ser Ile Glu Lys Ala Arg Lys Glu Gln Ser Arg Phe Phe
    130                 135                 140

Ile Asp Lys
145

<210> 4
<211> 932
<212> DNA

<213>  Homo sapiens

<400>  4
gagcgaggcc cggtccctgc agcgggcgaa aggagcccgg gcctggaggt ttgcgtaccg      60

gtcgcctggt cccggcacca gcgccgccca gtgtggtttc ccataaggaa gctcttcttc     120

ctgcttggct tccaccttta acccttccac ctgggagcgt cctctaacac attcagacta     180

caagtccaga cccaggagag caaggcccag aaagaggtca aaatggggtt tatattttca     240

aaatctatga atgaaagcat gaaaaatcaa aaggagttca tgcttatgaa tgctcgactt     300

cagctggaaa ggcagctcat catgcagagt gaaatgaggg aaagacaaat ggccatgcag     360

attgcgtggt ctcgggaatt cctcaaatat tttggaactt tttttggcct tgcagccatc     420

tctttaacag ctggagcgat taaaaaaaag aagccagcct tcctggtccc gattgttcca     480

ttaagcttta tcctcaccta ccagtatgac ttgggctatg gaaccctttt agaaagaatg     540

aaaggtgaag ctgaggacat actggaaaca gaaaagagta aattgcagct gccaagagga     600

atgatcactt ttgaaagcat tgaaaaagcc agaaaggaac agagtagatt cttcatagac     660

aaatgaaatc atgcttacca atcaaatctc aaagcacaga attattgact tgaatcatgg     720

tttttacagt tttttaaatg ctcaagattt tgatattata gattttattt taaatattta     780

aaatgcaaga tagttttgag ctattttaaa ataaaattta taacattcaa cacaaaatca     840

tggaggtgct ctaaataact tttagatttc ctctctctgt gtgcattacc aatatctaag     900

tgtaaaatta ataaattgtt ttgaattcct gg                                   932


<210>  5
<211>  131
<212>  PRT
<213>  Homo sapiens

<400>  5

Met Phe Leu Gly Leu Val Gly Leu Arg Thr Lys Gly Arg Arg Trp Ile
1               5                   10                  15


Ser Ser Trp Ser Glu Gly Glu Asp Arg Gly Gln Ser Pro Glu Gly Val
            20                  25                  30


Leu Leu Thr Trp Val Phe Gly Thr Lys Cys Val Met His Pro Cys Glu
        35                  40                  45


Glu Thr Thr Lys Gln Ala Leu Cys Glu Gln Gln Gly Cys Leu Phe His
    50                  55                  60


Leu Gly Ala Asp Glu Leu Ser Pro Lys Arg Glu Ser Ala Gln Ser Ile
65                  70                  75                  80

Ser Phe Lys Trp Glu Asn Ser Ile Tyr Leu His Ala Thr Leu Phe Leu
                85                  90                  95

Ile Gly Glu Tyr Leu His Leu Ala Phe Tyr Tyr Phe Leu Leu Val Leu
            100                 105                 110

Tyr Ile Leu Cys Ser Phe Leu Ser Tyr Cys Leu Leu Leu Trp Leu Gly
        115                 120                 125

Ser Phe Leu
    130

<210>  6
<211>  2950
<212>  DNA
<213>  Homo sapiens

<400>  6
attatctagg tctcggagga tggagaaatc aaaagtgcca ttttctggcc atttagaacc      60

attgtcgagt ttgtattggg gccaagcagt gttgcagaag aaaataagac atttagattt     120

tagttcaggt gatagttgaa gaaattttaa gttcttgaga acacaggcta agggagaaga     180

aggaggaatg gagggtggaa gtttgcccat agtgaaggag gcaagtttaa agagaaaggt     240

agagacatgg agaaagggtt ggggagcagc cctgggctgc aatgtgggtg agcagccaaa     300

gcaggcatcc ccgcaattga cttgccacca agggaatgtg gttgaatgac caaggcaggc     360

atccctgaag atatcagacg ccaatggaat gtgggtgaat aatcaggcag gcatccccgg     420

aatgattaaa cactaaggga aggctgcctt cctgagtaca tgaccagcac cagagttttg     480

ggtccatgga taaaatgtgt ctcctttgtc tctactagaa aatgaaagga attgaaatta     540

agagaagaga gggagtgaag ggtggcacca agaatgaaag gagaaagagg ttgagggata     600

gtgagaaagg ttggagaaga gagtaaaaag aggccactta cccgatttaa aatttgtgag     660

atgttccttg ggctggttgg tctgaggacc aaaggtcgta ggtggatctc ttcatggagt     720

gagggtgagg acaggggaca gtctcctgaa ggagtcctgc tgacctgggt ctttggcacc     780

aaatgtgtca tgcatccatg tgaagagacc accaaacagg ctttgtgtga gcaacagggc     840

tgtttgtttc acctgggtgc agacgagttg agtccaaaaa gagagtcagc ccagtctata     900

tcttttaaat gggaaaattc aatctattta catgcaacgt tattcttgat aggtgagtac     960

ttacatctag cattctatta ttttctgctt gttttgtata tcctttgttc cttcctctct    1020

tactgtttac ttttgtggtt gggcagtttt ctgtagggat aagatttgag tcttatctct    1080

ttctccctat gtgttagctt taccagtgag tttttatagt ttcacatatt tttatgatgc    1140

tggttatcat cttctctgtg gggaacaggc cccccaaaac ctggccataa actggcccca    1200

aaactggcca taaacaaaat ctctgcagca ctgtgacatg tacatgatgg tcttaacgcc    1260

cacgctggaa ggttgtgggt ttaccagaat gagggcaagg aacacctggc ccacccaggg 1320

tggaaaaccg cttaaaggca ttcttaaacc acaaacaata gcatgagtga tctgtgcctt 1380

aaggccatgc tcctgctgca gatagctagt ccaacccatc cctttatttc agcccatctc 1440

ttcatttccc ataaggaata attttagtta atctaatatc tatagaaaga atgctaatga 1500

ctagcttgct gttaataaat acatgggtaa acctctgttg gaggctctca gctctgaagg 1560

ctgtgagacc cttgatttcc tacttcactc ctctatattt ctgtgtcttt aattcctcta 1620

gtgccactgg gttagagtct ccccgaccaa gctggtctca gcaagtggtc tccatcatgg 1680

gggctcgaat ccaggttgaa gggtcaccag agtgatggtt ggagaacatg gaactagctg 1740

gaggacacct gagtactctt aaagcaaacc ccgtggtgag taagaagggg agctcagaag 1800

catcagggta acaatgggac aagtgtgggg tctggttcgt tccatcttgg aactttttca 1860

cactgatgat gaggaagaag gagagtataa tgaagtaaca gaagaggtta tagagcaggt 1920

ttatttgcca gctaaagcta aagtggcaaa ggagggagag gttcatccct acccttctgc 1980

accccctcat tattattttg aagaaaaaga gtggcctgac cctccagatc tttcttttcc 2040

agaggacagt gggcaaaaat tagttgcccc agtgactgtt caagcagcac ctcgagcgac 2100

tgctcttagt tctattcagt caggaattca gcaagctaga tgagaaggtg attaagaggc 2160

ttggcagttc cctgttagac tacactgccc agaccaacag ggaaatattg tagctacatt 2220

tgagcctttt tgttttaaat tactcaaaga atttaaacaa gctattaatc agtatggacc 2280

aggttctcct tttgtaatgg gactattaaa gaacattgct gtttccagtc agatgattcc 2340

tactgactgg gacgctctta ctcaagcttg tctaactcct gcttagttct tacaatttaa 2400

aacttggtgg gcagatgaag cttccattca ggcttctcac aacacgcagg accaacctca 2460

aattaatata actgcagacc aacttttggg ggttggcagt tgggctggtt tagatgcaca 2520

aatggtcatg caggatgatg ccatagaaca gcttagagga gcgtgcatta gagcttgggg 2580

aaaaaaaatc acttcaagtg gagaacaata ccctttcttt agtgctataa aacagggacc 2640

agaagaatca tatgtggatt ttatagctca gttacaggag tctcttaaaa agatgactgc 2700

agatttggct gctcaggata tagtgttgca attattagct ttcaacaatg ctaatcctga 2760

ttgccaggct gctctgtgac ctatcagagg gaaagcacat ttagttgatt atatcaaggc 2820

ctgtggtggt atcagaggta atctgcatca ggccacctgc tagcacgggc aatggcagga 2880

ctgagagtgg atacagaaag tactccattt cctggagctt gttttaactg tgggaagcat 2940

ggtcatactg 2950

<210> 7
<211> 316
<212> PRT

<213>  Homo sapiens

<400>  7

Met Ala Ser Ala Asp Glu Leu Thr Phe His Glu Phe Glu Glu Ala Thr
1               5                   10                  15

Asn Leu Leu Ala Asp Thr Pro Asp Ala Ala Thr Thr Ser Arg Ser Asp
            20                  25                  30

Gln Leu Thr Pro Gln Gly His Val Ala Val Ala Val Gly Ser Gly Gly
        35                  40                  45

Ser Tyr Gly Ala Glu Asp Glu Val Glu Glu Glu Ser Asp Lys Ala Ala
    50                  55                  60

Leu Leu Gln Glu Gln Gln Gln Gln Gln Gln Pro Gly Phe Trp Thr Phe
65                  70                  75                  80

Ser Tyr Tyr Gln Ser Phe Phe Asp Val Asp Thr Ser Gln Val Leu Asp
                85                  90                  95

Arg Ile Lys Gly Ser Leu Leu Pro Arg Pro Gly His Asn Phe Val Arg
            100                 105                 110

His His Leu Arg Asn Arg Pro Asp Leu Tyr Gly Pro Phe Trp Ile Cys
        115                 120                 125

Ala Thr Leu Ala Phe Val Leu Ala Val Thr Gly Asn Leu Thr Leu Val
    130                 135                 140

Leu Ala Gln Arg Arg Asp Pro Ser Ile His Tyr Ser Pro Gln Phe His
145                 150                 155                 160

Lys Val Thr Val Ala Gly Ile Ser Ile Tyr Cys Tyr Ala Trp Leu Val
                165                 170                 175

Pro Leu Ala Leu Trp Gly Phe Leu Arg Trp Arg Lys Gly Val Gln Glu
            180                 185                 190

Arg Met Gly Pro Tyr Thr Phe Leu Glu Thr Val Cys Ile Tyr Gly Tyr
        195                 200                 205

Ser Leu Phe Val Phe Ile Pro Met Val Val Leu Trp Leu Ile Pro Val
    210                 215                 220

Pro Trp Leu Gln Trp Leu Phe Gly Ala Leu Ala Leu Gly Leu Ser Ala
225                 230                 235                 240

Ala Gly Leu Val Phe Thr Leu Trp Pro Val Val Arg Glu Asp Thr Arg
                245                 250                 255

Leu Val Ala Thr Val Leu Leu Ser Val Val Val Leu Leu His Ala Leu
            260                 265                 270

Leu Ala Met Gly Cys Lys Leu Tyr Phe Phe Gln Ser Leu Pro Pro Glu
        275                 280                 285

Asn Val Ala Pro Pro Pro Gln Ile Thr Ser Leu Pro Ser Asn Ile Ala
    290                 295                 300

Leu Ser Pro Thr Leu Pro Gln Ser Leu Ala Pro Ser
305                 310                 315

<210>  8
<211>  316
<212>  PRT
<213>  Homo sapiens

<400>  8

Met Ala Ser Ala Asp Glu Leu Thr Phe His Glu Phe Glu Glu Ala Thr
1               5                   10                  15

Asn Leu Leu Ala Asp Thr Pro Asp Ala Ala Thr Thr Ser Arg Ser Asp
            20                  25                  30

Gln Leu Thr Pro Gln Gly His Val Ala Val Ala Val Gly Ser Gly Gly
        35                  40                  45

Ser Tyr Gly Ala Glu Asp Glu Val Glu Glu Glu Ser Asp Lys Ala Ala
    50                  55                  60

Leu Leu Gln Glu Gln Gln Gln Gln Gln Gln Pro Gly Phe Trp Thr Phe
65                  70                  75                  80

Ser Tyr Tyr Gln Ser Phe Phe Asp Val Asp Thr Ser Gln Val Leu Asp
                85                  90                  95

Arg Ile Lys Gly Ser Leu Leu Pro Arg Pro Gly His Asn Phe Val Arg
            100                 105                 110

His His Leu Arg Asn Arg Pro Asp Leu Tyr Gly Pro Phe Trp Ile Cys
        115                 120                 125

Ala Thr Leu Ala Phe Val Leu Ala Val Thr Gly Asn Leu Thr Leu Val
    130                 135                 140

Leu Ala Gln Arg Arg Asp Pro Ser Ile His Tyr Ser Pro Gln Phe His
145                 150                 155                 160

Lys Val Thr Val Ala Gly Ile Ser Ile Tyr Cys Tyr Ala Trp Leu Val
                165                 170                 175

Pro Leu Ala Leu Trp Gly Phe Leu Arg Trp Arg Lys Gly Val Gln Glu
            180                 185                 190

Arg Met Gly Pro Tyr Thr Phe Leu Glu Thr Val Cys Ile Tyr Gly Tyr
        195                 200                 205

Ser Leu Phe Val Phe Ile Pro Met Val Val Leu Trp Leu Ile Pro Val
    210                 215                 220

Pro Trp Leu Gln Trp Leu Phe Gly Ala Leu Ala Leu Gly Leu Ser Ala
225                 230                 235                 240

Ala Gly Leu Val Phe Thr Leu Trp Pro Val Val Arg Glu Asp Thr Arg
                245                 250                 255

Leu Val Ala Thr Val Leu Leu Ser Val Val Val Leu Leu His Ala Leu
            260                 265                 270

Leu Ala Met Gly Cys Lys Leu Tyr Phe Phe Gln Ser Leu Pro Pro Glu
        275                 280                 285

Asn Val Ala Pro Pro Pro Gln Ile Thr Ser Leu Pro Ser Asn Ile Ala
    290                 295                 300

Leu Ser Pro Thr Leu Pro Gln Ser Leu Ala Pro Ser
305                 310                 315

<210>  9
<211>  1210
<212>  DNA
<213>  Homo sapiens

<400>  9
aggtgagggg gggcggagcg cacctgtggg gacgggacga cgagttcaag cctccgtggg      60

tgcagttggt cgccagcgag ggatgcggag acgcccctga acgaccatgg catcggccga     120

cgagctgacc ttccatgaat tcgaggaggc cactaatctt ctggctgaca ccccagatgc     180

agccaccacc agcagaagcg atcagctgac cccacaaggg cacgtggctg tggccgtggg     240

ctcaggtggc agctatggag ccgaggatga ggtggaggag gagagtgaca aggccgcgct     300

cctgcaggag cagcagcagc agcagcagcc gggattctgg accttcagct actatcagag     360

cttctttgac gtggacacct cacaggtcct ggaccggatc aaaggctcac tgctgccccg 420

gcctggccac aactttgtgc ggcaccatct gcggaatcgg ccggatctgt atggcccctt 480

ctggatctgt gccacgttgg cctttgtcct ggccgtcact ggcaacctga cgctggtgct 540

ggcccagagg agggacccct ccatccacta cagcccccag ttccacaagg tgaccgtggc 600

aggcatcagc atctactgct atgcgtggct ggtgcccctg gccctgtggg gcttcctgcg 660

gtggcgcaag ggtgtccagg agcgcatggg gccctacacc ttcctggaga ctgtgtgcat 720

ctacggctac tccctctttg tcttcatccc catggtggtc ctgtggctca tccctgtgcc 780

ttggctgcag tggctctttg gggcgctggc cctgggcctg tcagccgccg ggctggtatt 840

caccctctgg cccgtggtcc gtgaggacac caggctggtg gccacagtgc tgctgtccgt 900

ggtcgtgctg ctccacgccc tcctggccat gggctgtaag ttgtacttct tccagtcgct 960

gcctccggag aacgtggctc ctccacccca aatcacatct ctgccctcaa acatcgcgct 1020

gtcccctacc ttgccgcagt ccctggcccc ctcctaggaa ggcccgggtc ccacaggcaa 1080

cacctaagtg gaccaacccc tctgcctgtc ctgcccccca gacgatgact gaaggctcct 1140

ttgacacctt gagatgattc tgctactttc cagacttttc ttacaaagca aacactttta 1200

ttttctatgc 1210

<210> 10
<211> 1502
<212> DNA
<213> Homo sapiens

<400> 10
ggatgttgct gtcaggtctg agtcggttgg agtctgacgg gtaggcgaga cgcgcaggcg 60

cagagagccc cagccacgcc ggcccaggtg gcctcagcga gggatgcgga gacgcccctg 120

aacgaccatg gcatcggccg acgagctgac cttccatgaa ttcgaggagg ccactaatct 180

tctggctgac accccagatg cagccaccac cagcagaagc gatcagctga ccccacaagg 240

gcacgtggct gtggccgtgg gctcaggtgg cagctatgga gccgaggatg aggtggagga 300

ggagagtgac aaggccgcgc tcctgcagga gcagcagcag cagcagcagc cgggattctg 360

gaccttcagc tactatcaga gcttctttga cgtggacacc tcacaggtcc tggaccggat 420

caaaggctca ctgctgcccc ggcctggcca caactttgtg cggcaccatc tgcggaatcg 480

gccggatctg tatggcccct tctggatctg tgccacgttg gcctttgtcc tggccgtcac 540

tggcaacctg acgctggtgc tggcccagag gagggacccc tccatccact acagccccca 600

gttccacaag gtgaccgtgg caggcatcag catctactgc tatgcgtggc tggtgcccct 660

ggccctgtgg ggcttcctgc ggtggcgcaa gggtgtccag gagcgcatgg ggccctacac 720

cttcctggag actgtgtgca tctacggcta ctccctcttt gtcttcatcc ccatggtggt 780

cctgtggctc atccctgtgc cttggctgca gtggctcttt ggggcgctgg ccctgggcct 840

gtcagccgcc gggctggtat tcaccctctg gcccgtggtc cgtgaggaca ccaggctggt 900

ggccacagtg ctgctgtccg tggtcgtgct gctccacgcc ctcctggcca tgggctgtaa 960

gttgtacttc ttccagtcgc tgcctccgga gaacgtggct cctccacccc aaatcacatc 1020

tctgccctca aacatcgcgc tgtcccctac cttgccgcag tccctggccc cctcctagga 1080

aggcccgggt cccacaggca acacctaagt ggaccaaccc ctctgcctgt cctgcccccc 1140

agacgatgac tgaaggctcc tttgacacct tgagatgatt ctgctacttt ccagactttt 1200

cttacaaagc aaacactttt attttctatg caaaggtgat tcagagaatt tatataaagg 1260

cgggcgaggg gcagccgagc agggagcttt gggacagggc tggggccccc atatcccccc 1320

cgggccacct gctttccctc ctatggctcc cctggaacag gagggagagc caagggggcg 1380

gcccagcctg gacagcgccc gctcctgcct gggtgcacac acggcgggcc tgagctccag 1440

catctgagtt tgggggtatg agaaacaggg gagcagaagg agaagaaaac tgcctgtgct 1500

gc 1502

<210> 11
<211> 157
<212> PRT
<213> Homo sapiens

<400> 11

Met Leu Pro Glu Gln Gly Pro Gln Pro Ser Thr Met Pro Leu Trp Cys
1               5                   10                  15

Leu Leu Ala Ala Cys Thr Ser Leu Pro Arg Gln Ala Ala Thr Met Leu
            20                  25                  30

Glu Glu Ala Ala Ser Pro Asn Glu Ala Val His Ala Ser Thr Ser Gly
        35                  40                  45

Ser Gly Ala Leu Thr Asp Gln Thr Phe Thr Asp Leu Ser Ala Ala Glu
    50                  55                  60

Ala Ser Ser Glu Glu Val Pro Asp Phe Met Glu Val Pro His Ser Val
65                  70                  75                  80

His His Lys Ile Asn Cys Phe Phe Tyr Leu Glu Lys Gln Leu Cys Gln
                85                  90                  95

Leu Pro Ser Pro Leu Cys Leu Ser Ser Leu Leu Thr Leu Lys Leu Lys
            100                 105                 110

Thr Thr Val Pro Ala Pro Gly Arg Trp Trp Ser Phe Gln Pro His Lys

115                 120                 125

Ala Phe Pro Leu Leu Val Gly Thr Pro Gly Ser Trp Gln Ser Thr Ile
    130                 135                 140

Asp Pro Ala Trp Ala Ala Pro Ser Gln Pro Ser Pro Gly
145                 150                 155

<210>  12
<211>  474
<212>  DNA
<213>  Homo sapiens

<400>  12
atgcttccag agcaagggcc ccagccttcc acgatgccgc tctggtgcct cctcgccgcc      60

tgcaccagcc tcccaaggca ggcagccacc atgctggagg aagctgcttc tcccaacgag     120

gctgtccacg catcaacatc aggcagtggc gcactcactg atcagacatt tacagacctc     180

tcagctgccg aggcctcctc agaggaggtt cctgacttca tggaggtgcc acactctgtt     240

caccataaaa ttaactgctt tttctactta gaaaaacaac tctgccaact gccgtcccca     300

ctgtgtctaa gcagcttgct tactttaaaa ttaaaaacaa cggtcccagc tcctggcagg     360

tggtggagct tccagcctca caaggcattc ccacttctgg tgggcactcc tggaagctgg     420

cagagcacaa tcgatcccgc gtgggcggcc ccctctcagc caagcccagg gtga           474

<210>  13
<211>  241
<212>  PRT
<213>  Homo sapiens

<400>  13

Met Lys Phe Ile Leu Leu Trp Ala Leu Leu Asn Leu Thr Val Ala Leu
1               5                   10                  15

Ala Phe Asn Pro Asp Tyr Thr Val Ser Ser Thr Pro Pro Tyr Leu Val
            20                  25                  30

Tyr Leu Lys Ser Asp Tyr Leu Pro Cys Ala Gly Val Leu Ile His Pro
        35                  40                  45

Leu Trp Val Ile Thr Ala Ala His Cys Asn Leu Pro Lys Leu Arg Val
    50                  55                  60

Ile Leu Gly Val Thr Ile Pro Ala Asp Ser Asn Glu Lys His Leu Gln
65                  70                  75                  80

Val Ile Gly Tyr Glu Lys Met Ile His His Pro His Phe Ser Val Thr
                85                  90                  95

Ser Ile Asp His Asp Ile Met Leu Ile Lys Leu Lys Thr Glu Ala Glu
            100                 105                 110

Leu Asn Asp Tyr Val Lys Leu Ala Asn Leu Pro Tyr Gln Thr Ile Ser
        115                 120                 125

Glu Asn Thr Met Cys Ser Val Ser Thr Trp Ser Tyr Asn Val Cys Asp
    130                 135                 140

Ile Tyr Lys Glu Pro Asp Ser Leu Gln Thr Val Asn Ile Ser Val Ile
145                 150                 155                 160

Ser Lys Pro Gln Cys Arg Asp Ala Tyr Lys Thr Tyr Asn Ile Thr Glu
                165                 170                 175

Asn Met Leu Cys Val Gly Ile Val Pro Gly Arg Arg Gln Pro Cys Lys
            180                 185                 190

Glu Val Ser Ala Ala Pro Ala Ile Cys Asn Gly Met Leu Gln Gly Ile
        195                 200                 205

Leu Ser Phe Ala Asp Gly Cys Val Leu Arg Ala Asp Val Gly Ile Tyr
    210                 215                 220

Ala Lys Ile Phe Tyr Tyr Ile Pro Trp Ile Glu Asn Val Ile Gln Asn
225                 230                 235                 240

Asn

<210>  14
<211>  912
<212>  DNA
<213>  Homo sapiens

<400>  14
aaggctggca aaaaggagac cagacaggag gcgtctgtag agatatcatg aacttcaact      60

tagctttgtt ttccagagac tggagctaaa ctgggctttc aacatcatca tgaagtttat     120

cctcctctgg gccctcttga atctgactgt tgctttggcc tttaatccag attacacagt     180

cagctccact cccccttact tggtctattt gaaatctgac tacttgccct gcgctggagt     240

cctgatccac ccgctttggg tgatcacagc tgcacactgc aatttaccaa agcttcgggt     300

gatattgggg gttacaatcc cagcagactc taatgaaaag catctgcaag tgattggcta     360

tgagaagatg attcatcatc cacacttctc agtcacttct attgatcatg acatcatgct     420

aatcaagctg aaaacagagg ctgaactcaa tgactatgtg aaattagcca acctgcccta     480

ccaaactatc tctgaaaata ccatgtgctc tgtctctacc tggagctaca atgtgtgtga 540

tatctacaaa gagcccgatt cactgcaaac tgtgaacatc tctgtaatct ccaagcctca 600

gtgtcgcgat gcctataaaa cctacaacat cacggaaaat atgctgtgtg tgggcattgt 660

gccaggaagg aggcagccct gcaaggaagt ttctgctgcc ccggcaatct gcaatgggat 720

gcttcaagga atcctgtctt ttgcggatgg atgtgttttg agagccgatg ttggcatcta 780

tgccaaaatt ttttactata taccctggat tgaaaatgta atccaaaata actgagctgt 840

ggcagttgtg gaccatatga cacagcttgt ccccatcgtt cacctttaga attaaatata 900

aattaactcc tc 912

<210> 15
<211> 279
<212> PRT
<213> Homo sapiens

<400> 15

Met Met Leu Met Gln Ala Leu Val Leu Phe Thr Leu Asp Ser Leu Asp
1               5                   10                  15

Met Leu Pro Ala Val Lys Ala Thr Trp Leu Tyr Gly Ile Gln Ile Thr
            20                  25                  30

Ser Leu Leu Leu Val Cys Ile Leu Gln Phe Phe Asn Ser Met Ile Leu
        35                  40                  45

Gly Ser Leu Leu Ile Ser Phe Asn Leu Ser Val Phe Ile Ala Arg Lys
    50                  55                  60

Leu Gln Lys Asn Leu Lys Thr Gly Ser Phe Leu Asn Arg Leu Gly Lys
65                  70                  75                  80

Leu Leu Leu His Leu Phe Met Val Leu Cys Leu Thr Leu Phe Leu Asn
                85                  90                  95

Asn Ile Ile Lys Lys Ile Leu Asn Leu Lys Ser Asp Glu His Ile Phe
            100                 105                 110

Lys Phe Leu Lys Ala Lys Phe Gly Leu Gly Ala Thr Arg Asp Phe Asp
        115                 120                 125

Ala Asn Leu Tyr Leu Cys Glu Glu Ala Phe Gly Leu Leu Pro Phe Asn
    130                 135                 140

Thr Phe Gly Arg Leu Ser Asp Thr Leu Leu Phe Tyr Ala Tyr Ile Phe
145                 150                 155                 160

Val Leu Ser Ile Thr Val Ile Val Ala Phe Val Val Ala Phe His Asn
                165                 170                 175

Leu Ser Asp Ser Thr Asn Gln Gln Ser Val Gly Lys Met Glu Lys Gly
            180                 185                 190

Thr Val Asp Leu Lys Pro Glu Thr Ala Tyr Asn Leu Ile His Thr Ile
        195                 200                 205

Leu Phe Gly Phe Leu Ala Leu Ser Thr Met Arg Met Lys Tyr Leu Trp
    210                 215                 220

Thr Ser His Met Cys Val Phe Ala Ser Phe Gly Leu Cys Ser Pro Glu
225                 230                 235                 240

Ile Trp Glu Leu Leu Leu Lys Ser Val His Leu Tyr Asn Pro Lys Arg
                245                 250                 255

Ile Cys Ile Met Arg Tyr Ser Val Pro Ile Leu Ile Leu Leu Tyr Leu
            260                 265                 270

Cys Tyr Lys Asn Gln Lys Ser
        275

<210>  16
<211>  102
<212>  PRT
<213>  Homo sapiens

<400>  16

Met Met Ser Ile Arg Gln Arg Arg Glu Ile Arg Ala Thr Glu Val Ser
1               5                   10                  15

Glu Asp Phe Pro Ala Gln Glu Glu Asn Val Lys Leu Glu Asn Lys Leu
            20                  25                  30

Pro Ser Gly Cys Thr Ser Arg Arg Leu Trp Lys Ile Leu Ser Leu Thr
        35                  40                  45

Ile Gly Gly Thr Ile Ala Leu Cys Ile Gly Leu Leu Thr Ser Val Tyr
    50                  55                  60

Leu Ala Thr Leu His Glu Asn Asp Leu Trp Phe Ser Asn Ile Lys Val
65                  70                  75                  80

Trp Ser Phe Phe Asp His Cys Ile Ile His Ser Val Gly Ser Pro Val
                85                  90                  95

Val Ser His Val Asp Glu
                100

<210>  17
<211>  716
<212>  PRT
<213>  Homo sapiens

<400>  17

Met Met Ser Ile Arg Gln Arg Arg Glu Ile Arg Ala Thr Glu Val Ser
1               5                   10                  15

Glu Asp Phe Pro Ala Gln Glu Glu Asn Val Lys Leu Glu Asn Lys Leu
            20                  25                  30

Pro Ser Gly Cys Thr Ser Arg Arg Leu Trp Lys Ile Leu Ser Leu Thr
        35                  40                  45

Ile Gly Gly Thr Ile Ala Leu Cys Ile Gly Leu Leu Thr Ser Val Tyr
    50                  55                  60

Leu Ala Thr Leu His Glu Asn Asp Leu Trp Phe Ser Asn Ile Lys Glu
65                  70                  75                  80

Val Glu Arg Glu Ile Ser Phe Arg Thr Glu Cys Gly Leu Tyr Tyr Ser
                85                  90                  95

Tyr Tyr Lys Gln Met Leu Gln Ala Pro Thr Leu Val Gln Gly Phe His
            100                 105                 110

Gly Leu Ile Tyr Asp Asn Lys Thr Glu Ser Met Lys Thr Ile Asn Leu
        115                 120                 125

Leu Gln Arg Met Asn Ile Tyr Gln Glu Val Phe Leu Ser Ile Leu Tyr
    130                 135                 140

Arg Val Leu Pro Ile Gln Lys Tyr Leu Glu Pro Val Tyr Phe Tyr Ile
145                 150                 155                 160

Tyr Thr Leu Phe Gly Leu Gln Ala Ile Tyr Val Thr Ala Leu Tyr Ile
                165                 170                 175

Thr Ser Trp Leu Leu Ser Gly Thr Trp Leu Ser Gly Leu Leu Ala Ala
            180                 185                 190

Phe Trp Tyr Val Thr Asn Arg Ile Asp Thr Thr Arg Val Glu Phe Thr
        195                 200                 205

Ile Pro Leu Arg Glu Asn Trp Ala Leu Pro Phe Phe Ala Ile Gln Ile
    210                 215                 220

Ala Ala Ile Thr Tyr Phe Leu Arg Pro Asn Leu Gln Pro Leu Ser Glu
225                 230                 235                 240

Arg Leu Thr Leu Leu Ala Ile Phe Ile Ser Thr Phe Leu Phe Ser Leu
                245                 250                 255

Thr Trp Gln Phe Asn Gln Phe Met Met Leu Met Gln Ala Leu Val Leu
            260                 265                 270

Phe Thr Leu Asp Ser Leu Asp Met Leu Pro Ala Val Lys Ala Thr Trp
        275                 280                 285

Leu Tyr Gly Ile Gln Ile Thr Ser Leu Leu Leu Val Cys Ile Leu Gln
    290                 295                 300

Phe Phe Asn Ser Met Ile Leu Gly Ser Leu Leu Ile Ser Phe Asn Leu
305                 310                 315                 320

Ser Val Phe Ile Ala Arg Lys Leu Gln Lys Asn Leu Lys Thr Gly Ser
                325                 330                 335

Phe Leu Asn Arg Leu Gly Lys Leu Leu Leu His Leu Phe Met Val Leu
            340                 345                 350

Cys Leu Thr Leu Phe Leu Asn Asn Ile Ile Lys Lys Ile Leu Asn Leu
        355                 360                 365

Lys Ser Asp Glu His Ile Phe Lys Phe Leu Lys Ala Lys Phe Gly Leu
    370                 375                 380

Gly Ala Thr Arg Asp Phe Asp Ala Asn Leu Tyr Leu Cys Glu Glu Ala
385                 390                 395                 400

Phe Gly Leu Leu Pro Phe Asn Thr Phe Gly Arg Leu Ser Asp Thr Leu
                405                 410                 415

Leu Phe Tyr Ala Tyr Ile Phe Val Leu Ser Ile Thr Val Ile Val Ala
            420                 425                 430

Phe Val Val Ala Phe His Asn Leu Ser Asp Ser Thr Asn Gln Gln Ser
        435                 440                 445

Val Gly Lys Met Glu Lys Gly Thr Val Asp Leu Lys Pro Glu Thr Ala

450                 455                 460

Tyr Asn Leu Ile His Thr Ile Leu Phe Gly Phe Leu Ala Leu Ser Thr
465                 470                 475                 480

Met Arg Met Lys Tyr Leu Trp Thr Ser His Met Cys Val Phe Ala Ser
                485                 490                 495

Phe Gly Leu Cys Ser Pro Glu Ile Trp Glu Leu Leu Leu Lys Ser Val
            500                 505                 510

His Leu Tyr Asn Pro Lys Arg Ile Cys Ile Met Arg Tyr Ser Val Pro
        515                 520                 525

Ile Leu Ile Leu Leu Tyr Leu Cys Tyr Lys Phe Trp Pro Gly Met Met
    530                 535                 540

Asp Glu Leu Ser Glu Leu Arg Glu Phe Tyr Asp Pro Asp Thr Val Glu
545                 550                 555                 560

Leu Met Asn Trp Ile Asn Ser Asn Thr Pro Arg Lys Ala Val Phe Ala
                565                 570                 575

Gly Ser Met Gln Leu Leu Ala Gly Val Lys Leu Cys Thr Gly Arg Thr
            580                 585                 590

Leu Thr Asn His Pro His Tyr Glu Asp Ser Ser Leu Arg Glu Arg Thr
        595                 600                 605

Arg Ala Val Tyr Gln Ile Tyr Ala Lys Arg Ala Pro Glu Glu Val His
    610                 615                 620

Ala Leu Leu Arg Ser Phe Gly Thr Asp Tyr Val Ile Leu Glu Asp Ser
625                 630                 635                 640

Ile Cys Tyr Glu Arg Arg His Arg Arg Gly Cys Arg Leu Arg Asp Leu
                645                 650                 655

Leu Asp Ile Ala Asn Gly His Met Met Asp Gly Pro Gly Glu Asn Asp
            660                 665                 670

Pro Asp Leu Lys Pro Ala Asp His Pro Arg Phe Cys Glu Glu Ile Lys
        675                 680                 685

Arg Asn Leu Pro Pro Tyr Val Ala Tyr Phe Thr Arg Val Phe Gln Asn
    690                 695                 700

Lys Thr Phe His Val Tyr Lys Leu Ser Arg Asn Lys
705                 710                 715

<210>  18
<211>  112
<212>  PRT
<213>  Homo sapiens

<400>  18

Met Met Ser Ile Arg Gln Arg Arg Glu Ile Arg Ala Thr Glu Val Ser
1               5                   10                  15

Glu Asp Phe Pro Ala Gln Glu Glu Asn Val Lys Leu Glu Asn Lys Leu
            20                  25                  30

Pro Ser Gly Cys Thr Ser Arg Arg Leu Trp Lys Ile Leu Ser Leu Thr
        35                  40                  45

Ile Gly Gly Thr Pro Phe Ala Leu Asp Phe Leu His Leu Ser Thr Leu
    50                  55                  60

Pro Arg Tyr Met Lys Met Ile Tyr Gly Phe Leu Ile Leu Arg Lys Trp
65                  70                  75                  80

Ser Glu Lys Ser His Ser Glu Gln Ser Val Ala Cys Ile Thr Pro Thr
                85                  90                  95

Thr Ser Arg Cys Cys Arg Leu Gln Pro Ser Cys Lys Val Ile Thr Thr
            100                 105                 110

<210>  19
<211>  1841
<212>  DNA
<213>  Homo sapiens

<400>  19
aagtttgcgg agcggcttct gctcgtcggc cgtgcggcga ggcagggcct gggctgcgac      60

cccggcggcc gctcgcggtc ttgggagagc tggggcgcgt gcctgaactt cccggctgcc     120

cctgtccttg gagacctacc tgatggggac gccaggtgtg caggggcgtg gcgcgtagga     180

gtgatttgga gaacaatgca tgtaagtctg acatcatgat gtccatccgg caaagaagag     240

aaataagagc cacagaagtt tctgaagact ttccagccca agaagaaaat gtgaagttgg     300

aaaataaatt gccatctggt tgtaccagta gaagattatg gaagattttg tcattgacaa     360

ttggtggaac cattgccctt tgcattggac ttcttacatc tgtctacctt gccacgttac     420

atgaaaatga tttatggttt tctaatatta aggaagtgga gcgagaaatc tcattcagaa     480

cagagtgtgg cctgtattac tcctactaca agcagatgct gcaggctcca accctcgtgc     540

aaggttttca tggcctaata tatgataata aaactgaatc tatgaagaca attaacctcc 600

ttcagcgaat gaatatttac caagaggttt ttctcagtat tttatataga gttctaccca 660

tacagaaata tttagagcca gtttattttt atatttacac cttatttggg ctccaggcga 720

tctatgtcac agctctctac ataaccagct ggctactcag tggtacatgg ctgtcaggac 780

tgttggcagc tttctggtat gtcacaaata gaatagatac cacaagagtt gagtttacca 840

tcccactgag ggagaactgg gcgctgccat tctttgcaat tcagatagca gcaattacat 900

atttcctgag accaaactta cagcctcttt ctgaaaggct gacacttctt gccattttca 960

tatcaacttt tctctttagt ctgacatggc aatttaatca atttatgatg ctgatgcaag 1020

cattagtgct gttcacactg gactccctgg acatgctgcc agcagtgaag gcgacatggc 1080

tgtatggaat acagataaca agtttactcc tggtctgcat tcttcagttt tttaattcca 1140

tgattcttgg atcactgctt atcagtttta acctttcagt attcattgca agaaaacttc 1200

agaaaaatct gaaaactgga agcttcctta ataggcttgg gaaacttttg ttacatttat 1260

ttatggtttt atgtttgaca ctttttctca acaacataat taagaaaatt cttaacctga 1320

agtcagatga acacatattt aaatttctga aggcaaaatt tgggcttgga gcaacaaggg 1380

attttgatgc aaatctctat ctgtgtgaag aagcttttgg cctcctgcct tttaatacat 1440

ttggaaggct ttcagatact ctgctttttt atgcttacat attcgttctg tccatcacag 1500

tgattgtagc attcgttgtt gcctttcata atctcagtga ttctacaaat caacaatccg 1560

tgggtaaaat ggaaaaaggc acagttgacc tgaaaccaga aactgcctac aacttaatac 1620

ataccattct gtttggattc ttggcattga gtacaatgag aatgaagtac ctctggacgt 1680

cacacatgtg tgtgttcgca tcattcggcc tatgtagccc tgaaatatgg gagttacttc 1740

tgaagtcagt ccatctttat aacccaaaga ggatatgtat aatgcgatat tcagtaccga 1800

tattaatact gctgtatcta tgctataaga atcagaaatc t 1841

<210> 20
<211> 2206
<212> DNA
<213> Homo sapiens

<400> 20
cggttctgcc ctccttgtac ccgcggcgcg ctgcggcccg tggcgcggcc ccgttcccgc 60

ctagccccgt cggcctcctt cccctcccgg agccgcgcgt gaggacggct gaggccgcag 120

gagtgatttg gagaacaatg catgtaagtc tgacatcatg atgtccatcc ggcaaagaag 180

agaaataaga gccacagaag tttctgaaga ctttccagcc caagaagaaa atgtgaagtt 240

ggaaaataaa ttgccatctg gttgtaccag tagaagatta tggaagattt tgtcattgac 300

aattggtgga accattgccc tttgcattgg acttcttaca tctgtctacc ttgccacgtt 360

acatgaaaat gatttatggt tttctaatat taaggtatgg agtttctttg accattgtat 420

cattcactca gtgggatctc cagtagtaag ccatgtggat gaatgaccaa ggcaacacag 480

ttttgccata aagaatccaa tctctagaaa ggttggacta tagagtgaaa taacttttgt 540

gtttattatt ttaaaataac atattagaat ctttttttaa atttttcttt attatttatt 600

tatttttgag atggagtctc actctgtcac ccaggctgga gtgcggtggc gcaatcttgg 660

ctcactacaa cctctgcctc gcaggttcag gtgattcttc tggcttagcc tcccaagtag 720

ctgggactat aggtgcgtgc caccacaccc agctaatttt tgtattttta ctagagacgg 780

ggtttcagca tattgaccag gctgatctcg aactcctgac cttgtgatct gcctgtctca 840

gcctcccaaa gtgctgggat tacaggcgtg agccactgcg tccagccaga atctttattt 900

ttcattttaa ttttttgaga tagggtattg ctctgtcacc caggctagaa tgcagtggtg 960

caaacatggg tcactgcagc ctcaacctcc tgggctcaag tgagtatcct gcctaagctt 1020

cctgtgtcac tgggacccca ggcatgcacc acctcaccaa gctaaatttg atttttttgt 1080

agagacaggg tctcactttg ttgcccatgc tggtctcgaa ctcctgggct caagcgatcc 1140

tactgccctg gtcttccaaa atatgagaat gagccatagc acccagccca gaatttttat 1200

aatcaagtga gtttttcttt tttcattaac ttattccatt tatttagcag ttattctaaa 1260

ttagtatttt tcaagttata gattgtgaaa ttagtgcagt aggtcatgag taacattttt 1320

cttaatgaaa tcaaaaagaa agaatactat cacatctagt agggttgagg attgttttgt 1380

gaaactttta attttatata tatatatata tgcacaaact gggtcacagt atacaaggta 1440

cttccttttc ttttttttct tgttggctac aacaggaaaa aaaaaaaaca gaaaaggaaa 1500

taaaaaagcc actgctttaa atcatggggt ctaaatgtgg ctccacagag ggtcctcagc 1560

atgttcatga ctatctaata ctctgtgcaa gtggttttgc agggcatagg gcgatgggga 1620

agccatatgt ttccagggaa aggaactgta attttaatca gattttcagg agggttagcc 1680

gggcgtcacg cctgtaatcc cagcactttg ggaggtcgag gcgggcagat cacttgaagt 1740

caggagttca agaccagcct ggccaacatg gtggaaccct atctctacta aaaatacaaa 1800

aattagccgg gcatggtgac acacacctgt aatctcagct actcaggagg ctgaggcaca 1860

agaatcactt gaactcggga ggaagaggtt gcagtgagct gagatcccac cactgcactc 1920

cagcctgggc aacagagcaa tactctttat caaaaaaaaa aagaaaaaag ttgagggggt 1980

ggtctgtgac tctttaaaca cgtttccttg ttttctttct ctctctcttt ttcaacattt 2040

ctagaactcc tcttggcatt gttttcagaa ctcgtatata acttacatgt ggaaatttgc 2100

atccaaatat accttacatt ttaatctaat atgtcatgat ctttaaccta aactgtggtg 2160

tctaatgact agttgcttgt aaaaataaac aaacaccttc aaagcc 2206

<210>  21
<211>  4456
<212>  DNA
<213>  Homo sapiens

<400>  21
aagtttgcgg agcggcttct gctcgtcggc cgtgcggcga ggcagggcct gggctgcgac      60

cccggcggcc gctcgcggtc ttgggagagc tggggcgcgt gcctgaactt cccggctgcc     120

cctgtccttg gagacctacc tgatggggac gccaggtgtg caggggcgtg gcgcgtagga     180

gtgatttgga gaacaatgca tgtaagtctg acatcatgat gtccatccgg caaagaagag     240

aaataagagc cacagaagtt tctgaagact ttccagccca agaagaaaat gtgaagttgg     300

aaaataaatt gccatctggt tgtaccagta gaagattatg gaagattttg tcattgacaa     360

ttggtggaac cattgccctt tgcattggac ttcttacatc tgtctacctt gccacgttac     420

atgaaaatga tttatggttt tctaatatta aggaagtgga gcgagaaatc tcattcagaa     480

cagagtgtgg cctgtattac tcctactaca agcagatgct gcaggctcca accctcgtgc     540

aaggttttca tggcctaata tatgataata aaactgaatc tatgaagaca attaacctcc     600

ttcagcgaat gaatatttac caagaggttt ttctcagtat tttatataga gttctaccca     660

tacagaaata tttagagcca gtttattttt atatttacac cttatttggg ctccaggcga     720

tctatgtcac agctctctac ataaccagct ggctactcag tggtacatgg ctgtcaggac     780

tgttggcagc tttctggtat gtcacaaata gaatagatac cacaagagtt gagtttacca     840

tcccactgag ggagaactgg gcgctgccat tctttgcaat tcagatagca gcaattacat     900

atttcctgag accaaactta cagcctcttt ctgaaaggct gacacttctt gccattttca     960

tatcaacttt tctctttagt ctgacatggc aatttaatca atttatgatg ctgatgcaag    1020

cattagtgct gttcacactg gactccctgg acatgctgcc agcagtgaag gcgacatggc    1080

tgtatggaat acagataaca agtttactcc tggtctgcat tcttcagttt tttaattcca    1140

tgattcttgg atcactgctt atcagtttta acctttcagt attcattgca agaaaacttc    1200

agaaaaatct gaaaactgga agcttcctta ataggcttgg gaaacttttg ttacatttat    1260

ttatggtttt atgtttgaca ctttttctca acaacataat taagaaaatt cttaacctga    1320

agtcagatga acacatattt aaatttctga aggcaaaatt tgggcttgga gcaacaaggg    1380

attttgatgc aaatctctat ctgtgtgaag aagcttttgg cctcctgcct tttaatacat    1440

ttggaaggct ttcagatact ctgctttttt atgcttacat attcgttctg tccatcacag    1500

tgattgtagc attcgttgtt gcctttcata atctcagtga ttctacaaat caacaatccg    1560

tgggtaaaat ggaaaaaggc acagttgacc tgaaaccaga aactgcctac aacttaatac    1620

ataccattct gtttggattc ttggcattga gtacaatgag aatgaagtac ctctggacgt    1680

cacacatgtg tgtgttcgca tcattcggcc tatgtagccc tgaaatatgg gagttacttc    1740

tgaagtcagt ccatctttat aacccaaaga ggatatgtat aatgcgatat tcagtaccga 1800

tattaatact gctgtatcta tgctataagt tctggccagg aatgatggat gaactctccg 1860

agttgagaga attctatgat ccagatacag tggagctgat gaactggatt aactctaaca 1920

ctccaagaaa ggctgtgttt gcgggaagca tgcagttgct ggccggagtc aagctgtgca 1980

cgggaaggac cctaaccaac cacccgcact atgaagacag cagcctgaga gagcggacca 2040

gagcggttta tcagatatat gccaagaggg caccagagga agtgcatgcc ctcctaaggt 2100

ccttcggcac tgactacgta atcctggaag acagcatctg ctacgagcgg aggcaccgcc 2160

ggggctgccg actccgggac ctgctggaca ttgccaacgg ccacatgatg gatggcccag 2220

gagagaatga tcctgatttg aaacctgcag accaccctcg cttctgtgaa gagatcaaaa 2280

gaaacctgcc tccctacgtg gcctacttca ccagagtgtt ccagaacaaa accttccacg 2340

tttacaagct gtccagaaac aagtagcgca gatttctgcc cagtgtctat tttgatacgg 2400

gagaaactgc atcatgatga aactcaatag atgacgtttc ctatgtaagt aggtagccca 2460

aaccttcaag ctgtgatatg agtaagttct acagatgttt acacaagtgt tgccatcttt 2520

gaaagcatct tctacaagca gaagtctttt tcgttgtgtg tctatctttc tcattaatgt 2580

tctttagcct aaatgttaac aactttctaa gagtgaccta gaattatgtt gttggagaga 2640

atgatgtgtg ttccatggat acctggatag gcacataaca tgttggaaga tgagcacctg 2700

ctcaggattt gaaatacgtt taattttcag gtgacttaag acagctatga ttgaatcaac 2760

tagagatgat gatcgactta tttaatatga tttcactggt gaagaccaat tggtagcttt 2820

ttaaaaagca ctttagtgtc ctgttttacc ttaaaatgtt ataatatttt ccagttgtca 2880

tgctgtcaac attaacaaaa aaaatcatgt taaggctttg tatcaaacat tttgttacac 2940

tctgtctgaa atgtaatgtg gagtacttca gcagtatgtg tcatgtattg tgtgtgtctg 3000

tgtgtgtgca tgtgcacaca tgtgttttaa tgctgggcac agaaaagtgt tacaagttcc 3060

atatcgtaag tccttaaagg ggcagaaata tatgtagcca agtagaattt attacatttt 3120

agtgttatta ttttaaaact tactgatact ctttaacctc tcctgcagta atagttttgc 3180

tttatttctt actcatttca atttattggg tttgcaaaat tttgtaaact ttttgtgttt 3240

ttagcctttg tattttttac agcctagaat cttgcaaagt ctgaatattt tttaaatgtt 3300

ctatcttaac tagttcacta atacagtatt tttagcagac agcattttca gacagcattt 3360

tcataccaag tttgacttgt ggtctccaat cttactggga aggccctggt agtgtaattc 3420

ttttccttat taaaaggtaa ccaagtgcct ctaagtcatg cttatttgta aacaacaaag 3480

aagagtatat gtacctgctc aaaatttttt tgataatcgc ttatataatt aatttctaat 3540

gatgaggaca tgtaaaagtt gccagtaaga acatagtatg catttaatta aatcaagatg 3600

gctaatggaa ttaactttct cccctgttct tgccaggtgg aaatgattta agcatttctc 3660

cttgcagttg tattgaagta aattaccata ggcatcaaga tggctgcatc acattttcaa 3720

atgattttat attcagttgc tacttataaa gcagcattca aaaagtcttt tacactgtca 3780

tgttggacac aagcagactc agcttttatc aaaacttgtt taaataaaaa attgacagta 3840

gctgggttat taaattatgc aactgaaact cctgaattat atcttttctg tatcccttaa 3900

taagattgga gaccactgcc gtttaggata atacaataat aaaacgtttt aatcagtact 3960

aaaactttaa ttaagccaat aatgatgcat gcctgttgta gctgacagca tgggtcagta 4020

catccttcag cgagtgcctt actctaattg aaaccaagca cacgtaaggt acaatatgtt 4080

agactctgtg attttgtttt caaaatcctc tgttatggct atatttaaat ttattttaaa 4140

tattcctgta tgtattcatc taagcatttg ggcatttgga gtcttaatat acaagaaaca 4200

cgtacttaaa tttttatgct tatcaccgca atgatggcaa acagtgattt tttttttcat 4260

agtttaggtg tcattgttgc cagcaccttt agtgctcagt cttcagtgaa aaatataaag 4320

tgccaaaaaa atcttgcaag acagaatcca tacttaacac tctttccaag acactgtgac 4380

catgtacagt agctatttcc tgatgaccaa atctctcaac gaatcatgtt attaataaat 4440

atttttagca ctcatc 4456

<210> 22
<211> 336
<212> DNA
<213> Homo sapiens

<400> 22
atgatgtcca tccggcaaag aagagaaata agagccacag aagtttctga agactttcca 60

gcccaagaag aaaatgtgaa gttggaaaat aaattgccat ctggttgtac cagtagaaga 120

ttatggaaga ttttgtcatt gacaattggt ggaaccccct ttgcattgga cttcttacat 180

ctgtctacct tgccacgtta catgaaaatg atttatggtt ttctaatatt aaggaagtgg 240

agcgagaaat ctcattcaga acagagtgtg gcctgtatta ctcctactac aagcagatgc 300

tgcaggctcc aaccctcgtg caaggtaatt acaact 336

<210> 23
<211> 831
<212> PRT
<213> Homo sapiens

<400> 23

Met Lys Val His Met His Thr Lys Phe Cys Leu Ile Cys Leu Leu Thr
1               5                   10                  15

Phe Ile Phe His His Cys Asn His Cys His Glu Glu His Asp His Gly
            20                  25                  30

Pro Glu Ala Leu His Arg Gln His Arg Gly Met Thr Glu Leu Glu Pro
        35                  40                  45

Ser Lys Phe Ser Lys Gln Ala Ala Glu Asn Glu Lys Lys Tyr Tyr Ile
    50                  55                  60

Glu Lys Leu Phe Glu Arg Tyr Gly Glu Asn Gly Arg Leu Ser Phe Phe
65                  70                  75                  80

Gly Leu Glu Lys Leu Leu Thr Asn Leu Gly Leu Gly Glu Arg Lys Val
                85                  90                  95

Val Glu Ile Asn His Glu Asp Leu Gly His Asp His Val Ser His Leu
            100                 105                 110

Asp Ile Leu Ala Val Gln Glu Gly Lys His Phe His Ser His Asn His
        115                 120                 125

Gln His Ser His Asn His Leu Asn Ser Glu Asn Gln Thr Val Thr Ser
    130                 135                 140

Val Ser Thr Lys Arg Asn His Lys Cys Asp Pro Glu Lys Glu Thr Val
145                 150                 155                 160

Glu Val Ser Val Lys Ser Asp Asp Lys His Met His Asp His Asn His
                165                 170                 175

Arg Leu Arg His His His Arg Leu His His His Leu Asp His Asn Asn
            180                 185                 190

Thr His His Phe His Asn Asp Ser Ile Thr Pro Ser Glu Arg Gly Glu
        195                 200                 205

Pro Ser Asn Glu Pro Ser Thr Glu Thr Asn Lys Thr Gln Glu Gln Ser
    210                 215                 220

Asp Val Lys Leu Pro Lys Gly Lys Arg Lys Lys Lys Gly Arg Lys Ser
225                 230                 235                 240

Asn Glu Asn Ser Glu Val Ile Thr Pro Gly Phe Pro Pro Asn His Asp
                245                 250                 255

Gln Gly Glu Gln Tyr Glu His Asn Arg Val His Lys Pro Asp Arg Val
            260                 265                 270

His Asn Pro Gly His Ser His Val His Leu Pro Glu Arg Asn Gly His

275                 280                 285

Asp Pro Gly Arg Gly His Gln Asp Leu Asp Pro Asp Asn Glu Gly Glu
    290                 295                 300

Leu Arg His Thr Arg Lys Arg Glu Ala Pro His Val Lys Asn Asn Ala
305                 310                 315                 320

Ile Ile Ser Leu Arg Lys Asp Leu Asn Glu Asp Asp His His His Glu
                325                 330                 335

Cys Leu Asn Val Thr Gln Leu Leu Lys Tyr Tyr Gly His Gly Ala Asn
            340                 345                 350

Ser Pro Ile Ser Thr Asp Leu Phe Thr Tyr Leu Cys Pro Ala Leu Leu
        355                 360                 365

Tyr Gln Ile Asp Ser Arg Leu Cys Ile Glu His Phe Asp Lys Leu Leu
    370                 375                 380

Val Glu Asp Ile Asn Lys Asp Lys Asn Leu Val Pro Glu Asp Glu Ala
385                 390                 395                 400

Asn Ile Gly Ala Ser Ala Trp Ile Cys Gly Ile Ile Ser Ile Thr Val
                405                 410                 415

Ile Ser Leu Leu Ser Leu Leu Gly Val Ile Leu Val Pro Ile Ile Asn
            420                 425                 430

Gln Gly Cys Phe Lys Phe Leu Leu Thr Phe Leu Val Ala Leu Ala Val
        435                 440                 445

Gly Thr Met Ser Gly Asp Ala Leu Leu His Leu Leu Pro His Ser Gln
    450                 455                 460

Gly Gly His Asp His Ser His Gln His Ala His Gly His Gly His Ser
465                 470                 475                 480

His Gly His Glu Ser Asn Lys Phe Leu Glu Glu Tyr Asp Ala Val Leu
                485                 490                 495

Lys Gly Leu Val Ala Leu Gly Gly Ile Tyr Leu Leu Phe Ile Ile Glu
            500                 505                 510

His Cys Ile Arg Met Phe Lys His Tyr Lys Gln Gln Arg Gly Lys Gln
        515                 520                 525

Lys Trp Phe Met Lys Gln Asn Thr Glu Glu Ser Thr Ile Gly Arg Lys
    530                 535                 540

Leu Ser Asp His Lys Leu Asn Asn Thr Pro Asp Ser Asp Trp Leu Gln
545                 550                 555                 560

Leu Lys Pro Leu Ala Gly Thr Asp Asp Ser Val Val Ser Glu Asp Arg
                565                 570                 575

Leu Asn Glu Thr Glu Leu Thr Asp Leu Glu Gly Gln Gln Glu Ser Pro
            580                 585                 590

Pro Lys Asn Tyr Leu Cys Ile Glu Glu Glu Lys Ile Ile Asp His Ser
        595                 600                 605

His Ser Asp Gly Leu His Thr Ile His Glu His Asp Leu His Ala Ala
    610                 615                 620

Ala His Asn His His Gly Glu Asn Lys Thr Val Leu Arg Lys His Asn
625                 630                 635                 640

His Gln Trp His His Lys His Ser His His Ser His Gly Pro Cys His
                645                 650                 655

Ser Gly Ser Asp Leu Lys Glu Thr Gly Ile Ala Asn Ile Ala Trp Met
            660                 665                 670

Val Ile Met Gly Asp Gly Ile His Asn Phe Ser Asp Gly Leu Ala Ile
        675                 680                 685

Gly Ala Ala Phe Ser Ala Gly Leu Thr Gly Gly Ile Ser Thr Ser Ile
    690                 695                 700

Ala Val Phe Cys His Glu Leu Pro His Glu Leu Gly Asp Phe Ala Val
705                 710                 715                 720

Leu Leu Lys Ala Gly Met Thr Val Lys Gln Ala Ile Val Tyr Asn Leu
                725                 730                 735

Leu Ser Ala Met Met Ala Tyr Ile Gly Met Leu Ile Gly Thr Ala Val
            740                 745                 750

Gly Gln Tyr Ala Asn Asn Ile Thr Leu Trp Ile Phe Ala Val Thr Ala
        755                 760                 765

Gly Met Phe Leu Tyr Val Ala Leu Val Asp Met Leu Pro Glu Met Leu
    770                 775                 780

His Gly Asp Gly Asp Asn Glu Glu His Gly Phe Cys Pro Val Gly Gln
785                 790                 795                 800

Phe Ile Leu Gln Asn Leu Gly Leu Leu Phe Gly Phe Ala Ile Met Leu
                805                 810                 815

Val Ile Ala Leu Tyr Glu Asp Lys Ile Val Phe Asp Ile Gln Phe
            820                 825                 830

<210>  24
<211>  831
<212>  PRT
<213>  Homo sapiens

<400>  24

Met Lys Val His Met His Thr Lys Phe Cys Leu Ile Cys Leu Leu Thr
1               5                   10                  15

Phe Ile Phe His His Cys Asn His Cys His Glu Glu His Asp His Gly
            20                  25                  30

Pro Glu Ala Leu His Arg Gln His Arg Gly Met Thr Glu Leu Glu Pro
        35                  40                  45

Ser Lys Phe Ser Lys Gln Ala Ala Glu Asn Glu Lys Lys Tyr Tyr Ile
    50                  55                  60

Glu Lys Leu Phe Glu Arg Tyr Gly Glu Asn Gly Arg Leu Ser Phe Phe
65                  70                  75                  80

Gly Leu Glu Lys Leu Leu Thr Asn Leu Gly Leu Gly Glu Arg Lys Val
                85                  90                  95

Val Glu Ile Asn His Glu Asp Leu Gly His Asp His Val Ser His Leu
            100                 105                 110

Asp Ile Leu Ala Val Gln Glu Gly Lys His Phe His Ser His Asn His
        115                 120                 125

Gln His Ser His Asn His Leu Asn Ser Glu Asn Gln Thr Val Thr Ser
    130                 135                 140

Val Ser Thr Lys Arg Asn His Lys Cys Asp Pro Glu Lys Glu Thr Val
145                 150                 155                 160

Glu Val Ser Val Lys Ser Asp Asp Lys His Met His Asp His Asn His
                165                 170                 175

Arg Leu Arg His His His Arg Leu His His His Leu Asp His Asn Asn
            180                 185                 190

Thr His His Phe His Asn Asp Ser Ile Thr Pro Ser Glu Arg Gly Glu
        195                 200                 205

Pro Ser Asn Glu Pro Ser Thr Glu Thr Asn Lys Thr Gln Glu Gln Ser
    210                 215                 220

Asp Val Lys Leu Pro Lys Gly Lys Arg Lys Lys Lys Gly Arg Lys Ser
225                 230                 235                 240

Asn Glu Asn Ser Glu Val Ile Thr Pro Gly Phe Pro Pro Asn His Asp
                245                 250                 255

Gln Gly Glu Gln Tyr Glu His Asn Arg Val His Lys Pro Asp Arg Val
            260                 265                 270

His Asn Pro Gly His Ser His Val His Leu Pro Glu Arg Asn Gly His
        275                 280                 285

Asp Pro Gly Arg Gly His Gln Asp Leu Asp Pro Asp Asn Glu Gly Glu
    290                 295                 300

Leu Arg His Thr Arg Lys Arg Glu Ala Pro His Val Lys Asn Asn Ala
305                 310                 315                 320

Ile Ile Ser Leu Arg Lys Asp Leu Asn Glu Asp Asp His His His Glu
                325                 330                 335

Cys Leu Asn Val Thr Gln Leu Leu Lys Tyr Tyr Gly His Gly Ala Asn
            340                 345                 350

Ser Pro Ile Ser Thr Asp Leu Phe Thr Tyr Leu Cys Pro Ala Leu Leu
        355                 360                 365

Tyr Gln Ile Asp Ser Arg Leu Cys Ile Glu His Phe Asp Lys Leu Leu
    370                 375                 380

Val Glu Asp Ile Asn Lys Asp Lys Asn Leu Val Pro Glu Asp Glu Ala
385                 390                 395                 400

Asn Ile Gly Ala Ser Ala Trp Ile Cys Gly Ile Ile Ser Ile Thr Val
                405                 410                 415

Ile Ser Leu Leu Ser Leu Leu Gly Val Ile Leu Val Pro Ile Ile Asn
            420                 425                 430

Gln Gly Cys Phe Lys Phe Leu Leu Thr Phe Leu Val Ala Leu Ala Val
        435                 440                 445

Gly Thr Met Ser Gly Asp Ala Leu Leu His Leu Leu Pro His Ser Gln
    450                 455                 460

Gly Gly His Asp His Ser His Gln His Ala His Gly His Gly His Ser
465                 470                 475                 480

His Gly His Glu Ser Asn Lys Phe Leu Glu Glu Tyr Asp Ala Val Leu
                485                 490                 495

Lys Gly Leu Val Ala Leu Gly Gly Ile Tyr Leu Leu Phe Ile Ile Glu
            500                 505                 510

His Cys Ile Arg Met Phe Lys His Tyr Lys Gln Gln Arg Gly Lys Gln
        515                 520                 525

Lys Trp Phe Met Lys Gln Asn Thr Glu Glu Ser Thr Ile Gly Arg Lys
    530                 535                 540

Leu Ser Asp His Lys Leu Asn Asn Thr Pro Asp Ser Asp Trp Leu Gln
545                 550                 555                 560

Leu Lys Pro Leu Ala Gly Thr Asp Asp Ser Val Val Ser Glu Asp Arg
                565                 570                 575

Leu Asn Glu Thr Glu Leu Thr Asp Leu Glu Gly Gln Gln Glu Ser Pro
            580                 585                 590

Pro Lys Asn Tyr Leu Cys Ile Glu Glu Glu Lys Ile Ile Asp His Ser
        595                 600                 605

His Ser Asp Gly Leu His Thr Ile His Glu His Asp Leu His Ala Ala
    610                 615                 620

Ala His Asn His His Gly Glu Asn Lys Thr Val Leu Arg Lys His Asn
625                 630                 635                 640

His Gln Trp His His Lys His Ser His His Ser His Gly Pro Cys His
                645                 650                 655

Ser Gly Ser Asp Leu Lys Glu Thr Gly Ile Ala Asn Ile Ala Trp Met
            660                 665                 670

Val Ile Met Gly Asp Gly Ile His Asn Phe Ser Asp Gly Leu Ala Ile

675                 680                 685

Gly Ala Ala Phe Ser Ala Gly Leu Thr Gly Gly Ile Ser Thr Ser Ile
    690                 695                 700

Ala Val Phe Cys His Glu Leu Pro His Glu Leu Gly Asp Phe Ala Val
705                 710                 715                 720

Leu Leu Lys Ala Gly Met Thr Val Lys Gln Ala Ile Val Tyr Asn Leu
                725                 730                 735

Leu Ser Ala Met Met Ala Tyr Ile Gly Met Leu Ile Gly Thr Ala Val
            740                 745                 750

Gly Gln Tyr Ala Asn Asn Ile Thr Leu Trp Ile Phe Ala Val Thr Ala
        755                 760                 765

Gly Met Phe Leu Tyr Val Ala Leu Val Asp Met Leu Pro Glu Met Leu
    770                 775                 780

His Gly Asp Gly Asp Asn Glu Glu His Gly Phe Cys Pro Val Gly Gln
785                 790                 795                 800

Phe Ile Leu Gln Asn Leu Gly Leu Leu Phe Gly Phe Ala Ile Met Leu
                805                 810                 815

Val Ile Ala Leu Tyr Glu Asp Lys Ile Val Phe Asp Ile Gln Phe
            820                 825                 830

<210>  25
<211>  5227
<212>  DNA
<213>  Homo sapiens

<400>  25
cacgatttgg tgcagccggg gtttggtacc gagcggagag gagatgcaca cggcactcga      60

gtgtgaggaa aaatagaaat gaaggtacat atgcacacaa aattttgcct catttgtttg     120

ctgacattta tttttcatca ttgcaaccat tgccatgaag aacatgacca tggccctgaa     180

gcgcttcaca gacagcatcg tggaatgaca gaattggagc caagcaaatt ttcaaagcaa     240

gctgctgaaa atgaaaaaaa atactatatt gaaaaacttt ttgagcgtta tggtgaaaat     300

ggaagattat ccttttttgg tttggagaaa cttttaacaa acttgggcct tggagagaga     360

aaagtagttg agattaatca tgaggatctt ggccacgatc atgtttctca tttagatatt     420

ttggcagttc aagagggaaa gcattttcac tcacataacc accagcattc ccataatcat     480

ttaaattcag aaaatcaaac tgtgaccagt gtatccacaa aaagaaacca taaatgtgat     540

ccagagaaag agacagttga agtgtctgta aaatctgatg ataaacatat gcatgaccat 600

aatcaccgcc tacgtcatca ccatcgtttg catcatcatc ttgatcataa caacactcac 660

cattttcata atgattccat tactcccagt gagcgtgggg agcctagcaa tgaaccttca 720

acagagacca ataaaaccca ggaacaatct gatgttaaac taccgaaagg aaagaggaag 780

aaaaaaggga ggaaaagtaa tgaaaattct gaggttatta caccaggttt tccccctaac 840

catgatcagg gtgaacagta tgagcataat cgggtccaca aacctgatcg tgtacataac 900

ccaggtcatt ctcatgtaca tcttccagaa cgtaatggtc atgatcctgg tcgtggacac 960

caagatcttg atcctgataa tgaaggtgaa cttcgacata ctagaaagag agaagcacca 1020

catgttaaaa ataatgcaat aatttctttg agaaaagatc taaatgaaga tgaccatcat 1080

catgaatgtt tgaacgtcac tcagttatta aaatactatg gtcatggtgc caactctccc 1140

atctcaactg atttatttac atacctttgc cctgcattgt tatatcaaat cgacagcaga 1200

ctttgtattg agcattttga caaactttta gttgaagata taaataagga taaaaacctg 1260

gttcctgaag atgaggcaaa tataggggca tcagcctgga tttgtggtat catttctatc 1320

actgtcatta gcctgctttc cttgctaggc gtgatcttgg ttcctatcat taaccaagga 1380

tgcttcaaat tccttcttac attccttgtt gcattagctg taggaacaat gagtggagac 1440

gcccttcttc atctactgcc ccattctcag ggtggacatg atcacagtca ccaacatgca 1500

catgggcatg gacattctca tggacatgaa tctaacaagt ttttggaaga atatgatgct 1560

gtattgaaag gacttgttgc tctaggaggc atttacttgc tatttatcat tgaacactgc 1620

attagaatgt ttaagcacta caaacaacaa agaggaaaac agaaatggtt tatgaaacag 1680

aacacagaag aatcaactat tggaagaaag ctttcagatc acaagttaaa caatacacca 1740

gattctgact ggcttcaact caagcctctt gccggaactg atgactcggt tgtttctgaa 1800

gatcgactta atgaaactga actgacagat ttagaaggcc aacaagaatc ccctcctaaa 1860

aattaccttt gtatagaaga ggagaaaatc atagaccatt ctcacagtga tggattacat 1920

accattcatg agcatgatct ccatgctgct gcacataacc accacggcga gaacaaaact 1980

gtgctgagga agcataatca ccagtggcac cacaagcatt ctcatcattc ccatggcccc 2040

tgtcattctg gatccgatct gaaagaaaca ggaatagcta atatagcctg gatggtgatc 2100

atgggggatg gcatccacaa cttcagtgat gggctcgcaa ttggtgcagc tttcagtgct 2160

ggattgacag gaggaatcag tacttctata gccgtcttct gtcatgaact gccacatgaa 2220

ttaggagatt ttgcagttct tcttaaagca ggcatgactg taaagcaagc aattgtatac 2280

aacctcctct ctgccatgat ggcttacata ggcatgctca taggcacagc tgttggtcag 2340

tatgccaata acatcacact ttggatcttt gcagtcactg caggcatgtt cctctatgta 2400

gccttggtgg atatgcttcc agaaatgttg catggtgatg gtgacaatga agaacatggc 2460

ttttgtcctg tggggcaatt catccttcag aatttaggat tgctctttgg atttgccatt 2520

atgctggtga ttgccctcta tgaagataaa attgtgtttg acatccagtt ttgacctttc 2580

ccagtaatca ctgttgatta cgagaatgtt accatgcagc tttgcatctg ttccttgtac 2640

tgtatgcaca ttgctcaaag gaaagtcagt ggcttgcact acttacaagt ttcatagatt 2700

tgagcctaac cacaagaggc tggtgcttag tactgttttc cctgcacgta ggggtctttt 2760

aaaaatataa agcttgtgat aaagagagga gaatatggga ctccatgaac cagtgttgat 2820

atgtttgatt aagacttttc acaaaataat catataaaac actagtctct ttattagtag 2880

aaacttctgt ggctatgcag aaatagagat cgaaccaaaa aaaatcattt aaactttaaa 2940

aatattttaa atggactttg gggagacatt ttttgtgtgt tttaagaatg aattgtagtg 3000

ctctttaatt cagctacata tattcatgtg gtgataggga tcaacttgac acaactttga 3060

aactgcataa agtagacata ggaactagag gaaagctcag gctgcattag agtatgaatt 3120

tagcattggg aaaagccctt attcttgaat ctagagttac tatttttgta tatatttgca 3180

tagtgtttaa acctgcagcc taaactactg aaatttgtga ttgtatgttt gtgtgagctt 3240

cagtttaatg aaagattcat aatggttctt tgtattatta taatacttgg tgttggggtg 3300

ttctttctgt tttgtttttt actttaattt tgttttgatt tttttttttt ttttttggcg 3360

ggggtaggtg agggtttgga gcatgtggtc tttttaaaaa attgtaaccc tctagaaaat 3420

atcaaagaaa tgaaccagac gtggtttaaa tagttgattt tcctatttta acagtaccaa 3480

ctagttaatt gggaaatgta agttctgaat gttcacattg ctttaccagt ttggcactgg 3540

aaccaagagc acatgtcgtg gctggctaca aggttgtaaa gcagaaaatc gaagtttacc 3600

atgtctgtaa tgtgtacatg aagtgtcaat ttagaacagt tactaggata aactccatta 3660

ttgccatggc tgtcatggta cccaagtgac ttggaagatg catttaaatt actcagctga 3720

aatcacttga tcatcttgtg ccaagatatg ctgttggtgc ctgataggga ttagtctttt 3780

aggtgccctg ttctcctacc ataattgtga atgatttgtg agaagtgcaa gccatgttta 3840

tcctgaattt ttacttaata atttgtatta ctagtcatat gcatgtagct ttctgtttac 3900

atcctatgcc acatggtctt catttatgcc aggtaaactg tatttgaact atgtgcagct 3960

agctttgttt taatctgctt ggcaaccagt gtagctgctg taacaatcta tcttattgtt 4020

caaatatata agagccaaac tcttttccat tccatctaaa atgttttcat ttagtactct 4080

tctttcctcc tactctatga acttcaaaac aaaaacaaaa ctttgagagc agcacatgca 4140

tccaggtatt tatagattat tgccagtgtc ttttctgtat gctataagca agggagctta 4200

ggtgttattt ctttaattta tgcttgaatc tgaaaaatta tttctgactt actccatggc 4260

ctccttataa taagtagaag ttttatatat aattaatttt cagcattggg cactgaatta 4320

ggacagtcct catctcattg cttggccctt caagcaacct agctaaaagg tgctgatatt 4380

ttatttagta ctgccaactt caagtgattt agatatctat ctatctagat ttctgaacca 4440

agatatattt atagttcact tttgggtttt tatacccacg gtaggattct gcattccagc 4500

attaaatctg cttcatttta gaacctttat aaaagcaata gctggaatat actcccagtt 4560

ttaaaataaa tgcctgattg atttaaagca agtaggttat gctgaagtat ataaagaagt 4620

tttatattct ctcaaaaatg gtattatctt tctttatttg ctagattctt acaaatcttt 4680

taagagggct gtaacagttg ctgctagtat tagggttcca catcattcta atgtatagtt 4740

tcaagtctta atagacaatc tgaattccac tacatttctt ttggctccaa cattcctttt 4800

agcttgacca gtctaattta aaatgtgttt gttggaggtc attaacgtta cttgtacaat 4860

gctgtcactg tgtgacatcc atatgaattt tggtatatat caatcaatca atcaatcaca 4920

ttgcattcaa tcaatcagct gtgattgatt gattatgctt agaaatacta tagtaactag 4980

atgcagtgtg aattttttcc attaacaaac aaacaagtca gtggcttaaa tgtgattatg 5040

gtcctgcaag gtgattcttg ctaaaatatc taaacttttg ttttgtttta actgaatcat 5100

tttttaactt aaaaagctgg aaaatatcaa atgctgtttt ttttttttca ttgtcaacag 5160

tggtgtgtca ttttatgtat gttcctaatg cttatggaac tcctccaaaa taaagttact 5220

caaagag 5227

<210> 26
<211> 5432
<212> DNA
<213> Homo sapiens

<400> 26
agttgatcac tctgaagctt tttggctaaa gcgtttgggt ttagagcttc cattactcat 60

tcgccttgcc caaggcctca gcaaccgacg ttcgaaagcc aggagaaaag gcgaatgata 120

aagggcgctc cacgcatgcg ttaagaagcc gccccaactc ccccgcggcg ttctttcttg 180

gaacaaaact agcgcggagc cacggaactc cgcagtttgc gtagacttga atttcctatt 240

cctcggacga tccatgtgga atccgaaaaa tagaaatgaa ggtacatatg cacacaaaat 300

tttgcctcat ttgtttgctg acatttattt ttcatcattg caaccattgc catgaagaac 360

atgaccatgg ccctgaagcg cttcacagac agcatcgtgg aatgacagaa ttggagccaa 420

gcaaattttc aaagcaagct gctgaaaatg aaaaaaaata ctatattgaa aaactttttg 480

agcgttatgg tgaaaatgga agattatcct tttttggttt ggagaaactt ttaacaaact 540

tgggccttgg agagagaaaa gtagttgaga ttaatcatga ggatcttggc cacgatcatg 600

tttctcattt agatattttg gcagttcaag agggaaagca ttttcactca cataaccacc 660

agcattccca taatcattta aattcagaaa atcaaactgt gaccagtgta tccacaaaaa 720

gaaaccataa atgtgatcca gagaaagaga cagttgaagt gtctgtaaaa tctgatgata 780

aacatatgca tgaccataat caccgcctac gtcatcacca tcgtttgcat catcatcttg 840

atcataacaa cactcaccat tttcataatg attccattac tcccagtgag cgtggggagc 900

ctagcaatga accttcaaca gagaccaata aaacccagga acaatctgat gttaaactac 960

cgaaaggaaa gaggaagaaa aaagggagga aaagtaatga aaattctgag gttattacac 1020

caggttttcc ccctaaccat gatcagggtg aacagtatga gcataatcgg gtccacaaac 1080

ctgatcgtgt acataaccca ggtcattctc atgtacatct tccagaacgt aatggtcatg 1140

atcctggtcg tggacaccaa gatcttgatc ctgataatga aggtgaactt cgacatacta 1200

gaaagagaga agcaccacat gttaaaaata atgcaataat ttctttgaga aaagatctaa 1260

atgaagatga ccatcatcat gaatgtttga acgtcactca gttattaaaa tactatggtc 1320

atggtgccaa ctctcccatc tcaactgatt tatttacata cctttgccct gcattgttat 1380

atcaaatcga cagcagactt tgtattgagc attttgacaa acttttagtt gaagatataa 1440

ataaggataa aaacctggtt cctgaagatg aggcaaatat aggggcatca gcctggattt 1500

gtggtatcat ttctatcact gtcattagcc tgctttcctt gctaggcgtg atcttggttc 1560

ctatcattaa ccaaggatgc ttcaaattcc ttcttacatt ccttgttgca ttagctgtag 1620

gaacaatgag tggagacgcc cttcttcatc tactgcccca ttctcagggt ggacatgatc 1680

acagtcacca acatgcacat gggcatggac attctcatgg acatgaatct aacaagtttt 1740

tggaagaata tgatgctgta ttgaaaggac ttgttgctct aggaggcatt tacttgctat 1800

ttatcattga acactgcatt agaatgttta agcactacaa acaacaaaga ggaaaacaga 1860

aatggtttat gaaacagaac acagaagaat caactattgg aagaaagctt tcagatcaca 1920

agttaaacaa tacaccagat tctgactggc ttcaactcaa gcctcttgcc ggaactgatg 1980

actcggttgt ttctgaagat cgacttaatg aaactgaact gacagattta gaaggccaac 2040

aagaatcccc tcctaaaaat tacctttgta tagaagagga gaaaatcata gaccattctc 2100

acagtgatgg attacatacc attcatgagc atgatctcca tgctgctgca cataaccacc 2160

acggcgagaa caaaactgtg ctgaggaagc ataatcacca gtggcaccac aagcattctc 2220

atcattccca tggcccctgt cattctggat ccgatctgaa agaaacagga atagctaata 2280

tagcctggat ggtgatcatg ggggatggca tccacaactt cagtgatggg ctcgcaattg 2340

gtgcagcttt cagtgctgga ttgacaggag gaatcagtac ttctatagcc gtcttctgtc 2400

atgaactgcc acatgaatta ggagattttg cagttcttct taaagcaggc atgactgtaa 2460

agcaagcaat tgtatacaac ctcctctctg ccatgatggc ttacataggc atgctcatag 2520

gcacagctgt tggtcagtat gccaataaca tcacactttg gatctttgca gtcactgcag 2580

gcatgttcct ctatgtagcc ttggtggata tgcttccaga aatgttgcat ggtgatggtg 2640

acaatgaaga acatggcttt tgtcctgtgg ggcaattcat ccttcagaat ttaggattgc 2700

tctttggatt tgccattatg ctggtgattg ccctctatga agataaaatt gtgtttgaca 2760

tccagttttg acctttccca gtaatcactg ttgattacga gaatgttacc atgcagcttt 2820

gcatctgttc cttgtactgt atgcacattg ctcaaaggaa agtcagtggc ttgcactact 2880

tacaagtttc atagatttga gcctaaccac aagaggctgg tgcttagtac tgttttccct 2940

gcacgtaggg gtcttttaaa aatataaagc ttgtgataaa gagaggagaa tatgggactc 3000

catgaaccag tgttgatatg tttgattaag acttttcaca aaataatcat ataaaacact 3060

agtctcttta ttagtagaaa cttctgtggc tatgcagaaa tagagatcga accaaaaaaa 3120

atcatttaaa ctttaaaaat attttaaatg gactttgggg agacattttt tgtgtgtttt 3180

aagaatgaat tgtagtgctc tttaattcag ctacatatat tcatgtggtg atagggatca 3240

acttgacaca actttgaaac tgcataaagt agacatagga actagaggaa agctcaggct 3300

gcattagagt atgaatttag cattgggaaa agcccttatt cttgaatcta gagttactat 3360

ttttgtatat atttgcatag tgtttaaacc tgcagcctaa actactgaaa tttgtgattg 3420

tatgtttgtg tgagcttcag tttaatgaaa gattcataat ggttctttgt attattataa 3480

tacttggtgt tggggtgttc tttctgtttt gtttttactt ttaattttgt tttgattttt 3540

tttttttttt tttggcgggg gtaggtgagg gtttggagca tgtggtcttt ttaaaaaatt 3600

gtaaccctct agaaaatatc aaagaaatga accagacgtg gtttaaatag ttgattttcc 3660

tattttaaca gtaccaacta gttaattggg aaatgtaagt tctgaatgtt cacattgctt 3720

taccagtttg gcactggaac caagagcaca tgtcgtggct ggctacaagg ttgtaaagca 3780

gaaaatcgaa gtttaccatg tctgtaatgt gtacatgaag tgtcaattta gaacagttac 3840

taggataaac tccattattg ccatggctgt catggtaccc aagtgacttg gaagatgcat 3900

ttaaattact cagctgaaat cacttgatca tcttgtgcca agatatgctg ttggtgcctg 3960

atagggatta gtcttttagg tgccctgttc tcctaccata attgtgaatg atttgtgaga 4020

agtgcaagcc atgtttatcc tgaattttta cttaataatt tgtattacta gtcatatgca 4080

tgtagctttc tgtttacatc ctatgccaca tggtcttcat ttatgccagg taaactgtat 4140

ttgaactatg tgcagctagc tttgttttaa tctgcttggc aaccagtgta gctgctgtaa 4200

caatctatct tattgttcaa atatataaga gccaaactct tttccattcc atctaaaatg 4260

ttttcattta gtactcttct ttcctcctac tctatgaact tcaaaacaaa aacaaaactt 4320

tgagagcagc acatgcatcc aggtatttat agattattgc cagtgtcttt tctgtatgct 4380

ataagcaagg gagcttaggt gttatttctt taatttatgc ttgaatctga aaaattattt 4440

ctgacttact ccatggcctc cttataataa gtagaagttt tatatataat taattttcag 4500

cattgggcac tgaattagga cagtcctcat ctcattgctt ggcccttcaa gcaacctagc 4560

taaaaggtgc tgatatttta tttagtactg ccaacttcaa gtgatttaga tatctatcta 4620

tctagatttc tgaaccaaga tatatttata gttcactttt gggtttttat acccacggta 4680

ggattctgca ttccagcatt aaatctgctt cattttagaa cctttataaa agcaatagct 4740

ggaatatact cccagtttta aaataaatgc ctgattgatt taaagcaagt aggttatgct 4800

gaagtatata aagaagtttt atattctctc aaaaatggta ttatctttct ttatttgcta 4860

gattcttaca aatcttttaa gagggctgta acagttgctg ctagtattag ggttccacat 4920

cattctaatg tatagtttca agtcttaata gacaatctga attccactac atttcttttg 4980

gctccaacat tccttttagc ttgaccagtc taatttaaaa tgtgtttgtt ggaggtcatt 5040

aacgttactt gtacaatgct gtcactgtgt gacatccata tgaattttgg tatatatcaa 5100

tcaatcaatc aatcacattg cattcaatca atcagctgtg attgattgat tatgcttaga 5160

aatactatag taactagatg cagtgtgaat tttttccatt aacaaacaaa caagtcagtg 5220

gcttaaatgt gattatggtc ctgcaaggtg attcttgcta aaatatctaa actttgtttt 5280

tgttttaact gaatcatttt ttaacttaaa aagctggaaa atatcaaatg ctgttttttt 5340

tttttcattg tcaacagtgg tgtgtcattt tatgtatgtt cctaatgctt atggaactcc 5400

tccaaaataa agttactcaa agagagcaaa ta 5432

<210> 27
<211> 543
<212> PRT
<213> Homo sapiens

<400> 27

<210>  28
<211>  938
<212>  PRT
<213>  Homo sapiens

<400>  28

Met Pro Ala Leu Glu His Met Asn Gln Ile Leu His Ile Leu Phe Val
1               5                   10                  15

Phe Leu Pro Phe Leu Trp Ala Leu Gly Thr Leu Pro Pro Pro Asp Ala
            20                  25                  30

Leu Leu Leu Trp Ala Met Glu Gln Val Leu Glu Phe Gly Leu Gly Gly
        35                  40                  45

Ser Ser Met Ser Thr His Leu Arg Leu Leu Val Met Phe Ile Met Ser
    50                  55                  60

Ala Gly Thr Ala Ile Ala Ser Tyr Phe Ile Pro Ser Thr Val Gly Val
65                  70                  75                  80

Val Leu Phe Met Thr Gly Phe Gly Phe Leu Leu Ser Leu Asn Leu Ser
                85                  90                  95

Asp Met Gly His Lys Ile Gly Thr Lys Ser Lys Asp Leu Pro Ser Gly
            100                 105                 110

Pro Glu Lys His Phe Ser Trp Lys Glu Cys Leu Phe Tyr Ile Ile Ile
        115                 120                 125

Leu Val Leu Ala Leu Leu Glu Thr Ser Leu Leu His His Phe Ala Gly
    130                 135                 140

Phe Ser Gln Ile Ser Lys Ser Asn Ser Gln Ala Ile Val Gly Tyr Gly
145                 150                 155                 160

Leu Met Ile Leu Leu Ile Ile Leu Trp Ile Leu Arg Glu Ile Gln Ser
                165                 170                 175

Val Tyr Ile Ile Gly Ile Phe Arg Asn Pro Phe Tyr Pro Lys Asp Val
            180                 185                 190

Gln Thr Val Thr Val Phe Phe Glu Lys Gln Thr Arg Leu Met Lys Ile
        195                 200                 205

Gly Ile Val Arg Arg Ile Leu Leu Thr Leu Val Ser Pro Phe Ala Met
    210                 215                 220

Ile Ala Phe Leu Ser Leu Asp Ser Ser Leu Gln Gly Leu His Ser Val
225                 230                 235                 240

Ser Val Cys Ile Gly Phe Thr Arg Ala Phe Arg Met Val Trp Gln Asn
                245                 250                 255

Thr Glu Asn Ala Leu Leu Glu Thr Val Ile Val Ser Thr Val His Leu
            260                 265                 270

Ile Ser Ser Thr Asp Ile Trp Trp Asn Arg Ser Leu Asp Thr Gly Leu

275                 280                 285

Arg Leu Leu Leu Val Gly Ile Ile Arg Asp Arg Leu Ile Gln Phe Ile
    290                 295                 300

Ser Lys Leu Gln Phe Ala Val Thr Val Leu Leu Thr Ser Trp Thr Glu
305                 310                 315                 320

Lys Lys Gln Arg Arg Lys Thr Thr Ala Thr Leu Cys Ile Leu Asn Ile
                325                 330                 335

Val Phe Ser Pro Phe Val Leu Val Ile Ile Val Phe Ser Thr Leu Leu
            340                 345                 350

Ser Ser Pro Leu Leu Pro Leu Phe Thr Leu Pro Val Phe Leu Val Gly
        355                 360                 365

Phe Pro Arg Pro Ile Gln Ser Trp Pro Gly Ala Ala Gly Thr Thr Ala
    370                 375                 380

Cys Val Cys Ala Asp Thr Val Tyr Tyr Tyr Gln Met Val Pro Arg Leu
385                 390                 395                 400

Thr Ala Val Leu Gln Thr Ala Met Ala Ala Gly Ser Leu Gly Leu Leu
                405                 410                 415

Leu Pro Gly Ser His Tyr Leu Gly Arg Phe Gln Asp Arg Leu Met Trp
            420                 425                 430

Ile Met Ile Leu Glu Cys Gly Tyr Thr Tyr Cys Ser Ile Asn Ile Lys
        435                 440                 445

Gly Leu Glu Leu Gln Glu Thr Ser Cys His Thr Ala Glu Ala Arg Arg
    450                 455                 460

Val Asp Glu Val Phe Glu Asp Ala Phe Glu Gln Glu Tyr Thr Arg Val
465                 470                 475                 480

Cys Ser Leu Asn Glu His Phe Gly Asn Val Leu Thr Pro Cys Thr Val
                485                 490                 495

Leu Pro Val Lys Leu Tyr Ser Asp Ala Arg Asn Val Leu Ser Gly Ile
            500                 505                 510

Ile Asp Ser His Glu Asn Leu Lys Glu Phe Lys Gly Asp Leu Ile Lys
        515                 520                 525

Val Leu Val Trp Ile Leu Val Gln Tyr Cys Ser Lys Arg Pro Gly Met
    530                 535                 540

Lys Glu Asn Val His Asn Thr Glu Asn Lys Gly Lys Ala Pro Leu Met
545                 550                 555                 560

Leu Pro Ala Leu Asn Thr Leu Pro Pro Pro Lys Ser Pro Glu Asp Ile
                565                 570                 575

Asp Ser Leu Asn Ser Glu Thr Phe Asn Asp Trp Ser Asp Asp Asn Ile
            580                 585                 590

Phe Asp Asp Glu Pro Thr Ile Lys Lys Val Ile Glu Glu Lys His Gln
        595                 600                 605

Leu Lys Asp Leu Pro Gly Thr Asn Leu Phe Ile Pro Gly Ser Val Glu
    610                 615                 620

Ser Gln Arg Val Gly Asp His Ser Thr Gly Thr Val Pro Glu Asn Asp
625                 630                 635                 640

Leu Tyr Lys Ala Val Leu Leu Gly Tyr Pro Ala Val Asp Lys Gly Lys
                645                 650                 655

Gln Glu Asp Met Pro Tyr Ile Pro Leu Met Glu Phe Ser Cys Ser His
            660                 665                 670

Ser His Leu Val Cys Leu Pro Ala Glu Trp Arg Thr Ser Cys Met Pro
        675                 680                 685

Ser Ser Lys Met Lys Glu Met Ser Ser Leu Phe Pro Glu Asp Trp Tyr
    690                 695                 700

Gln Phe Val Leu Arg Gln Leu Glu Cys Tyr His Ser Glu Glu Lys Ala
705                 710                 715                 720

Ser Asn Val Leu Glu Glu Ile Ala Lys Asp Lys Val Leu Lys Asp Phe
                725                 730                 735

Tyr Val His Thr Val Met Thr Cys Tyr Phe Ser Leu Phe Gly Ile Asp
            740                 745                 750

Asn Met Ala Pro Ser Pro Gly His Ile Leu Arg Val Tyr Gly Gly Val
        755                 760                 765

Leu Pro Trp Ser Val Ala Leu Asp Trp Leu Thr Glu Lys Pro Glu Leu
    770                 775                 780

Phe Gln Leu Ala Leu Lys Ala Phe Arg Tyr Thr Leu Lys Leu Met Ile
785                 790                 795                 800

Asp Lys Ala Ser Leu Gly Pro Ile Glu Asp Phe Arg Glu Leu Ile Lys
                805                 810                 815

Tyr Leu Glu Glu Tyr Glu Arg Asp Trp Tyr Ile Gly Leu Val Ser Asp
            820                 825                 830

Glu Lys Trp Lys Glu Ala Ile Leu Gln Glu Lys Pro Tyr Leu Phe Ser
        835                 840                 845

Leu Gly Tyr Asp Ser Asn Met Gly Ile Tyr Thr Gly Arg Val Leu Ser
    850                 855                 860

Leu Gln Glu Leu Leu Ile Gln Val Gly Lys Leu Asn Pro Glu Ala Val
865                 870                 875                 880

Arg Gly Gln Trp Ala Asn Leu Ser Trp Glu Leu Leu Tyr Ala Thr Asn
                885                 890                 895

Asp Asp Glu Glu Arg Tyr Ser Ile Gln Ala His Pro Leu Leu Leu Arg
            900                 905                 910

Asn Leu Thr Val Gln Ala Ala Glu Pro Pro Leu Gly Tyr Pro Ile Tyr
        915                 920                 925

Ser Ser Lys Pro Leu His Ile His Leu Tyr
    930                 935

<210>  29
<211>  230
<212>  PRT
<213>  Homo sapiens

<400>  29

Met Ser Pro Asp Val Pro Leu Leu Asn Asp Tyr Lys Gln Asp Phe Phe
1               5                   10                  15

Leu Lys Arg Phe Pro Gln Thr Val Leu Gly Gly Pro Arg Phe Lys Leu
            20                  25                  30

Gly Tyr Cys Ala Pro Pro Tyr Ile Tyr Val Asn Gln Ile Ile Leu Phe
        35                  40                  45

Leu Met Pro Trp Val Trp Gly Gly Val Gly Thr Leu Leu Tyr Gln Leu
    50                  55                  60

Gly Ile Leu Lys Asp Tyr Tyr Thr Ala Ala Leu Ser Gly Gly Leu Met
65                  70                  75                  80

Leu Phe Thr Ala Phe Val Ile Gln Phe Thr Ser Leu Tyr Ala Lys Asn
                85                  90                  95

Lys Ser Thr Thr Val Glu Arg Ile Leu Thr Thr Asp Ile Leu Ala Glu
            100                 105                 110

Glu Asp Glu His Glu Phe Thr Ser Cys Thr Gly Ala Glu Thr Val Lys
        115                 120                 125

Phe Leu Ile Pro Gly Lys Lys Tyr Val Ala Asn Thr Val Phe His Ser
    130                 135                 140

Ile Leu Ala Gly Leu Ala Cys Gly Leu Gly Thr Trp Tyr Leu Leu Pro
145                 150                 155                 160

Asn Arg Ile Thr Leu Leu Tyr Gly Ser Thr Gly Gly Thr Ala Leu Leu
                165                 170                 175

Phe Phe Phe Gly Trp Met Thr Leu Cys Ile Ala Glu Tyr Ser Leu Ile
            180                 185                 190

Val Asn Thr Ala Thr Glu Thr Ala Thr Phe Gln Thr Gln Asp Thr Tyr
        195                 200                 205

Glu Ile Ile Pro Leu Met Arg Pro Leu Tyr Ile Phe Phe Phe Val Ser
    210                 215                 220

Val Asp Leu Ala His Arg
225                 230

<210>  30
<211>  1172
<212>  PRT
<213>  Homo sapiens

<400>  30

Met Ser Pro Asp Val Pro Leu Leu Asn Asp Tyr Lys Gln Asp Phe Phe
1               5                   10                  15

Leu Lys Arg Phe Pro Gln Thr Val Leu Gly Gly Pro Arg Phe Lys Leu
            20                  25                  30

Gly Tyr Cys Ala Pro Pro Tyr Ile Tyr Val Asn Gln Ile Ile Leu Phe
        35                  40                  45

Leu Met Pro Trp Val Trp Gly Gly Val Gly Thr Leu Leu Tyr Gln Leu
    50                  55                  60

Gly Ile Leu Lys Asp Tyr Tyr Thr Ala Ala Leu Ser Gly Gly Leu Met
65                  70                  75                  80

Leu Phe Thr Ala Phe Val Ile Gln Phe Thr Ser Leu Tyr Ala Lys Asn
                85                  90                  95

Lys Ser Thr Thr Val Glu Arg Ile Leu Thr Thr Asp Ile Leu Ala Glu
            100                 105                 110

Glu Asp Glu His Glu Phe Thr Ser Cys Thr Gly Ala Glu Thr Val Lys
        115                 120                 125

Phe Leu Ile Pro Gly Lys Lys Tyr Val Ala Asn Thr Val Phe His Ser
    130                 135                 140

Ile Leu Ala Gly Leu Ala Cys Gly Leu Gly Thr Trp Tyr Leu Leu Pro
145                 150                 155                 160

Asn Arg Ile Thr Leu Leu Tyr Gly Ser Thr Gly Gly Thr Ala Leu Leu
                165                 170                 175

Phe Phe Phe Gly Trp Met Thr Leu Cys Ile Ala Glu Tyr Ser Leu Ile
            180                 185                 190

Val Asn Thr Ala Thr Glu Thr Ala Thr Phe Gln Thr Gln Asp Thr Tyr
        195                 200                 205

Glu Ile Ile Pro Leu Met Arg Pro Leu Tyr Ile Phe Phe Phe Val Ser
    210                 215                 220

Val Asp Leu Ala His Arg Phe Val Val Asn Met Pro Ala Leu Glu His
225                 230                 235                 240

Met Asn Gln Ile Leu His Ile Leu Phe Val Phe Leu Pro Phe Leu Trp
                245                 250                 255

Ala Leu Gly Thr Leu Pro Pro Pro Asp Ala Leu Leu Leu Trp Ala Met
            260                 265                 270

Glu Gln Val Leu Glu Phe Gly Leu Gly Gly Ser Ser Met Ser Thr His
        275                 280                 285

Leu Arg Leu Leu Val Met Phe Ile Met Ser Ala Gly Thr Ala Ile Ala
    290                 295                 300

Ser Tyr Phe Ile Pro Ser Thr Val Gly Val Val Leu Phe Met Thr Gly
305                 310                 315                 320

Phe Gly Phe Leu Leu Ser Leu Asn Leu Ser Asp Met Gly His Lys Ile
                325                 330                 335

Gly Thr Lys Ser Lys Asp Leu Pro Ser Gly Pro Glu Lys His Phe Ser
            340                 345                 350

Trp Lys Glu Cys Leu Phe Tyr Ile Ile Ile Leu Val Leu Ala Leu Leu
        355                 360                 365

Glu Thr Ser Leu Leu His His Phe Ala Gly Phe Ser Gln Ile Ser Lys
    370                 375                 380

Ser Asn Ser Gln Ala Ile Val Gly Tyr Gly Leu Met Ile Leu Leu Ile
385                 390                 395                 400

Ile Leu Trp Ile Leu Arg Glu Ile Gln Ser Val Tyr Ile Ile Gly Ile
                405                 410                 415

Phe Arg Asn Pro Phe Tyr Pro Lys Asp Val Gln Thr Val Thr Val Phe
            420                 425                 430

Phe Glu Lys Gln Thr Arg Leu Met Lys Ile Gly Ile Val Arg Arg Ile
        435                 440                 445

Leu Leu Thr Leu Val Ser Pro Phe Ala Met Ile Ala Phe Leu Ser Leu
    450                 455                 460

Asp Ser Ser Leu Gln Gly Leu His Ser Val Ser Val Cys Ile Gly Phe
465                 470                 475                 480

Thr Arg Ala Phe Arg Met Val Trp Gln Asn Thr Glu Asn Ala Leu Leu
                485                 490                 495

Glu Thr Val Ile Val Ser Thr Val His Leu Ile Ser Ser Thr Asp Ile
            500                 505                 510

Trp Trp Asn Arg Ser Leu Asp Thr Gly Leu Arg Leu Leu Leu Val Gly
        515                 520                 525

Ile Ile Arg Asp Arg Leu Ile Gln Phe Ile Ser Lys Leu Gln Phe Ala
    530                 535                 540

Val Thr Val Leu Leu Thr Ser Trp Thr Glu Lys Lys Gln Arg Arg Lys

545                 550                 555                 560

Thr Thr Ala Thr Leu Cys Ile Leu Asn Ile Val Phe Ser Pro Phe Val
                565                 570                 575

Leu Val Ile Ile Val Phe Ser Thr Leu Leu Ser Ser Pro Leu Leu Pro
            580                 585                 590

Leu Phe Thr Leu Pro Val Phe Leu Val Gly Phe Pro Arg Pro Ile Gln
        595                 600                 605

Ser Trp Pro Gly Ala Ala Gly Thr Thr Ala Cys Val Cys Ala Asp Thr
    610                 615                 620

Val Tyr Tyr Tyr Gln Met Val Pro Arg Leu Thr Ala Val Leu Gln Thr
625                 630                 635                 640

Ala Met Ala Ala Gly Ser Leu Gly Leu Leu Leu Pro Gly Ser His Tyr
                645                 650                 655

Leu Gly Arg Phe Gln Asp Arg Leu Met Trp Ile Met Ile Leu Glu Cys
            660                 665                 670

Gly Tyr Thr Tyr Cys Ser Ile Asn Ile Lys Gly Leu Glu Leu Gln Glu
        675                 680                 685

Thr Ser Cys His Thr Ala Glu Ala Arg Arg Val Asp Glu Val Phe Glu
    690                 695                 700

Asp Ala Phe Glu Gln Glu Tyr Thr Arg Val Cys Ser Leu Asn Glu His
705                 710                 715                 720

Phe Gly Asn Val Leu Thr Pro Cys Thr Val Leu Pro Val Lys Leu Tyr
                725                 730                 735

Ser Asp Ala Arg Asn Val Leu Ser Gly Ile Ile Asp Ser His Glu Asn
            740                 745                 750

Leu Lys Glu Phe Lys Gly Asp Leu Ile Lys Val Leu Val Trp Ile Leu
        755                 760                 765

Val Gln Tyr Cys Ser Lys Arg Pro Gly Met Lys Glu Asn Val His Asn
    770                 775                 780

Thr Glu Asn Lys Gly Lys Ala Pro Leu Met Leu Pro Ala Leu Asn Thr
785                 790                 795                 800

Leu Pro Pro Pro Lys Ser Pro Glu Asp Ile Asp Ser Leu Asn Ser Glu
                805                 810                 815

Thr Phe Asn Asp Trp Ser Asp Asp Asn Ile Phe Asp Asp Glu Pro Thr
            820                 825                 830

Ile Lys Lys Val Ile Glu Glu Lys His Gln Leu Lys Asp Leu Pro Gly
        835                 840                 845

Thr Asn Leu Phe Ile Pro Gly Ser Val Glu Ser Gln Arg Val Gly Asp
    850                 855                 860

His Ser Thr Gly Thr Val Pro Glu Asn Asp Leu Tyr Lys Ala Val Leu
865                 870                 875                 880

Leu Gly Tyr Pro Ala Val Asp Lys Gly Lys Gln Glu Asp Met Pro Tyr
                885                 890                 895

Ile Pro Leu Met Glu Phe Ser Cys Ser His Ser His Leu Val Cys Leu
            900                 905                 910

Pro Ala Glu Trp Arg Thr Ser Cys Met Pro Ser Ser Lys Met Lys Glu
        915                 920                 925

Met Ser Ser Leu Phe Pro Glu Asp Trp Tyr Gln Phe Val Leu Arg Gln
    930                 935                 940

Leu Glu Cys Tyr His Ser Glu Glu Lys Ala Ser Asn Val Leu Glu Glu
945                 950                 955                 960

Ile Ala Lys Asp Lys Val Leu Lys Asp Phe Tyr Val His Thr Val Met
                965                 970                 975

Thr Cys Tyr Phe Ser Leu Phe Gly Ile Asp Asn Met Ala Pro Ser Pro
            980                 985                 990

Gly His Ile Leu Arg Val Tyr Gly  Gly Val Leu Pro Trp  Ser Val Ala
        995                 1000                1005

Leu Asp  Trp Leu Thr Glu Lys  Pro Glu Leu Phe Gln  Leu Ala Leu
    1010                1015                1020

Lys Ala  Phe Arg Tyr Thr Leu  Lys Leu Met Ile Asp  Lys Ala Ser
    1025                1030                1035

Leu Gly  Pro Ile Glu Asp Phe  Arg Glu Leu Ile Lys  Tyr Leu Glu
    1040                1045                1050

Glu Tyr Glu Arg Asp Trp Tyr Ile Gly Leu Val Ser Asp Glu Lys
    1055                1060                1065

Trp Lys Glu Ala Ile Leu Gln Glu Lys Pro Tyr Leu Phe Ser Leu
    1070                1075                1080

Gly Tyr Asp Ser Asn Met Gly Ile Tyr Thr Gly Arg Val Leu Ser
    1085                1090                1095

Leu Gln Glu Leu Leu Ile Gln Val Gly Lys Leu Asn Pro Glu Ala
    1100                1105                1110

Val Arg Gly Gln Trp Ala Asn Leu Ser Trp Glu Leu Leu Tyr Ala
    1115                1120                1125

Thr Asn Asp Asp Glu Glu Arg Tyr Ser Ile Gln Ala His Pro Leu
    1130                1135                1140

Leu Leu Arg Asn Leu Thr Val Gln Ala Ala Glu Pro Pro Leu Gly
    1145                1150                1155

Tyr Pro Ile Tyr Ser Ser Lys Pro Leu His Ile His Leu Tyr
    1160                1165                1170

<210>  31
<211>  873
<212>  PRT
<213>  Homo sapiens

<400>  31

Met Pro Ala Leu Glu His Met Asn Gln Ile Leu His Ile Leu Phe Val
1               5                   10                  15

Phe Leu Pro Phe Leu Trp Ala Leu Gly Thr Leu Pro Pro Pro Asp Ala
            20                  25                  30

Leu Leu Leu Trp Ala Met Glu Gln Val Leu Glu Phe Gly Leu Gly Gly
        35                  40                  45

Ser Ser Met Ser Thr His Leu Arg Leu Leu Val Met Phe Ile Met Ser
    50                  55                  60

Ala Gly Thr Ala Ile Ala Ser Tyr Phe Ile Pro Ser Thr Val Gly Val
65                  70                  75                  80

Val Leu Phe Met Thr Gly Phe Gly Phe Leu Leu Ser Leu Asn Leu Ser
                85                  90                  95

Asp Met Gly His Lys Ile Gly Thr Lys Ser Lys Asp Leu Pro Ser Gly
            100                 105                 110

Pro Glu Lys His Phe Ser Trp Lys Glu Cys Leu Phe Tyr Ile Ile Ile
        115                 120                 125

Leu Val Leu Ala Leu Leu Glu Thr Ser Leu Leu His His Phe Ala Gly
    130                 135                 140

Phe Ser Gln Ile Ser Lys Ser Asn Ser Gln Ala Ile Val Gly Tyr Gly
145                 150                 155                 160

Leu Met Ile Leu Leu Ile Ile Leu Trp Ile Leu Arg Glu Ile Gln Ser
                165                 170                 175

Val Tyr Ile Ile Gly Ile Phe Arg Asn Pro Phe Tyr Pro Lys Asp Val
            180                 185                 190

Gln Thr Val Thr Val Phe Phe Glu Lys Gln Thr Arg Leu Met Lys Ile
        195                 200                 205

Gly Ile Val Arg Arg Ile Leu Leu Thr Leu Val Ser Pro Phe Ala Met
    210                 215                 220

Ile Ala Phe Leu Ser Leu Asp Ser Ser Leu Gln Gly Leu His Ser Val
225                 230                 235                 240

Ser Val Cys Ile Gly Phe Thr Arg Ala Phe Arg Met Val Trp Gln Asn
                245                 250                 255

Thr Glu Asn Ala Leu Leu Glu Thr Val Ile Val Ser Thr Val His Leu
            260                 265                 270

Ile Ser Ser Thr Asp Ile Trp Trp Asn Arg Ser Leu Asp Thr Gly Leu
        275                 280                 285

Arg Leu Leu Leu Val Gly Ile Ile Arg Asp Arg Leu Ile Gln Phe Ile
    290                 295                 300

Ser Lys Leu Gln Phe Ala Val Thr Val Leu Leu Thr Ser Trp Thr Glu
305                 310                 315                 320

Lys Lys Gln Arg Arg Lys Thr Thr Ala Thr Leu Cys Ile Leu Asn Ile
                325                 330                 335

Val Phe Ser Pro Phe Val Leu Val Ile Ile Val Phe Ser Thr Leu Leu
            340                 345                 350

Ser Ser Pro Leu Leu Pro Leu Phe Thr Leu Pro Val Phe Leu Val Gly
        355                 360                 365

Phe Pro Arg Pro Ile Gln Ser Trp Pro Gly Ala Ala Gly Thr Thr Ala
    370                 375                 380

Cys Val Cys Ala Asp Thr Val Tyr Tyr Tyr Gln Met Val Pro Arg Leu
385                 390                 395                 400

Thr Ala Val Leu Gln Thr Ala Met Ala Ala Gly Ser Leu Gly Leu Leu
                405                 410                 415

Leu Pro Gly Ser His Tyr Leu Gly Arg Phe Gln Asp Arg Leu Met Trp
            420                 425                 430

Ile Met Ile Leu Glu Cys Gly Tyr Thr Tyr Cys Ser Ile Asn Ile Lys
        435                 440                 445

Gly Leu Glu Leu Gln Glu Thr Ser Cys His Thr Ala Glu Ala Arg Arg
    450                 455                 460

Val Asp Glu Val Phe Glu Asp Ala Phe Glu Gln Glu Tyr Thr Arg Val
465                 470                 475                 480

Cys Ser Leu Asn Glu His Phe Gly Asn Val Leu Thr Pro Cys Thr Val
                485                 490                 495

Leu Pro Val Lys Leu Tyr Ser Asp Ala Arg Asn Val Leu Ser Gly Ile
            500                 505                 510

Ile Asp Ser His Glu Asn Leu Lys Glu Phe Lys Gly Asp Leu Ile Lys
        515                 520                 525

Val Leu Val Trp Ile Leu Val Gln Tyr Cys Ser Lys Arg Pro Gly Met
    530                 535                 540

Lys Glu Asn Val His Asn Thr Glu Asn Lys Gly Lys Ala Pro Leu Met
545                 550                 555                 560

Leu Pro Ala Leu Asn Thr Leu Pro Pro Pro Lys Ser Pro Glu Asp Ile
                565                 570                 575

Asp Ser Leu Asn Ser Glu Thr Phe Asn Asp Trp Ser Asp Asp Asn Ile
            580                 585                 590

Phe Asp Asp Glu Pro Thr Ile Lys Lys Val Ile Glu Glu Lys His Gln

595                 600                 605

Leu Lys Asp Leu Pro Gly Thr Asn Leu Phe Ile Pro Gly Ser Val Glu
    610                 615                 620

Ser Gln Arg Val Gly Asp His Ser Thr Gly Thr Val Pro Glu Asn Asp
625                 630                 635                 640

Leu Tyr Lys Ala Val Leu Leu Gly Tyr Pro Ala Val Asp Lys Gly Lys
                645                 650                 655

Gln Glu Asp Met Pro Tyr Ile Pro Leu Met Glu Phe Ser Cys Ser His
            660                 665                 670

Ser His Leu Val Cys Leu Pro Ala Glu Trp Arg Thr Ser Cys Met Pro
        675                 680                 685

Ser Ser Lys Met Lys Glu Met Ser Ser Leu Phe Pro Glu Asp Trp Tyr
    690                 695                 700

Gln Phe Val Leu Arg Gln Leu Glu Cys Tyr His Ser Glu Glu Lys Ala
705                 710                 715                 720

Ser Asn Val Leu Glu Glu Ile Ala Lys Asp Lys Val Leu Lys Asp Phe
                725                 730                 735

Tyr Val His Thr Val Met Thr Cys Tyr Phe Ser Leu Phe Gly Ile Asp
            740                 745                 750

Asn Met Ala Pro Ser Pro Gly His Ile Leu Arg Val Tyr Gly Gly Val
        755                 760                 765

Leu Pro Trp Ser Val Ala Leu Asp Trp Leu Thr Glu Lys Pro Glu Leu
    770                 775                 780

Phe Gln Leu Ala Leu Lys Ala Phe Arg Tyr Thr Leu Lys Leu Met Ile
785                 790                 795                 800

Asp Lys Ala Ser Leu Gly Pro Ile Glu Asp Phe Arg Glu Leu Ile Lys
                805                 810                 815

Tyr Leu Glu Glu Tyr Glu Arg Asp Trp Tyr Ile Gly Leu Val Ser Asp
            820                 825                 830

Glu Lys Trp Lys Glu Ala Ile Leu Gln Glu Lys Pro Tyr Leu Phe Ser
        835                 840                 845

Leu Gly Tyr Asp Ser Asn Met Pro Gly Pro Ala Leu Glu Ile Ser Arg
    850                 855                 860

Val Asn Arg Asn Leu Trp Ser Gln Ile
865                 870

<210>  32
<211>  3927
<212>  DNA
<213>  Homo sapiens

<400>  32
aacgacgctc ttgcgtaaag gcccggccca agggaacgtt cagggcgtct cggctttccc      60

cgctgctgct tctgctaggc ccagtgcgag accagagcac gagcgactcc cgtcgtcccc     120

ggccaggcag atgttggcct agtcctggcg cgaacgaagc gcgctatttc cctgcttcct     180

ctaggccaag cctgctttac ggcagggccc gcctcgggag cgagcacaga ccggggcagc     240

gaggccagcc aggcgccgac gaggtccccg aacgcgcacg cgctccgttc agctccgggt     300

ggcggccgcc ggagtagacg ttagccatgg aaaccgagag ctggcccggg cggggccgcg     360

gtgagctcgt tattcggccg ccgcagcttt tctgcctccg cattcgggca ctaaccaacc     420

tcccggcggg agcgcccagc ccgagtttac ctgcaaaaat gcggtccctg ggatgccttc     480

gcgtcttctc ttccctcggg tgacttgagg tttgtggtaa atatgccagc tctagaacac     540

atgaatcaga ttttacacat cttgtttgta tttttaccct ttctgtgggc acttgggact     600

ctgcccccac ccgatgcact tctcttatgg gcaatggagc aggttttaga gttcggcctt     660

ggaggctcat ctatgtcaac ccacttacgg ttattagtaa tgttcatcat gtctgctgga     720

acagctatag catcatattt cattccaagc actgttggtg tggttctttt catgactgga     780

tttggtttct tgctgagtct gaacttaagt gatatgggtc acaaaattgg aaccaaatct     840

aaggatttac ccagtggtcc ggaaaaacat ttttcatgga aggaatgcct tttctacatc     900

attatattag tcttggctct tttagaaact agcttgcttc atcactttgc tggcttctca     960

cagatttcta aaagcaattc ccaggctatt gtgggctatg gtttgatgat attacttata    1020

atactgtgga tacttagaga aattcaaagc gtatatatca ttggaatttt ccgaaatccc    1080

ttttatccga aggatgtgca aactgtgact gtattctttg agaagcaaac taggctcatg    1140

aagattggta ttgtcagacg gattttgcta actttagtat caccttttgc catgatagca    1200

tttctttcat tggacagttc cttacaaggg ctccactcag tgtctgtctg tattggattc    1260

acaagagcct ttagaatggt atggcagaat acagaaaatg ctttattgga gacagtcatt    1320

gtatcaacag tacacttgat ctccagtaca gacatatggt ggaacagaag cctggataca    1380

ggactcagac tcttactggt tggtatcata cgtgatcgtt tgattcagtt catctctaaa    1440

ttgcagtttg ccgtgactgt gcttttgaca tcatggacag agaaaaaaca acgtcgaaaa    1500

acaactgcca ctttatgtat actcaacatt gtcttttctc cattcgtgtt ggtcatcata 1560

gtttttctta cactactctc ttctccctta ctccctcttt tcacccttcc tgtgttcttg 1620

gtggggtttc cccgacctat tcagagttgg ccaggagcag caggcaccac agcctgtgtg 1680

tgtgcagata cagtgtacta ctaccaaatg gtgcccaggt tgactgctgt actgcagact 1740

gcaatggcag ctggaagttt aggtctcctc ctacctggat ctcattactt gggccgtttt 1800

caggatcgtt taatgtggat aatgattctg gaatgtggct atacttactg ctctattaac 1860

attaaggggt tagaattgca ggaaacatcc tgtcatactg cagaagctcg cagagttgat 1920

gaagtttttg aagatgcttt tgagcaagaa tacacaagag tatgttccct taatgaacac 1980

tttggaaatg tcttgacacc ctgtactgtt ttgcctgtga aattgtattc tgatgccagg 2040

aatgttctat caggcataat tgattctcat gaaaacttaa aagaatttaa aggtgacctc 2100

attaaagtac ttgtgtggat acttgttcaa tactgctcca aaaggcctgg catgaaagag 2160

aatgttcaca acactgaaaa taaaggaaaa gcacctctaa tgttgcctgc tttgaacact 2220

ttgccacctc ccaaatcccc agaagacata gacagtttaa attcagaaac ttttaatgac 2280

tggtctgatg ataatatttt tgatgatgag ccaactatca aaaaagtaat agaagaaaaa 2340

catcagttga aagatttgcc aggtacaaat ttgtttattc caggatcagt agaatcacag 2400

agggttggtg atcattctac aggcactgtt cctgaaaacg atctttacaa agcagttcta 2460

ttaggatacc ctgctgttga caaaggaaaa caagaggaca tgccatatat tcctctcatg 2520

gagttcagtt gttcacattc tcacttagta tgcttacccg cagagtggag gactagctgt 2580

atgcccagtt ccaaaatgaa ggagatgagc tcgttatttc cagaagactg gtaccaattt 2640

gttctaaggc agttggaatg ttatcattca gaagagaagg cctcaaatgt actggaagaa 2700

attgccaagg acaaagtttt aaaagacttt tatgttcata cagtaatgac ttgttatttt 2760

agtttatttg gaatagacaa tatggctcct agtcctggtc atatattgag agtttacggt 2820

ggtgttttgc cttggtctgt tgctttggac tggctcacag aaaagccaga actgtttcaa 2880

ctagcactga aagcattcag gtatactctg aaactaatga ttgataaagc aagtttaggt 2940

ccaatagaag actttagaga actgattaag taccttgaag aatatgaacg tgactggtac 3000

attggtttgg tatctgatga aaagtggaag gaagcaattt tacaagaaaa gccatacttg 3060

ttttctctgg ggtatgattc taatatggga atttacactg ggagagtgct tagccttcaa 3120

gaattattga tccaagtggg aaagttaaat cctgaagctg ttagaggtca gtgggccaat 3180

ctttcatggg aattacttta tgccacaaac gatgatgaag aacgttatag tatacaagct 3240

catccactac ttttaagaaa tcttacggta caagcagcag aacctcccct gggatatccg 3300

atttattctt caaaacctct ccacatacat ttgtattaga gctcattttg actgtaatgt 3360

catcaaatgc aatgttttta ttttttcatc ctaaaaaagt aactgtgatt cttgtaactt 3420

gaggacttct ccacaccccc attcagatgc ctgagaacag ctaagctccg taaagttggt 3480

tctcttagcc atcttaatgg ttctaaaaaa cagcaaaaac atctttatgt ctaagataaa 3540

agaactattt ggccaatatt tgtgccctct ggactttagt aggctttggt aaatgtgaga 3600

aaacttttgt agaattatca tataatgaat tttgtaatgc tttcttaaat gtgttatagg 3660

tgaattgcca tacaaagtta acagctatgt aatttttaca tacttaagag ataaacatat 3720

cagtgttcta agtagtgata atggatcctg ttgaaggtta acataatgtg tatatatttg 3780

tttgaaatat aatttatagt attttcaaat gtgctgattt attttgacat ctaatatctg 3840

aatgtttttg tatcaagtag tttgttttca tagacttcaa ttcataaact ttaaaaaact 3900

tttaataaaa tattttcctt ccttttc 3927

<210> 33
<211> 3932
<212> DNA
<213> Homo sapiens

<400> 33
aacgacgctc ttgcgtaaag gcccggccca agggaacgtt cagggcgtct cggctttccc 60

cgctgctgct tctgctaggc ccagtgcgag accagagcac gagcgactcc cgtcgtcccc 120

ggccaggcag atgttggcct agtcctggcg cgaacgaagc gcgctatttc cctgcttcct 180

ctaggccaag cctgctttac ggcagggccc gcctcgggag cgagcacaga ccggggcagc 240

gaggccagcc aggcgccgac gaggtccccg aacgcgcacg cgctccgttc agctccgggt 300

ggcggccgcc ggagtagacg ttagccatgg aaaccgagag ctggcccggg cggggccgcg 360

gtgagctcgt tattcggccg ccgcagcttt tctgcctccg cattcgggca ctaaccaacc 420

tcccggcggg agcgcccagc ccgagtttac ctgcaaaaat gcggtccctg ggatgccttc 480

gcgtcttctc ttccctcggg tgacttgagg tttgtggtaa atatgccagc tctagaacac 540

atgaatcaga ttttacacat cttgtttgta tttttaccct ttctgtgggc acttgggact 600

ctgcccccac ccgatgcact tctcttatgg gcaatggagc aggttttaga gttcggcctt 660

ggaggctcat ctatgtcaac ccacttacgg ttattagtaa tgttcatcat gtctgctgga 720

acagctatag catcatattt cattccaagc actgttggtg tggttctttt catgactgga 780

tttggtttct tgctgagtct gaacttaagt gatatgggtc acaaaattgg aaccaaatct 840

aaggatttac ccagtggtcc ggaaaaacat ttttcatgga aggaatgcct tttctacatc 900

attatattag tcttggctct tttagaaact agcttgcttc atcactttgc tggcttctca 960

cagatttcta aaagcaattc ccaggctatt gtgggctatg gtttgatgat attacttata 1020

atactgtgga tacttagaga aattcaaagc gtatatatca ttggaatttt ccgaaatccc 1080

ttttatccga aggatgtgca aactgtgact gtattctttg agaagcaaac taggctcatg 1140

aagattggta ttgtcagacg gattttgcta actttagtat caccttttgc catgatagca 1200

tttctttcat tggacagttc cttacaaggg ctccactcag tgtctgtctg tattggattc 1260

acaagagcct ttagaatggt atggcagaat acagaaaatg ctttattgga gacagtcatt 1320

gtatcaacag tacacttgat ctccagtaca gacatatggt ggaacagaag cctggataca 1380

ggactcagac tcttactggt tggtatcata cgtgatcgtt tgattcagtt catctctaaa 1440

ttgcagtttg ccgtgactgt gcttttgaca tcatggacag agaaaaaaca acgtcgaaaa 1500

acaactgcca ctttatgtat actcaacatt gtcttttctc cattcgtgtt ggtcatcata 1560

gttttttcta cactactctc ttctccctta ctccctcttt tcacccttcc tgtgttcttg 1620

gtggggtttc cccgacctat tcagagttgg ccaggagcag caggcaccac agcctgtgtg 1680

tgtgcagata cagtgtacta ctaccaaatg gtgcccaggt tgactgctgt actgcagact 1740

gcaatggcag ctggaagttt aggtctcctc ctacctggat ctcattactt gggccgtttt 1800

caggatcgtt taatgtggat aatgattctg gaatgtggct atacttactg ctctattaac 1860

attaaggggt tagaattgca ggaaacatcc tgtcatactg cagaagctcg cagagttgat 1920

gaagtttttg aagatgcttt tgagcaagaa tacacaagag tatgttccct taatgaacac 1980

tttggaaatg tcttgacacc ctgtactgtt ttgcctgtga aattgtattc tgatgccagg 2040

aatgttctat caggcataat tgattctcat gaaaacttaa aagaatttaa aggtgacctc 2100

attaaagtac ttgtgtggat acttgttcaa tactgctcca aaaggcctgg catgaaagag 2160

aatgttcaca acactgaaaa taaaggaaaa gcacctctaa tgttgcctgc tttgaacact 2220

ttgccacctc ccaaatcccc agaagacata gacagtttaa attcagaaac ttttaatgac 2280

tggtctgatg ataatatttt tgatgatgag ccaactatca aaaaagtaat agaagaaaaa 2340

catcagttga aagatttgcc aggtacaaat ttgtttattc caggatcagt agaatcacag 2400

agggttggtg atcattctac aggcactgtt cctgaaaacg atctttacaa agcagttcta 2460

ttaggatacc ctgctgttga caaaggaaaa caagaggaca tgccatatat tcctctcatg 2520

gagttcagtt gttcacattc tcacttagta tgcttacccg cagagtggag gactagctgt 2580

atgcccagtt ccaaaatgaa ggagatgagc tcgttatttc cagaagactg gtaccaattt 2640

gttctaaggc agttggaatg ttatcattca gaagagaagg cctcaaatgt actggaagaa 2700

attgccaagg acaaagtttt aaaagacttt tatgttcata cagtaatgac ttgttatttt 2760

agtttatttg gaatagacaa tatggctcct agtcctggtc atatattgag agtttacggt 2820

ggtgttttgc cttggtctgt tgctttggac tggctcacag aaaagccaga actgtttcaa 2880

ctagcactga aagcattcag gtatactctg aaactaatga ttgataaagc aagtttaggt 2940

ccaatagaag actttagaga actgattaag taccttgaag aatatgaacg tgactggtac 3000

attggtttgg tatctgatga aaagtggaag gaagcaattt tacaagaaaa gccatacttg 3060

ttttctctgg ggtatgattc taatatggga atttacactg ggagagtgct tagccttcaa 3120

gaattattga tccaagtggg aaagttaaat cctgaagctg ttagaggtca gtgggccaat 3180

ctttcatggg aattacttta tgccacaaac gatgatgaag aacgttatag tatacaagct 3240

catccactac ttttaagaaa tcttacggta caagcagcag aacctcccct gggatatccg 3300

atttattctt caaaacctct ccacatacat ttgtattaga gctcattttg actgtaatgt 3360

catcaaatgc aatgttttta ttttttcatc ctaaaaaagt aactgtgatt cttgtaactt 3420

gaggacttct ccacaccccc attcagatgc ctgagaacag ctaagctccg taaagttggt 3480

tctcttagcc atcttaatgg ttctaaaaaa cagcaaaaac atctttatgt ctaagataaa 3540

agaactattt ggccaatatt tgtgccctct ggactttagt aggctttggt aaatgtgaga 3600

aaacttttgt agaattatca tataatgaat tttgtaatgc tttcttaaat gtgttatagg 3660

tgaattgcca tacaaagtta acagctatgt aatttttaca tacttaagag ataaacatat 3720

cagtgttcta agtagtgata atggatcctg ttgaaggtta acataatgtg tatatatttg 3780

tttgaaatat aatttatagt attttcaaat gtgctgattt attttgacat ctaatatctg 3840

aatgtttttg tatcaagtag tttgttttca tagacttcaa ttcataaact ttaaaaaact 3900

tttaataaaa tattttcctt ccttttcaaa ta 3932

<210> 34
<211> 4073
<212> DNA
<213> Homo sapiens

<400> 34
tcttgcgtaa aggcccggcc caagggaacg ttcagggcgt ctcggctttc cccgctgctg 60

cttctgctag gcccagtgcg agaccagagc acgagcgact cccgtcgtcc ccggccaggc 120

agatgttggc ctagtcctgg cgcgaacgaa gcgcgctatt tccctgcttc ctctaggcca 180

agcctgcttt acggcagggc ccgcctcggg agcgagcaca gaccggggca gcgaggccag 240

ccaggcgccg acgaggtccc cgaacgcgca cgcgctccgt tcagctccgg gtggcggccg 300

ccggagtaga cgttagccat ggaaaccgag agctggcccg ggcggggccg cggtgagctc 360

gttattcggc cgccgcagct tttctgcctc cgcattcggg cactaaccaa cctcccggcg 420

ggagcgccca gcccgagttt acctgcaaaa atgcggtccc tgggatgcct tcgcgtcttc 480

tcttccctcg ggtgacttga gaaactgctg tgttacagaa aagcatgtga ctttcagaat 540

aatcccgagt gaggatgagt ccagatgtgc ctctactgaa tgattacaag caggacttct 600

ttctgaagcg ctttccacag actgttcttg gaggccctcg attcaaatta ggctattgtg 660

cccctcctta catatatgtt aatcaaatta ttctttttct aatgccatgg gtttggggtg 720

gagtcggaac acttttatac cagttaggca tcctgaaaga ctattataca gcagcacttt 780

caggtggatt aatgcttttt actgcatttg tcatccagtt cacaagttta tacgccaaaa 840

acaaatcaac aacagtagaa agaatactaa ccacggatat cttagcagag gaggatgagc 900

atgaatttac cagttgtact ggtgctgaga ctgtcaaatt tctcattcct ggcaagaaat 960

atgtagccaa tacagttttt cattctattc ttgctggatt agcgtgtggt cttggaacat 1020

ggtatctgct cccaaataga ataaccttgc tgtatggcag tacaggaggc actgctctac 1080

tattcttctt tggatggatg acactatgta tagcagaata ttctttaatt gtaaacacag 1140

ctacagagac tgcgactttc caaacacagg atacttatga aattattcct cttatgagac 1200

ctctttatat ttttttcttt gtttctgtgg atctggcaca caggtaaaaa cctaccaaat 1260

actttgtaac taactttgtt tttaagtata cagagtaaga gagctttcct tttagtgtta 1320

caaaaaaatg aatccatgga ttaaaaatca tcaaaccatt gggtgacagg ttattttgat 1380

aattattctt ttaggattaa tctctgtaaa acatactaaa gcaatagtta aaacttatta 1440

aagagttttt ttaaaaaacc ctttttgaga taaggaactt ttcaattttg tgtttcactt 1500

taaataagga gctttgagtt tttaagatag cctggctaaa acctgtgtaa ggagatggaa 1560

ctttcctgtg gggggaaaga agaaattaaa atttatacat ataaatatta tatacagatt 1620

gaatgaattt aagacaaata caaaatttat ttctaatttt atgatagcaa caatagtaga 1680

agtaatattg attttttaaa aaccaacttg ttacagaaga aaagtagaaa atagtttttt 1740

taacagacat aattgttcac aaaattgttt gataacccct tttacttgcc ttttcaagtt 1800

tgacttttct ttctccgtct ccgtagattg cagctttctt ttcttaggtt agtgtccagg 1860

tagaaatgtt cagcatgtta tggactgaat atttgtgtct ctccagaatt catatattac 1920

agtcttaaac cccaatgtga tggtattttg agatgaggcc tttgggaggt aattaggtca 1980

tgaaggtggg tccttggtat gatgggacta gtccccttat gagaagaggt accagagagc 2040

ttgatttgtc cctctctgtg ccatctaagg acacagcaaa aatgtggcca cctgcaagtc 2100

aaagaagaga gctctcatca aaacctgacc atactggcag cctgatcatg gtctttcggc 2160

cttcagaagt gtgagaaagt aaattgatgt tgtttaagcc actcagctta tggcattttt 2220

ttatggcagc ccatgctgat taagattttg ctaccaagaa gtgggatgcc tttgtaccaa 2280

atacctaaaa atgtggaaat ggctttgtaa ctgttggtaa tgggtagagg ctgaaagagg 2340

tttttttgtt tgtttctttg ttttgttttg tttgagatgg agtcttcact ctcttgccca 2400

ggctggagtg cagtggcaca atcttggccc actgcaacgt ccgcctcctg gcttcaagtg 2460

atactcctcc ctgagcctcc tgagtagctg gaattacaga catgcgccac catgcccggc 2520

taatttttat atttttaata gagacagggt ttcgccatgt tggccaggct ggtcccaaag 2580

tcctgacctc aagtgatcca cccgcctcgg cctcccaaag ttctgggatt acaggcttga 2640

gccatcacac ccagccaagg gttttgatgt gcatgctaga aatatggaca ttaagggtga 2700

ttctgatgaa gtctgaagtc ccctgaaccc agaggaaagg cagtccttgt tacaaagcgt 2760

caaagaactt agctgaactg tgtcctagtg ttttgtggag ccatgaagtt ggatacctac 2820

gtaaggagag ttcaaagcag tgttgaaagg agcagttggc ttctcctgtt tctagtaaaa 2880

tgtgaaagga gagagatgga ttgaagaagg gattgttaag caagagaagg aaccagaact 2940

tgaagatttg gaaaattttc agcctggatt atccatattg taaaacatga gaaagcatat 3000

tctgaagaga acaccaaaag tgtggggctg gactgtccct cagtaaagag cttttggcat 3060

tatgtgagta gaaacactgc cagtttgaat tgaagtggtt ggagacagga agtaatgaag 3120

gccgacagtt gaacttcttg gatttgacag gatgtaatga tagaactgtt cagctacaaa 3180

agtgcagtat tcttcaagaa aaggggaaaa ttatgccaaa ggtgatttaa gggtcttgag 3240

ggctaccacc tgtttcaaca agtcagctag cctctaccca aagcctcggg agcagaactg 3300

aacttcagag ccacagaagc aggaccctca cctagagcac tggcggtgac ctgccacccc 3360

agtggcctgg tgggcagagt atggaaccaa acagaattat tctcaagctc taagatctaa 3420

tggaatttgc cttgctaggt tttgacttcc ttgggatcat cacccttttt ttcctgtttc 3480

tcctattgga gtggggatgt ctcttctata cctgccctac ccttagattt tggaagcacg 3540

taactcatct ggtttcacag attcacagct ggagaggaat tttgcctcag gatggattgt 3600

acctaggtct catccatatc tgatttagat gagactttga attttagcca tcagagttaa 3660

tgctagaatg agtgaagact ttgggggata tggggatgga atgaatgtct tttgcatttg 3720

aatttggaaa ggacatgagt tttgaggggc cagggatgga atgttataga ctaattgtgt 3780

tccctcaaaa atgtttatgt tgaagcccta acccccaatg tgttggtatt tggagatggg 3840

cctctgggag gtagtttatg aaggtgagac cctagtctga taggattagt gcccttagga 3900

gagatgccaa agagcttgat gtctctcttt ttgctacaaa aagacacagc aaaaaggcag 3960

ccatgtgtaa gccaggaaga gagtcttcac cagaacctga ctatactggc agcctgatct 4020

tgtacttgta gcccccagaa ctgttagaaa ataaatttct gttgtttaag cca 4073

<210> 35
<211> 4666
<212> DNA
<213> Homo sapiens

<400> 35
tcttgcgtaa aggcccggcc caagggaacg ttcagggcgt ctcggctttc cccgctgctg 60

cttctgctag gcccagtgcg agaccagagc acgagcgact cccgtcgtcc ccggccaggc 120

agatgttggc ctagtcctgg cgcgaacgaa gcgcgctatt tccctgcttc ctctaggcca 180

agcctgcttt acggcagggc ccgcctcggg agcgagcaca gaccggggca gcgaggccag 240

ccaggcgccg acgaggtccc cgaacgcgca cgcgctccgt tcagctccgg gtggcggccg 300

ccggagtaga cgttagccat ggaaaccgag agctggcccg ggcggggccg cggtgagctc 360

gttattcggc cgccgcagct tttctgcctc cgcattcggg cactaaccaa cctcccggcg 420

ggagcgccca gcccgagttt acctgcaaaa atgcggtccc tgggatgcct tcgcgtcttc 480

tcttccctcg ggtgacttga gaaactgctg tgttacagaa aagcatgtga ctttcagaat 540

aatcccgagt gaggatgagt ccagatgtgc ctctactgaa tgattacaag caggacttct 600

ttctgaagcg ctttccacag actgttcttg gaggccctcg attcaaatta ggctattgtg 660

cccctcctta catatatgtt aatcaaatta ttctttttct aatgccatgg gtttggggtg 720

gagtcggaac acttttatac cagttaggca tcctgaaaga ctattataca gcagcacttt 780

caggtggatt aatgcttttt actgcatttg tcatccagtt cacaagttta tacgccaaaa 840

acaaatcaac aacagtagaa agaatactaa ccacggatat cttagcagag gaggatgagc 900

atgaatttac cagttgtact ggtgctgaga ctgtcaaatt tctcattcct ggcaagaaat 960

atgtagccaa tacagttttt cattctattc ttgctggatt agcgtgtggt cttggaacat 1020

ggtatctgct cccaaataga ataaccttgc tgtatggcag tacaggaggc actgctctac 1080

tattcttctt tggatggatg acactatgta tagcagaata ttctttaatt gtaaacacag 1140

ctacagagac tgcgactttc caaacacagg atacttatga aattattcct cttatgagac 1200

ctctttatat tttttctttt gtttctgtgg atctggcaca caggtttgtg gtaaatatgc 1260

cagctctaga acacatgaat cagattttac acatcttgtt tgtattttta ccctttctgt 1320

gggcacttgg gactctgccc ccacccgatg cacttctctt atgggcaatg gagcaggttt 1380

tagagttcgg ccttggaggc tcatctatgt caacccactt acggttatta gtaatgttca 1440

tcatgtctgc tggaacagct atagcatcat atttcattcc aagcactgtt ggtgtggttc 1500

ttttcatgac tggatttggt ttcttgctga gtctgaactt aagtgatatg ggtcacaaaa 1560

ttggaaccaa atctaaggat ttacccagtg gtccggaaaa acatttttca tggaaggaat 1620

gccttttcta catcattata ttagtcttgg ctcttttaga aactagcttg cttcatcact 1680

ttgctggctt ctcacagatt tctaaaagca attcccaggc tattgtgggc tatggtttga 1740

tgatattact tataatactg tggatactta gagaaattca aagcgtatat atcattggaa 1800

ttttccgaaa tcccttttat ccgaaggatg tgcaaactgt gactgtattc tttgagaagc 1860

aaactaggct catgaagatt ggtattgtca gacggatttt gctaacttta gtatcacctt 1920

ttgccatgat agcatttctt tcattggaca gttccttaca agggctccac tcagtgtctg 1980

tctgtattgg attcacaaga gcctttagaa tggtatggca gaatacagaa aatgctttat 2040

tggagacagt cattgtatca acagtacact tgatctccag tacagacata tggtggaaca 2100

gaagcctgga tacaggactc agactcttac tggttggtat catacgtgat cgtttgattc 2160

agttcatctc taaattgcag tttgccgtga ctgtgctttt gacatcatgg acagagaaaa 2220

aacaacgtcg aaaaacaact gccactttat gtatactcaa cattgtcttt tctccattcg 2280

tgttggtcat catagttttt tctacactac tctcttctcc cttactccct cttttcaccc 2340

ttcctgtgtt cttggtgggg tttccccgac ctattcagag ttggccagga gcagcaggca 2400

ccacagcctg tgtgtgtgca gatacagtgt actactacca aatggtgccc aggttgactg 2460

ctgtactgca gactgcaatg gcagctggaa gtttaggtct cctcctacct ggatctcatt 2520

acttgggccg ttttcaggat cgtttaatgt ggataatgat tctggaatgt ggctatactt 2580

actgctctat taacattaag gggttagaat tgcaggaaac atcctgtcat actgcagaag 2640

ctcgcagagt tgatgaagtt tttgaagatg cttttgagca agaatacaca agagtatgtt 2700

cccttaatga acactttgga aatgtcttga caccctgtac tgttttgcct gtgaaattgt 2760

attctgatgc caggaatgtt ctatcaggca taattgattc tcatgaaaac ttaaaagaat 2820

ttaaaggtga cctcattaaa gtacttgtgt ggatacttgt tcaatactgc tccaaaaggc 2880

ctggcatgaa agagaatgtt cacaacactg aaaataaagg gaaagcacct ctaatgttgc 2940

ctgctttgaa cactttgcca cctcccaaat ccccagaaga catagacagt ttaaattcag 3000

aaacttttaa tgactggtct gatgataata tttttgatga tgagccaact atcaaaaaag 3060

taatagaaga aaaacatcag ttgaaagatt tgccaggtac aaatttgttt attccaggat 3120

cagtagaatc acagagggtt ggtgatcatt ctacaggcac tgttcctgaa aacgatcttt 3180

acaaagcagt tctattagga taccctgctg ttgacaaagg aaaacaagag gacatgccat 3240

atattcctct catggagttc agttgttcac attctcactt agtatgctta cccgcagagt 3300

ggaggactag ctgtatgccc agttccaaaa tgaaggagat gagctcgtta tttccagaag 3360

actggtacca atttgttcta aggcagttgg aatgttatca ttcagaagag aaggcctcaa 3420

atgtactgga agaaattgcc aaggacaaag ttttaaaaga cttttatgtt catacagtaa 3480

tgacttgtta ttttagttta tttggaatag acaatatggc tcctagtcct ggtcatatat 3540

tgagagttta cggtggtgtt ttgccttggt ctgttgcttt ggactggctc acagaaaagc 3600

cagaactgtt tcaactagca ctgaaagcat tcaggtatac tctgaaacta atgattgata 3660

aagcaagttt aggtccaata gaagacttta gagaactgat taagtacctt gaagaatatg 3720

aacgtgactg gtacattggt ttggtatctg atgaaaagtg gaaggaagca attttacaag 3780

aaaagccata cttgttttct ctggggtatg attctaatat gggaatttac actgggagag 3840

tgcttagcct tcaagaatta ttgatccaag tgggaaagtt aaatcctgaa gctgttagag 3900

gtcagtgggc caatctttca tgggaattac tttatgccac aaacgatgat gaagaacgtt 3960

atagtataca agctcatcca ctacttttaa gaaatcttac ggtacaagca gcagaacctc 4020

ccctgggata tccgatttat tcttcaaaac ctctccacat acatttgtat tagagctcat 4080

tttgactgta atgtcatcaa atgcaatgtt tttatttttt catcctaaaa aagtaactgt 4140

gattcttgta acttgaggac ttctccacac ccccattcag atgcctgaga acagctaagc 4200

tccgtaaagt tggttctctt agccatctta atggttctaa aaaacagcaa aaacatcttt 4260

atgtctaaga taaaagaact atttggccaa tatttgtgcc ctctggactt tagtaggctt 4320

tggtaaatgt gagaaaactt ttgtagaatt atcatataat gaattttgta atgctttctt 4380

aaatgtgtta taggtgaatt gccatacaaa gttaacagct atgtaatttt tacatactta 4440

agagataaac atatcagtgt tctaagtagt gataatggat cctgttgaag gttaacataa 4500

tgtgtatata tttgtttgaa atataattta tagtattttc aaatgtgctg atttattttg 4560

acatctaata tctgaatgtt tttgtatcaa gtagtttgtt ttcatagact tcaattcata 4620

aactttaaaa aacttttaat aaaatatttt ccttcctttt caaata 4666

<210> 36
<211> 3513
<212> DNA
<213> Homo sapiens

<400> 36
aaaggcccgg cccaagggaa cgttcagggc gtctcggctt tccccgctgc tgcttctgct 60

aggcccagtg cgagaccaga gcacgagcga ctcccgtcgt ccccggccag gcagatgttg 120

gcctagtcct ggcgcgaacg aagcgcgcta tttccctgct tcctctaggc caagcctgct 180

ttacggcagg gcccgcctcg ggagcgagca cagaccgggg cagcgaggcc agccaggcgc 240

cgacgaggtc cccgaacgcg cacgcgctcc gttcagctcc gggtggcggc cgccggagta 300

gacgttagcc atggaaaccg agagctggcc cgggcggggc cgcggtgagc tcgttattcg 360

gccgccgcag cttttctgcc tccgcattcg ggcactaacc aacctcccgg cgggagcgcc 420

cagcccgagt ttacctgcaa aaatgcggtc cctgggatgc cttcgcgtct tctcttccct 480

cgggtgactt gaggtttgtg gtaaatatgc cagctctaga acacatgaat cagattttac 540

acatcttgtt tgtattttta ccctttctgt gggcacttgg gactctgccc ccacccgatg 600

cacttctctt atgggcaatg gagcaggttt tagagttcgg ccttggaggc tcatctatgt 660

caacccactt acggttatta gtaatgttca tcatgtctgc tggaacagct atagcatcat 720

atttcattcc aagcactgtt ggtgtggttc ttttcatgac tggatttggt ttcttgctga 780

gtctgaactt aagtgatatg ggtcacaaaa ttggaaccaa atctaaggat ttacccagtg 840

gtccggaaaa acatttttca tggaaggaat gccttttcta catcattata ttagtcttgg 900

ctcttttaga aactagcttg cttcatcact ttgctggctt ctcacagatt tctaaaagca 960

attcccaggc tattgtgggc tatggtttga tgatattact tataatactg tggatactta 1020

gagaaattca aagcgtatat atcattggaa ttttccgaaa tcccttttat ccgaaggatg 1080

tgcaaactgt gactgtattc tttgagaagc aaactaggct catgaagatt ggtattgtca 1140

gacggatttt gctaacttta gtatcacctt ttgccatgat agcatttctt tcattggaca 1200

gttccttaca agggctccac tcagtgtctg tctgtattgg attcacaaga gcctttagaa 1260

tggtatggca gaatacagaa aatgctttat tggagacagt cattgtatca acagtacact 1320

tgatctccag tacagacata tggtggaaca gaagcctgga tacaggactc agactcttac 1380

tggttggtat catacgtgat cgtttgattc agttcatctc taaattgcag tttgccgtga 1440

ctgtgctttt gacatcatgg acagagaaaa aacaacgtcg aaaaacaact gccactttat 1500

gtatactcaa cattgtcttt tctccattcg tgttggtcat catagttttt tctacactac 1560

tctcttctcc cttactccct cttttcaccc ttcctgtgtt cttggtgggg tttccccgac 1620

ctattcagag ttggccagga gcagcaggca ccacagcctg tgtgtgtgca gatacagtgt 1680

actactacca aatggtgccc aggttgactg ctgtactgca gactgcaatg gcagctggaa 1740

gtttaggtct cctcctacct ggatctcatt acttgggccg ttttcaggat cgtttaatgt 1800

ggataatgat tctggaatgt ggctatactt actgctctat taacattaag gggttagaat 1860

tgcaggaaac atcctgtcat actgcagaag ctcgcagagt tgatgaagtt tttgaagatg 1920

cttttgagca agaatacaca agagtatgtt cccttaatga acactttgga aatgtcttga 1980

caccctgtac tgttttgcct gtgaaattgt attctgatgc caggaatgtt ctatcaggca 2040

taattgattc tcatgaaaac ttaaaagaat ttaaaggtga cctcattaaa gtacttgtgt 2100

ggatacttgt tcaatactgc tccaaaaggc ctggcatgaa agagaatgtt cacaacactg 2160

aaaataaagg gaaagcacct ctaatgttgc ctgctttgaa cactttgcca cctcccaaat 2220

ccccagaaga catagacagt ttaaattcag aaacttttaa tgactggtct gatgataata 2280

tttttgatga tgagccaact atcaaaaaag taatagaaga aaaacatcag ttgaaagatt 2340

tgccaggtac aaatttgttt attccaggat cagtagaatc acagagggtt ggtgatcatt 2400

ctacaggcac tgttcctgaa aacgatcttt acaaagcagt tctattagga taccctgctg 2460

ttgacaaagg aaaacaagag gacatgccat atattcctct catggagttc agttgttcac 2520

attctcactt agtatgctta cccgcagagt ggaggactag ctgtatgccc agttccaaaa 2580

tgaaggagat gagctcgtta tttccagaag actggtacca atttgttcta aggcagttgg 2640

aatgttatca ttcagaagag aaggcctcaa atgtactgga agaaattgcc aaggacaaag 2700

ttttaaaaga cttttatgtt catacagtaa tgacttgtta ttttagttta tttggaatag 2760

acaatatggc tcctagtcct ggtcatatat tgagagttta cggtggtgtt ttgccttggt 2820

ctgttgcttt ggactggctc acagaaaagc cagaactgtt tcaactagca ctgaaagcat 2880

tcaggtatac tctgaaacta atgattgata aagcaagttt aggtccaata gaagacttta 2940

gagaactgat taagtacctt gaagaatatg aacgtgactg gtacattggt ttggtatctg 3000

atgaaaagtg gaaggaagca attttacaag aaaagccata cttgttttct ctggggtatg 3060

attctaatat gccaggacca gccttggaga taagcagagt gaacagaaat ttatggtctc 3120

agatttaaga aaaacaaaat tctttcttgc ttcttaaatc atactccatc ccattggctt 3180

gcaaacatgc tgacactcct cataatttca ctcttcataa accaaagcat aaggtcagag 3240

gagaacttga catattagaa cacttaggca ttgaaagtgg ttagtctaac taacccattg 3300

aagttttgga gaacctggga ctcaaatttt ggaagatgtg acagatgata tgttaacata 3360

cattgcaccg aggctgaagt gggaggattg cttgagactg cctggaaggc agaagttgca 3420

gtgagccgag actgatggtg tcactgcact ccagcctggg caacagagca agaccctgtc 3480

ttaaaaaaac aaaacaaaca aacaaaagaa acc 3513

<210> 37
<211> 406
<212> PRT
<213> Homo sapiens

<400> 37

Met Asp Cys Arg Thr Lys Ala Asn Pro Asp Arg Thr Phe Asp Leu Val
1               5                   10                  15

Leu Lys Val Lys Cys His Ala Ser Glu Asn Glu Asp Pro Val Val Leu
            20                  25                  30

Trp Lys Phe Pro Glu Asp Phe Gly Asp Gln Glu Ile Leu Gln Ser Val
        35                  40                  45

Pro Lys Phe Cys Phe Pro Phe Asp Val Glu Arg Val Ser Gln Asn Gln
    50                  55                  60

Val Gly Gln His Phe Thr Phe Val Leu Thr Asp Ile Glu Ser Lys Gln
65                  70                  75                  80

Arg Phe Gly Phe Cys Arg Leu Thr Ser Gly Gly Thr Ile Cys Leu Cys
                85                  90                  95

Ile Leu Ser Tyr Leu Pro Trp Phe Glu Val Tyr Tyr Lys Leu Leu Asn
            100                 105                 110

Thr Leu Ala Asp Tyr Leu Ala Lys Glu Leu Glu Asn Asp Leu Asn Glu
        115                 120                 125

Thr Leu Arg Ser Leu Tyr Asn His Pro Val Pro Lys Ala Asn Thr Pro
    130                 135                 140

Val Asn Leu Ser Val His Ser Tyr Phe Ile Ala Pro Asp Val Thr Gly
145                 150                 155                 160

Leu Pro Thr Ile Pro Glu Ser Arg Asn Leu Thr Glu Tyr Phe Val Ala
                165                 170                 175

Val Asp Val Asn Asn Met Leu Gln Leu Tyr Ala Ser Met Leu His Glu
            180                 185                 190

Arg Arg Ile Val Ile Ile Ser Ser Lys Leu Ser Thr Leu Thr Ala Cys
        195                 200                 205

Ile His Gly Ser Ala Ala Leu Leu Tyr Pro Met Tyr Trp Gln His Ile
    210                 215                 220

Tyr Ile Pro Val Leu Pro Pro His Leu Leu Asp Tyr Cys Cys Ala Pro
225                 230                 235                 240

Met Pro Tyr Leu Ile Gly Ile His Ser Ser Leu Ile Glu Arg Val Lys
                245                 250                 255

Asn Lys Ser Leu Glu Asp Val Val Met Leu Asn Val Asp Thr Asn Thr
            260                 265                 270

Leu Glu Ser Pro Phe Ser Asp Leu Asn Asn Leu Pro Ser Asp Val Val
        275                 280                 285

Ser Ala Leu Lys Asn Lys Leu Lys Lys Gln Ser Thr Ala Thr Gly Asp
    290                 295                 300

Gly Val Ala Arg Ala Phe Leu Arg Ala Gln Ala Ala Leu Phe Gly Ser
305                 310                 315                 320

Tyr Arg Asp Ala Leu Arg Tyr Lys Pro Gly Glu Pro Ile Thr Phe Cys
                325                 330                 335

Glu Glu Ser Phe Val Lys His Arg Ser Ser Val Met Lys Gln Phe Leu
            340                 345                 350

Glu Thr Ala Ile Asn Leu Gln Leu Phe Lys Gln Phe Ile Asp Gly Arg
        355                 360                 365

Leu Ala Lys Leu Asn Ala Gly Arg Gly Phe Ser Asp Val Phe Glu Glu
    370                 375                 380

Glu Ile Thr Ser Gly Gly Phe Cys Gly Gly Lys Asp Lys Leu Gln Tyr
385                 390                 395                 400

Asp Tyr Pro Phe Ser Gln
                405

<210>  38
<211>  426
<212>  PRT
<213>  Homo sapiens

<400>  38

Met Asp Cys Arg Thr Lys Ala Asn Pro Asp Arg Thr Phe Asp Leu Val
1               5                   10                  15

Leu Lys Val Lys Cys His Ala Ser Glu Asn Glu Asp Pro Val Val Leu
            20                  25                  30

Trp Lys Phe Pro Glu Asp Phe Gly Asp Gln Glu Ile Leu Gln Ser Val
        35                  40                  45

Pro Lys Phe Cys Phe Pro Phe Asp Val Glu Arg Val Ser Gln Asn Gln
    50                  55                  60

Val Gly Gln His Phe Thr Phe Val Leu Thr Asp Ile Glu Ser Lys Gln
65                  70                  75                  80

Arg Phe Gly Phe Cys Arg Leu Thr Ser Gly Gly Thr Ile Cys Leu Cys
                85                  90                  95

Ile Leu Ser Tyr Leu Pro Trp Phe Glu Val Tyr Tyr Lys Leu Leu Asn
            100                 105                 110

Thr Leu Ala Asp Tyr Leu Ala Lys Glu Leu Glu Asn Asp Leu Asn Glu
        115                 120                 125

Thr Leu Arg Ser Leu Tyr Asn His Pro Val Pro Lys Ala Asn Thr Pro
    130                 135                 140

Val Asn Leu Ser Val Asn Gln Glu Ile Phe Ile Ala Cys Glu Gln Val
145                 150                 155                 160

Leu Lys Asp Gln Pro Ala Leu Val Pro His Ser Tyr Phe Ile Ala Pro
                165                 170                 175

Asp Val Thr Gly Leu Pro Thr Ile Pro Glu Ser Arg Asn Leu Thr Glu
            180                 185                 190

Tyr Phe Val Ala Val Asp Val Asn Asn Met Leu Gln Leu Tyr Ala Ser
        195                 200                 205

Met Leu His Glu Arg Arg Ile Val Ile Ile Ser Ser Lys Leu Ser Thr
    210                 215                 220

Leu Thr Ala Cys Ile His Gly Ser Ala Ala Leu Leu Tyr Pro Met Tyr
225                 230                 235                 240

Trp Gln His Ile Tyr Ile Pro Val Leu Pro Pro His Leu Leu Asp Tyr
                245                 250                 255

Cys Cys Ala Pro Met Pro Tyr Leu Ile Gly Ile His Ser Ser Leu Ile
            260                 265                 270

Glu Arg Val Lys Asn Lys Ser Leu Glu Asp Val Val Met Leu Asn Val
        275                 280                 285

Asp Thr Asn Thr Leu Glu Ser Pro Phe Ser Asp Leu Asn Asn Leu Pro
    290                 295                 300

Ser Asp Val Val Ser Ala Leu Lys Asn Lys Leu Lys Lys Gln Ser Thr
305                 310                 315                 320

Ala Thr Gly Asp Gly Val Ala Arg Ala Phe Leu Arg Ala Gln Ala Ala
                325                 330                 335

Leu Phe Gly Ser Tyr Arg Asp Ala Leu Arg Tyr Lys Pro Gly Glu Pro
            340                 345                 350

Ile Thr Phe Cys Glu Glu Ser Phe Val Lys His Arg Ser Ser Val Met
        355                 360                 365

Lys Gln Phe Leu Glu Thr Ala Ile Asn Leu Gln Leu Phe Lys Gln Phe
    370                 375                 380

Ile Asp Gly Arg Leu Ala Lys Leu Asn Ala Gly Arg Gly Phe Ser Asp
385                 390                 395                 400

Val Phe Glu Glu Glu Ile Thr Ser Gly Gly Phe Cys Gly Gly Lys Asp
                405                 410                 415

Lys Leu Gln Tyr Asp Tyr Pro Phe Ser Gln
            420                 425

<210>  39
<211>  426

<212>  PRT
<213>  Homo sapiens

<400>  39

Ile Glu Thr Lys Thr Arg Ala Asn Pro Asp Arg Thr Phe Asp Leu Val
1               5                   10                  15

Leu Lys Val Lys Cys His Ala Ser Glu Asn Glu Asp Pro Val Val Leu
            20                  25                  30

Trp Lys Phe Pro Glu Asp Phe Gly Asp Gln Glu Ile Leu Gln Ser Val
        35                  40                  45

Pro Lys Phe Cys Phe Pro Phe Asp Val Glu Arg Val Ser Gln Asn Gln
    50                  55                  60

Val Gly Gln His Phe Thr Phe Val Leu Thr Asp Ile Glu Ser Lys Gln
65                  70                  75                  80

Arg Phe Gly Phe Cys Arg Leu Thr Ser Gly Gly Thr Ile Cys Leu Cys
                85                  90                  95

Ile Leu Ser Tyr Leu Pro Trp Phe Glu Val Tyr Tyr Lys Leu Leu Asn
            100                 105                 110

Thr Leu Ala Asp Tyr Leu Ala Lys Glu Leu Glu Asn Asp Leu Asn Glu
        115                 120                 125

Thr Leu Arg Ser Leu Tyr Asn His Pro Val Pro Lys Ala Asn Thr Pro
    130                 135                 140

Val Asn Leu Ser Val Asn Gln Glu Ile Phe Ile Ala Cys Glu Gln Val
145                 150                 155                 160

Leu Lys Asp Gln Pro Ala Leu Val Pro His Ser Tyr Phe Ile Ala Pro
                165                 170                 175

Asp Val Thr Gly Leu Pro Thr Ile Pro Glu Ser Arg Asn Leu Thr Glu
            180                 185                 190

Tyr Phe Val Ala Val Asp Val Asn Asn Met Leu Gln Leu Tyr Ala Ser
        195                 200                 205

Met Leu His Glu Arg Arg Ile Val Ile Ile Ser Ser Lys Leu Ser Thr
    210                 215                 220

Leu Thr Ala Cys Ile His Gly Ser Ala Ala Leu Leu Tyr Pro Met Tyr
225                 230                 235                 240

Trp Gln His Ile Tyr Ile Pro Val Leu Pro Pro His Leu Leu Asp Tyr
                245                 250                 255

Cys Cys Ala Pro Met Pro Tyr Leu Ile Gly Ile His Ser Ser Leu Ile
            260                 265                 270

Glu Arg Val Lys Asn Lys Ser Leu Glu Asp Val Val Met Leu Asn Val
        275                 280                 285

Asp Thr Asn Thr Leu Glu Ser Pro Phe Ser Asp Leu Asn Asn Leu Pro
    290                 295                 300

Ser Asp Val Val Ser Ala Leu Lys Asn Lys Leu Lys Lys Gln Ser Thr
305                 310                 315                 320

Ala Thr Gly Asp Gly Val Ala Arg Ala Phe Leu Arg Ala Gln Ala Ala
                325                 330                 335

Leu Phe Gly Ser Tyr Arg Asp Ala Leu Arg Tyr Lys Pro Gly Glu Pro
            340                 345                 350

Ile Thr Phe Cys Glu Glu Ser Phe Val Lys His Arg Ser Ser Val Met
        355                 360                 365

Lys Gln Phe Leu Glu Thr Ala Ile Asn Leu Gln Leu Phe Lys Gln Phe
    370                 375                 380

Ile Asp Gly Arg Leu Ala Lys Leu Asn Ala Gly Arg Gly Phe Ser Asp
385                 390                 395                 400

Val Phe Glu Glu Glu Ile Thr Ser Gly Gly Phe Cys Gly Gly Lys Asp
                405                 410                 415

Lys Leu Gln Tyr Asp Tyr Pro Phe Ser Gln
            420                 425

<210> 40
<211> 396
<212> PRT
<213> Homo sapiens

<400> 40

Met Ala Ala Ala Pro Arg Glu Glu Lys Arg Trp Pro Gln Pro Val Phe
1               5                   10                  15

Ser Asn Pro Val Val Leu Trp Lys Phe Pro Glu Asp Phe Gly Asp Gln
            20                  25                  30

Glu Ile Leu Gln Ser Val Pro Lys Phe Cys Phe Pro Phe Asp Val Glu
        35                  40                  45

Arg Val Ser Gln Asn Gln Val Gly Gln His Phe Thr Phe Val Leu Thr
    50                  55                  60

Asp Ile Glu Ser Lys Gln Arg Phe Gly Phe Cys Arg Leu Thr Ser Gly
65                  70                  75                  80

Gly Thr Ile Cys Leu Cys Ile Leu Ser Tyr Leu Pro Trp Phe Glu Val
                85                  90                  95

Tyr Tyr Lys Leu Leu Asn Thr Leu Ala Asp Tyr Leu Ala Lys Glu Leu
            100                 105                 110

Glu Asn Asp Leu Asn Glu Thr Leu Arg Ser Leu Tyr Asn His Pro Val
        115                 120                 125

Pro Lys Ala Asn Thr Pro Val Asn Leu Ser Val His Ser Tyr Phe Ile
    130                 135                 140

Ala Pro Asp Val Thr Gly Leu Pro Thr Ile Pro Glu Ser Arg Asn Leu
145                 150                 155                 160

Thr Glu Tyr Phe Val Ala Val Asp Val Asn Asn Met Leu Gln Leu Tyr
                165                 170                 175

Ala Ser Met Leu His Glu Arg Arg Ile Val Ile Ile Ser Ser Lys Leu
            180                 185                 190

Ser Thr Leu Thr Ala Cys Ile His Gly Ser Ala Ala Leu Leu Tyr Pro
        195                 200                 205

Met Tyr Trp Gln His Ile Tyr Ile Pro Val Leu Pro Pro His Leu Leu
    210                 215                 220

Asp Tyr Cys Cys Ala Pro Met Pro Tyr Leu Ile Gly Ile His Ser Ser
225                 230                 235                 240

Leu Ile Glu Arg Val Lys Asn Lys Ser Leu Glu Asp Val Val Met Leu
                245                 250                 255

Asn Val Asp Thr Asn Thr Leu Glu Ser Pro Phe Ser Asp Leu Asn Asn
            260                 265                 270

Leu Pro Ser Asp Val Val Ser Ala Leu Lys Asn Lys Leu Lys Lys Gln

275                 280                 285

Ser Thr Ala Thr Gly Asp Gly Val Ala Arg Ala Phe Leu Arg Ala Gln
    290                 295                 300

Ala Ala Leu Phe Gly Ser Tyr Arg Asp Ala Leu Arg Tyr Lys Pro Gly
305                 310                 315                 320

Glu Pro Ile Thr Phe Cys Glu Glu Ser Phe Val Lys His Arg Ser Ser
                325                 330                 335

Val Met Lys Gln Phe Leu Glu Thr Ala Ile Asn Leu Gln Leu Phe Lys
            340                 345                 350

Gln Phe Ile Asp Gly Arg Leu Ala Lys Leu Asn Ala Gly Arg Gly Phe
        355                 360                 365

Ser Asp Val Phe Glu Glu Glu Ile Thr Ser Gly Gly Phe Cys Gly Gly
    370                 375                 380

Lys Asp Lys Leu Gln Tyr Asp Tyr Pro Phe Ser Gln
385                 390                 395

<210>  41
<211>  2117
<212>  DNA
<213>  Homo sapiens

<400>  41
gccgggggcg cagccgacat gggcccgccg ccacggctgc tgtgagcagc ctctttccct      60

gtgtggccgc cggcgtgggc ggggacggcg cgaccctcgc gcggccgggc tgcgggcttc     120

caggccagcg cgcgggggcc ggacggacag ccccacaccg acatgtaacc atggactgca     180

ggaccaaggc aaatccagac agaacctttg acttggtgtt gaaagtgaaa tgtcatgcct     240

ctgaaaatga agatcctgtg gtattgtgga aattcccaga ggactttgga gaccaggaaa     300

tactacagag tgtgccaaag ttctgttttc cctttgacgt tgaaagggtg tctcagaatc     360

aagttggaca gcactttacc tttgtactga cagacattga aagtaaacag agatttggat     420

tctgcagact gacgtcagga ggcacaattt gtttatgcat ccttagttac cttccctggt     480

ttgaagtgta ttacaagctt ctaaatactc ttgcagatta cttggctaag gaactggaaa     540

atgatttgaa tgaaactctc agatcactgt ataaccaccc agtaccaaag gcaaatactc     600

ctgtaaattt gagtgtgcat tcctacttca ttgcccctga tgtaactgga ctcccaacaa     660

tacccgagag tagaaatctt acagaatatt ttgttgccgt ggatgtgaac aacatgctgc     720

agctgtatgc cagtatgctg catgaaaggc gcatcgtgat tatctcgagc aaattaagca     780

ctttaactgc ctgtatccat ggatcagctg ctcttctata cccaatgtat tggcaacaca 840

tatacatccc agtgcttcct ccacacctgc tggactactg ctgtgcccca atgccatacc 900

tgattggaat acactccagc ctcatagaga gagtgaaaaa caaatcattg gaagatgttg 960

ttatgttaaa tgttgataca aacacattag aatcaccatt tagtgacttg aacaacctac 1020

caagtgatgt ggtctcggcc ttgaaaaata aactgaagaa gcagtctaca gctacgggtg 1080

atggagtagc tagggccttt cttagagcac aggctgcttt gtttggatcc tacagagatg 1140

cactgagata caaacctggt gagcccatca ctttctgtga ggagagtttt gtaaagcacc 1200

gctcaagcgt gatgaaacag ttcctggaaa ctgccattaa cctccagctt tttaagcagt 1260

ttatcgatgg tcgactggca aaactaaatg caggaagggg tttctctgat gtatttgaag 1320

aagagatcac ttcaggtggc ttttgtggag gtaaagacaa gttacaatat gattatccat 1380

tttctcaata acaattttct tggtctttgc acttgtgtct gataaaacct atttcataaa 1440

caactaatga tttcctccta aatatgtaat gtcttaaata catttttcat cttataaaag 1500

ctatggaatt agcttatttt gcctgatacc tgttactcaa ggcattaagt tggcctcctg 1560

aattggcagc tgttggcctc gataatctct taatattgct ggaaattagt aatacagaaa 1620

tccaatcaac tcatatcttc ctgtctttcc ttctgaatag tagtattctc tgctagaaaa 1680

ctactagtga tggttattac tgagtatgaa tttaagaact gaggttatga ttggtaatac 1740

aatccaaaaa gaagggtctg aacaccaaaa ttctttatac atatttaagt aactgtatta 1800

ttattataca gatgtcttta cctttttgac tttatagatc actgcagcat taagaaagtt 1860

tccagtttac cattccataa gtacaattaa tccttctagt gtaaatgttc aaatactgtt 1920

ataattatct aggcaattaa taatttacaa actgatattt ttgcacgatt gtagtggtgt 1980

atagtcttga cttgcagagc attttgcttg agtccttgaa atgtcgtgtt cattcattat 2040

ttgctgagtg cttacaatgt attaggcact gttctaaata ttaagtgtac taaataaaca 2100

aaaatccttg tattctg 2117

<210> 42
<211> 2177
<212> DNA
<213> Homo sapiens

<400> 42
gccgggggcg cagccgacat gggcccgccg ccacggctgc tgtgagcagc ctctttccct 60

gtgtggccgc cggcgtgggc ggggacggcg cgaccctcgc gcggccgggc tgcgggcttc 120

caggccagcg cgcgggggcc ggacggacag ccccacaccg acatgtaacc atggactgca 180

ggaccaaggc aaatccagac agaacctttg acttggtgtt gaaagtgaaa tgtcatgcct 240

ctgaaaatga agatcctgtg gtattgtgga aattcccaga ggactttgga gaccaggaaa 300

tactacagag tgtgccaaag ttctgttttc cctttgacgt tgaaagggtg tctcagaatc 360

aagttggaca gcactttacc tttgtactga cagacattga aagtaaacag agatttggat 420

tctgcagact gacgtcagga ggcacaattt gtttatgcat ccttagttac cttccctggt 480

ttgaagtgta ttacaagctt ctaaatactc ttgcagatta cttggctaag gaactggaaa 540

atgatttgaa tgaaactctc agatcactgt ataaccaccc agtaccaaag gcaaatactc 600

ctgtaaattt gagtgtgaac caagagatat ttattgcctg tgagcaagtt ctgaaagatc 660

agcctgctct agtaccgcat tcctacttca ttgcccctga tgtaactgga ctcccaacaa 720

tacccgagag tagaaatctt acagaatatt ttgttgccgt ggatgtgaac aacatgctgc 780

agctgtatgc cagtatgctg catgaaaggc gcatcgtgat tatctcgagc aaattaagca 840

ctttaactgc ctgtatccat ggatcagctg ctcttctata cccaatgtat tggcaacaca 900

tatacatccc agtgcttcct ccacacctgc tggactactg ctgtgcccca atgccatacc 960

tgattggaat acactccagc ctcatagaga gagtgaaaaa caaatcattg gaagatgttg 1020

ttatgttaaa tgttgataca aacacattag aatcaccatt tagtgacttg aacaacctac 1080

caagtgatgt ggtctcggcc ttgaaaaata aactgaagaa gcagtctaca gctacgggtg 1140

atggagtagc tagggccttt cttagagcac aggctgcttt gtttggatcc tacagagatg 1200

cactgagata caaacctggt gagcccatca ctttctgtga ggagagtttt gtaaagcacc 1260

gctcaagcgt gatgaaacag ttcctggaaa ctgccattaa cctccagctt tttaagcagt 1320

ttatcgatgg tcgactggca aaactaaatg caggaagggg tttctctgat gtatttgaag 1380

aagagatcac ttcaggtggc ttttgtggag gtaaagacaa gttacaatat gattatccat 1440

tttctcaata acaattttct tggtctttgc acttgtgtct gataaaacct atttcataaa 1500

caactaatga tttcctccta aatatgtaat gtcttaaata catttttcat cttataaaag 1560

ctatggaatt agcttatttt gcctgatacc tgttactcaa ggcattaagt tggcctcctg 1620

aattggcagc tgttggcctc gataatctct taatattgct ggaaattagt aatacagaaa 1680

tccaatcaac tcatatcttc ctgtctttcc ttctgaatag tagtattctc tgctagaaaa 1740

ctactagtga tggttattac tgagtatgaa tttaagaact gaggttatga ttggtaatac 1800

aatccaaaaa gaagggtctg aacaccaaaa ttctttatac atatttaagt aactgtatta 1860

ttattataca gatgtcttta cctttttgac tttatagatc actgcagcat taagaaagtt 1920

tccagtttac cattccataa gtacaattaa tccttctagt gtaaatgttc aaatactgtt 1980

ataattatct aggcaattaa taatttacaa actgatattt ttgcacgatt gtagtggtgt 2040

atagtcttga cttgcagagc attttgcttg agtccttgaa atgtcgtgtt cattcattat 2100

ttgctgagtg cttacaatgt attaggcact gttctaaata ttaagtgtac taaataaaca 2160

aaaatccttg tattctg 2177

<210>  43
<211>  2007
<212>  DNA
<213>  Homo sapiens

<400>  43
attgagacaa aaacaagggc aaatccagac agaacctttg acttggtgtt gaaagtgaaa      60

tgtcatgcct ctgaaaatga agatcctgtg gtattgtgga aattcccaga ggactttgga     120

gaccaggaaa tactacagag tgtgccaaag ttctgttttc cctttgacgt tgaaagggtg     180

tctcagaatc aagttggaca gcactttacc tttgtactga cagacattga aagtaaacag     240

agatttggat tctgcagact gacgtcagga ggcacaattt gtttatgcat ccttagttac     300

cttccctggt ttgaagtgta ttacaagctt ctaaatactc ttgcagatta cttggctaag     360

gaactggaaa atgatttgaa tgaaactctc agatcactgt ataaccaccc agtaccaaag     420

gcaaatactc ctgtaaattt gagtgtgaac caagagatat ttattgcctg tgagcaagtt     480

ctgaaagatc agcctgctct agtaccgcat tcctacttca ttgcccctga tgtaactgga     540

ctcccaacaa tacccgagag tagaaatctt acagaatatt ttgttgccgt ggatgtgaac     600

aacatgctgc agctgtatgc cagtatgctg catgaaaggc gcatcgtgat tatctcgagc     660

aaattaagca ctttaactgc ctgtatccat ggatcagctg ctcttctata cccaatgtat     720

tggcaacaca tatacatccc agtgcttcct ccacacctgc tggactactg ctgtgcccca     780

atgccatacc tgattggaat acactccagc ctcatagaga gagtgaaaaa caaatcattg     840

gaagatgttg ttatgttaaa tgttgataca aacacattag aatcaccatt tagtgacttg     900

aacaacctac caagtgatgt ggtctcggcc ttgaaaaata aactgaagaa gcagtctaca     960

gctacgggtg atggagtagc tagggccttt cttagagcac aggctgcttt gtttggatcc    1020

tacagagatg cactgagata caaacctggt gagcccatca ctttctgtga ggagagtttt    1080

gtaaagcacc gctcaagcgt gatgaaacag ttcctggaaa ctgccattaa cctccagctt    1140

tttaagcagt ttatcgatgg tcgactggca aaactaaatg caggaagggg tttctctgat    1200

gtatttgaag aagagatcac ttcaggtggc ttttgtggag gtaaagacaa gttacaatat    1260

gattatccat tttctcaata acaattttct tggtctttgc acttgtgtct gataaaacct    1320

atttcataaa caactaatga tttcctccta aatatgtaat gtcttaaata catttttcat    1380

cttataaaag ctatggaatt agcttatttt gcctgatacc tgttactcaa ggcattaagt    1440

tggcctcctg aattggcagc tgttggcctc gataatctct taatattgct ggaaattagt    1500

aatacagaaa tccaatcaac tcatatcttc ctgtctttcc ttctgaatag tagtattctc    1560

tgctagaaaa ctactagtga tggttattac tgagtatgaa tttaagaact gaggttatga    1620

ttggtaatac aatccaaaaa gaagggtctg aacaccaaaa ttctttatac atatttaagt    1680

aactgtatta ttattataca gatgtcttta cctttttgac tttatagatc actgcagcat 1740

taagaaagtt tccagtttac cattccataa gtacaattaa tccttctagt gtaaatgttc 1800

aaatactgtt ataattatct aggcaattaa taatttacaa actgatattt ttgcacgatt 1860

gtagtggtgt atagtcttga cttgcagagc attttgcttg agtccttgaa atgtcgtgtt 1920

cattcattat ttgctgagtg cttacaatgt attaggcact gttctaaata ttaagtgtac 1980

taaataaaca aaaatccttg tattctg 2007

<210> 44
<211> 2197
<212> DNA
<213> Homo sapiens

<400> 44
gcgggggccg gacggacagc cccacaccga catgtaacca tggactgcag gaccaaggca 60

aatccagaca gaacctttga cttggtgttg aaagtgaaat gtcatgcctc tgaaaatgaa 120

gaggacagtc cagcttatct gccgaggatt ccccctggaa aagtacgccg attcgcattt 180

tgcattaaga aactggaaaa ctttcctgtc ggtcctggcg tagcgcctcc cgtgtccggg 240

gtagaccttg taccggctga aaccgcatag ctcgaccttc atggcggcag ctccacggga 300

ggagaaaaga tggccccaac ctgtattttc gaatcctgtg gtattgtgga aattcccaga 360

ggactttgga gaccaggaaa tactacagag tgtgccaaag ttctgttttc cctttgacgt 420

tgaaagggtg tctcagaatc aagttggaca gcactttacc tttgtactga cagacattga 480

aagtaaacag agatttggat tctgcagact gacgtcagga ggcacaattt gtttatgcat 540

ccttagttac cttccctggt ttgaagtgta ttacaagctt ctaaatactc ttgcagatta 600

cttggctaag gaactggaaa atgatttgaa tgaaactctc agatcactgt ataaccaccc 660

agtaccaaag gcaaatactc ctgtaaattt gagtgtgcat tcctacttca ttgcccctga 720

tgtaactgga ctcccaacaa tacccgagag tagaaatctt acagaatatt ttgttgccgt 780

ggatgtgaac aacatgctgc agctgtatgc cagtatgctg catgaaaggc gcatcgtgat 840

tatctcgagc aaattaagca ctttaactgc ctgtatccat ggatcagctg ctcttctata 900

cccaatgtat tggcaacaca tatacatccc agtgcttcct ccacacctgc tggactactg 960

ctgtgcccca atgccatacc tgattggaat acactccagc ctcatagaga gagtgaaaaa 1020

caaatcattg gaagatgttg ttatgttaaa tgttgataca aacacattag aatcaccatt 1080

tagtgacttg aacaacctac caagtgatgt ggtctcggcc ttgaaaaata aactgaagaa 1140

gcagtctaca gctacgggtg atggagtagc tagggccttt cttagagcac aggctgcttt 1200

gtttggatcc tacagagatg cactgagata caaacctggt gagcccatca ctttctgtga 1260

ggagagtttt gtaaagcacc gctcaagcgt gatgaaacag ttcctggaaa ctgccattaa 1320

cctccagctt tttaagcagt ttatcgatgg tcgactggca aaactaaatg caggaagggg 1380

tttctctgat gtatttgaag aagagatcac ttcaggtggc ttttgtggag gtaaagacaa 1440

gttacaatat gattatccat tttctcaata acaattttct tggtctttgc acttgtgtct 1500

gataaaacct atttcataaa caactaatga tttcctccta aatatgtaat gtcttaaata 1560

catttttcat cttataaaag ctatggaatt agcttatttt gcctgatacc tgttactcaa 1620

ggcattaagt tggcctcctg aattggcagc tgttggcctc gataatctct taatattgct 1680

ggaaattagt aatacagaaa tccaatcaac tcatatcttc ctgtctttcc ttctgaatag 1740

tagtattctc tgctagaaaa ctactagtga tggttattac tgagtatgaa tttaagaact 1800

gaggttatga ttggtaatac aatccaaaaa gaagggtctg aacaccaaaa ttctttatac 1860

atatttaagt aactgtatta ttattataca gatgtcttta cctttttgac tttatagatc 1920

actgcagcat taagaaagtt tccagtttac cattccataa gtacaattaa tccttctagt 1980

gtaaatgttc aaatactgtt ataattatct aggcaattaa taatttacaa actgatattt 2040

ttgcacgatt gtagtggtgt atagtcttga cttgcagagc attttgcttg agtccttgaa 2100

atgtcgtgtt cattcattat ttgctgagtg cttacaatgt attaggcact gttctaaata 2160

ttaagtgtac taaataaaca aaaatccttg tattctg 2197

<210> 45
<211> 423
<212> PRT
<213> Homo sapiens

<400> 45

Met Gly Gly Pro Arg Ala Trp Ala Leu Leu Cys Leu Gly Leu Leu Leu
1               5                   10                  15

Pro Gly Gly Gly Ala Ala Trp Ser Ile Gly Ala Ala Pro Phe Ser Gly
            20                  25                  30

Arg Arg Asn Trp Cys Ser Tyr Val Val Thr Arg Thr Ile Ser Cys His
        35                  40                  45

Val Gln Asn Gly Thr Tyr Leu Gln Arg Val Leu Gln Asn Cys Pro Trp
    50                  55                  60

Pro Met Ser Cys Pro Gly Ser Arg Thr Val Val Arg Pro Thr Tyr Lys
65                  70                  75                  80

Val Met Tyr Lys Ile Val Thr Ala Pro Ser Ser Ala Ser Leu Glu Pro
                85                  90                  95

Met Trp Ser Gly Ser Thr Met Arg Arg Met Ala Leu Arg Pro Thr Ala
                100                 105                 110

Phe Ser Gly Cys Leu Asn Cys Ser Lys Val Ser Glu Leu Thr Glu Arg
            115                 120                 125

Leu Lys Val Leu Glu Ala Lys Met Thr Met Leu Thr Val Ile Glu Gln
        130                 135                 140

Pro Val Pro Pro Thr Pro Ala Thr Pro Glu Asp Pro Ala Pro Leu Trp
145                 150                 155                 160

Gly Pro Pro Pro Ala Gln Gly Ser Pro Gly Asp Gly Gly Leu Gln Asp
                165                 170                 175

Gln Val Gly Ala Trp Gly Leu Pro Gly Pro Thr Gly Pro Lys Gly Asp
            180                 185                 190

Ala Gly Ser Arg Gly Pro Met Gly Met Arg Gly Pro Pro Gly Pro Gln
        195                 200                 205

Gly Pro Pro Gly Ser Pro Gly Arg Ala Gly Ala Val Gly Thr Pro Gly
    210                 215                 220

Glu Arg Gly Pro Pro Gly Pro Pro Gly Pro Pro Gly Pro Pro Gly Pro
225                 230                 235                 240

Pro Ala Pro Val Gly Pro Pro His Ala Arg Ile Ser Gln His Gly Asp
                245                 250                 255

Pro Leu Leu Ser Asn Thr Phe Thr Glu Thr Asn Asn His Trp Pro Gln
            260                 265                 270

Gly Pro Thr Gly Pro Pro Gly Pro Pro Gly Pro Met Gly Pro Pro Gly
        275                 280                 285

Pro Pro Gly Pro Thr Gly Val Pro Gly Ser Pro Gly His Ile Gly Pro
    290                 295                 300

Pro Gly Pro Thr Gly Pro Lys Gly Ile Ser Gly His Pro Gly Glu Lys
305                 310                 315                 320

Gly Glu Arg Gly Leu Arg Gly Glu Pro Gly Pro Gln Gly Ser Ala Gly
                325                 330                 335

Gln Arg Gly Glu Pro Gly Pro Lys Gly Asp Pro Gly Glu Lys Ser His
            340                 345                 350

Trp Gly Glu Gly Leu His Gln Leu Arg Glu Ala Leu Lys Ile Leu Ala
        355                 360                 365

Glu Arg Val Leu Ile Leu Glu Thr Met Ile Gly Leu Tyr Glu Pro Glu
    370                 375                 380

Leu Gly Ser Gly Ala Gly Pro Ala Gly Thr Gly Thr Pro Ser Leu Leu
385                 390                 395                 400

Arg Gly Lys Arg Gly Gly His Ala Thr Asn Tyr Arg Ile Val Ala Pro
                405                 410                 415

Arg Ser Arg Asp Glu Arg Gly
            420

<210>  46
<211>  212
<212>  PRT
<213>  Homo sapiens

<400>  46

Met Gly Gly Pro Arg Ala Trp Ala Leu Leu Cys Leu Gly Leu Leu Leu
1               5                   10                  15

Pro Gly Gly Gly Ala Ala Trp Ser Ile Gly Ala Ala Pro Phe Ser Gly
            20                  25                  30

Arg Arg Asn Trp Cys Ser Tyr Val Val Thr Arg Thr Ile Ser Cys His
        35                  40                  45

Val Gln Asn Gly Thr Tyr Leu Gln Arg Val Leu Gln Asn Cys Pro Trp
    50                  55                  60

Pro Met Ser Cys Pro Gly Ser Ser Tyr Arg Thr Val Val Arg Pro Thr
65                  70                  75                  80

Tyr Lys Val Met Tyr Lys Ile Val Thr Ala Arg Glu Trp Arg Cys Cys
                85                  90                  95

Pro Gly His Ser Gly Val Ser Cys Glu Glu Val Ala Ala Ser Ser Ala
            100                 105                 110

Ser Leu Glu Pro Met Trp Ser Gly Ser Thr Met Arg Arg Met Ala Leu
        115                 120                 125

Arg Pro Thr Ala Phe Ser Gly Cys Leu Asn Cys Ser Lys Val Ser Glu
    130                 135                 140

Leu Thr Glu Arg Leu Lys Val Leu Glu Ala Lys Met Thr Met Leu Thr
145                 150                 155                 160

Val Ile Glu Gln Pro Val Pro Pro Thr Pro Ala Thr Pro Glu Asp Pro
                165                 170                 175

Ala Pro Leu Trp Gly Pro Pro Pro Ala Gln Gly Ser Pro Gly Asp Gly
            180                 185                 190

Gly Leu Gln Gly Asp Pro Leu Leu Ser Asn Thr Phe Thr Glu Thr Asn
        195                 200                 205

Asn His Trp Pro
    210

<210>  47
<211>  175
<212>  PRT
<213>  Homo sapiens

<400>  47

Met Gly Gly Pro Arg Ala Trp Ala Leu Leu Cys Leu Gly Leu Leu Leu
1               5                   10                  15

Pro Gly Gly Gly Ala Ala Trp Ser Ile Gly Ala Ala Pro Phe Ser Gly
            20                  25                  30

Arg Arg Asn Trp Cys Ser Tyr Val Val Thr Arg Thr Ile Ser Cys His
        35                  40                  45

Val Gln Asn Gly Thr Tyr Leu Gln Arg Val Leu Gln Asn Cys Pro Trp
    50                  55                  60

Pro Met Ser Cys Pro Gly Ser Ser Tyr Arg Thr Val Val Arg Pro Thr
65                  70                  75                  80

Tyr Lys Val Met Tyr Lys Ile Val Thr Ala Arg Glu Trp Arg Cys Cys
                85                  90                  95

Pro Gly His Ser Gly Val Ser Cys Glu Glu Gly Cys Leu Asn Cys Ser
            100                 105                 110

Lys Val Ser Glu Leu Thr Glu Arg Leu Lys Val Leu Glu Ala Lys Met
        115                 120                 125

Thr Met Leu Thr Val Ile Glu Gln Pro Val Pro Pro Thr Pro Ala Thr
    130                 135                 140

Pro Glu Asp Pro Ala Pro Leu Trp Gly Pro Pro Pro Ala Gln Gly Ser
145                 150                 155                 160

Pro Gly Asp Gly Gly Leu Gln Asp Gln Val Gly Ala Trp Gly Leu
                165                 170                 175

<210>  48
<211>  34
<212>  PRT
<213>  Homo sapiens

<400>  48

Met Gly Gly Pro Arg Ala Trp Ala Leu Leu Cys Leu Gly Leu Leu Leu
1               5                   10                  15

Pro Gly Gly Gly Ala Ala Trp Ser Ile Gly Ala Ala Pro Phe Ser Gly
            20                  25                  30

Arg Arg

<210>  49
<211>  445
<212>  PRT
<213>  Homo sapiens

<400>  49

Met Gly Gly Pro Arg Ala Trp Ala Leu Leu Cys Leu Gly Leu Leu Leu
1               5                   10                  15

Pro Gly Gly Gly Ala Ala Trp Ser Ile Gly Ala Ala Pro Phe Ser Gly
            20                  25                  30

Arg Arg Asn Trp Cys Ser Tyr Val Val Thr Arg Thr Ile Ser Cys His
        35                  40                  45

Val Gln Asn Gly Thr Tyr Leu Gln Arg Val Leu Gln Asn Cys Pro Trp
    50                  55                  60

Pro Met Ser Cys Pro Gly Ser Ser Tyr Arg Thr Val Val Arg Pro Thr
65                  70                  75                  80

Tyr Lys Val Met Tyr Lys Ile Val Thr Ala Arg Glu Trp Arg Cys Cys
                85                  90                  95

Pro Gly His Ser Gly Val Ser Cys Glu Glu Val Ala Ala Ser Ser Ala
            100                 105                 110

Ser Leu Glu Pro Met Trp Ser Gly Ser Thr Met Arg Arg Met Ala Leu
        115                 120                 125

Arg Pro Thr Ala Phe Ser Gly Cys Leu Asn Cys Ser Lys Val Ser Glu
    130                 135                 140

Leu Thr Glu Arg Leu Lys Val Leu Glu Ala Lys Met Thr Met Leu Thr
145                 150                 155                 160

Val Ile Glu Gln Pro Val Pro Pro Thr Pro Ala Thr Pro Glu Asp Pro
                165                 170                 175

Ala Pro Leu Trp Gly Pro Pro Pro Ala Gln Gly Ser Pro Gly Asp Gly
            180                 185                 190

Gly Leu Gln Asp Gln Val Gly Ala Trp Gly Leu Pro Gly Pro Thr Gly
        195                 200                 205

Pro Lys Gly Asp Ala Gly Ser Arg Gly Pro Met Gly Met Arg Gly Pro
    210                 215                 220

Pro Gly Pro Gln Gly Pro Pro Gly Ser Pro Gly Arg Ala Gly Ala Val
225                 230                 235                 240

Gly Thr Pro Gly Glu Arg Gly Pro Pro Gly Pro Pro Gly Pro Pro Gly
                245                 250                 255

Pro Pro Gly Pro Pro Ala Pro Val Gly Pro Pro His Ala Arg Ile Ser
            260                 265                 270

Gln His Gly Asp Pro Leu Leu Ser Asn Thr Phe Thr Glu Thr Asn Asn
        275                 280                 285

His Trp Pro Gln Gly Pro Thr Gly Pro Pro Gly Pro Pro Gly Pro Met
    290                 295                 300

Gly Pro Pro Gly Pro Pro Gly Pro Thr Gly Val Pro Gly Ser Pro Gly
305                 310                 315                 320

His Ile Gly Pro Pro Gly Pro Thr Gly Pro Lys Gly Ile Ser Gly His
                325                 330                 335

Pro Gly Glu Lys Gly Glu Arg Gly Leu Arg Gly Glu Pro Gly Pro Gln
            340                 345                 350

Gly Ser Ala Gly Gln Arg Gly Glu Pro Gly Pro Lys Gly Asp Pro Gly
        355                 360                 365

Glu Lys Ser His Trp Ala Pro Ser Leu Gln Ser Phe Leu Gln Gln Gln
    370                 375                 380

Ala Gln Leu Glu Leu Leu Ala Arg Arg Val Thr Leu Leu Glu Ala Ile
385                 390                 395                 400

Ile Trp Pro Glu Pro Glu Leu Gly Ser Gly Ala Gly Pro Ala Gly Thr
                405                 410                 415

Gly Thr Pro Ser Leu Leu Arg Gly Lys Arg Gly Gly His Ala Thr Asn
            420                 425                 430

Tyr Arg Ile Val Ala Pro Arg Ser Arg Asp Glu Arg Gly
        435                 440                 445

<210>  50
<211>  443
<212>  PRT
<213>  Homo sapiens

<400>  50

Met Gly Gly Pro Arg Ala Trp Ala Leu Leu Cys Leu Gly Leu Leu Leu
1               5                   10                  15

Pro Gly Gly Gly Ala Ala Trp Ser Ile Gly Ala Ala Pro Phe Ser Gly
            20                  25                  30

Arg Arg Asn Trp Cys Ser Tyr Val Val Thr Arg Thr Ile Ser Cys His
        35                  40                  45

Val Gln Asn Gly Thr Tyr Leu Gln Arg Val Leu Gln Asn Cys Pro Trp
    50                  55                  60

Pro Met Ser Cys Pro Gly Ser Ser Tyr Arg Thr Val Val Arg Pro Thr
65                  70                  75                  80

Tyr Lys Val Met Tyr Lys Ile Val Thr Ala Arg Glu Trp Arg Cys Cys
                85                  90                  95

Pro Gly His Ser Gly Val Ser Cys Glu Glu Val Ala Ala Ser Ser Ala
            100                 105                 110

Ser Leu Glu Pro Met Trp Ser Gly Ser Thr Met Arg Arg Met Ala Leu
        115                 120                 125

Arg Pro Thr Ala Phe Ser Gly Cys Leu Asn Cys Ser Lys Val Ser Glu
    130                 135                 140

Leu Thr Glu Arg Leu Lys Val Leu Glu Ala Lys Met Thr Met Leu Thr
145                 150                 155                 160

Val Ile Glu Gln Pro Val Pro Pro Thr Pro Ala Thr Pro Glu Asp Pro
                165                 170                 175

Ala Pro Leu Trp Gly Pro Pro Pro Ala Gln Gly Ser Pro Gly Asp Gly
            180                 185                 190

Gly Leu Gln Asp Gln Val Gly Ala Trp Gly Leu Pro Gly Pro Thr Gly
        195                 200                 205

Pro Lys Gly Asp Ala Gly Ser Arg Gly Pro Met Gly Met Arg Gly Pro
    210                 215                 220

Pro Gly Pro Gln Gly Pro Pro Gly Ser Pro Gly Arg Ala Gly Ala Val
225                 230                 235                 240

Gly Thr Pro Gly Glu Arg Gly Pro Pro Gly Pro Pro Gly Pro Pro Gly
                245                 250                 255

Pro Pro Gly Pro Pro Ala Pro Val Gly Pro Pro His Ala Arg Ile Ser
            260                 265                 270

Gln His Gly Asp Pro Leu Leu Ser Asn Thr Phe Thr Glu Thr Asn Asn
        275                 280                 285

His Trp Pro Gln Gly Pro Thr Gly Pro Pro Gly Pro Pro Gly Pro Met
    290                 295                 300

Gly Pro Pro Gly Pro Pro Gly Pro Thr Gly Val Pro Gly Ser Pro Gly
305                 310                 315                 320

His Ile Gly Pro Pro Gly Pro Thr Gly Pro Lys Gly Ile Ser Gly His
                325                 330                 335

Pro Gly Glu Lys Gly Glu Arg Gly Leu Arg Gly Glu Pro Gly Pro Gln
            340                 345                 350

Gly Ser Ala Gly Gln Arg Gly Glu Pro Gly Pro Lys Gly Asp Pro Gly
        355                 360                 365

Glu Lys Ser His Trp Gly Glu Gly Leu His Gln Leu Arg Glu Ala Leu
    370                 375                 380

Lys Ile Leu Ala Glu Arg Val Leu Ile Leu Glu Thr Met Ile Gly Leu
385                 390                 395                 400

Tyr Glu Pro Glu Leu Gly Ser Gly Ala Gly Pro Ala Gly Thr Gly Thr
                405                 410                 415

Pro Ser Leu Leu Arg Gly Lys Arg Gly Gly His Ala Thr Asn Tyr Arg
            420                 425                 430

Ile Val Ala Pro Arg Ser Arg Asp Glu Arg Gly
        435                 440

<210>  51
<211>  441
<212>  PRT
<213>  Homo sapiens

<400>  51

Met Gly Gly Pro Arg Ala Trp Ala Leu Leu Cys Leu Gly Leu Leu Leu
1               5                   10                  15

Pro Gly Gly Gly Ala Ala Trp Ser Ile Gly Ala Ala Pro Phe Ser Gly
            20                  25                  30

Arg Arg Asn Trp Cys Ser Tyr Val Val Thr Arg Thr Ile Ser Cys His
        35                  40                  45

Val Gln Asn Gly Thr Tyr Leu Gln Arg Val Leu Gln Asn Cys Pro Trp
    50                  55                  60

Pro Met Ser Cys Pro Gly Ser Ser Tyr Arg Thr Val Val Arg Pro Thr
65                  70                  75                  80

Tyr Lys Val Met Tyr Lys Ile Val Thr Ala Arg Glu Trp Arg Cys Cys
                85                  90                  95

Pro Gly His Ser Gly Val Ser Cys Glu Glu Ala Ser Ser Ala Ser Leu
            100                 105                 110

Glu Pro Met Trp Ser Gly Ser Thr Met Arg Arg Met Ala Leu Arg Pro
        115                 120                 125

Thr Ala Phe Ser Gly Cys Leu Asn Cys Ser Lys Val Ser Glu Leu Thr
    130                 135                 140

Glu Arg Leu Lys Val Leu Glu Ala Lys Met Thr Met Leu Thr Val Ile
145                 150                 155                 160

Glu Gln Pro Val Pro Pro Thr Pro Ala Thr Pro Glu Asp Pro Ala Pro
                165                 170                 175

Leu Trp Gly Pro Pro Pro Ala Gln Gly Ser Pro Gly Asp Gly Gly Leu
            180                 185                 190

Gln Asp Gln Val Gly Ala Trp Gly Leu Pro Gly Pro Thr Gly Pro Lys
        195                 200                 205

Gly Asp Ala Gly Ser Arg Gly Pro Met Gly Met Arg Gly Pro Pro Gly
    210                 215                 220

Pro Gln Gly Pro Pro Gly Ser Pro Gly Arg Ala Gly Ala Val Gly Thr
225                 230                 235                 240

Pro Gly Glu Arg Gly Pro Pro Gly Pro Pro Gly Pro Pro Gly Pro Pro
                245                 250                 255

Gly Pro Pro Ala Pro Val Gly Pro Pro His Ala Arg Ile Ser Gln His
            260                 265                 270

Gly Asp Pro Leu Leu Ser Asn Thr Phe Thr Glu Thr Asn Asn His Trp
        275                 280                 285

Pro Gln Gly Pro Thr Gly Pro Pro Gly Pro Pro Gly Pro Met Gly Pro
    290                 295                 300

Pro Gly Pro Pro Gly Pro Thr Gly Val Pro Gly Ser Pro Gly His Ile
305                 310                 315                 320

Gly Pro Pro Gly Pro Thr Gly Pro Lys Gly Ile Ser Gly His Pro Gly
                325                 330                 335

Glu Lys Gly Glu Arg Gly Leu Arg Gly Glu Pro Gly Pro Gln Gly Ser
            340                 345                 350

Ala Gly Gln Arg Gly Glu Pro Gly Pro Lys Gly Asp Pro Gly Glu Lys
        355                 360                 365

Ser His Trp Gly Glu Gly Leu His Gln Leu Arg Glu Ala Leu Lys Ile
    370                 375                 380

Leu Ala Glu Arg Val Leu Ile Leu Glu Thr Met Ile Gly Leu Tyr Glu
385                 390                 395                 400

Pro Glu Leu Gly Ser Gly Ala Gly Pro Ala Gly Thr Gly Thr Pro Ser
                405                 410                 415

Leu Leu Arg Gly Lys Arg Gly Gly His Ala Thr Asn Tyr Arg Ile Val

            420                 425                 430

Ala Pro Arg Ser Arg Asp Glu Arg Gly
        435                 440

<210> 52
<211> 439
<212> PRT
<213> Homo sapiens

<400> 52

Met Gly Gly Pro Arg Ala Trp Ala Leu Leu Cys Leu Gly Leu Leu Leu
1               5                   10                  15

Pro Gly Gly Gly Ala Ala Trp Ser Ile Gly Ala Ala Pro Phe Ser Gly
            20                  25                  30

Arg Arg Asn Trp Cys Ser Tyr Val Val Thr Arg Thr Ile Ser Cys His
        35                  40                  45

Val Gln Asn Gly Thr Tyr Leu Gln Arg Val Leu Gln Asn Cys Pro Trp
    50                  55                  60

Pro Met Ser Cys Pro Gly Ser Arg Thr Val Val Arg Pro Thr Tyr Lys
65                  70                  75                  80

Val Met Tyr Lys Ile Val Thr Ala Arg Glu Trp Arg Cys Cys Pro Gly
                85                  90                  95

His Ser Gly Val Ser Cys Glu Glu Ala Ser Ser Ala Ser Leu Glu Pro
            100                 105                 110

Met Trp Ser Gly Ser Thr Met Arg Arg Met Ala Leu Arg Pro Thr Ala
        115                 120                 125

Phe Ser Gly Cys Leu Asn Cys Ser Lys Val Ser Glu Leu Thr Glu Arg
    130                 135                 140

Leu Lys Val Leu Glu Ala Lys Met Thr Met Leu Thr Val Ile Glu Gln
145                 150                 155                 160

Pro Val Pro Pro Thr Pro Ala Thr Pro Glu Asp Pro Ala Pro Leu Trp
                165                 170                 175

Gly Pro Pro Pro Ala Gln Gly Ser Pro Gly Asp Gly Gly Leu Gln Asp
            180                 185                 190

Gln Val Gly Ala Trp Gly Leu Pro Gly Pro Thr Gly Pro Lys Gly Asp

195                 200                 205

Ala Gly Ser Arg Gly Pro Met Gly Met Arg Gly Pro Pro Gly Pro Gln
    210                 215                 220

Gly Pro Pro Gly Ser Pro Gly Arg Ala Gly Ala Val Gly Thr Pro Gly
225                 230                 235                 240

Glu Arg Gly Pro Pro Gly Pro Pro Gly Pro Pro Gly Pro Pro Gly Pro
                245                 250                 255

Pro Ala Pro Val Gly Pro Pro His Ala Arg Ile Ser Gln His Gly Asp
            260                 265                 270

Pro Leu Leu Ser Asn Thr Phe Thr Glu Thr Asn Asn His Trp Pro Gln
        275                 280                 285

Gly Pro Thr Gly Pro Pro Gly Pro Pro Gly Pro Met Gly Pro Pro Gly
    290                 295                 300

Pro Pro Gly Pro Thr Gly Val Pro Gly Ser Pro Gly His Ile Gly Pro
305                 310                 315                 320

Pro Gly Pro Thr Gly Pro Lys Gly Ile Ser Gly His Pro Gly Glu Lys
                325                 330                 335

Gly Glu Arg Gly Leu Arg Gly Glu Pro Gly Pro Gln Gly Ser Ala Gly
            340                 345                 350

Gln Arg Gly Glu Pro Gly Pro Lys Gly Asp Pro Gly Glu Lys Ser His
        355                 360                 365

Trp Gly Glu Gly Leu His Gln Leu Arg Glu Ala Leu Lys Ile Leu Ala
    370                 375                 380

Glu Arg Val Leu Ile Leu Glu Thr Met Ile Gly Leu Tyr Glu Pro Glu
385                 390                 395                 400

Leu Gly Ser Gly Ala Gly Pro Ala Gly Thr Gly Thr Pro Ser Leu Leu
                405                 410                 415

Arg Gly Lys Arg Gly Gly His Ala Thr Asn Tyr Arg Ile Val Ala Pro
            420                 425                 430

Arg Ser Arg Asp Glu Arg Gly
        435

<210>  53
<211>  422
<212>  PRT
<213>  Homo sapiens

<400>  53

Met Gly Gly Pro Arg Ala Trp Ala Leu Leu Cys Leu Gly Leu Leu Leu
1               5                   10                  15

Pro Gly Gly Gly Ala Ala Trp Ser Ile Gly Ala Ala Pro Phe Ser Gly
            20                  25                  30

Arg Arg Asn Trp Cys Ser Tyr Val Val Thr Arg Thr Ile Ser Cys His
        35                  40                  45

Val Gln Asn Gly Thr Tyr Leu Gln Arg Val Leu Gln Asn Cys Pro Trp
    50                  55                  60

Pro Met Ser Cys Pro Gly Ser Ser Tyr Arg Thr Val Val Arg Pro Thr
65                  70                  75                  80

Tyr Lys Val Met Tyr Lys Ile Val Thr Ala Arg Glu Trp Arg Cys Cys
                85                  90                  95

Pro Gly His Ser Gly Val Ser Cys Glu Glu Val Ala Ala Ser Ser Ala
            100                 105                 110

Ser Leu Glu Pro Met Trp Ser Gly Ser Thr Met Arg Arg Met Ala Leu
        115                 120                 125

Arg Pro Thr Ala Phe Ser Gly Cys Leu Asn Cys Ser Lys Val Ser Glu
    130                 135                 140

Leu Thr Glu Arg Leu Lys Val Leu Glu Ala Lys Met Thr Met Leu Thr
145                 150                 155                 160

Val Ile Glu Gln Pro Val Pro Pro Thr Pro Ala Thr Pro Glu Asp Pro
                165                 170                 175

Ala Pro Leu Trp Gly Pro Pro Pro Ala Gln Gly Ser Pro Gly Asp Gly
            180                 185                 190

Gly Leu Gln Asp Gln Val Gly Ala Trp Gly Leu Pro Gly Pro Thr Gly
        195                 200                 205

Pro Lys Gly Asp Ala Gly Ser Arg Gly Pro Met Gly Met Arg Gly Pro
    210                 215                 220

Pro Gly Pro Gln Gly Pro Pro Gly Ser Pro Gly Arg Ala Gly Ala Val
225                 230                 235                 240

Gly Thr Pro Gly Glu Arg Gly Pro Pro Gly Pro Pro Gly Pro Pro Gly
                245                 250                 255

Pro Pro Gly Pro Pro Ala Pro Val Gly Pro Pro His Ala Arg Ile Ser
            260                 265                 270

Gln His Gly Asp Pro Leu Leu Ser Asn Thr Phe Thr Glu Thr Asn Asn
        275                 280                 285

His Trp Pro Gln Gly Pro Thr Gly Pro Pro Gly Pro Pro Gly Pro Met
    290                 295                 300

Gly Pro Pro Gly Pro Pro Gly Pro Thr Gly Val Pro Gly Ser Pro Gly
305                 310                 315                 320

His Ile Gly Leu Arg Gly Glu Pro Gly Pro Gln Gly Ser Ala Gly Gln
                325                 330                 335

Arg Gly Glu Pro Gly Pro Lys Gly Asp Pro Gly Glu Lys Ser His Trp
            340                 345                 350

Gly Glu Gly Leu His Gln Leu Arg Glu Ala Leu Lys Ile Leu Ala Glu
        355                 360                 365

Arg Val Leu Ile Leu Glu Thr Met Ile Gly Leu Tyr Glu Pro Glu Leu
    370                 375                 380

Gly Ser Gly Ala Gly Pro Ala Gly Thr Gly Thr Pro Ser Leu Leu Arg
385                 390                 395                 400

Gly Lys Arg Gly Gly His Ala Thr Asn Tyr Arg Ile Val Ala Pro Arg
                405                 410                 415

Ser Arg Asp Glu Arg Gly
            420

<210>  54
<211>  212
<212>  PRT
<213>  Homo sapiens

<400>  54

Met Gly Gly Pro Arg Ala Trp Ala Leu Leu Cys Leu Gly Leu Leu Leu
1               5                   10                  15

Pro Gly Gly Gly Ala Ala Trp Ser Ile Gly Ala Ala Pro Phe Ser Gly
            20                  25                  30

Arg Arg Asn Trp Cys Ser Tyr Val Val Thr Arg Thr Ile Ser Cys His
        35                  40                  45

Val Gln Asn Gly Thr Tyr Leu Gln Arg Val Leu Gln Asn Cys Pro Trp
    50                  55                  60

Pro Met Ser Cys Pro Gly Ser Ser Tyr Arg Thr Val Val Arg Pro Thr
65                  70                  75                  80

Tyr Lys Val Met Tyr Lys Ile Val Thr Ala Arg Glu Trp Arg Cys Cys
                85                  90                  95

Pro Gly His Ser Gly Val Ser Cys Glu Glu Val Ala Ala Ser Ser Ala
            100                 105                 110

Ser Leu Glu Pro Met Trp Ser Gly Ser Thr Met Arg Arg Met Ala Leu
        115                 120                 125

Arg Pro Thr Ala Phe Ser Gly Cys Leu Asn Cys Ser Lys Val Ser Glu
    130                 135                 140

Leu Thr Glu Arg Leu Lys Val Leu Glu Ala Lys Met Thr Met Leu Thr
145                 150                 155                 160

Val Ile Glu Gln Pro Val Pro Pro Thr Pro Ala Thr Pro Glu Asp Pro
                165                 170                 175

Ala Pro Leu Trp Gly Pro Pro Pro Ala Gln Gly Ser Pro Gly Asp Gly
            180                 185                 190

Gly Leu Gln Gly Asp Pro Leu Leu Ser Asn Thr Phe Thr Glu Thr Asn
        195                 200                 205

Asn His Trp Pro
    210

<210>  55
<211>  175
<212>  PRT
<213>  Homo sapiens

<400>  55

Met Gly Gly Pro Arg Ala Trp Ala Leu Leu Cys Leu Gly Leu Leu Leu
1               5                   10                  15

Pro Gly Gly Gly Ala Ala Trp Ser Ile Gly Ala Ala Pro Phe Ser Gly
            20                  25                  30

Arg Arg Asn Trp Cys Ser Tyr Val Val Thr Arg Thr Ile Ser Cys His
        35                  40                  45

Val Gln Asn Gly Thr Tyr Leu Gln Arg Val Leu Gln Asn Cys Pro Trp
    50                  55                  60

Pro Met Ser Cys Pro Gly Ser Ser Tyr Arg Thr Val Val Arg Pro Thr
65                  70                  75                  80

Tyr Lys Val Met Tyr Lys Ile Val Thr Ala Arg Glu Trp Arg Cys Cys
                85                  90                  95

Pro Gly His Ser Gly Val Ser Cys Glu Glu Gly Cys Leu Asn Cys Ser
            100                 105                 110

Lys Val Ser Glu Leu Thr Glu Arg Leu Lys Val Leu Glu Ala Lys Met
        115                 120                 125

Thr Met Leu Thr Val Ile Glu Gln Pro Val Pro Pro Thr Pro Ala Thr
    130                 135                 140

Pro Glu Asp Pro Ala Pro Leu Trp Gly Pro Pro Pro Ala Gln Gly Ser
145                 150                 155                 160

Pro Gly Asp Gly Gly Leu Gln Asp Gln Val Gly Ala Trp Gly Leu
                165                 170                 175

<210>  56
<211>  236
<212>  PRT
<213>  Homo sapiens

<220>
<221>  misc_feature
<222>  (1)..(1)
<223>  Xaa can be any naturally occurring amino acid

<400>  56

Xaa Met Thr Met Leu Thr Val Ile Glu Gln Pro Val Pro Pro Thr Pro
1               5                   10                  15

Ala Thr Pro Glu Asp Pro Ala Pro Leu Trp Gly Pro Pro Pro Ala Gln
            20                  25                  30

Gly Ser Pro Gly Asp Gly Gly Leu Gln Gly Leu Pro Gly Ala Ile Glu
        35                  40                  45

Ser Val Arg Val Pro Leu Leu Pro Arg Asn Asp Gln Val Gly Ala Trp
    50                  55                  60

Gly Leu Pro Gly Pro Thr Gly Pro Lys Gly Asp Ala Gly Ser Arg Gly
65                  70                  75                  80

Pro Met Gly Met Arg Gly Pro Pro Gly Pro Gln Gly Pro Pro Gly Ser
                85                  90                  95

Pro Gly Arg Ala Gly Ala Val Gly Thr Pro Gly Glu Arg Gly Pro Pro
            100                 105                 110

Gly Pro Pro Gly Pro Pro Gly Pro Pro Gly Pro Pro Ala Pro Val Gly
        115                 120                 125

Pro Pro His Ala Arg Ile Ser Gln His Gly Asp Pro Leu Leu Ser Asn
    130                 135                 140

Thr Phe Thr Glu Thr Asn Asn His Trp Pro Gln Gly Pro Thr Gly Pro
145                 150                 155                 160

Pro Gly Pro Pro Gly Pro Met Gly Pro Pro Gly Pro Pro Gly Pro Thr
                165                 170                 175

Gly Val Pro Gly Ser Pro Gly His Ile Gly Pro Pro Gly Pro Thr Gly
            180                 185                 190

Pro Lys Gly Ile Ser Gly His Pro Gly Glu Lys Gly Glu Arg Gly Leu
        195                 200                 205

Arg Gly Glu Pro Gly Pro Gln Gly Ser Ala Gly Gln Arg Gly Glu Pro
    210                 215                 220

Gly Pro Lys Gly Asp Pro Gly Glu Lys Ser His Trp
225                 230                 235

<210>  57
<211>  305
<212>  PRT
<213>  Homo sapiens

<400>  57

Met Thr Met Leu Thr Val Ile Glu Gln Pro Val Pro Pro Thr Pro Ala
1               5                   10                  15

Thr Pro Glu Asp Pro Ala Pro Leu Trp Gly Pro Pro Pro Ala Gln Gly
            20                  25                  30

Ser Pro Gly Asp Gly Gly Leu Gln Gly Leu Pro Gly Ala Ile Glu Ser
        35                  40                  45

Val Arg Val Pro Leu Leu Pro Arg Asn Asp Gln Val Gly Ala Trp Gly
    50                  55                  60

Leu Pro Gly Pro Thr Gly Pro Lys Gly Asp Ala Gly Ser Arg Gly Pro
65                  70                  75                  80

Met Gly Met Arg Gly Pro Pro Gly Pro Gln Gly Pro Pro Gly Ser Pro
                85                  90                  95

Gly Arg Ala Gly Ala Val Gly Thr Pro Gly Glu Arg Gly Pro Pro Gly
            100                 105                 110

Pro Pro Gly Pro Pro Gly Pro Pro Gly Pro Pro Ala Pro Val Gly Pro
        115                 120                 125

Pro His Ala Arg Ile Ser Gln His Gly Asp Pro Leu Leu Ser Asn Thr
    130                 135                 140

Phe Thr Glu Thr Asn Asn His Trp Pro Gln Gly Pro Thr Gly Pro Pro
145                 150                 155                 160

Gly Pro Pro Gly Pro Met Gly Pro Pro Gly Pro Pro Gly Pro Thr Gly
                165                 170                 175

Val Pro Gly Ser Pro Gly His Ile Gly Pro Pro Gly Pro Thr Gly Pro
            180                 185                 190

Lys Gly Ile Ser Gly His Pro Gly Glu Lys Gly Glu Arg Gly Leu Arg
        195                 200                 205

Gly Glu Pro Gly Pro Gln Gly Ser Ala Gly Gln Arg Gly Glu Pro Gly
    210                 215                 220

Pro Lys Gly Asp Pro Gly Glu Lys Ser His Trp Gly Glu Gly Leu His
225                 230                 235                 240

Gln Leu Arg Glu Ala Leu Lys Ile Leu Ala Glu Arg Val Leu Ile Leu
                245                 250                 255

Glu Thr Met Ile Gly Leu Tyr Glu Pro Glu Leu Gly Ser Gly Ala Gly
            260                 265                 270

Pro Ala Gly Thr Gly Thr Pro Ser Leu Leu Arg Gly Lys Arg Gly Gly

275                 280                 285

His Ala Thr Asn Tyr Arg Ile Val Ala Pro Arg Ser Arg Asp Glu Arg
    290                 295                 300

Gly
305

<210>  58
<211>  226
<212>  PRT
<213>  Homo sapiens

<400>  58

<210>  59
<211>  2031
<212>  DNA
<213>  Homo sapiens

<400>  59
gggctccgcg cgtccggggc ggctggcggc gcgggcaggc aggcggggag gacaggctgg      60

gggcggcgac cgcgaggggc cgcgcgcgga gggcgcctgg tgcagcatgg gcggcccgcg     120

ggcttgggcg ctgctctgcc tcgggctcct gctcccggga ggcggcgctg cgtggagcat     180

cggggcagct ccgttctccg gacgcaggaa ctggtgctcc tatgtggtga cccgcaccat     240

ctcatgccat gtgcagaatg gcacctacct tcagcgagtg ctgcagaact gcccctggcc     300

catgagctgt ccggggagca gaactgtggt gagacccaca tacaaggtga tgtacaagat     360

agtgaccgcc ccttcctctg cctccttgga gcccatgtgg tcgggcagta ccatgcggcg     420

gatggcgctt cggcccacag ccttctcagg ttgtctcaac tgcagcaaag tgtcagagct     480

gacagagcgg ctgaaggtgc tggaggccaa gatgaccatg ctgactgtca tagagcagcc     540

agtacctcca acaccagcta cccctgagga ccctgccccg ctctggggtc cccctcctgc     600

ccagggcagc cccggagatg gaggcctcca ggaccaagtc ggtgcttggg ggcttcccgg     660

gcccaccggc cccaagggag atgccggcag tcggggccca atggggatga gaggcccacc     720

aggtccacag ggccccccag ggagccctgg ccgggctgga gctgtgggca cccctggaga     780

gaggggacct cctgggccac cagggcctcc tggcccccct gggcccccag cccctgttgg     840

gccaccccat gcccggatct cccagcatgg agacccattg ctgtccaaca ccttcactga     900

gaccaacaac cactggcccc agggacccac tgggcctcca ggccctccag ggcccatggg     960

tccccctggg cctcctggcc ccacaggtgt ccctgggagt cctggtcaca taggaccccc    1020

aggccccact ggacccaaag gaatctctgg ccacccagga gagaagggcg agagaggact    1080

gcgtggggag cctggccccc aaggctctgc tgggcagcgg ggggaacctg gccctaaggg    1140

agaccctggt gagaagagcc actgggggga ggggttgcac cagctacgcg aggctttgaa    1200

gattttagct gagagggttt taatcttgga aacaatgatt gggctctatg aaccagagct    1260

ggggtctggg gcgggccctg ccggcacagg cacccccagc ctccttcggg gcaagagggg 1320

cggacatgca accaactacc ggatcgtggc ccccaggagc cgggacgaga gaggctgagg 1380

gtggtggcgg cccctgaggc agaccaggcc aggcttcccc tcctacctgg actcggccag 1440

ctgcctccag ggaccgcccg tccatattta ttaatgtcct cagggtccct tctgccatct 1500

aggccttagg ggtaagcagg tctcagtcct ggcaccatgc acatgtctga ggctgagcaa 1560

gggctgagag gagaggcttg ggcctcagtt tccctctgtg aagtgggggg aggcaggcct 1620

tcaaggaggg atagaggtac aaggcttcgt ctcatctgct gtctgagcat ccaggcccaa 1680

aggcactgag ggagtcagga gctggggctc ggcacatgca gagatgacag ggcagggggc 1740

agtcttcctc cccctccccg accaaacctc ggggagccct cctgtgcccc tccctccttg 1800

ttgtccagtg ctgggctccc caccccgagg tcaggctgcc caatcctctg actggatcac 1860

cgggggcttc ttgcctcagt tcttccctct gagcccccag gccctcccgc atctcaggtt 1920

ggggatgggg acatggagag gaaggggccg cctactcctg caaatgcttg tgacagatgc 1980

caggaggtag atgtgtgctg gccaataaag gcccctacct gattccccgc a 2031

<210> 60
<211> 769
<212> DNA
<213> Homo sapiens

<400> 60
cgccctccgg ccgcggagct ggaaaccggg ctccgcgcgt ccggggcggc tggcggcgcg 60

ggcaggcagg cggggaggac aggctggggg cggcgaccgc gaggggccgc gcgcggaggg 120

cgcctggtgc agcatgggcg gcccgcgggc ttgggcgctg ctctgcctcg ggctcctgct 180

cccgggaggc ggcgctgcgt ggagcatcgg ggcagctccg ttctccggac gcaggaactg 240

gtgctcctat gtggtgaccc gcaccatctc atgccatgtg cagaatggca cctaccttca 300

gcgagtgctg cagaactgcc cctggcccat gagctgtccg gggagcagct acagaactgt 360

ggtgagaccc acatacaagg tgatgtacaa gatagtgacc gcccgtgagt ggaggtgctg 420

ccctgggcac tcaggagtga gctgcgagga agttgcagct tcctctgcct ccttggagcc 480

catgtggtcg ggcagtacca tgcggcggat ggcgcttcgg cccacagcct tctcaggttg 540

tctcaactgc agcaaagtgt cagagctgac agagcggctg aaggtgctgg aggccaagat 600

gaccatgctg actgtcatag agcagccagt acctccaaca ccagctaccc ctgaggaccc 660

tgccccgctc tggggtcccc ctcctgccca gggcagcccc ggagatggag gcctccaggg 720

agacccattg ctgtccaaca ccttcactga gaccaacaac cactggccc 769

<210> 61
<211> 641
<212> DNA

<213>  Homo sapiens

<400>  61
gctggaaacc gggctccgcg cgtccggggc ggctggcggc gcgggcaggc aggcggggag      60

gacaggctgg gggcggcgac cgcgaggggc cgcgcgcgga gggcgcctgg tgcagcatgg     120

gcggcccgcg ggcttgggcg ctgctctgcc tcgggctcct gctcccggga ggcggcgctg     180

cgtggagcat cggggcagct ccgttctccg gacgcaggaa ctggtgctcc tatgtggtga     240

cccgcaccat ctcatgccat gtgcagaatg gcacctacct tcagcgagtg ctgcagaact     300

gcccctggcc catgagctgt ccggggagca gctacagaac tgtggtgaga cccacataca     360

aggtgatgta caagatagtg accgcccgtg agtggaggtg ctgccctggg cactcaggag     420

tgagctgcga ggaaggttgt ctcaactgca gcaaagtgtc agagctgaca gagcggctga     480

aggtgctgga ggccaagatg accatgctga ctgtcataga gcagccagta cctccaacac     540

cagctacccc tgaggaccct gccccgctct ggggtccccc tcctgcccag ggcagccccg     600

gagatggagg cctccaggac caagtcggtg cttgggggct t                        641

<210>  62
<211>  482
<212>  DNA
<213>  Homo sapiens

<400>  62
cggcgcgggc aggcaggcgg ggaggacagg ctgggggcgg cgaccgcgag gggccgcgcg      60

cggagggcgc ctggtgcagc atgggcggcc cgcgggcttg ggcgctgctc tgcctcgggc     120

tcctgctccc gggaggcggc gctgcgtgga gcatcggggc agctccgttc tccggacgca     180

gatgaccatg ctgactgtca tagagcagcc agtacctcca acaccagcta cccctgagga     240

ccctgccccg ctctggggtc cccctcctgc ccagggcagc cccggagatg gaggcctcca     300

ggaccaagtc ggtgcttggg ggcttcccgg gcccaccggc cccaagggag atgccggcag     360

tcggggccca atggggatga gaggcccacc aggtccacag ggccccccag ggagccctgg     420

ccgggctgga gctgtgggca cccctggaga gaggggacct cctgggccac cagggcctcc     480

tg                                                                    482

<210>  63
<211>  2066
<212>  DNA
<213>  Homo sapiens

<400>  63
cgggcaggca ggcggggagg acaggctggg ggcggcgacc gcgaggggcc gcgcgcggag      60

ggcgcctggt gcagcatggg cggcccgcgg gcttgggcgc tgctctgcct cgggctcctg     120

ctcccgggag gcggcgctgc gtggagcatc ggggcagctc cgttctccgg acgcaggaac     180

tggtgctcct atgtggtgac ccgcaccatc tcatgccatg tgcagaatgg cacctacctt 240

cagcgagtgc tgcagaactg cccctggccc atgagctgtc cggggagcag ctacagaact 300

gtggtgagac ccacatacaa ggtgatgtac aagatagtga ccgcccgtga gtggaggtgc 360

tgccctgggc actcaggagt gagctgcgag gaagttgcag cttcctctgc ctccttggag 420

cccatgtggt cgggcagtac catgcggcgg atggcgcttc ggcccacagc cttctcaggt 480

tgtctcaact gcagcaaagt gtcagagctg acagagcggc tgaaggtgct ggaggccaag 540

atgaccatgc tgactgtcat agagcagcca gtacctccaa caccagctac ccctgaggac 600

cctgccccgc tctggggtcc ccctcctgcc cagggcagcc ccggagatgg aggcctccag 660

gaccaagtcg gtgcttgggg gcttcccggg cccaccggcc ccaagggaga tgccggcagt 720

cggggcccaa tggggatgag aggcccacca ggtccacagg gccccccagg gagccctggc 780

cgggctggag ctgtgggcac ccctggagag aggggacctc ctgggccacc agggcctcct 840

ggcccccctg ggcccccagc ccctgttggg ccaccccatg cccggatctc ccagcatgga 900

gacccattgc tgtccaacac cttcactgag accaacaacc actggcccca gggacccact 960

gggcctccag gccctccagg gcccatgggt ccccctgggc ctcctggccc cacaggtgtc 1020

cctgggagtc ctggtcacat aggaccccca ggccccactg gacccaaagg aatctctggc 1080

cacccaggag agaagggcga gagaggactg cgtggggagc ctggccccca aggctctgct 1140

gggcagcggg gggaacctgg ccctaaggga gaccctggtg agaagagcca ctgggctcct 1200

agcttacaga gcttcctgca gcagcaggct cagctggagc tcctggccag acgggtcacc 1260

ctcctggaag ccatcatctg gccagaacca gagctggggt ctgggcgggg ccctgccggc 1320

acaggcaccc ccagcctcct tcggggcaag aggggcggac atgcaaccaa ctaccggatc 1380

gtggccccca ggagccggga cgagagaggc tgagggtggt ggcggcccct gaggcagacc 1440

aggccaggct tcccctccta cctggactcg gccagctgcc tccagggacc gcccgtccat 1500

atttattaat gtcctcaggg tcccttctgc catctaggcc ttaggggtaa gcaggtctca 1560

gtcctggcac catgcacatg tctgaggctg agcaagggct gagaggagag gcttgggcct 1620

cagtttccct ctgtgaagtg gggggaggca ggccttcaag gagggataga ggtacaaggc 1680

ttcgtctcat ctgctgtctg agcatccagg cccaaaggca ctgagggagt caggagctgg 1740

ggctcggcac atgcagagat gacagggcag ggggcagtct tcctccccct ccccgaccaa 1800

acctcgggga gccctcctgt gcccctccct ccttgttgtc cagtgctggg ctccccaccc 1860

cgaggtcagg ctgcccaatc ctctgactgg atcaccgggg gcttcttgcc tcagttcttc 1920

cctctgagcc cccaggccct cccgcatctc aggttgggga tggggacatg gagaggaagg 1980

ggccgcctac tcctgcaaat gcttgtgaca gatgccagga ggtagatgtg tgctggccaa 2040

taaaggcccc tacctgattc cccgca 2066

<210> 64
<211> 2060
<212> DNA
<213> Homo sapiens

<400> 64
cgggcaggca ggcggggagg acaggctggg ggcggcgacc gcgaggggcc gcgcgcggag 60

ggcgcctggt gcagcatggg cggcccgcgg gcttgggcgc tgctctgcct cgggctcctg 120

ctcccgggag gcggcgctgc gtggagcatc ggggcagctc cgttctccgg acgcaggaac 180

tggtgctcct atgtggtgac ccgcaccatc tcatgccatg tgcagaatgg cacctacctt 240

cagcgagtgc tgcagaactg cccctggccc atgagctgtc cggggagcag ctacagaact 300

gtggtgagac ccacatacaa ggtgatgtac aagatagtga ccgcccgtga gtggaggtgc 360

tgccctgggc actcaggagt gagctgcgag gaagttgcag cttcctctgc ctccttggag 420

cccatgtggt cgggcagtac catgcggcgg atggcgcttc ggcccacagc cttctcaggt 480

tgtctcaact gcagcaaagt gtcagagctg acagagcggc tgaaggtgct ggaggccaag 540

atgaccatgc tgactgtcat agagcagcca gtacctccaa caccagctac ccctgaggac 600

cctgccccgc tctggggtcc ccctcctgcc cagggcagcc ccggagatgg aggcctccag 660

gaccaagtcg gtgcttgggg gcttcccggg cccaccggcc ccaaggagga tgccggcagt 720

cggggcccaa tggggatgag aggcccacca ggtccacagg gccccccagg gagccctggc 780

cgggctggag ctgtgggcac ccctggagag aggggacctc ctgggccacc agggcctcct 840

ggcccccctg ggcccccagc ccctgttggg ccaccccatg cccggatctc ccagcatgga 900

gacccattgc tgtccaacac cttcactgag accaacaacc actggcccca gggacccact 960

gggcctccag gccctccagg gcccatgggt ccccctgggc ctcctggccc cacaggtgtc 1020

cctgggagtc ctggtcacat aggaccccca ggccccactg gacccaaagg aatctctggc 1080

cacccaggag agaagggcga gagaggactg cgtggggagc ctggccccca aggctctgct 1140

gggcagcggg gggaacctgg ccctaaggga gaccctggtg agaagagcca ctggggggag 1200

gggttgcacc agctacgcga ggctttgaag attttagctg agagggtttt aatcttggaa 1260

acaatgattg ggctctatga accagagctg gggtctgggg cgggccctgc cggcacaggc 1320

acccccagcc tccttcgggg caagaggggc ggacatgcaa ccaactaccg gatcgtggcc 1380

cccaggagcc gggacgagag aggctgaggg tggtggcggc ccctgaggca gaccaggcca 1440

ggcttcccct cctacctgga ctcggccagc tgcctccagg gaccgcccgt ccatatttat 1500

taatgtcctc agggtccctt ctgccatcta ggccttaggg gtaagcaggt ctcagtcctg 1560

gcaccatgca catgtctgag gctgagcaag ggctgagagg agaggcttgg gcctcagttt 1620

ccctctgtga agtgggggga ggcaggcctt caaggaggga tagaggtaca aggcttcgtc 1680

tcatctgctg tctgagcatc caggcccaaa ggcactgagg gagtcaggag ctggggctcg 1740

gcacatgcag agatgacagg gcagggggca gtcttcctcc ccctccccga ccaaacctcg 1800

gggagccctc ctgtgcccct ccctccttgt tgtccagtgc tgggctcccc accccgaggt 1860

caggctgccc aatcctctga ctggatcacc gggggcttct tgcctcagtt cttccctctg 1920

agcccccagg ccctcccgca tctcaggttg gggatgggga catggagagg aaggggccgc 1980

ctactcctgc aaatgcttgt gacagatgcc aggaggtaga tgtgtgctgg ccaataaagg 2040

cccctacctg attccccgca 2060

<210> 65
<211> 2054
<212> DNA
<213> Homo sapiens

<400> 65
cgggcaggca ggcggggagg acaggctggg ggcggcgacc gcgaggggcc gcgcgcggag 60

ggcgcctggt gcagcatggg cggcccgcgg gcttgggcgc tgctctgcct cgggctcctg 120

ctcccgggag gcggcgctgc gtggagcatc ggggcagctc cgttctccgg acgcaggaac 180

tggtgctcct atgtggtgac ccgcaccatc tcatgccatg tgcagaatgg cacctacctt 240

cagcgagtgc tgcagaactg cccctggccc atgagctgtc cggggagcag ctacagaact 300

gtggtgagac ccacatacaa ggtgatgtac aagatagtga ccgcccgtga gtggaggtgc 360

tgccctgggc actcaggagt gagctgcgag gaagcttcct ctgcctcctt ggagcccatg 420

tggtcgggca gtaccatgcg gcggatggcg cttcggccca cagccttctc aggttgtctc 480

aactgcagca aagtgtcaga gctgacagag cggctgaagg tgctggaggc caagatgacc 540

atgctgactg tcatagagca gccagtacct ccaacaccag ctacccctga ggaccctgcc 600

ccgctctggg gtccccctcc tgcccagggc agccccggag atggaggcct ccaggaccaa 660

gtcggtgctt gggggcttcc cgggcccacc ggccccaagg gagatgccgg cagtcggggc 720

ccaatgggga tgagaggccc accaggtcca cagggccccc cagggagccc tggccgggct 780

ggagctgtgg gcacccctgg agagagggga cctcctgggc caccagggcc tcctggcccc 840

cctgggcccc cagcccctgt tgggccaccc catgcccgga tctcccagca tggagaccca 900

ttgctgtcca acaccttcac tgagaccaac aaccactggc cccagggacc cactgggcct 960

ccaggccctc cagggcccat gggtccccct gggcctcctg gccccacagg tgtccctggg 1020

agtcctggtc acataggacc cccaggcccc actggaccca aaggaatctc tggccaccca 1080

ggagagaagg gcgagagagg actgcgtggg gagcctggcc cccaaggctc tgctgggcag 1140

cggggggaac ctggccctaa gggagaccct ggtgagaaga gccactgggg ggaggggttg 1200

caccagctac gcgaggcttt gaagatttta gctgagaggg ttttaatctt ggaaacaatg 1260

attgggctct atgaaccaga gctggggtct ggggcgggcc ctgccggcac aggcaccccc 1320

agcctccttc ggggcaagag gggcggacat gcaaccaact accggatcgt ggcccccagg 1380

agccgggacg agagaggctg agggtggtgg cggcccctga ggcagaccag gccaggcttc 1440

ccctcctacc tggactcggc cagctgcctc cagggaccgc ccgtccatat ttattaatgt 1500

cctcagggtc ccttctgcca tctaggcctt aggggtaagc aggtctcagt cctggcacca 1560

tgcacatgtc tgaggctgag caagggctga gaggagaggc ttgggcctca gtttccctct 1620

gtgaagtggg gggaggcagg ccttcaagga gggatagagg tacaaggctt cgtctcatct 1680

gctgtctgag catccaggcc caaaggcact gagggagtca ggagctgggg ctcggcacat 1740

gcagagatga cagggcaggg ggcagtcttc ctccccctcc ccgaccaaac ctcggggagc 1800

cctcctgtgc ccctccctcc ttgttgtcca gtgctgggct ccccaccccg aggtcaggct 1860

gcccaatcct ctgactggat caccgggggc ttcttgcctc agttcttccc tctgagcccc 1920

caggccctcc cgcatctcag gttggggatg gggacatgga gaggaagggg ccgcctactc 1980

ctgcaaatgc ttgtgacaga tgccaggagg tagatgtgtg ctggccaata aaggccccta 2040

cctgattccc cgca 2054

<210> 66
<211> 2048
<212> DNA
<213> Homo sapiens

<400> 66
cgggcaggca ggcggggagg acaggctggg ggcggcgacc gcgaggggcc gcgcgcggag 60

ggcgcctggt gcagcatggg cggcccgcgg gcttgggcgc tgctctgcct cgggctcctg 120

ctcccgggag gcggcgctgc gtggagcatc ggggcagctc cgttctccgg acgcaggaac 180

tggtgctcct atgtggtgac ccgcaccatc tcatgccatg tgcagaatgg cacctacctt 240

cagcgagtgc tgcagaactg cccctggccc atgagctgtc cggggagcag aactgtggtg 300

agacccacat acaaggtgat gtacaagata gtgaccgccc gtgagtggag gtgctgccct 360

gggcactcag gagtgagctg cgaggaagct tcctctgcct ccttggagcc catgtggtcg 420

ggcagtacca tgcggcggat ggcgcttcgg cccacagcct tctcaggttg tctcaactgc 480

agcaaagtgt cagagctgac agagcggctg aaggtgctgg aggccaagat gaccatgctg 540

actgtcatag agcagccagt acctccaaca ccagctaccc ctgaggaccc tgccccgctc 600

tggggtcccc ctcctgccca gggcagcccc ggagatggag gcctccagga ccaagtcggt 660

gcttgggggc ttcccgggcc caccggcccc aagggagatg ccggcagtcg gggcccaatg 720

gggatgagag gcccaccagg tccacagggc cccccaggga gccctggccg ggctggagct 780

gtgggcaccc ctggagagag gggacctcct gggccaccag ggcctcctgg cccccctggg 840

cccccagccc ctgttgggcc accccatgcc cggatctccc agcatggaga cccattgctg 900

tccaacacct tcactgagac caacaaccac tggccccagg gacccactgg gcctccaggc 960

cctccagggc ccatgggtcc ccctgggcct cctggcccca caggtgtccc tgggagtcct 1020

ggtcacatag gacccccagg ccccactgga cccaaaggaa tctctggcca cccaggagag 1080

aagggcgaga gaggactgcg tggggagcct ggcccccaag gctctgctgg gcagcggggg 1140

gaacctggcc ctaagggaga ccctggtgag aagagccact gggggagggg gttgcaccag 1200

ctacgcgagg ctttgaagat tttagctgag agggttttaa tcttggaaac aatgattggg 1260

ctctatgaac cagagctggg gtctggggcg ggccctgccg gcacaggcac ccccagcctc 1320

cttcggggca agaggggcgg acatgcaacc aactaccgga tcgtggcccc caggagccgg 1380

gacgagagag gctgagggtg gtggcggccc ctgaggcaga ccaggccagg cttcccctcc 1440

tacctggact cggccagctg cctccaggga ccgcccgtcc atatttatta atgtcctcag 1500

ggtcccttct gccatctagg ccttaggggt aagcaggtct cagtcctggc accatgcaca 1560

tgtctgaggc tgagcaaggg ctgagaggag aggcttgggc ctcagtttcc ctctgtgaag 1620

tggggggagg caggccttca aggagggata gaggtacaag gcttcgtctc atctgctgtc 1680

tgagcatcca ggcccaaagg cactgaggga gtcaggagct ggggctcggc acatgcagag 1740

atgacagggc agggggcagt cttcctcccc ctccccgacc aaacctcggg gagccctcct 1800

gtgcccctcc ctccttgttg tccagtgctg ggctccccac cccgaggtca ggctgcccaa 1860

tcctctgact ggatcaccgg gggcttcttg cctcagttct tccctctgag cccccaggcc 1920

ctcccgcatc tcaggttggg gatggggaca tggagaggaa ggggccgcct actcctgcaa 1980

atgcttgtga cagatgccag gaggtagatg tgtgctggcc aataaaggcc cctacctgat 2040

tccccgca 2048

<210> 67
<211> 1997
<212> DNA
<213> Homo sapiens

<400> 67
cgggcaggca ggcggggagg acaggctggg ggcggcgacc gcgaggggcc gcgcgcggag 60

ggcgcctggt gcagcatggg cggcccgcgg gcttgggcgc tgctctgcct cgggctcctg 120

ctcccgggag gcggcgctgc gtggagcatc ggggcagctc cgttctccgg acgcaggaac 180

tggtgctcct atgtggtgac ccgcaccatc tcatgccatg tgcagaatgg cacctacctt 240

cagcgagtgc tgcagaactg cccctggccc atgagctgtc cggggagcag ctacagaact 300

gtggtgagac ccacatacaa ggtgatgtac aagatagtga ccgcccgtga gtggaggtgc 360

tgccctgggc actcaggagt gagctgcgag gaagttgcag cttcctctgc ctccttggag 420

cccatgtggt cgggcagtac catgcggcgg atggcgcttc ggcccacagc cttctcaggt 480

tgtctcaact gcagcaaagt gtcagagctg acagagcggc tgaaggtgct ggaggccaag 540

atgaccatgc tgactgtcat agagcagcca gtacctccaa caccagctac ccctgaggac 600

cctgccccgc tctggggtcc ccctcctgcc cagggcagcc ccggagatgg aggcctccag 660

gaccaagtcg gtgcttgggg gcttcccggg cccaccggcc ccaagggaga tgccggcagt 720

cggggcccaa tggggatgag aggcccacca ggtccacagg gccccccagg gagccctggc 780

cgggctggag ctgtgggcac ccctggagag aggggacctc ctgggccacc agggcctcct 840

ggcccccctg ggcccccagc ccctgttggg ccaccccatg cccggatctc ccagcatgga 900

gacccattgc tgtccaacac cttcactgag accaacaacc actggcccca gggacccact 960

gggcctccag gccctccagg gcccatgggt ccccctgggc ctcctggccc cacaggtgtc 1020

cctgggagtc ctggtcacat aggactgcgt ggggagcctg gcccccaagg ctctgctggg 1080

cagcgggggg aacctggccc taagggagac cctggtgaga agagccactg gggggagggg 1140

ttgcaccagc tacgcgaggc tttgaagatt ttagctgaga gggttttaat cttggaaaca 1200

atgattgggc tctatgaacc agagctgggg tctggggcgg gccctgccgg cacaggcacc 1260

cccagcctcc ttcggggcaa gaggggcgga catgcaacca actaccggat cgtggccccc 1320

aggagccggg acgagagagg ctgagggtgg tggcggcccc tgaggcagac caggccaggc 1380

ttcccctcct acctggactc ggccagctgc ctccagggac cgcccgtcca tatttattaa 1440

tgtcctcagg gtcccttctg ccatctaggc cttaggggta agcaggtctc agtcctggca 1500

ccatgcacat gtctgaggct gagcaagggc tgagaggaga ggcttgggcc tcagtttccc 1560

tctgtgaagt ggggggaggc aggccttcaa ggagggatag aggtacaagg cttcgtctca 1620

tctgctgtct gagcatccag gcccaaaggc actgagggag tcaggagctg gggctcggca 1680

catgcagaga tgacagggca gggggcagtc ttcctccccc tccccgacca aacctcgggg 1740

agccctcctg tgcccctccc tccttgttgt ccagtgctgg gctccccacc ccgaggtcag 1800

gctgcccaat cctctgactg gatcaccggg ggcttcttgc ctcagttctt ccctctgagc 1860

ccccaggccc tcccgcatct caggttgggg atggggacat ggagaggaag gggccgccta 1920

ctcctgcaaa tgcttgtgac agatgccagg aggtagatgt gtgctggcca ataaaggccc 1980

ctacctgatt ccccgca 1997

<210> 68
<211> 1823
<212> DNA
<213> Homo sapiens

<400> 68
cgggcaggca ggcggggagg acaggctggg ggcggcgacc gcgaggggcc gcgcgcggag 60

ggcgcctggt gcagcatggg cggcccgcgg gcttgggcgc tgctctgcct cgggctcctg 120

ctcccgggag gcggcgctgc gtggagcatc ggggcagctc cgttctccgg acgcaggaac 180

tggtgctcct atgtggtgac ccgcaccatc tcatgccatg tgcagaatgg cacctacctt 240

cagcgagtgc tgcagaactg cccctggccc atgagctgtc cggggagcag ctacagaact 300

gtggtgagac ccacatacaa ggtgatgtac aagatagtga ccgcccgtga gtggaggtgc 360

tgccctgggc actcaggagt gagctgcgag gaagttgcag cttcctctgc ctccttggag 420

cccatgtggt cgggcagtac catgcggcgg atggcgcttc ggcccacagc cttctcaggt 480

tgtctcaact gcagcaaagt gtcagagctg acagagcggc tgaaggtgct ggaggccaag 540

atgaccatgc tgactgtcat agagcagcca gtacctccaa caccagctac ccctgaggac 600

cctgccccgc tctggggtcc ccctcctgcc cagggcagcc ccggagatgg aggcctccag 660

ggagacccat tgctgtccaa caccttcact gagaccaaca accactggcc ccagggaccc 720

actgggcctc caggccctcc agggcccatg ggtccccctg ggcctcctgg ccccacaggt 780

gtccctggga gtcctggtca cataggaccc ccaggcccca ctggacccaa aggaatctct 840

ggccacccag gagagaaggg cgagagagga ctgcgtgggg agcctggccc ccaaggctct 900

gctgggcagc ggggggaacc tggccctaag ggagaccctg gtgagaagag ccactggggg 960

gaggggttgc accagctacg cgaggctttg aagattttag ctgagagggt tttaatcttg 1020

gaaacaatga ttgggctcta tgaaccagag ctggggtctg ggcgggcccc tgccggcaca 1080

ggcaccccca gcctccttcg ggcaagaggg ggcggacatg caaccaacta ccggatcgtg 1140

gcccccagga gccgggacga gagaggctga gggtggtggc ggcccctgag gcagaccagg 1200

ccaggcttcc cctcctacct ggactcggcc agctgcctcc agggaccgcc cgtccatatt 1260

tattaatgtc ctcagggtcc cttctgccat ctaggcctta ggggtaagca ggtctcagtc 1320

ctggcaccat gcacatgtct gaggctgagc aagggctgag aggagaggct tgggcctcag 1380

tttccctctg tgaagtgggg ggaggcaggc cttcaaggag ggatagaggt acaaggcttc 1440

gtctcatctg ctgtctgagc atccaggccc aaaggcactg agggagtcag gagctggggc 1500

tcggcacatg cagagatgac agggcagggg gcagtcttcc tccccctccc cgaccaaacc 1560

tcggggagcc ctcctgtgcc cctccctcct tgttgtccag tgctgggctc cccaccccga 1620

ggtcaggctg cccaatcctc tgactggatc accgggggct tcttgcctca gttcttccct 1680

ctgagccccc aggccctccc gcatctcagg ttggggatgg ggacatggag aggaaggggc 1740

cgcctactcc tgcaaatgct tgtgacagat gccaggaggt agatgtgtgc tggccaataa 1800

aggcccctac ctgattcccc gca 1823

<210> 69

<211>  1976
<212>  DNA
<213>  Homo sapiens

<400>  69
cgggcaggca ggcggggagg acaggctggg ggcggcgacc gcgaggggcc gcgcgcggag      60

ggcgcctggt gcagcatggg cggcccgcgg gcttgggcgc tgctctgcct cgggctcctg     120

ctcccgggag gcggcgctgc gtggagcatc ggggcagctc cgttctccgg acgcaggaac     180

tggtgctcct atgtggtgac ccgcaccatc tcatgccatg tgcagaatgg cacctacctt     240

cagcgagtgc tgcagaactg cccctggccc atgagctgtc cggggagcag ctacagaact     300

gtggtgagac ccacatacaa ggtgatgtac aagatagtga ccgcccgtga gtggaggtgc     360

tgccctgggc actcaggagt gagctgcgag gaaggttgtc tcaactgcag caaagtgtca     420

gagctgacag agcggctgaa ggtgctggag gccaagatga ccatgctgac tgtcatagag     480

cagccagtac ctccaacacc agctacccct gaggaccctg ccccgctctg gggtccccct     540

cctgcccagg gcagccccgg agatggaggc ctccaggacc aagtcggtgc ttgggggctt     600

cccgggccca ccggccccaa gggagatgcc ggcagtcggg gcccaatggg gatgagaggc     660

ccaccaggtc cacagggccc cccagggagc cctggccggg ctggagctgt gggcacccct     720

ggagagaggg gacctcctgg gccaccaggg cctcctggcc cccctgggcc cccagcccct     780

gttgggccac cccatgcccg gatctcccag catggagacc cattgctgtc caacaccttc     840

actgagacca acaaccactg gccccaggga cccactgggc ctccaggccc tccagggccc     900

atgggtcccc ctgggcctcc tggccccaca ggtgtccctg ggagtcctgg tcacatagga     960

cccccaggcc ccactggacc caaaggaatc tctggccacc caggagagaa gggcgagaga    1020

ggactgcgtg gggagcctgg cccccaaggc tctgctgggc agcgggggga acctggccct    1080

aagggagacc ctggtgagaa gagccactgg ggggaggggt tgcaccagct acgcgaggct    1140

ttgaagattt tagctgagag ggttttaatc ttggaaacaa tgattgggct ctatgaacca    1200

gagctggggt ctggggcggg ccctgccggc acaggcaccc ccagcctcct tcggggcaag    1260

aggggcggac atgcaaccaa ctaccggatc gtggccccca ggagccggga cgagagaggc    1320

tgagggtggt ggcggcccct gaggcagacc aggccaggct tcccctccta cctggactcg    1380

gccagctgcc tccagggacc gcccgtccat atttattaat gtcctcaggg tcccttctgc    1440

catctaggcc ttaggggtaa gcaggtctca gtcctggcac catgcacatg tctgaggctg    1500

agcaagggct gagaggagag gcttgggcct cagtttccct ctgtgaagtg gggggaggca    1560

ggccttcaag gagggataga ggtacaaggc ttcgtctcat ctgctgtctg agcatccagg    1620

cccaaaggca ctgagggagt caggagctgg ggctcggcac atgcagagat gacagggcag    1680

ggggcagtct tcctccccct ccccgaccaa acctcgggga gccctcctgt gcccctccct    1740

ccttgttgtc cagtgctggg ctccccaccc cgaggtcagg ctgcccaatc ctctgactgg 1800

atcaccgggg gcttcttgcc tcagttcttc cctctgagcc cccaggccct cccgcatctc 1860

aggttgggga tggggacatg gagaggaagg ggccgcctac tcctgcaaat gcttgtgaca 1920

gatgccagga ggtagatgtg tgctggccaa taaaggcccc tacctgattc cccgca 1976

<210> 70
<211> 2111
<212> DNA
<213> Homo sapiens

<400> 70
cgggcaggca ggcggggagg acaggctggg ggcggcgacc gcgaggggcc gcgcgcggag 60

ggcgcctggt gcagcatggg cggcccgcgg gcttgggcgc tgctctgcct cgggctcctg 120

ctcccgggag gcggcgctgc gtggagcatc ggggcagctc cgttctccgg acgcaggaac 180

tggtgctcct atgtggtgac ccgcaccatc tcatgccatg tgcagaatgg cacctacctt 240

cagcgagtgc tgcagaactg cccctggccc atgagctgtc cggggagcag ctacagaact 300

gtggtgagac ccacatacaa ggtgatgtac aagatagtga ccgcccgtga gtggaggtgc 360

tgccctgggc actcaggagt gagctgcgag gaagttgcag cttcctctgc ctccttggag 420

cccatgtggt cgggcagtac catgcggcgg atggcgcttc ggcccacagc cttctcaggt 480

tgtctcaact gcagcaaagt gtcagagctg acagagcggc tgaaggtgct ggaggccaag 540

atgaccatgc tgactgtcat agagcagcca gtacctccaa caccagctac ccctgaggac 600

cctgccccgc tctggggtcc ccctcctgcc cagggcagcc ccggagatgg aggcctccag 660

gggctgccag gagccataga gagtgtgagg gtcccgctgc ttccccgaaa tgaccaagtc 720

ggtgcttggg ggcttcccgg gcccaccggc cccaagggag atgccggcag tcggggccca 780

atggggatga gaggcccacc aggtccacag ggccccccag ggagccctgg ccgggctgga 840

gctgtgggca cccctggaga gaggggacct cctgggccac cagggcctcc tggcccccct 900

gggcccccag cccctgttgg gccaccccat gcccggatct cccagcatgg agacccattg 960

ctgtccaaca ccttcactga gaccaacaac cactggcccc agggacccac tgggcctcca 1020

ggccctccag ggcccatggg tccccctggg cctcctggcc ccacaggtgt ccctgggagt 1080

cctggtcaca taggaccccc aggccccact ggacccaaag gaatctctgg ccacccagga 1140

gagaagggcg agagaggact gcgtggggag cctggccccc aaggctctgc tgggcagcgg 1200

ggggaacctg gccctaaggg agaccctggt gagaagagcc actgggggga ggggttgcac 1260

cagctacgcg aggctttgaa gattttagct gagagggttt taatcttgga aacaatgatt 1320

gggctctatg aaccagagct ggggtctggg gcgggccctg ccggcacagg cacccccagc 1380

ctccttcggg gcaagagggg cggacatgca accaactacc ggatcgtggc ccccaggagc 1440

cgggacgaga gaggctgagg gtggtggcgg cccctgaggc agaccaggcc aggcttcccc 1500

tcctacctgg actcggccag ctgcctccag ggaccgcccg tccatattta ttaatgtcct 1560

cagggtccct tctgccatct aggccttagg ggtaagcagg tctcagtcct ggcaccatgc 1620

acatgtctga ggctgagcaa gggctgagag gagaggcttg ggcctcagtt tccctctgtg 1680

aagtgggggg aggcaggcct tcaaggaggg atagaggtac aaggcttcgt ctcatctgct 1740

gtctgagcat ccaggcccaa aggcactgag ggagtcagga gctggggctc ggcacatgca 1800

gagatgacag ggcagggggc agtcttcctc cccctccccg accaaacctc ggggagccct 1860

cctgtgcccc tccctccttg ttgtccagtg ctgggctccc caccccgagg tcaggctgcc 1920

caatcctctg actggatcac cgggggcttc ttgcctcagt tcttccctct gagcccccag 1980

gccctcccgc atctcaggtt ggggatgggg acatggagag gaaggggccg cctactcctg 2040

caaatgcttg tgacagatgc caggaggtag atgtgtgctg gccaataaag gcccctacct 2100

gattccccgc a 2111

<210> 71
<211> 707
<212> DNA
<213> Homo sapiens

<400> 71
agatgaccat gctgactgtc atagagcagc cagtacctcc aacaccagct acccctgagg 60

accctgcccc gctctggggt ccccctcctg cccagggcag ccccggagat ggaggcctcc 120

aggggctgcc aggagccata gagagtgtga gggtcccgct gcttccccga aatgaccaag 180

tcggtgcttg ggggcttccc gggcccaccg gccccaaggg agatgccggc agtcggggcc 240

caatggggat gagaggccca ccaggtccac agggcccccc agggagccct ggccgggctg 300

gagctgtggg cacccctgga gagaggggac ctcctgggcc accagggcct cctggccccc 360

ctgggccccc agcccctgtt gggccacccc atgcccggat ctcccagcat ggagacccat 420

tgctgtccaa caccttcact gagaccaaca accactggcc ccagggaccc actgggcctc 480

caggccctcc agggcccatg ggtccccctg ggcctcctgg ccccacaggt gtccctggga 540

gtcctggtca cataggaccc ccaggcccca ctggacccaa aggaatctct ggccacccag 600

gagagaaggg cgagagagga ctgcgtgggg agcctggccc ccaaggctct gctgggcagc 660

ggggggaacc tggccctaag ggagaccctg gtgagaagag ccactgg 707

<210> 72
<211> 2034
<212> DNA
<213> Homo sapiens

<400> 72
ggtgagtgcc cgcaatgctg ccccacagct cctctggcca tcccctccac caggtgggcc 60

cttccctgct cctgacatgg ccaggatgac ctgggccctt tcatctactt gcctcttcac 120

tcagcacccc accacggagt gccctgccca cgcctgggct ccatgaagtc ctctcttatg 180

ttcactgacc cacattccct gggcacctac acttatcagg ctctgagctg ggcactgggt 240

ggggtcagac atgtccctgc ccttctggag cttccatgct gctgggagca gggctgggca 300

gaggagaagc agcaatgctt gccccatgtg accagggttt ctatgagggg ttttgggggt 360

ttgggagccc caaggaagga agactcagcc tggacgaggt ggagaactag gtgctgtgct 420

catccccctg ttagactacc aggcagccta ggcctgtgga ctccggggcc ctctctcatg 480

cccactgctc caggctgcct tgtcctgtcg ctcaaggcca ccctgggcct ccttgcctcc 540

tgtataccca caaatccgtg tgattccatt gcaggtccac agggcccccc agggagccct 600

ggccgggctg gagctgtggg cacccctgga gagaggggac ctcctgggcc accagggcct 660

cctggccccc ctgggccccc agcccctgtt gggccacccc atgcccggat ctcccagcat 720

ggtgagtccc cctgggatcc cagcaggtgg aggtgggggt ggagtagcca tcagcacagt 780

gcccgctacc atctgccacg tgccttctgt gtgccagccc tgctcacgat aggccacatg 840

tgacccagtc ctccagcagg cgccgttgtc ctcctgtggt tacaggtgag gaacactgag 900

gaccagagag ggaaggtggc ttgccagggt cccacagcct gggcgtaggg gaacggcttc 960

aaacccaggc tgcctccaga acctgtgctt agagccaccg ggcatcaggc cctcccaagc 1020

cttggaactg gctggaatcc agttctcgga acactgggac gcaaaagacc cggcggcagg 1080

aagtgagtcc tgaactccca aggccacagg cccggcccct cctccaggcc ctgacgtgcg 1140

tccttggctt cttccctttg gcagcccagc ctgacctgcc catgggctgc caggggtcag 1200

agtgtggagc gccaggtttc agcctcttct ccactgtgtt tttggtgcac aacccagcac 1260

accattcatt cattctgcca tcccagcatt cattccatct cactatccat acgatgggga 1320

caatgacagt gccagcctcc cagagctgcg taacatccat gtacagaagc ctggcacaca 1380

gtaggtggtg gataaatggt atcttttatt gtcattccca tttgacaggt gacagtacag 1440

gctctgaaaa gtagaaagtg ttgctggatg tcaccagctg gattgcagtg gggttagaac 1500

ccacatctcc ctgcctcctg gtcttgcggg accaacactc tccacactcc tcaccctgga 1560

gcaggtgccc aggtggtacc agccatgctg caggctgccc catagggcag tccaagctgt 1620

cttggcagag gtggcaggtg aagactaacc accccactct acccagctct actcactcat 1680

catctttgct cacccaggag acccattgct gtccaacacc ttcactgaga ccaacaacca 1740

ctggccccag ggacccactg ggcctccagg ccctccaggg cccatgggtc cccctggccc 1800

tcctggcccc acaggtgtcc ctgggagtcc tggtcacata gtgagtagtt ctccttgtac 1860

tctcacccat gtgtctgtcc atctttccat ctatgcatac atccatacat ctgtccatca 1920

tccacccttg tatccatcta tccatccatc cattcatcct tccattcatt cattcaacaa 1980

gtatttattg agcacttaat atgcaaacta ccttccataa atcttattca atcc 2034

<210> 73
<211> 518
<212> PRT
<213> Homo sapiens

<400> 73

Met Leu Ala Ala Ala Ser Lys Tyr Arg His Gly Asn Met Val Phe Phe
1               5                   10                  15

Asp Val Leu Gly Leu Phe Val Ile Ala Tyr Pro Ser Arg Ile Gly Ser
            20                  25                  30

Ile Ile Asn Tyr Met Val Val Met Gly Val Val Leu Tyr Leu Gly Lys
        35                  40                  45

Lys Phe Leu Gln Pro Lys His Lys Thr Gly Asn Tyr Lys Lys Asp Phe
    50                  55                  60

Leu Cys Gly Leu Gly Ile Thr Leu Ile Ser Trp Phe Thr Ser Leu Val
65                  70                  75                  80

Thr Val Leu Ile Ile Ala Val Phe Ile Ser Leu Ile Gly Gln Ser Leu
                85                  90                  95

Ser Trp Tyr Asn His Phe Tyr Val Ser Val Cys Leu Tyr Gly Thr Ala
            100                 105                 110

Thr Val Ala Lys Ile Ile Leu Ile His Thr Leu Ala Lys Arg Phe Tyr
        115                 120                 125

Tyr Met Asn Ala Ser Ala Gln Tyr Leu Gly Glu Val Phe Phe Asp Ile
    130                 135                 140

Ser Leu Phe Val His Cys Cys Phe Leu Val Thr Leu Thr Tyr Gln Gly
145                 150                 155                 160

Leu Cys Ser Ala Phe Ile Ser Ala Val Trp Val Ala Phe Pro Leu Leu
                165                 170                 175

Thr Lys Leu Cys Val His Lys Asp Phe Lys Gln His Gly Ala Gln Gly
            180                 185                 190

Lys Phe Ile Ala Phe Tyr Leu Leu Gly Met Phe Ile Pro Tyr Leu Tyr
        195                 200                 205

Ala Leu Tyr Leu Ile Trp Ala Val Phe Glu Met Phe Thr Pro Ile Leu
    210                 215                 220

Gly Arg Ser Gly Ser Glu Ile Pro Pro Asp Val Val Leu Ala Ser Ile
225                 230                 235                 240

Leu Ala Gly Cys Thr Met Ile Leu Ser Ser Tyr Phe Ile Asn Phe Ile
                245                 250                 255

Tyr Leu Ala Lys Ser Thr Lys Lys Thr Met Leu Thr Leu Thr Leu Val
            260                 265                 270

Cys Ala Ile Thr Phe Leu Leu Val Cys Ser Gly Thr Phe Phe Pro Tyr
        275                 280                 285

Ser Ser Asn Pro Ala Asn Pro Lys Pro Lys Arg Val Phe Leu Gln His
    290                 295                 300

Met Thr Arg Thr Phe His Asp Leu Glu Gly Asn Ala Val Lys Arg Asp
305                 310                 315                 320

Ser Gly Ile Trp Ile Asn Gly Phe Asp Tyr Thr Gly Ile Ser His Ile
                325                 330                 335

Thr Pro His Ile Pro Glu Ile Asn Asp Ser Ile Arg Ala His Cys Glu
            340                 345                 350

Glu Asn Ala Pro Leu Cys Gly Phe Pro Trp Tyr Leu Pro Val His Phe
        355                 360                 365

Leu Ile Arg Lys Asn Trp Tyr Leu Pro Ala Pro Glu Val Ser Pro Arg
    370                 375                 380

Asn Pro Pro His Phe Arg Leu Ile Ser Lys Glu Gln Thr Pro Trp Asp
385                 390                 395                 400

Ser Ile Lys Leu Thr Phe Glu Ala Thr Gly Pro Ser His Met Ser Phe
                405                 410                 415

Tyr Val Arg Ala His Lys Gly Ser Thr Leu Ser Gln Trp Ser Leu Gly
            420                 425                 430

Asn Gly Thr Pro Val Thr Ser Lys Gly Gly Asp Tyr Phe Val Phe Tyr
        435                 440                 445

Ser His Gly Leu Gln Ala Ser Ala Trp Gln Phe Trp Ile Glu Val Gln
    450                 455                 460

Val Ser Glu Glu His Pro Glu Gly Met Val Thr Val Ala Ile Ala Ala
465                 470                 475                 480

His Tyr Leu Ser Gly Glu Asp Lys Arg Ser Pro Gln Leu Asp Ala Leu
                485                 490                 495

Lys Glu Lys Phe Pro Asp Trp Thr Phe Pro Ser Ala Trp Val Cys Thr
            500                 505                 510

Tyr Asp Leu Phe Val Phe
        515

<210>  74
<211>  904
<212>  PRT
<213>  Homo sapiens

<400>  74

Met Glu Trp Gly Ser Glu Ser Ala Ala Val Arg Arg His Arg Val Gly
1               5                   10                  15

Val Glu Arg Arg Glu Gly Ala Ala Ala Ala Pro Pro Pro Glu Arg Glu
            20                  25                  30

Ala Arg Ala Gln Glu Pro Leu Val Asp Gly Cys Ser Gly Gly Gly Arg
        35                  40                  45

Thr Arg Lys Arg Ser Pro Gly Gly Ser Gly Gly Ala Ser Arg Gly Ala
    50                  55                  60

Gly Thr Gly Leu Ser Glu Val Arg Ala Ala Leu Gly Leu Ala Leu Tyr
65                  70                  75                  80

Leu Ile Ala Leu Arg Thr Leu Val Gln Leu Ser Leu Gln Gln Leu Val
                85                  90                  95

Leu Arg Gly Ala Ala Gly His Arg Gly Glu Phe Asp Ala Leu Gln Ala
            100                 105                 110

Arg Asp Tyr Leu Glu His Ile Thr Ser Ile Gly Pro Arg Thr Thr Gly
        115                 120                 125

Ser Pro Glu Asn Glu Ile Leu Thr Val His Tyr Leu Leu Glu Gln Ile
    130                 135                 140

Lys Leu Ile Glu Val Gln Ser Asn Ser Leu His Lys Ile Ser Val Asp
145                 150                 155                 160

Val Gln Arg Pro Thr Gly Ser Phe Ser Ile Asp Phe Leu Gly Gly Phe
                165                 170                 175

Thr Ser Tyr Tyr Asp Asn Ile Thr Asn Val Val Val Lys Leu Glu Pro
            180                 185                 190

Arg Asp Gly Ala Gln His Ala Val Leu Ala Asn Cys His Phe Asp Ser
        195                 200                 205

Val Ala Asn Ser Pro Gly Ala Ser Asp Asp Ala Val Ser Cys Ser Val
    210                 215                 220

Met Leu Glu Val Leu Arg Val Leu Ser Thr Ser Ser Glu Ala Leu His
225                 230                 235                 240

His Ala Val Ile Phe Leu Phe Asn Gly Ala Glu Glu Asn Val Leu Gln
                245                 250                 255

Ala Ser His Gly Phe Ile Thr Gln His Pro Trp Ala Ser Leu Ile Arg
            260                 265                 270

Ala Phe Ile Asn Leu Glu Ala Ala Gly Val Gly Gly Lys Glu Leu Val
        275                 280                 285

Phe Gln Thr Gly Pro Glu Asn Pro Trp Leu Val Gln Ala Tyr Val Ser
    290                 295                 300

Ala Ala Lys His Pro Phe Ala Ser Val Val Ala Gln Glu Val Phe Gln
305                 310                 315                 320

Ser Gly Ile Ile Pro Ser Asp Thr Asp Phe Arg Ile Tyr Arg Asp Phe
                325                 330                 335

Gly Asn Ile Pro Gly Ile Asp Leu Ala Phe Ile Glu Asn Gly Tyr Ile
            340                 345                 350

Tyr His Thr Lys Tyr Asp Thr Ala Asp Arg Ile Leu Thr Asp Ser Ile
        355                 360                 365

Gln Arg Ala Gly Asp Asn Ile Leu Ala Val Leu Lys His Leu Ala Thr
    370                 375                 380

Ser Asp Met Leu Ala Ala Ala Ser Lys Tyr Arg His Gly Asn Met Val
385                 390                 395                 400

Phe Phe Asp Val Leu Gly Leu Phe Val Ile Ala Tyr Pro Ser Arg Ile

                405                 410                 415

Gly Ser Ile Ile Asn Tyr Met Val Val Met Gly Val Val Leu Tyr Leu
            420                 425                 430

Gly Lys Lys Phe Leu Gln Pro Lys His Lys Thr Gly Asn Tyr Lys Lys
        435                 440                 445

Asp Phe Leu Cys Gly Leu Gly Ile Thr Leu Ile Ser Trp Phe Thr Ser
    450                 455                 460

Leu Val Thr Val Leu Ile Ile Ala Val Phe Ile Ser Leu Ile Gly Gln
465                 470                 475                 480

Ser Leu Ser Trp Tyr Asn His Phe Tyr Val Ser Val Cys Leu Tyr Gly
                485                 490                 495

Thr Ala Thr Val Ala Lys Ile Ile Leu Ile His Thr Leu Ala Lys Arg
            500                 505                 510

Phe Tyr Tyr Met Asn Ala Ser Ala Gln Tyr Leu Gly Glu Val Phe Phe
        515                 520                 525

Asp Ile Ser Leu Phe Val His Cys Cys Phe Leu Val Thr Leu Thr Tyr
    530                 535                 540

Gln Gly Leu Cys Ser Ala Phe Ile Ser Ala Val Trp Val Ala Phe Pro
545                 550                 555                 560

Leu Leu Thr Lys Leu Cys Val His Lys Asp Phe Lys Gln His Gly Ala
                565                 570                 575

Gln Gly Lys Phe Ile Ala Phe Tyr Leu Leu Gly Met Phe Ile Pro Tyr
            580                 585                 590

Leu Tyr Ala Leu Tyr Leu Ile Trp Ala Val Phe Glu Met Phe Thr Pro
        595                 600                 605

Ile Leu Gly Arg Ser Gly Ser Glu Ile Pro Pro Asp Val Val Leu Ala
    610                 615                 620

Ser Ile Leu Ala Gly Cys Thr Met Ile Leu Ser Ser Tyr Phe Ile Asn
625                 630                 635                 640

Phe Ile Tyr Leu Ala Lys Ser Thr Lys Lys Thr Met Leu Thr Leu Thr
                645                 650                 655

Leu Val Cys Ala Ile Thr Phe Leu Leu Val Cys Ser Gly Thr Phe Phe
            660                 665                 670

Pro Tyr Ser Ser Asn Pro Ala Asn Pro Lys Pro Lys Arg Val Phe Leu
        675                 680                 685

Gln His Met Thr Arg Thr Phe His Asp Leu Glu Gly Asn Ala Val Lys
    690                 695                 700

Arg Asp Ser Gly Ile Trp Ile Asn Gly Phe Asp Tyr Thr Gly Ile Ser
705                 710                 715                 720

His Ile Thr Pro His Ile Pro Glu Ile Asn Asp Ser Ile Arg Ala His
                725                 730                 735

Cys Glu Glu Asn Ala Pro Leu Cys Gly Phe Pro Trp Tyr Leu Pro Val
            740                 745                 750

His Phe Leu Ile Arg Lys Asn Trp Tyr Leu Pro Ala Pro Glu Val Ser
        755                 760                 765

Pro Arg Asn Pro Pro His Phe Arg Leu Ile Ser Lys Glu Gln Thr Pro
    770                 775                 780

Trp Asp Ser Ile Lys Leu Thr Phe Glu Ala Thr Gly Pro Ser His Met
785                 790                 795                 800

Ser Phe Tyr Val Arg Ala His Lys Gly Ser Thr Leu Ser Gln Trp Ser
                805                 810                 815

Leu Gly Asn Gly Thr Pro Val Thr Ser Lys Gly Gly Asp Tyr Phe Val
            820                 825                 830

Phe Tyr Ser His Gly Leu Gln Ala Ser Ala Trp Gln Phe Trp Ile Glu
        835                 840                 845

Val Gln Val Ser Glu Glu His Pro Glu Gly Met Val Thr Val Ala Ile
    850                 855                 860

Ala Ala His Tyr Leu Ser Gly Glu Asp Lys Arg Ser Pro Gln Leu Asp
865                 870                 875                 880

Ala Leu Lys Glu Lys Phe Pro Asp Trp Thr Phe Pro Ser Ala Trp Val
                885                 890                 895

Cys Thr Tyr Asp Leu Phe Val Phe
            900

<210>  75
<211>  419
<212>  PRT
<213>  Homo sapiens

<400>  75

Met Val Val Met Gly Val Val Leu Tyr Leu Gly Lys Lys Phe Leu Gln
1               5                   10                  15

Pro Lys His Lys Thr Gly Asn Tyr Lys Lys Asp Phe Leu Cys Gly Leu
            20                  25                  30

Gly Ile Thr Leu Ile Ser Trp Phe Thr Ser Leu Val Thr Val Leu Ile
        35                  40                  45

Ile Ala Val Phe Ile Ser Leu Ile Gly Gln Ser Leu Ser Trp Tyr Asn
    50                  55                  60

His Phe Tyr Val Ser Val Cys Leu Tyr Gly Thr Ala Thr Val Ala Lys
65                  70                  75                  80

Ile Ile Leu Ile His Thr Leu Ala Lys Arg Phe Tyr Tyr Met Asn Ala
                85                  90                  95

Ser Ala Gln Tyr Leu Gly Glu Val Phe Phe Asp Ile Ser Leu Phe Val
            100                 105                 110

His Cys Cys Phe Leu Val Thr Leu Thr Tyr Gln Gly Leu Cys Ser Ala
        115                 120                 125

Phe Ile Ser Ala Val Trp Val Ala Phe Pro Leu Leu Thr Lys Leu Cys
    130                 135                 140

Val His Lys Asp Phe Lys Gln His Gly Ala Gln Gly Lys Phe Ile Ala
145                 150                 155                 160

Phe Tyr Leu Leu Gly Met Phe Ile Pro Tyr Leu Tyr Ala Leu Tyr Leu
                165                 170                 175

Ile Trp Ala Val Phe Glu Met Phe Thr Pro Ile Leu Gly Arg Ser Gly
            180                 185                 190

Ser Glu Ile Pro Pro Asp Val Val Leu Ala Ser Ile Leu Ala Gly Cys
        195                 200                 205

Thr Met Ile Leu Ser Ser Tyr Phe Ile Asn Phe Ile Tyr Leu Ala Lys
    210                 215                 220

Ser Thr Lys Lys Thr Met Leu Thr Leu Thr Leu Val Cys Ala Ile Thr
225                 230                 235                 240

Phe Leu Leu Val Cys Ser Gly Thr Phe Phe Pro Tyr Ser Ser Asn Pro
                245                 250                 255

Ala Asn Pro Lys Pro Lys Arg Val Phe Leu Gln His Met Thr Arg Thr
            260                 265                 270

Phe His Asp Leu Glu Gly Asn Ala Val Lys Arg Asp Ser Gly Ile Trp
        275                 280                 285

Ile Asn Gly Phe Asp Tyr Thr Gly Ile Ser His Ile Thr Pro His Ile
    290                 295                 300

Pro Glu Ile Asn Asp Ser Ile Arg Ala His Cys Glu Glu Asn Ala Pro
305                 310                 315                 320

Leu Cys Gly Phe Pro Trp Tyr Leu Pro Val His Phe Leu Ile Arg Lys
                325                 330                 335

Asn Trp Tyr Leu Pro Ala Pro Glu Val Ser Pro Arg Asn Pro Pro His
            340                 345                 350

Phe Arg Leu Ile Ser Lys Glu Gln Thr Pro Trp Asp Ser Ile Lys Leu
        355                 360                 365

Thr Phe Glu Ala Thr Ala Cys Leu Pro Ile Leu Gln Ile Leu Asp Leu
    370                 375                 380

Pro Ala Ser Thr Ile Met Thr Lys Pro Tyr Val Leu Leu Cys Ser Ser
385                 390                 395                 400

Pro Gln Arg Val Asn Thr Phe Ser Val Val Ser Trp Gln Trp His Pro
                405                 410                 415

Ser His Lys

<210>  76
<211>  4974
<212>  DNA
<213>  Homo sapiens

<400>  76
ggcgcgggga ccgggctgtc tgaggtgcgc gccgcgctgg ggctcgcgct ctacctgatc      60

gcgctgcgga cgctggtgca gctctcgctg cagcagctcg tgctacgcgg ggccgctgga     120

caccgcgggg agttcgacgc gctccaagcc agggattatc ttgaacacat aacctccatt 180

ggccccagga ctacaggaag tccagaaaat gaaattctga ccgtgcacta ccttttggaa 240

cagattaaac tgattgaagt gcaaagcaac agccttcata agatttcagt agatgtacaa 300

cggcccacag gctcttttag cattgatttc ttgggaggtt ttacaagcta ttatgacaac 360

atcaccaatg ttgtggtaaa gctggaaccc agagatggag cccagcatgc tgtcttggct 420

aattgtcatt ttgactcagt agcaaactca ccaggccagt catggtttca ttactcagca 480

cccctgggct agcttgattc gtgcattcat taacctagag gcagcaggtg taggagggaa 540

agaacttgta ttccaaacag gtcctgaaaa tccttggttg gttcaagctt atgtttcagc 600

agctaaacac ccttttgctt ctgtggtggc tcaggaggtt tttcagagtg gaatcattcc 660

ttcagatact gactttcgta tctacaggga ttttgggaac attccaggaa tagacttagc 720

ttttattgag aatggataca tttatcacac caagtatgac acagcggaca gaattctaac 780

agattccatt cagagagcag gtgacaacat tttagcagtt cttaagcatc tagctacatc 840

tgatatgctg gctgctgctt ctaagtatcg acatggaaac atggtcttct ttgatgtgct 900

gggcctgttt gtcattgcct acccctctcg tattggctca atcataaact acatggtggt 960

aatgggtgtt gttttgtacc tgggcaaaaa atttttgcag cccaaacata agactggtaa 1020

ctacaagaag gacttcttgt gtggacttgg catcactttg atcagctggt tcactagcct 1080

tgttaccgtt ctcattatag cagtgttcat ctctcttatt ggacagtctc tctcatggta 1140

taaccacttc tatgtctccg tttgtctgta tggaactgca actgtagcca aaataatact 1200

tatacatact cttgcgaaaa gattttatta catgaatgcc agtgcccagt atctgggaga 1260

agtatttttt gacatttcgc tgtttgtcca ttgctgtttt cttgttaccc tcacttacca 1320

aggactttgc tcggcgttta ttagtgctgt ctgggtagca ttcccattgc tcacaaagct 1380

ctgtgtgcat aaggacttca agcagcatgg tgcccaagga aaatttattg ctttttacct 1440

tttggggatg tttattcctt atctttatgc attgtacctc atctgggcag tatttgagat 1500

gtttacccct atcctcggga gaagtggttc tgaaatccca cctgatgttg tgctggcatc 1560

cattttggct ggctgtacaa tgattctctc gtcctatttt attaacttca tctaccttgc 1620

caagagcaca aaaaaaacca tgctaacttt aactttggta tgtgcaatta cattcctcct 1680

tgtttgcagt ggaacatttt ttccatatag ctccaatcct gctaatccga agccaaagag 1740

agtgtttctt cagcatatga ctagaacatt ccatgacttg gaaggaaatg cagttaaacg 1800

ggactctgga atatggatca atgggtttga ttatactgga atttctcaca taacccctca 1860

cattcctgag atcaatgata gtatccgagc tcactgtgag gagaatgcac ctctttgtgg 1920

ttttccttgg tatcttccag tgcactttct gatcaggaaa aactggtatc ttcctgcccc 1980

agaagtttct ccaagaaatc ctcctcattt ccgactcata tccaaagaac agacaccttg 2040

ggattctata aaattgactt ttgaagcaac aggaccaagc catatgtcct tctatgttcg 2100

agcccacaaa gggtcaacac tttctcagtg gtctcttggc aatggcaccc cagtcacaag 2160

taaaggagga gactactttg tcttttactc ccatggactc caggcctctg catggcagtt 2220

ctggatagaa gtgcaggttt cagaagaaca tcctgaagga atggtcaccg tggccattgc 2280

tgcccactat ctgtctgggg aagacaagag atcccctcaa ctggatgctc tgaaggaaaa 2340

gttcccagat tggacatttc cctctgcctg ggtgtgcacc tacgatctct ttgtatttta 2400

atcttgtgga tgagctctaa gtacatgccc agtggatact ccatgtgaca tggtttctcc 2460

ctatgttacg tggatgtttg taacgtaagt caatgaattt taatgatcat atgttcaaag 2520

agctttctgg gttaacgctt ttcagggcca agcactataa gggtttagct gtggcgcagt 2580

gatgcatggc ctgttgacac ttgaaaatgc cagtcttttg gcacttcagc acatgtgggt 2640

actgccacta cacacacgtc attttatatg accttaagga caaagccaac aatccacttc 2700

aatagctgcc cctttaggat caagaaagat gtacactgtc agagcattgt taatgagaca 2760

aaagttgttt ccaatttaag ccccaaaacc atttgttgta ttagtggatg gtgggtaaaa 2820

tatcattcac tgaggtaatg attccccttg agaatataac tctgtgtagg tcactggaaa 2880

gtgattgcca tagggctggg agagaagcat tgcactcttg aggctgtagc ctgtgtcaag 2940

ctgtttcttc aggcagcctc tcaaatgtgc tttgtctctc tgtgctgagg cctggaccct 3000

gtgctgagct ggtgactcac tgtcctgaca agtggacaca cagatgcact gctgtgctgc 3060

tttcctgagg tggttttcta tgcctgtttt cctctgaaac atgtctgtta cccctctcca 3120

tcttaccaag ttgaaaaggg gaatatttgg ccacataccc ctctggtttt cgtaggttct 3180

tttggttcag aatattgttt gtgccagtac atgaccttaa cttccttcct cagagcactg 3240

agctgccatc tgggctattc tggggtagaa ggaaggctgg gagtggtggg aattttataa 3300

atatttattc tcttttcttt gtttcatagg agtcttgtgt tatacaaggt tagtccttca 3360

tggtataatc ttactgatgc actgggccta tctttttgtt ttccagccag ttgaatagat 3420

tagtttttct cagtaactta ctatccagca gactggcttt cctgagactt gaggttgtgg 3480

cttatactgg aatgagacca ctgtacgtgt aggtggttca gatcctgcgt aatggcagca 3540

tgaggactta aaaggtggtt ttcattttga agatggctat gtagcttgta aggtgtatca 3600

cagcagtacc tctcatggct ttttggttcc agcagtgagg gcattggtga gatcaatggt 3660

aaactgtgca agctttcttt ttatcattag gaaatgtgaa acgttggaca aattttgagt 3720

tttaacaagg acaaaaagtt gaaagaaaag gcacagttaa caaaaaaggg tggctagatt 3780

tatcttgggt gatggaggaa atgagagagg aatgctcttg aaaggtggtc tgtggatctg 3840

tctgaataga aagagcacag taagtatgca ttgccggaga aaacgtcctt gaagctgctt 3900

gtctcatgtg tatgatgtgc tttttaaatc atgcccctcg ttgcctgcct aatctgtgac 3960

tccctaaaaa ctaactgggc ccatgtagat ggggctgcaa ccagagctga ataacatgtt 4020

aggctcacac atgcatcagc actgcacact ggaatcattg ctcttcctgg actttgtaga 4080

aatcagtctc aagtgcttca agagtctggc tcctgctact tttatctgtc aggtagcaca 4140

taaggtttgc agggtttata ttttgtatag aatcacagtt gtggagaaaa agtaataatt 4200

tctcaatgaa ttttaaaaat gggcctattt tctatccccg tggttcatct gatataatta 4260

gtgttccctg tgaattcccc ccctctatgg gaaggatgcc tttactcttt atcagtaata 4320

aattatgact gttttcatat tgccttaggg ttatttccct gtgtaaacca ttgtcttttg 4380

ttttggtttt ctttagcatt atgaagcttt ggtattgtac aaggtcagta gtaagatgct 4440

cactagtctc agggcttgtg taatattctg ggaggtcatt taaatgccag aaatggtcaa 4500

gcaattatac acagtattta tgactctgtt aagcataccg tttgtctgtc acattagtag 4560

attctgagat taaaaaaaat ttttaaagag tgatcattta aataatttct aaaagggtct 4620

tttcaagctc taacaaagtc actaacaaat gcattatttt ctacagaatt agatgttagt 4680

agtacagtac tgcatattca gggaaaaagt gtgaggaatt gatttcaaaa tagttcgttc 4740

ttgtgtttga cctaagaatg attgtcgcat gaagtgtttg tttttacagt ttagcatata 4800

taaacaaaca tgataggatt ccttaagatg ttaccaccca gggggccaca agccagcctg 4860

ctgtctcagg aagctgtaga aggagtgttt gtcaatttct tgtcactggt ttgctgactt 4920

actgaggatt aattgttgcc ttacaatgtt actgaaataa actgtttaat atac 4974

<210> 77
<211> 5338
<212> DNA
<213> Homo sapiens

<400> 77
ggccggggct gtcgcgggtt ggggcggttg ggctggcagc tgaggctcgt ggccatggag 60

tggggttctg agtcggctgc tgtgaggcgg caccgcgtcg gagtagagcg tcgagaggga 120

gcggcggccg cgccaccgcc ggagagggag gcccgagcgc aggagcctct ggtggatggg 180

tgcagcggcg gcgggaggac gcggaagagg agccccgggg gtagcggcgg cgcgagcagg 240

ggcgcgggga ccgggctgtc tgaggtgcgc gccgcgctgg ggctcgcgct ctacctgatc 300

gcgctgcgga cgctggtgca gctctcgctg cagcagctcg tgctacgcgg ggccgctgga 360

caccgcgggg agttcgacgc gctccaagcc agggattatc ttgaacacat aacctccatt 420

ggccccagga ctacaggaag tccagaaaat gaaattctga ccgtgcacta ccttttggaa 480

cagattaaac tgattgaagt gcaaagcaac agccttcata agatttcagt agatgtacaa 540

cggcccacag gctcttttag cattgatttc ttgggaggtt ttacaagcta ttatgacaac 600

atcaccaatg ttgtggtaaa gctggaaccc agagatggag cccagcatgc tgtcttggct 660
aattgtcatt ttgactcagt agcaaactca ccaggtgcca gtgatgatgc agttagctgc 720
tcagtgatgc tggaagtcct tcgcgtcttg tcaacatctt cagaagcctt gcatcatgct 780
gtcatatttc tctttaatgg tgctgaggaa aatgtcttgc aagccagtca tggtttcatt 840
actcagcacc cctgggctag cttgattcgt gcattcatta acctagaggc agcaggtgta 900
ggagggaaag aacttgtatt ccaaacaggt cctgaaaatc cttggttggt tcaagcttat 960
gtttcagcag ctaaacaccc ttttgcttct gtggtggctc aggaggtttt tcagagtgga 1020
atcattcctt cagatactga ctttcgtatc tacagggatt ttgggaacat tccaggaata 1080
gacttagctt ttattgagaa tggatacatt tatcacacca agtatgacac agcggacaga 1140
attctaacag attccattca gagagcaggt gacaacattt tagcagttct taagcatcta 1200
gctacatctg atatgctggc tgctgcttct aagtatcgac atggaaacat ggtcttcttt 1260
gatgtgctgg gcctgtttgt cattgcctac ccctctcgta ttggctcaat cataaactac 1320
atggtggtaa tgggtgttgt tttgtacctg ggcaaaaaat ttttgcagcc caaacataag 1380
actggtaact acaagaagga cttcttgtgt ggacttggca tcactttgat cagctggttc 1440
actagccttg ttaccgttct cattatagca gtgttcatct ctcttattgg acagtctctc 1500
tcatggtata accacttcta tgtctccgtt tgtctgtatg gaactgcaac tgtagccaaa 1560
ataatactta tacatactct tgcgaaaaga ttttattaca tgaatgccag tgcccagtat 1620
ctgggagaag tattttttga catttcgctg tttgtccatt gctgttttct tgttaccctc 1680
acttaccaag gactttgctc ggcgtttatt agtgctgtct gggtagcatt cccattgctc 1740
acaaagctct gtgtgcataa ggacttcaag cagcatggtg cccaaggaaa atttattgct 1800
ttttaccttt tggggatgtt tattccttat ctttatgcat tgtacctcat ctgggcagta 1860
tttgagatgt ttacccctat cctcgggaga agtggttctg aaatcccacc tgatgttgtg 1920
ctggcatcca ttttggctgg ctgtacaatg attctctcgt cctattttat taacttcatc 1980
taccttgcca agagcacaaa aaaaaccatg ctaactttaa ctttggtatg tgcaattaca 2040
ttcctccttg tttgcagtgg aacatttttt ccatatagct ccaatcctgc taatccgaag 2100
ccaaagagag tgtttcttca gcatatgact agaacattcc atgacttgga aggaaatgca 2160
gttaaacggg actctggaat atggatcaat gggtttgatt atactggaat ttctcacata 2220
acccctcaca ttcctgagat caatgatagt atccgagctc actgtgagga gaatgcacct 2280
ctttgtggtt ttccttggta tcttccagtg cactttctga tcaggaaaaa ctggtatctt 2340
cctgccccag aagtttctcc aagaaatcct cctcatttcc gactcatatc caaagaacag 2400
acaccttggg attctataaa attgactttt gaagcaacag gaccaagcca tatgtccttc 2460
tatgttcgag cccacaaagg gtcaacactt tctcagtggt ctcttggcaa tggcacccca 2520

gtcacaagta aaggaggaga ctactttgtc ttttactccc atggactcca ggcctctgca 2580

tggcagttct ggatagaagt gcaggtttca gaagaacatc ctgaaggaat ggtcaccgtg 2640

gccattgctg cccactatct gtctggggaa gacaagagat cccctcaact ggatgctctg 2700

aaggaaaagt tcccagattg gacatttccc tctgcctggg tgtgcaccta cgatctcttt 2760

gtattttaat cttgtggatg agctctaagt acatgcccag tggatactcc atgtgacatg 2820

gtttctccct atgttacgtg gatgtttgta acgtaagtca atgaatttta atgatcatat 2880

gttcaaagag ctttctgggt taacgctttt cagggccaag cactataagg gtttagctgt 2940

ggcgcagtga tgcatggcct gttgacactt gaaaatgcca gtcttttggc acttcagcac 3000

atgtgggtac tgccactaca cacacgtcat tttatatgac cttaaggaca aagccaacaa 3060

tccacttcaa tagctgcccc tttaggatca agaaagatgt acactgtcag agcattgtta 3120

atgagacaaa agttgtttcc aatttaagcc ccaaaaccat ttgttgtatt agtggatggt 3180

gggtaaaata tcattcactg aggtaatgat tccccttgag aatataactc tgtgtaggtc 3240

actggaaagt gattgccata gggctgggag agaagcattg cactcttgag gctgtagcct 3300

gtgtcaagct gtttcttcag gcagcctctc aaatgtgctt tgtctctctg tgctgaggcc 3360

tggaccctgt gctgagctgg tgactcactg tcctgacaag tggacacaca gatgcactgc 3420

tgtgctgctt tcctgaggtg gttttctatg cctgttttcc tctgaaacat gtctgttacc 3480

cctctccatc ttaccaagtt gaaaagggga atatttggcc acatacccct ctggttttcg 3540

taggttcttt tggttcagaa tattgtttgt gccagtacat gaccttaact tccttcctca 3600

gagcactgag ctgccatctg ggctattctg gggtagaagg aaggctggga gtggtgggaa 3660

ttttataaat atttattctc ttttctttgt ttcataggag tcttgtgtta tacaaggtta 3720

gtccttcatg gtataatctt actgatgcac tgggcctatc tttttgtttt ccagccagtt 3780

gaatagatta gtttttctca gtaacttact atccagcaga ctggctttcc tgagacttga 3840

ggttgtggct tatactggaa tgagaccact gtacgtgtag gtggttcaga tcctgcgtaa 3900

tggcagcatg aggacttaaa aggtggtttt cattttgaag atggctatgt agcttgtaag 3960

gtgtatcaca gcagtacctc tcatggcttt ttggttccag cagtgagggc attggtgaga 4020

tcaatggtaa actgtgcaag ctttcttttt atcattagga aatgtgaaac gttggacaaa 4080

ttttgagttt taacaaggac aaaaagttga aagaaaaggc acagttaaca aaaaagggtg 4140

gctagattta tcttgggtga tggaggaaat gagagaggaa tgctcttgaa aggtggtctg 4200

tggatctgtc tgaatagaaa gagcacagta agtatgcatt gccggagaaa acgtccttga 4260

agctgcttgt ctcatgtgta tgatgtgctt tttaaatcat gcccctcgtt gcctgcctaa 4320

tctgtgactc cctaaaaact aactgggccc atgtagatgg ggctgcaacc agagctgaat 4380

aacatgttag gctcacacat gcatcagcac tgcacactgg aatcattgct cttcctggac 4440

tttgtagaaa tcagtctcaa gtgcttcaag agtctggctc ctgctacttt tatctgtcag 4500

gtagcacata aggtttgcag ggtttatatt ttgtatagaa tcacagttgt ggagaaaaag 4560

taataatttc tcaatgaatt ttaaaaatgg gcctattttc tatccccgtg gttcatctga 4620

tataattagt gttccctgtg aattcccccc ctctatggga aggatgcctt tactctttat 4680

cagtaataaa ttatgactgt tttcatattg ccttagggtt atttccctgt gtaaaccatt 4740

gtcttttgtt ttggttttct ttagcattat gaagctttgg tattgtacaa ggtcagtagt 4800

aagatgctca ctagtctcag ggcttgtgta atattctggg aggtcattta aatgccagaa 4860

atggtcaagc aattatacac agtatttatg actctgttaa gcataccgtt tgtctgtcac 4920

attagtagat tctgagatta aaaaaaattt ttaaagagtg atcatttaaa taatttctaa 4980

aagggtcttt tcaagctcta acaaagtcac taacaaatgc attattttct acagaattag 5040

atgttagtag tacagtactg catattcagg gaaaaagtgt gaggaattga tttcaaaata 5100

gttcgttctt gtgtttgacc taagaatgat tgtcgcatga agtgtttgtt tttacagttt 5160

agcatatata aacaaacatg ataggattcc ttaagatgtt accacccagg gggccacaag 5220

ccagcctgct gtctcaggaa gctgtagaag gagtgtttgt caatttcttg tcactggttt 5280

gctgacttac tgaggattaa ttgttgcctt acaatgttac tgaaataaac tgtttaat 5338

<210> 78
<211> 5387
<212> DNA
<213> Homo sapiens

<400> 78
   ggccggggct gtcgcgggtt ggggcggttg ggctggcagc tgaggctcgt ggccatggag 60
   tggggttctg agtcggctgc tgtgaggcgg caccgcgtcg gagtagagcg tcgagaggga 120
   gcggcggccg cgccaccgcc ggagagggag gcccgagcgc aggagcctct ggtggatggg 180
   tgcagcggcg gcgggaggac gcggaagagg agccccgggg gtagcggcgg cgcgagcagg 240
   ggcgcgggga ccgggctgtc tgaggtgcgc gccgcgctgg ggctcgcgct ctacctgatc 300
   gcgctgcgga cgctggtgca gctctcgctg cagcagctcg tgctacgcgg ggccgctgga 360
   caccgcgggg agttcgacgc gctccaagcc agggattatc ttgaacacat aacctccatt 420
   ggccccagga ctacaggaag tccagaaaat gaaattctga ccgtgcacta ccttttggaa 480
   cagattaaac tgattgaagt gcaaagcaac agccttcata agatttcagt agatgtacaa 540
   cggcccacag gctcttttag cattgatttc ttgggaggtt ttacaagcta ttatgacaac 600
   atcaccaatg ttgtggtaaa gctggaaccc agagatggag cccagcatgc tgtcttggct 660
   aattgtcatt ttgactcagt agcaaactca ccaggtgcca gtgatgatgc agttagctgc 720
   tcagtgatgc tggaagtcct tcgcgtcttg tcaacatctt cagaagcctt gcatcatgct 780
   gtcatatttc tctttaatgg tgctgaggaa aatgtcttgc aagccagtca tggtttcatt 840
   actcagcacc cctgggctag cttgattcgt gcattcatta acctagaggc agcaggtgta 900
   ggagggaaag aacttgtatt ccaaacaggt cctgaaaatc cttggttggt tcaagcttat 960
   gtttcagcag ctaaacaccc ttttgcttct gtggtggctc aggaggtttt tcagagtgga 1020
   atcattcctt cagatactga ctttcgtatc tacagggatt ttgggaacat tccaggaata 1080
   gacttagctt ttattgagaa tggatacatt tatcacacca agtatgacac agcggacaga 1140
   attctaacag attccattca gagagcaggt gacaacattt tagcagttct taagcatcta 1200
   gctacatctg atatgctggc tgctgcttct aagtatcgac atggaaacat ggtcttcttt 1260
   gatgtgctgg gcctgtttgt cattgcctac ccctctcgta ttggctcaat cataaactac 1320
   atggtggtaa tgggtgttgt tttgtacctg ggcaaaaaat ttttgcagcc caaacataag 1380
   actggtaact acaagaagga cttcttgtgt ggacttggca tcactttgat cagctggttc 1440
   actagccttg ttaccgttct cattatagca gtgttcatct ctcttattgg acagtctctc 1500
   tcatggtata accacttcta tgtctccgtt tgtctgtatg gaactgcaac tgtagccaaa 1560
   ataatactta tacatactct tgcgaaaaga ttttattaca tgaatgccag tgcccagtat 1620
   ctgggagaag tattttttga catttcgctg tttgtccatt gctgttttct tgttaccctc 1680
   acttaccaag gactttgctc ggcgtttatt agtgctgtct gggtagcatt cccattgctc 1740
   acaaagctct gtgtgcataa ggacttcaag cagcatggtg cccaaggaaa atttattgct 1800
   ttttaccttt tggggatgtt tattccttat ctttatgcat tgtacctcat ctgggcagta 1860
   tttgagatgt ttacccctat cctcgggaga agtggttctg aaatcccacc tgatgttgtg 1920
   ctggcatcca ttttggctgg ctgtacaatg attctctcgt cctattttat taacttcatc 1980
   taccttgcca agagcacaaa aaaaaccatg ctaactttaa ctttggtatg tgcaattaca 2040
   ttcctccttg tttgcagtgg aacatttttt ccatatagct ccaatcctgc taatccgaag 2100
   ccaaagagag tgtttcttca gcatatgact agaacattcc atgacttgga aggaaatgca 2160
   gttaaacggg actctggaat atggatcaat gggtttgatt atactggaat ttctcacata 2220
   acccctcaca ttcctgagat caatgatagt atccgagctc actgtgagga gaatgcacct 2280
   ctttgtggtt ttccttggta tcttccagtg cactttctga tcaggaaaaa ctggtatctt 2340
   cctgccccag aagtttctcc aagaaatcct cctcatttcc gactcatatc caaagaacag 2400
   acaccttggg attctataaa attgactttt gaagcaacag cctgcctgcc tatccttcag 2460
   attttggact tgccagcctc aacaatcatg accaagccat atgtccttct atgttcgagc 2520
   ccacaaaggg tcaacacttt ctcagtggtc tcttggcaat ggcaccccag tcacaagtaa 2580
   aggaggagac tactttgtct tttactccca tggactccag gcctctgcat ggcagttctg 2640
   gatagaagtg caggtttcag aagaacatcc tgaaggaatg gtcaccgtgg ccattgctgc 2700
   ccactatctg tctggggaag acaagagatc ccctcaactg gatgctctga aggaaaagtt 2760
   cccagattgg acatttccct ctgcctgggt gtgcacctac gatctctttg tattttaatc 2820
   ttgtggatga gctctaagta catgcccagt ggatactcca tgtgacatgg tttctcccta 2880
   tgttacgtgg atgtttgtaa cgtaagtcaa tgaattttaa tgatcatatg ttcaaagagc 2940
   tttctgggtt aacgcttttc agggccaagc actataaggg tttagctgtg gcgcagtgat 3000
   gcatggcctg ttgacacttg aaaatgccag tcttttggca cttcagcaca tgtgggtact 3060
   gccactacac acacgtcatt ttatatgacc ttaaggacaa agccaacaat ccacttcaat 3120
   agctgcccct ttaggatcaa gaaagatgta cactgtcaga gcattgttaa tgagacaaaa 3180
   gttgtttcca atttaagccc caaaaccatt tgttgtatta gtggatggtg ggtaaaatat 3240
   cattcactga ggtaatgatt ccccttgaga atataactct gtgtaggtca ctggaaagtg 3300
   attgccatag ggctgggaga gaagcattgc actcttgagg ctgtagcctg tgtcaagctg 3360
   tttcttcagg cagcctctca aatgtgcttt gtctctctgt gctgaggcct ggaccctgtg 3420
   ctgagctggt gactcactgt cctgacaagt ggacacacag atgcactgct gtgctgcttt 3480
   cctgaggtgg ttttctatgc ctgttttcct ctgaaacatg tctgttaccc ctctccatct 3540
   taccaagttg aaaaggggaa tatttggcca catacccctc tggttttcgt aggttctttt 3600
   ggttcagaat attgtttgtg ccagtacatg accttaactt ccttcctcag agcactgagc 3660
   tgccatctgg gctattctgg ggtagaagga aggctgggag tggtgggaat tttataaata 3720
   tttattctct tttctttgtt tcataggagt cttgtgttat acaaggttag tccttcatgg 3780
   tataatctta ctgatgcact gggcctatct ttttgttttc cagccagttg aatagattag 3840
   tttttctcag taacttacta tccagcagac tggctttcct gagacttgag gttgtggctt 3900
   atactggaat gagaccactg tacgtgtagg tggttcagat cctgcgtaat ggcagcatga 3960
   ggacttaaaa ggtggttttc attttgaaga tggctatgta gcttgtaagg tgtatcacag 4020
   cagtacctct catggctttt tggttccagc agtgagggca ttggtgagat caatggtaaa 4080
   ctgtgcaagc tttcttttta tcattaggaa atgtgaaacg ttggacaaat tttgagtttt 4140
   aacaaggaca aaaagttgaa agaaaaggca cagttaacaa aaaagggtgg ctagatttat 4200
   cttgggtgat ggaggaaatg agagaggaat gctcttgaaa ggtggtctgt ggatctgtct 4260
   gaatagaaag agcacagtaa gtatgcattg ccggagaaaa cgtccttgaa gctgcttgtc 4320
   tcatgtgtat gatgtgcttt ttaaatcatg cccctcgttg cctgcctaat ctgtgactcc 4380
   ctaaaaacta actgggccca tgtagatggg gctgcaacca gagctgaata acatgttagg 4440
   ctcacacatg catcagcact gcacactgga atcattgctc ttcctggact ttgtagaaat 4500
   cagtctcaag tgcttcaaga gtctggctcc tgctactttt atctgtcagg tagcacataa 4560
   ggtttgcagg gtttatattt tgtatagaat cacagttgtg gagaaaaagt aataatttct 4620
   caatgaattt taaaaatggg cctattttct atccccgtgg ttcatctgat ataattagtg 4680
   ttccctgtga attccccccc tctatgggaa ggatgccttt actctttatc agtaataaat 4740
   tatgactgtt ttcatattgc cttagggtta tttccctgtg taaaccattg tcttttgttt 4800
   tggttttctt tagcattatg aagctttggt attgtacaag gtcagtagta agatgctcac 4860
   tagtctcagg gcttgtgtaa tattctggga ggtcatttaa atgccagaaa tggtcaagca 4920
   attatacaca gtatttatga ctctgttaag cataccgttt gtctgtcaca ttagtagatt 4980
   ctgagattaa aaaaaatttt taaagagtga tcatttaaat aatttctaaa agggtctttt 5040
   caagctctaa caaagtcact aacaaatgca ttattttcta cagaattaga tgttagtagt 5100
   acagtactgc atattcaggg aaaaagtgtg aggaattgat ttcaaaatag ttcgttcttg 5160
   tgtttgacct aagaatgatt gtcgcatgaa gtgtttgttt ttacagttta gcatatataa 5220
   acaaacatga taggattcct taagatgtta ccacccaggg ggccacaagc cagcctgctg 5280
   tctcaggaag ctgtagaagg agtgtttgtc aatttcttgt cactggtttg ctgacttact 5340
   gaggattaat tgttgcctta caatgttact gaaataaact gtttaat 5387

<210> 79
<211> 245
<212> PRT
<213> Homo sapiens

<400> 79

Met Thr Leu Phe Pro Val Leu Leu Phe Leu Val Ala Gly Leu Leu Pro
1               5                   10                  15

Ser Phe Pro Ala Asn Glu Asp Lys Asp Pro Ala Phe Thr Ala Leu Leu
            20                  25                  30

Thr Thr Gln Thr Gln Val Gln Arg Glu Ile Val Asn Lys His Asn Glu
        35                  40                  45

Leu Arg Arg Ala Val Ser Pro Pro Ala Arg Asn Met Leu Lys Met Glu
    50                  55                  60

Trp Asn Lys Glu Ala Ala Ala Asn Ala Gln Lys Trp Ala Asn Gln Cys
65                  70                  75                  80

Asn Tyr Arg His Ser Asn Pro Lys Asp Arg Met Thr Ser Leu Lys Cys
                85                  90                  95

Gly Glu Asn Leu Tyr Met Ser Ser Ala Ser Ser Ser Trp Ser Gln Ala

            100                 105                 110

Ile Gln Ser Trp Phe Asp Glu Tyr Asn Asp Phe Asp Phe Gly Val Gly
        115                 120                 125

Pro Lys Thr Pro Asn Ala Val Val Gly His Tyr Thr Gln Val Val Trp
    130                 135                 140

Tyr Ser Ser Tyr Leu Val Gly Cys Gly Asn Ala Tyr Cys Pro Asn Gln
145                 150                 155                 160

Lys Val Leu Lys Tyr Tyr Tyr Val Cys Gln Tyr Cys Pro Ala Gly Asn
                165                 170                 175

Trp Ala Asn Arg Leu Tyr Val Pro Tyr Glu Gln Gly Ala Pro Cys Ala
            180                 185                 190

Ser Cys Pro Asp Asn Cys Asp Asp Gly Leu Cys Thr Asn Gly Cys Lys
        195                 200                 205

Tyr Glu Asp Leu Tyr Ser Asn Cys Lys Ser Leu Lys Leu Thr Leu Thr
    210                 215                 220

Cys Lys His Gln Leu Val Arg Asp Ser Cys Lys Ala Ser Cys Asn Cys
225                 230                 235                 240

Ser Asn Ser Ile Tyr
                245

<210> 80
<211> 245
<212> PRT
<213> Homo sapiens

<400> 80

Met Thr Leu Phe Pro Val Leu Leu Phe Leu Val Ala Gly Leu Leu Pro
1               5                   10                  15

Ser Phe Pro Ala Asn Glu Asp Lys Asp Pro Ala Phe Thr Ala Leu Leu
            20                  25                  30

Thr Thr Gln Thr Gln Val Gln Arg Glu Ile Val Asn Lys His Asn Glu
        35                  40                  45

Leu Arg Arg Ala Val Ser Pro Pro Ala Arg Asn Met Leu Lys Met Glu
    50                  55                  60

Trp Asn Lys Glu Ala Ala Ala Asn Ala Gln Lys Trp Ala Asn Gln Cys

65                  70                  75                  80

Asn Tyr Arg His Ser Asn Pro Lys Asp Arg Met Thr Ser Leu Lys Cys
                85                  90                  95

Gly Glu Asn Leu Tyr Met Ser Ser Ala Ser Ser Ser Trp Ser Gln Ala
            100                 105                 110

Ile Gln Ser Trp Phe Asp Glu Tyr Asn Asp Phe Asp Phe Gly Val Gly
        115                 120                 125

Pro Lys Thr Pro Asn Ala Val Val Gly His Tyr Thr Gln Val Val Trp
    130                 135                 140

Tyr Ser Ser Tyr Leu Val Gly Cys Gly Asn Ala Tyr Cys Pro Asn Gln
145                 150                 155                 160

Lys Val Leu Lys Tyr Tyr Tyr Val Cys Gln Tyr Cys Pro Ala Gly Asn
                165                 170                 175

Trp Ala Asn Arg Leu Tyr Val Pro Tyr Glu Gln Gly Ala Pro Cys Ala
            180                 185                 190

Ser Cys Pro Asp Asn Cys Asp Asp Gly Leu Cys Thr Asn Gly Cys Lys
        195                 200                 205

Tyr Glu Asp Leu Tyr Ser Asn Cys Lys Ser Leu Lys Leu Thr Leu Thr
    210                 215                 220

Cys Lys His Gln Leu Val Arg Asp Ser Cys Lys Ala Ser Cys Asn Cys
225                 230                 235                 240

Ser Asn Ser Ile Tyr
                245

<210>  81
<211>  245
<212>  PRT
<213>  Homo sapiens

<400>  81

Met Thr Leu Phe Pro Val Leu Leu Phe Leu Val Ala Gly Leu Leu Pro
1               5                   10                  15

Ser Phe Pro Ala Asn Glu Asp Lys Asp Pro Ala Phe Thr Ala Leu Leu
            20                  25                  30

Thr Thr Gln Thr Gln Val Gln Arg Glu Ile Val Asn Lys His Asn Glu

35                  40                  45

Leu Arg Arg Ala Val Ser Pro Pro Ala Arg Asn Met Leu Lys Met Glu
    50                  55                  60

Trp Asn Lys Glu Ala Ala Ala Asn Ala Gln Lys Trp Ala Asn Gln Cys
65                  70                  75                  80

Asn Tyr Arg His Ser Asn Pro Lys Asp Arg Met Thr Ser Leu Lys Cys
                85                  90                  95

Gly Glu Asn Leu Tyr Met Ser Ser Ala Ser Ser Ser Trp Ser Gln Ala
            100                 105                 110

Ile Gln Ser Trp Phe Asp Glu Tyr Asn Asp Phe Asp Phe Gly Val Gly
        115                 120                 125

Pro Lys Thr Pro Asn Ala Val Val Gly His Tyr Thr Gln Val Val Trp
    130                 135                 140

Tyr Ser Ser Tyr Leu Val Gly Cys Gly Asn Ala Tyr Cys Pro Asn Gln
145                 150                 155                 160

Lys Val Leu Lys Tyr Tyr Tyr Val Cys Gln Tyr Cys Pro Ala Gly Asn
                165                 170                 175

Trp Ala Asn Arg Leu Tyr Val Pro Tyr Glu Gln Gly Ala Pro Cys Ala
            180                 185                 190

Ser Cys Pro Asp Asn Cys Asp Asp Gly Leu Cys Thr Asn Gly Cys Lys
        195                 200                 205

Tyr Glu Asp Leu Tyr Ser Asn Cys Lys Ser Leu Lys Leu Thr Leu Thr
    210                 215                 220

Cys Lys His Gln Leu Val Arg Asp Ser Cys Lys Ala Ser Cys Asn Cys
225                 230                 235                 240

Ser Asn Ser Ile Tyr
                245

<210>  82
<211>  2073
<212>  DNA
<213>  Homo sapiens

<400>  82
cccagcaatg acattattcc cagtgctgtt gttcctggtt gctgggctgc ttccatcttt      60

tccagcaaat gaagataagg atcccgcttt tactgctttg ttaaccaccc aaacacaagt 120

gcaaagggag attgtgaata agcacaatga actgaggaga gcagtatctc cccctgccag 180

aaacatgctg aagatggaat ggaacaaaga ggctgcagca aatgcccaaa agtgggcaaa 240

ccagtgcaat tacagacaca gtaacccaaa ggatcgaatg acaagtctaa aatgtggtga 300

gaatctctac atgtcaagtg cctccagctc atggtcacaa gcaatccaaa gctggtttga 360

tgagtacaat gattttgact ttggtgtagg gccaaagact cccaacgcag tggttggaca 420

ttatacacag gttgtttggt actcttcata cctcgttgga tgtggaaatg cctactgtcc 480

caatcaaaaa gttctaaaat actactatgt ttgccaatat tgtcctgctg gtaattgggc 540

taatagacta tatgtccctt atgaacaagg agcaccttgt gccagttgcc cagataactg 600

tgacgatgga ctatgcacca atggttgcaa gtacgaagat ctctatagta actgtaaaag 660

tttgaagctc acattaacct gtaaacatca gttggtcagg gacagttgca aggcctcctg 720

caattgttca aacagcattt attaaatacg cattacacac cgagtagggc tatgtagaga 780

ggagtcagat tatctactta gatttggcat ctacttagat ttaacatata ctagctgaga 840

aattgtaggc atgtttgata cacatttgat ttcaaatgtt tttcttctgg atctgctttt 900

tattttacaa aaatattttt catacaaatg gttaaaaaga aacaaaatct ataacaacaa 960

ctttggattt ttatatataa actttgtgat ttaaatttac tgaatttaat tagggtgaaa 1020

attttgaaag ttgtattctc atatgactaa gttcactaaa accctggatt gaaagtgaaa 1080

attatgttcc tagaacaaaa tgtacaaaaa gaacaatata attttcacat gaacccttgg 1140

ctgtagttgc ctttcctagc tccactctaa ggctaagcat cttcaaagac gttttcccat 1200

atgctgtctt aattcttttc actcattcac ccttcttccc aatcatctgg ctggcatcct 1260

cacaattgag ttgaagctgt tcctcctaaa acaatcctga cttttatttt gccaaaatca 1320

atacaatcct ttgaattttt tatctgcata aattttacag tagaatatga tcaaaccttc 1380

atttttaaac ctctcttctc tttgacaaaa cttccttaaa aaagaataca agataatata 1440

ggtaaatacc ctccactcaa ggaggtagaa ctcagtcctc tcccttgtga gtcttcacta 1500

aaatcagtga ctcacttcca aagagtggag tatggaaagg gaaacatagt aactttacag 1560

gggagaaaaa tgacaaatga cgtcttcacc aagtgatcaa aattaacgtc accagtgata 1620

agtcattcag atttgttcta gataatcttt ctaaaaattc ataatcccaa tctaattatg 1680

agctaaaaca tccagcaaac tcaagttgaa ggacattcta caaaatatcc ctggggtatt 1740

ttagagtatt cctcaaaact gtaaaaatca tggaaaataa gggaatcctg agaaacaatc 1800

acagaccaca tgagactaag gagacatgtg agccaaatgc aatgtgcttc ttggatcaga 1860

tcctggaaca gaaaaagatc agtaatgaaa aaactgatga agtctgaata gaatctggag 1920

tatttttaac agtagtgttg atttcttaat cttgataaat atagcagggt aatgtaagat 1980

gataacgtta gagaaactga aactgggtga gggctatcta ggaattctct gtactatctt 2040

accaaatttt cggtaagtct aagaaagcaa tgc 2073

<210> 83
<211> 2081
<212> DNA
<213> Homo sapiens

<400> 83
ctggaaacca ctgcaatgac attattccca gtgctgttgt tcctggttgc tgggctgctt 60

ccatcttttc cagcaaatga agataaggat cccgctttta ctgctttgtt aaccacccaa 120

acacaagtgc aaagggagat tgtgaataag cacaatgaac tgaggagagc agtatctccc 180

cctgccagaa acatgctgaa gatggaatgg aacaaagagg ctgcagcaaa tgcccaaaag 240

tgggcaaacc agtgcaatta cagacacagt aacccaaagg atcgaatgac aagtctaaaa 300

tgtggtgaga atctctacat gtcaagtgcc tccagctcat ggtcacaagc aatccaaagc 360

tggtttgatg agtacaatga ttttgacttt ggtgtagggc caaagactcc caacgcagtg 420

gttggacatt atacacaggt tgtttggtac tcttcatacc tcgttggatg tggaaatgcc 480

tactgtccca atcaaaaagt tctaaaatac tactatgttt gccaatattg tcctgctggt 540

aattgggcta atagactata tgtcccttat gaacaaggag caccttgtgc cagttgccca 600

gataactgtg acgatggact atgcaccaat ggttgcaagt acgaagatct ctatagtaac 660

tgtaaaagtt tgaagctcac attaacctgt aaacatcagt tggtcaggga cagttgcaag 720

gcctcctgca attgttcaaa cagcatttat taaatacgca ttacacaccg agtagggcta 780

tgtagagagg agtcagatta tctacttaga tttggcatct acttagattt aacatatact 840

agctgagaaa ttgtaggcat gtttgataca catttgattt caaatgtttt tcttctggat 900

ctgcttttta ttttacaaaa atatttttca tacaaatggt taaaaagaaa caaaatctat 960

aacaacaact ttggattttt atatataaac tttgtgattt aaatttactg aatttaatta 1020

gggtgaaaat tttgaaagtt gtattctcat atgactaagt tcactaaaac cctggattga 1080

aagtgaaaat tatgttccta gaacaaaatg tacaaaaaga acaatataat tttcacatga 1140

acccttggct gtagttgcct ttcctagctc cactctaagg ctaagcatct tcaaagacgt 1200

tttcccatat gctgtcttaa ttcttttcac tcattcaccc ttcttcccaa tcatctggct 1260

ggcatcctca caattgagtt gaagctgttc ctcctaaaac aatcctgact tttattttgc 1320

caaaatcaat acaatccttt gaatttttta tctgcataaa ttttacagta gaatatgatc 1380

aaaccttcat ttttaaacct ctcttctctt tgacaaaact tccttaaaaa agaatacaag 1440

ataatatagg taaataccct ccactcaagg aggtagaact cagtcctctc ccttgtgagt 1500

cttcactaaa atcagtgact cacttccaaa gagtggagta tggaaaggga aacatagtaa 1560

ctttacaggg gagaaaaatg acaaatgacg tcttcaccaa gtgatcaaaa ttaacgtcac 1620

cagtgataag tcattcagat ttgttctaga taatctttct aaaaattcat aatcccaatc 1680

taattatgag ctaaaacatc cagcaaactc aagttgaagg acattctaca aaatatccct 1740

ggggtatttt agagtattcc tcaaaactgt aaaaatcatg gaaaataagg gaatcctgag 1800

aaacaatcac agaccacatg agactaagga gacatgtgag ccaaatgcaa tgtgcttctt 1860

ggatcagatc ctggaacaga aaaagatcag taatgaaaaa actgatgaag tctgaataga 1920

atctggagta tttttaacag tagtgttgat ttcttaatct tgataaatat agcagggtaa 1980

tgtaagatga taacgttaga gaaactgaaa ctgggtgagg gctatctagg aattctctgt 2040

actatcttac caaattttcg gtaagtctaa gaaagcaatg c 2081

<210> 84
<211> 2122
<212> DNA
<213> Homo sapiens

<400> 84
gatgaaacaa atacttcatc ctgctctgga aaccactgat gacattattc ccagtgctgt 60

tgttcctggt tgctgggctg cttccatctt ttccagcaaa tgaagataag gatcccgctt 120

ttactgcttt gttaaccacc caaacacaag tgcaaaggga gattgtgaat aagcacaatg 180

aactgaggag agcagtatct ccccctgcca gaaacatgct gaagatggaa tggaacaaag 240

aggctgcagc aaatgcccaa aagtgggcaa accagtgcaa ttacagacac agtaacccaa 300

aggatcgaat gacaagtcta aaatgtggtg agaatctcta catgtcaagt gcctccagct 360

catggtcaca agcaatccaa agctggtttg atgagtacaa tgattttgac tttggtgtag 420

ggccaaagac tcccaacgca gtggttggac attatacaca ggttgtttgg tactcttcat 480

acctcgttgg atgtggaaat gcctactgtc ccaatcaaaa agttctaaaa tactactatg 540

tttgccaata ttgtcctgct ggtaattggg ctaatagact atatgtccct tatgaacaag 600

gagcaccttg tgccagttgc ccagataact gtgacgatgg actatgcacc aatggttgca 660

agtacgaaga tctctatagt aactgtaaaa gtttgaagct cacattaacc tgtaaacatc 720

agttggtcag ggacagttgc aaggcctcct gcaattgttc aaacagcatt tattaaatac 780

gcattacaca ccgagtaggg ctatgtagag aggagtcaga ttatctactt agatttggca 840

tctacttaga tttaacatat actagctgag aaattgtagg catgtttgat acacatttga 900

tttcaaatgt ttttcttctg gatctgcttt ttattttaca aaaatatttt tcatacaaat 960

ggttaaaaag aaacaaaatc tataacaaca actttggatt tttatatata aactttgtga 1020

tttaaattta ctgaatttaa ttagggtgaa aattttgaaa gttgtattct catatgacta 1080

agttcactaa aaccctggat tgaaagtgaa aattatgttc ctagaacaaa atgtacaaaa 1140

agaacaatat aattttcaca tgaacccttg gctgtagttg cctttcctag ctccactcta 1200

aggctaagca tcttcaaaga cgttttccca tatgctgtct taattctttt cactcattca 1260

cccttcttcc caatcatctg gctggcatcc tcacaattga gttgaagctg ttcctcctaa 1320

aacaatcctg acttttattt tgccaaaatc aatacaatcc tttgaatttt ttatctgcat 1380

aaattttaca gtagaatatg atcaaacctt catttttaaa cctctcttct ctttgacaaa 1440

acttccttaa aaaagaatac aagataatat aggtaaatac cctccactca aggaggtaga 1500

actcagtcct ctcccttgtg agtcttcact aaaatcagtg actcacttcc aaagagtgga 1560

gtatggaaag ggaaacatag taactttaca ggggagaaaa atgacaaatg acgtcttcac 1620

caagtgatca aaattaacgt caccagtgat aagtcattca gatttgttct agataatctt 1680

tctaaaaatt cataatccca atctaattat gagctaaaac atccagcaaa ctcaagttga 1740

aggacattct acaaaatatc cctggggtat tttagagtat tcctcaaaac tgtaaaaatc 1800

atggaaaata agggaatcct gagaaacaat cacagaccac atgagactaa ggagacatgt 1860

gagccaaatg caatgtgctt cttggatcag atcctggaac agaaaaagat cagtaatgaa 1920

aaaactgatg aagtctgaat agaatctgga gtatttttaa cagtagtgtt gatttcttaa 1980

tcttgataaa tatagcaggg taatgtaaga tgataacgtt agagaaactg aaactgggtg 2040

agggctatct aggaattctc tgtactatct taccaaattt tcggtaagtc taagaaagca 2100

atgcaaaata aaaagtgtct tg 2122

<210> 85
<211> 409
<212> PRT
<213> Homo sapiens

<400> 85

Met Glu Glu Ser Trp Glu Ala Ala Pro Gly Gly Gln Ala Gly Ala Glu
1               5                   10                  15

Leu Pro Met Glu Pro Val Gly Ser Leu Val Pro Thr Leu Glu Gln Pro
            20                  25                  30

Gln Val Pro Ala Lys Val Arg Gln Pro Glu Gly Pro Glu Ser Ser Pro
        35                  40                  45

Ser Pro Ala Gly Ala Val Glu Lys Ala Ala Gly Ala Gly Leu Glu Pro
    50                  55                  60

Ser Ser Lys Lys Lys Pro Pro Ser Pro Arg Pro Gly Ser Pro Arg Val
65                  70                  75                  80

Pro Pro Leu Ser Leu Gly Tyr Gly Val Cys Pro Glu Pro Pro Ser Pro
                85                  90                  95

Gly Pro Ala Leu Val Lys Leu Pro Arg Asn Gly Glu Ala Pro Gly Ala
            100                 105                 110

Glu Pro Ala Pro Ser Ala Trp Ala Pro Met Glu Leu Gln Val Asp Val
        115                 120                 125

Arg Val Lys Pro Val Gly Ala Ala Gly Gly Ser Ser Thr Pro Ser Pro
    130                 135                 140

Arg Pro Ser Thr Arg Phe Leu Lys Val Pro Val Pro Glu Ser Pro Ala
145                 150                 155                 160

Phe Ser Arg His Ala Asp Pro Ala His Gln Leu Leu Leu Arg Ala Pro
                165                 170                 175

Ser Gln Gly Gly Thr Trp Gly Arg Arg Ser Pro Leu Ala Ala Ala Arg
            180                 185                 190

Thr Glu Ser Gly Cys Asp Ala Glu Gly Arg Ala Ser Pro Ala Glu Gly
        195                 200                 205

Ser Ala Gly Ser Pro Gly Ser Pro Thr Cys Cys Arg Cys Lys Glu Leu
    210                 215                 220

Gly Leu Glu Lys Glu Asp Ala Ala Leu Leu Pro Arg Ala Gly Leu Asp
225                 230                 235                 240

Gly Asp Glu Lys Leu Pro Arg Ala Val Thr Leu Thr Gly Leu Pro Met
                245                 250                 255

Tyr Val Lys Ser Leu Tyr Trp Ala Leu Ala Phe Met Ala Val Leu Leu
            260                 265                 270

Ala Val Ser Gly Val Val Ile Val Val Leu Ala Ser Arg Ala Gly Ala
        275                 280                 285

Arg Cys Gln Gln Cys Pro Pro Gly Trp Val Leu Ser Glu Glu His Cys
    290                 295                 300

Tyr Tyr Phe Ser Ala Glu Ala Gln Ala Trp Glu Ala Ser Gln Ala Phe
305                 310                 315                 320

Cys Ser Ala Tyr His Ala Thr Leu Pro Leu Leu Ser His Thr Gln Asp
                325                 330                 335

Phe Leu Gly Arg Tyr Pro Val Ser Arg His Ser Trp Val Gly Ala Trp
            340                 345                 350

Arg Gly Pro Gln Gly Trp His Trp Ile Asp Glu Ala Pro Leu Pro Pro
        355                 360                 365

Gln Leu Leu Pro Glu Asp Gly Glu Asp Asn Leu Asp Ile Asn Cys Gly
    370                 375                 380

Ala Leu Glu Glu Gly Thr Leu Val Ala Ala Asn Cys Ser Thr Pro Arg
385                 390                 395                 400

Pro Trp Val Cys Ala Lys Gly Thr Gln
                405

<210>  86
<211>  314
<212>  PRT
<213>  Homo sapiens

<400>  86

Met Glu Glu Ser Trp Glu Ala Ala Pro Gly Gly Gln Ala Gly Ala Glu
1               5                   10                  15

Leu Pro Met Glu Pro Val Gly Ser Leu Val Pro Thr Leu Glu Gln Pro
            20                  25                  30

Gln Val Pro Ala Lys Val Arg Gln Pro Glu Gly Pro Glu Ser Ser Pro
        35                  40                  45

Ser Pro Ala Gly Ala Val Glu Lys Ala Ala Gly Ala Gly Leu Glu Pro
    50                  55                  60

Ser Ser Lys Lys Lys Pro Pro Ser Pro Arg Pro Gly Ser Pro Arg Val
65                  70                  75                  80

Pro Pro Leu Ser Leu Gly Tyr Gly Val Cys Pro Glu Pro Pro Ser Pro
                85                  90                  95

Gly Pro Ala Leu Val Lys Leu Pro Arg Asn Gly Glu Ala Pro Gly Ala
            100                 105                 110

Glu Pro Ala Pro Ser Ala Trp Ala Pro Met Glu Leu Gln Val Asp Val
        115                 120                 125

Arg Val Lys Pro Val Gly Ala Ala Gly Gly Ser Ser Thr Pro Ser Pro
    130                 135                 140

Arg Pro Ser Thr Arg Phe Leu Lys Val Pro Val Pro Glu Ser Pro Ala
145                 150                 155                 160

Phe Ser Arg His Ala Asp Pro Ala His Gln Leu Leu Leu Arg Ala Pro
                165                 170                 175

Ser Gln Gly Gly Thr Trp Gly Arg Arg Ser Pro Leu Ala Ala Ala Arg
            180                 185                 190

Thr Glu Ser Gly Cys Asp Ala Glu Gly Arg Ala Ser Pro Ala Glu Gly
        195                 200                 205

Ser Ala Gly Ser Pro Gly Ser Pro Thr Cys Cys Arg Cys Lys Glu Leu
    210                 215                 220

Gly Leu Glu Lys Glu Asp Ala Ala Leu Leu Pro Arg Ala Gly Leu Asp
225                 230                 235                 240

Gly Asp Glu Lys Leu Pro Arg Ala Val Thr Leu Thr Asp Ser Leu Arg
                245                 250                 255

Thr Ala Arg Thr Ile Trp Ile Ser Thr Val Gly Pro Trp Arg Lys Ala
            260                 265                 270

Arg Trp Trp Leu Gln Thr Ala Ala Leu Gln Asp Pro Gly Ser Val Pro
        275                 280                 285

Arg Gly Pro Ser Asp Leu Gly Ser Ala Trp Ser Ser Ala Cys Gln Ala
    290                 295                 300

Asp Ala Ala Pro Pro Thr Gly Glu Ala Ser
305                 310

<210>  87
<211>  1544
<212>  DNA
<213>  Homo sapiens

<400>  87
gagagcgaag ctcctctgca ctgggcccag gtgcgctcct cagcgtctcc gggtggcggg      60

gcgcgcggga tggaggagtc ttgggaggct gcgcccggag gccaagccgg ggcagagctc     120

ccaatggagc ccgtgggaag cctggtcccc acgctggagc agccgcaggt gcccgcgaag     180

gtgcgacaac ctgaaggtcc cgaaagcagc ccaagtccgg ccggggccgt ggagaaggcg     240

gcgggcgcag gcctggagcc ctcgagcaag aaaaagccgc cttcgcctcg ccccgggtcc     300

ccgcgcgtgc cgccgctcag cctgggctac ggggtctgcc ccgagccgcc gtcaccgggc     360

cctgccttgg tcaagctgcc ccggaatggc gaggcgcccg gggctgagcc tgcgcccagc 420

gcctgggcgc ccatggagct gcaggtagat gtgcgcgtga agcccgtggg cgcggccggt 480

ggcagcagca cgccatcgcc caggccctcc acgcgcttcc tcaaggtgcc ggtgcccgag 540

tcccctgcct tctcccgcca cgcggacccg gcgcaccagc tcctgctgcg cgcaccatcc 600

cagggcggca cgtggggccg ccgctcgccg ctggctgcag cccggacgga gagcggctgc 660

gacgcagagg gccgggccag ccccgcggaa ggaagcgccg gctccccggg ctcccccacg 720

tgctgccgct gcaaggagct ggggctggag aaggaggatg cggcgctgtt gccccgcgcg 780

gggttggacg gcgacgagaa gctgccccgg gccgtaacgc ttacggggct acccatgtac 840

gtgaagtccc tgtactgggc cctggcgttc atggctgtgc tcctggcagt ctctggggtt 900

gtcattgtgg tcctggcctc aagagcagga gccagatgcc agcagtgccc cccaggctgg 960

gtgttgtccg aggagcactg ttactacttc tctgcagaag cgcaggcctg ggaagccagc 1020

caggctttct gctcagccta ccacgctacc ctccccctgc taagccacac ccaggacttc 1080

ctgggcagat acccagtctc caggcactcc tgggtggggg cctggcgagg cccccagggc 1140

tggcactgga tcgacgaggc cccactcccg ccccagctac tccctgagga cggcgaggac 1200

aatctggata tcaactgtgg ggccctggag gaaggcacgc tggtggctgc aaactgcagc 1260

actccaagac cctgggtctg tgccaagggg acccagtgat ctgggctctg cctggtcctc 1320

agcctgccag gcagatgcag caccccctac aggggaggcc agttgagagc ttgggcagcc 1380

tcttcctgga cccagttatc caggtcttca tgctctgctc aagggggcca catgagcgag 1440

cctaggagct ggacttcaac ccaggaagat gcatccgagg gaaaggagat tttctatggc 1500

ctcaggcctg agtgccaata ttagtctcca gcttctgtgg atga 1544

<210> 88
<211> 1192
<212> DNA
<213> Homo sapiens

<400> 88
gagagcgaag ctcctctgca ctgggcccag gtgcgctcct cagcgtctcc gggtggcggg 60

gcgcgcggga tggaggagtc ttgggaggct gcgcccggag gccaagccgg ggcagagctc 120

ccaatggagc ccgtgggaag cctggtcccc acgctggagc agccgcaggt gcccgcgaag 180

gtgcgacaac ctgaaggtcc cgaaagcagc ccaagtccgg ccggggccgt ggagaaggcg 240

gcgggcgcag gcctggagcc ctcgagcaag aaaaagccgc cttcgcctcg ccccgggtcc 300

ccgcgcgtgc cgccgctcag cctgggctac ggggtctgcc ccgagccgcc gtcaccgggc 360

cctgccttgg tcaagctgcc ccggaatggc gaggcgcccg gggctgagcc tgcgcccagc 420

gcctgggcgc ccatggagct gcaggtagat gtgcgcgtga agcccgtggg cgcggccggt 480

ggcagcagca cgccatcgcc caggccctcc acgcgcttcc tcaaggtgcc ggtgcccgag 540

tcccctgcct tctcccgcca cgcggacccg gcgcaccagc tcctgctgcg cgcaccatcc 600

cagggcggca cgtggggccg ccgctcgccg ctggctgcag cccggacgga gagcggctgc 660

gacgcagagg gccgggccag ccccgcggaa ggaagcgccg gctccccggg ctcccccacg 720

tgctgccgct gcaaggagct ggggctggag aaggaggatg cggcgctgtt gccccgcgcg 780

gggttggacg gcgacgagaa gctgccccgg gccgtaacgc ttacggactc cctgaggacg 840

gcgaggacaa tctggatatc aactgtgggg ccctggagga aggcacgctg gtggctgcaa 900

actgcagcac tccaagaccc tgggtctgtg ccaaggggac ccagtgatct gggctctgcc 960

tggtcctcag cctgccaggc agatgcagca ccccctacag gggaggccag ttgagagctt 1020

gggcagcctc ttcctggacc cagttatcca ggtcttcatg ctctgctcaa gggggccaca 1080

tgagcgagcc taggagctgg acttcaaccc aggaagatgc atccgaggga aaggagattt 1140

tctatggcct caggcctgag tgccaatatt agtctccagc ttctgtggat ga 1192

<210> 89
<211> 21
<212> DNA
<213> Homo sapiens

<400> 89
cgaggacaat ctggatatca a 21

<210> 90
<211> 21
<212> DNA
<213> Homo sapiens

<400> 90
ctggagccct cgagcaagaa a 21

<210> 91
<211> 21
<212> DNA
<213> Homo sapiens

<400> 91
cccgtggttc atctgatata a 21

<210> 92
<211> 21
<212> DNA
<213> Homo sapiens

<400> 92
aaggactttg ctcggcgttt a 21

<210> 93
<211> 21

<212>  DNA
<213>  Homo sapiens

<400>  93
tacgtggatg tttgtaacgt a                                                21


<210>  94
<211>  21
<212>  DNA
<213>  Homo sapiens

<400>  94
ctcgtattgg ctcaatcata a                                                21

## Description

The present invention relates to newly identified proteins as markers for the detection of breast tumors, or as therapeutic targets for treatment thereof. Also provided are affinity ligands capable of selectively interacting with the newly identified markers, as well as methods for tumor diagnosis and therapy using such ligands.

### Background of the invention

### Tumor markers (or biomarkers)

Tumor markers are substances that can be produced by tumor cells or by other cells of the body in response to cancer. In particular, a protein biomarker is either a single protein or a panel of different proteins, that could be used to unambiguously distinguish a disease state. Ideally, a biomarker would have both a high specificity and sensitivity, being represented in a significant percentage of the cases of given disease and not in healthy state.

Biomarkers can be identified in different biological samples, like tissue biopsies or preferably biological fluids (saliva, urine, blood-derivatives and other body fluids), whose collection does not necessitate invasive treatments. Tumor marker levels may be categorized in three major classes on the basis of their clinical use. Diagnostic markers can be used in the detection and diagnosis of cancer. Prognostics markers are indicative of specific outcomes of the disease and can be used to define predictive models that allow the clinicians to predict the likely prognosis of the disease at time of diagnosis. Moreover, prognosis markers are helpful to monitor the patient response to a drug therapy and facilitate a more personalized patient management. A decrease or return to a normal level may indicate that the cancer is responding to therapy, whereas an increase may indicate that the cancer is not responding. After treatment has ended, tumor marker levels may be used to check for recurrence of the tumor. Finally, therapeutic markers can be used to develop tumor-specific drugs or affinity ligand (i.e. antibodies) for a prophylactic intervention.

Currently, although an abnormal tumor marker level may suggest cancer, this alone is usually not enough to accurately diagnose cancer and their measurement in body fluids is frequently combined with other tests, such as a biopsy and radioscopic examination. Frequently, tumor marker levels are not altered in all of people with a certain cancer disease, especially if the cancer is at early stage. Some tumor marker levels can also be altered in patients with noncancerous conditions. Most biomarkers commonly used in clinical practice do not reach a sufficiently high level of specificity and sensitivity to unambiguously distinguish a tumor from a normal state.

To date the number of markers that are expressed abnormally is limited to certain types/subtypes of cancer, some of which are also found in other diseases. (http://www.cancer.gov/cancertopics/factsheet).

For instance, the human epidermal growth factor receptor (HER2) is a marker protein overproduced in about 20% of breast cancers, whose expression is typically associated with a more aggressive and recurrent tumors of this class.

### Routine screening test for tumor diagnosis

Screening tests are a way of detecting cancer early, before there are any symptoms. For a screening test to be helpful, it should have high sensitivity and specificity. Sensitivity refers to the test's ability to identify people who have the disease. Specificity refers to the test's ability to identify people who do not have the disease. Different molecular biology approaches such as analysis of DNA sequencing, small nucleotide polymorphyms, in situ hybridization and whole transcriptional profile analysis have done remarkable progresses to discriminate a tumor state from a normal state and are accelerating the knowledge process in the tumor field. However so far different reasons are delaying their use in the common clinical practice, including the higher analysis complexity and their expensiveness. Other diagnosis tools whose application is increasing in clinics include in situ hybridization and gene sequencing.

Currently, Immuno-HistoChemistry (IHC), a technique that allows the detection of proteins expressed in tissues and cells using specific antibodies, is the most commonly used method for the clinical diagnosis of tumor samples. This technique enables the analysis of cell morphology and the classification of tissue samples on the basis of their immunoreactivity. However, at present, IHC can be used in clinical practice to detect cancerous cells of tumor types for which protein markers and specific antibodies are available. In this context, the identification of a large panel of markers for the most frequent cancer classes would have a great impact in the clinical diagnosis of the disease.

### Anti-cancer therapies

In the last decades, an overwhelming number of studies remarkably contributed to the comprehension of the molecular mechanisms leading to cancer. However, this scientific progress in the molecular oncology field has not been paralleled by a comparable progress in cancer diagnosis and therapy. Surgery and/or radiotherapy are the still the main modality of local treatment of cancer in the majority of patients. However, these treatments are effective only at initial phases of the disease and in particular for solid tumors of epithelial origin, as is the case of colon, lung, breast, prostate and others, while they are not effective for distant recurrence of the disease. In some tumor classes, chemotherapy treatments have been developed, which generally relies on drugs, hormones and antibodies, targeting specific biological processes used by cancers to grow and spread. However, so far many cancer therapies had limited efficacy due to severity of side effects and overall toxicity. Indeed, a major effort in cancer therapy is the development of treatments able to target specifically tumor cells causing limited damages to surrounding normal cells thereby decreasing adverse side effects. Recent developments in cancer therapy in this direction are encouraging, indicating that in some cases a cancer specific therapy is feasible. In particular, the development and commercialization of humanized monoclonal antibodies that recognize specifically tumor-associated markers and promote the elimination of cancer is one of the most promising solutions that appears to be an extremely favorable market opportunity for pharmaceutical companies. However, at present the number of therapeutic antibodies available on the market or under clinical studies is very limited and restricted to specific cancer classes. So far licensed monoclonal antibodies currently used in clinics for the therapy of specific tumor classes, show only a partial efficacy and are frequently associated with chemotherapies to increase their therapeutic effect. Administration of Trastuzumab (Herceptin), a commercial monoclonal antibody targeting HER2, a protein overproduced in about 20% of breast cancers, in conjunction with Taxol adjuvant chemotherapy induces tumor remission in about 42% of the cases. Bevacizumab (Avastin) and Cetuximab (Erbitux) are two monoclonal antibodies recently licensed for use in humans, targeting the endothelial and epithelial growth factors respectively that, combined with adjuvant chemotherapy, proved to be effective against different tumor diseases. Bevacizumab proved to be effective in prolonging the life of patients with metastatic colorectal, breast and lung cancers. Cetuximab demostrated efficacy in patients with tumor types refractory to standard chemotherapeutic treatments (Adams G.P. and Weiner L.M. (2005) Monoclonal antibody therapy cancer. Nat Biotechnol. 23:1147-57).

In summary, available screening tests for tumor diagnosis are uncomfortable or invasive and this sometimes limits their applications. Moreover tumor markers available today have a limited utility in clinics due to either their incapability to detect all tumor subtypes of the defined cancers types and/or to distinguish unambiguously tumor vs. normal tissues. Similarly, licensed monoclonal antibodies combined with standard chemotherapies are not effective against the majority of cases. Therefore, there is a great demand for new tools to advance the diagnosis and treatment of cancer.

### Experimental approaches commonly used to identify tumor markers

Most popular approaches used to discover new tumor markers are based on genome-wide transcription profile or total protein content analyses of tumor. These studies usually lead to the identification of groups of mRNAs and proteins which are differentially expressed in tumors. Validation experiments then follow to eventually single out, among the hundreds of RNAs/proteins identified, the very few that have the potential to become useful markers. Although often successful, these approaches have several limitations and often, do not provide firm indications on the association of protein markers with tumor. A first limitation is that, since frequently mRNA levels not always correlate with corresponding protein abundance (approx. 50% correlation), studies based on transcription profile do not provide solid information regarding the expression of protein markers in tumor (1, 2, 3, 4).

A second limitation is that neither transcription profiles nor analysis of total protein content discriminate post-translation modifications, which often occur during oncogenesis. These modifications, including phosphorylations, acetylations, and glycosylations, or protein cleavages influence significantly protein stability, localization, interactions, and functions (5).

As a consequence, large scale studies generally result in long lists of differentially expressed genes that would require complex experimental paths in order to validate the potential markers. However, large scale genomic/proteomic studies reporting novel tumor markers frequently lack of confirmation data on the reported potential novel markers and thus do not provide solid demonstration on the association of the described protein markers with tumor.

The approach that we used to identify the protein markers included in the present invention is based on an innovative immuno-proteomic technology. In essence, a library of recombinant human proteins has been produced from E. coli and is being used to generate polyclonal antibodies against each of the recombinant proteins.

The screening of the antibodies library on Tissue microarrays (TMAs) carrying clinical samples from different patients affected by the tumor under investigation leads to the identification of specific tumor marker proteins. Therefore, by screening TMAs with the antibody library, the tumor markers are visualized by immuno-histochemistry, the classical technology applied in all clinical pathology laboratories. Since TMAs also include healthy tissues, the specificity of the antibodies for the tumors can be immediately appreciated and information on the relative level of expression and cellular localization of the markers can be obtained. In our approach the markers are subjected to a validation process consisting in a molecular and cellular characterization.

Altogether, the detection the marker proteins disclosed in the present invention selectively in tumor samples and the subsequent validation experiments leads to an unambiguous confirmation of the marker identity and confirm its association with defined tumor classes. Moreover this process provides an indication of the possible use of the proteins as tools for diagnostic or therapeutic intervention. For instance, markers showing a surface cellular localization could be both diagnostic and therapeutic markers against which both chemical and antibody therapies can be developed. Differently, markers showing a cytoplasmic expression could be more likely considered for the development of tumor diagnostic tests and chemotherapy/small molecules treatments.

### Summary of the invention

The present invention provides new means for the detection and treatment of breast tumors, based on the identification of protein markers specific for these tumor types, namely:
i) Endoplasmic reticulum metallopeptidase 1 (ERMP1)
ii) Chromosome 6 open reading frame 98 (C6orf98);
iii) Chromosome 9 open reading frame 46 (c9orf46);
iv) Putative uncharacterized protein (FLJ37107);
v) Yip1 domain family, member 2 (YIPF2);
vi) Uncharacterized protein UNQ6126/PRO20091 (UNQ6126);
vii) Trypsin-X3 Precursor (TRYX3);
viii) DPY -19-like 3 (DPY19L3);
ix) solute carrier family 39 (zinc transporter), member 10 (SLC39A10);
x) Chromosome 14 open reading frame 135 (c14orf135);
xi) DENN/MADD domain containing 1B (DENND1B);
xii) EMI domain-containing protein 1 Precursor (EMID1);
xiii) Cysteine-rich secretory protein 3 Precursor (CRISP3)
xiv) Killer cell lectin-like receptor subfamily G member 2 (C-type lectin domain family 15 member B) (KLRG2).

In preferred embodiments, the invention provides the use of, alone or in combination, C6orf98, C9orf46, FLJ37107, YIPF2, UNQ6126, TRYX3, DPY19L3, SLC39A10, C14orf135; DENND1B, EMID1, ERMP1, CRISP3 and KLRG2 as markers or targets for breast tumor.

The invention also provides a method for the diagnosis of these cancer types, comprising a step of detecting the above-identified markers in a biological sample, e.g. in a tissue sample of a subject suspected of having or at risk of developing malignancies or susceptible to cancer recurrences.

In addition, the tumor markers identify novel targets for affinity ligands, which can be used for therapeutic applications. Also provided are affinity ligands, particularly antibodies, capable of selectively interacting with the newly identified protein markers.

### Detailed disclosure of the invention

The present invention is based on the surprising finding of antibodies that are able to specifically stain breast tumor tissues from patients, while negative or very poor staining is observed in normal breast tissues from the same patients. These antibodies have been found to specifically bind to proteins for which no previous association with tumor has been reported. Hence, in a first aspect, the invention provides a breast tumor marker, which is selected from the group consisting of:
i) C6orf98 in one of its variant isoforms SEQ ID NO:1, or a different isoform having sequence identity of at least 80%, preferably at least 90%, more preferably at least 95% to SEQ ID NO:1; or a nucleic acid molecule containing a sequence coding for a C6orf98 protein, said encoding sequence being preferably SEQ ID NO: 2;
ii) C9orf46, in one of its variant isoforms SEQ ID NO:3, or a different isoform having sequence identity of at least 80%, preferably at least 90%, more preferably at least 95% to SEQ ID NO:3, or a nucleic acid molecule containing a sequence coding for a C9orf46 protein, said encoding sequence being preferably SEQ ID NO:4;
iii) FLJ37107, in one of its variant isoforms SEQ ID NO:5, or a different isoform having sequence identity of at least 80%, preferably at least 90%, more preferably at least 95% to SEQ ID NO:5; or a nucleic acid molecule containing a sequence coding for a FLJ37107 protein, said encoding sequence being preferably SEQ ID NO: 6;
iv) YIPF2, SEQ ID NO:7, SEQ ID NO:8, or a different isoform having sequence identity of at least 80%, preferably at least 90%, more preferably at least 95% to SEQ ID NO:7 or SEQ ID NO:8, or a nucleic acid molecule containing a sequence coding for a YIPF2 protein, said encoding sequence being preferably selected from SEQ ID NO:9 and SEQ ID NO: 10;
v) UNQ6126, in one of its variant isoforms SEQ ID NO:11, or a different isoform having sequence identity of at least 80%, preferably at least 90%, more preferably at least 95% to SEQ ID NO:11, or a nucleic acid molecule containing a sequence coding for a UNQ6126 protein, said encoding sequence being preferably SEQ ID NO: 12;
vi) TRYX3, in one of its variant isoforms SEQ ID NO:13, or a different isoform having sequence identity of at least 80%, preferably at least 90%, more preferably at least 95% to SEQ ID NO: 13, or a nucleic acid molecule containing a sequence coding for a TRYX3 protein, said encoding sequence being preferably SEQ ID NO:14;
vii) DPY19L3, in one of its variant isoforms SEQ ID NO:15, SEQ ID NO:16, SEQ ID NO:17, SEQ ID NO:18, or a different isoform having sequence identity of at least 80%, preferably at least 90%, more preferably at least 95% to any of SEQ ID NO:15, SEQ ID NO:16, SEQ ID NO:17 or SEQ ID NO: 18, or a nucleic acid molecule containing a sequence coding for a DPY19L3 protein, said encoding sequence being preferably selected from SEQ ID NO:19, SEQ ID NO:20, SEQ ID NO:21, SEQ ID NO:22;
viii) SLC39A10, SEQ ID NO:23, SEQ ID NO:24 or a different isoform having sequence identity of at least 80%, preferably at least 90%, more preferably at least 95% to SEQ ID NO:23 or SEQ ID NO:24, or a nucleic acid molecule containing a sequence coding for a SLC39A10 protein, said encoding sequence being preferably selected from SEQ ID NO:25 and SEQ ID NO:26;
ix) C14orf135, SEQ ID NO:27, SEQ ID NO:28, SEQ ID NO:29, SEQ ID NO:30, SEQ ID NO:31, or a different isoform having sequence identity of at least 80%, preferably at least 90%, more preferably at least 95% to any of SEQ ID NO:27, SEQ ID NO:28, SEQ ID NO:29, SEQ ID NO:30 or SEQ ID NO:31, or a nucleic acid molecule containing a sequence coding for a C14orf135 protein, said encoding sequence being preferably selected from SEQ ID NO:32, SEQ ID NO:33, SEQ ID NO:34, SEQ ID NO:35 and SEQ ID NO:36;
x) DENND1B; in one of its variant isoforms SEQ ID NO:37, SEQ ID NO:38, SEQ ID NO:39, SEQ ID NO:40, or a different isoform having sequence identity of at least 80%, preferably at least 90%, more preferably at least 95% to any of SEQ ID NO:37, SEQ ID NO:38, SEQ ID NO:39 or SEQ ID NO:40, or a nucleic acid molecule containing a sequence coding for a DENND1B protein, said encoding sequence being preferably selected from SEQ ID NO:41, SEQ ID NO:42, SEQ ID NO:43 and SEQ ID NO:44;
xi) EMID1, in one of its variant isoforms SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58,or a different isoform having sequence identity of at least 80%, preferably at least 90%, more preferably at least 95% to any of SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57 or SEQ ID NO:58, or a nucleic acid molecule containing a sequence coding for a DENND1B protein, said encoding sequence being preferably selected from SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, SEQ ID NO:68, SEQ ID NO:69, SEQ ID NO:70, SEQ ID NO:71 and SEQ ID NO:72;
xii) ERMP1, SEQ ID NO:73, SEQ ID NO:74, SEQ ID NO:75, or a different isoform having sequence identity of at least 80%, preferably at least 90%, more preferably at least 95% to SEQ ID NO:73, SEQ ID NO:74, SEQ ID NO:75, or a nucleic acid molecule containing a sequence coding for a ERMP1, protein, said encoding sequence being preferably selected from SEQ ID NO:76 SEQ ID NO: 77 and SEQ ID NO:78;
xiii) CRISP-3, SEQ ID NO: 79, SEQ ID NO:80, SEQ ID NO:81, or a different isoform having sequence identity of at least 80%, preferably at least 90%, more preferably at least 95% to SEQ ID NO: 79, SEQ ID NO:80, SEQ ID NO:81, or a nucleic acid molecule containing a sequence coding for a CRISP-3, protein, said encoding sequence being preferably selected from SEQ ID NO:82, SEQ ID NO:83 and SEQ ID NO:84.
xiv) KLRG2, SEQ ID: NO 85, SEQ ID NO:86 or a different isoform having sequence identity of at least 80%, preferably at least 90%, more preferably at least 95% to SEQ ID: NO 85 or SEQ ID: NO 86, or a nucleic acid molecule containing a sequence coding for a KLRG2 protein, said encoding sequence being preferably selected from SEQ ID NO: 87 and SEQ ID NO: 88;

As used herein, "Percent (%) amino acid sequence identity" with respect to the marker protein sequences identified herein indicates the percentage of amino acid residues in a full-length protein variant or isoform according to the invention, or in a portion thereof, that are identical with the amino acid residues in the specific marker sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. Identity between nucleotide sequences is preferably determined by the Smith-Waterman homology search algorithm as implemented in the MPSRCH program (Oxford Molecular), using an affine gap search with parameters gap open penalty = 12 and gap extension penalty = 1.

Solute carrier family 39 member 10 (SLC39A10, synonyms: Zinc transporter ZIP10 Precursor, Zrt- and Irt-like protein 10, ZIP-10, Solute carrier family 39 member 10; gene ID: ENSG00000196950; transcript IDs: ENST00000359634, ENST00000409086; protein ID: ENSP00000352655, ENSP00000386766) belongs to a subfamily of proteins that show structural characteristics of zinc transporters. It is an integral membrane protein likely involved in zinc transport. While other members of the zinc transport family have been at least partially studied in tumors, little is known about the association of SLC39A10 with these diseases. SLC39A10 mRNA has been shown to increase moderately in breast cancer tissues as compared to normal samples (approximately 1.5 fold). Loss of SLC39A10 transcription in breast cell lines has been shown to reduce cell migratory activity (6). However, published studies on the expression of SLC39A10 in breast tumor cells are limited to the analysis of SLC39A10 transcript whilst, to the best of our knowledge, no data have been reported documenting the presence of SLC39A10 protein in these tumor cells.

SLC39A10 is mentioned in a patent application reporting long lists of differentially transcribed genes in tumor cells based on the use of genome-scale transcription profile analysis (e.g. in Publication Number: US20070237770A1). However, since mRNA levels not always correlate with protein levels, studies solely based on transcription profile do not provide solid information regarding the expression of protein markers. Moreover, the lack of correlation between mRNA and protein expression has been specifically demonstrated for LIV-1, another member of the zinc transporter family, suggesting that a similar phenomenon could be extended to other proteins of this class (7).

In the present invention we disclose SLC39A10 as a protein without previous known association with breast tumor classes and preferably used as a marker for breast tumors and in general for cancers of these types. As described below, an antibody generated towards the SLC39A10 protein shows a selective immunoreactivity in histological preparation of breast cancer tissues which indicates the presence of SLC39A10 in these cancer samples and makes SLC39A10 protein and its antibody highly interesting tools for specifically distinguishing these cancer types from a normal state. Moreover, we show that the protein is localized on the surface of tumor cell lines, indicating that this protein is an ideal candidate target for anti-tumor therapies.

Chromosome 6 open reading frame 98 (C6orf98; synonym: dJ45H2.2; Gene ID: EG:387079, da ENSG00000222029 has 1 transcript: ENST00000409023, associated peptide: ENSP00000386324 and 1 exon: ENSE00001576965) is an uncharacterized protein. Analysis of human genome databases (E.g. Ensembl) erroneously assigns C6orf98 as SYNE1. Although SYNE nucleic acid sequences overlap with C6ORF98 transcript, the encoded proteins show no match. In fact C6orf98 locus maps on an SYNE1 untranslated region (intron) and its product derives from a different reading frame than those annotated for SYNE1 isoforms in public databases. C6orf98 is a protein without previous known association with tumor and is preferably used as a marker for breast tumor and in general for these cancer types. As described below, an antibody generated towards C6orf98 protein shows a selective immune-reactivity in histological preparation of breast cancer tissues, which indicates the presence of this protein in these cancer samples.

Chromosome 9 open reading frame 46 (C9orf46; synonyms: Transmembrane protein C9orf46; Gene ID: ENSG00000107020; Transcript ID:ENST00000223864; Protein ID: ENSP00000223864) is a poorly characterized protein. So far expression of C9orf46 has only been shown at transcriptional level in metastasis in oral squamous cell carcinoma (8) while no data are available on the expression of its encoded product in tumor. Based on available scientific publications, C9orf46 is a protein without previous known association with breast tumor and is preferably used as a marker for breast tumors. As described below, an antibody generated towards C9orf46 protein shows a selective immunoreactivity in histological preparation of breast cancer tissues, which indicates the presence of this protein in these cancer samples.

Putative uncharacterized protein FLJ37107 - (FLJ37107; synonyms: LOC284581; Gene ID: ENSG00000177990, Transcript ID: gi|58218993|ref|NM_001010882.1, Protein ID: gi|58218994|ref|NP_001010882.1| hypothetical protein LOC284581 [Homo sapiens], gi|74729692|sp|Q8N9I1.1|YA028_HUMAN) is an uncharacterized protein without previous known association with tumor and is preferably used as a marker for breast tumor and in general for these cancer types. As described below, an antibody generated towards FLJ37107 protein shows a selective immunoreactivity in histological preparation of breast cancer tissues, which indicates the presence of this protein in these cancer samples.

Yip1 domain family, member 2 (YIPF2; synonyms: FinGER2; Gene ID: ENSG00000130733; Transcript IDs: ENST00000393508, ENST00000253031; Protein IDs: ENSP00000377144, ENSP00000253031) is an uncharacterized without previous known association with tumor and is preferably used as a marker for breast tumor in general for these cancer types. As described below, an antibody generated towards YIPF2 protein shows a selective immunoreactivity in histological preparation of breast cancer tissues, which indicates the presence of this protein in these cancer samples.

Uncharacterized protein UNQ6126/PRO20091 (UNQ6126, LPEQ6126, synonyms: LOC100128818; Gene ID: gi|169216088; Transcript ID: GB:AY358194, Protein ID: SP:Q6UXV3); is an uncharacterized protein without previous known association with tumor and is preferably used as a marker for breast tumor, and in general for cancers of this type. As described below, an antibody generated towards UNQ6126 protein shows a selective immunoreactivity in histological preparation of breast cancer tissues.

A TRYX3 sequence has been listed in several generic patents, in which no solid data are reported showing the association of TRYX3 protein with tumor (e.g. US7105335, US7285626). Based on the above, TRYX3 is a protein without previous known association with tumor and preferably used as a marker for breast tumor and in general for cancers of this type. As described below, an antibody generated towards TRYX3 protein shows a selective immunoreactivity in histological preparation of breast cancer tissues, which indicates the presence of this protein in these cancer samples.

Protein dpy-19 homolog 3 (DPY19L3; synonym: Dpy-19-like protein 3; Gene ID: ENSG00000178904; Transcript IDs: ENST00000319326, ENST00000392250, ENST00000342179, ENST00000392248; Protein IDs: ENSP00000315672, ENSP00000376081. ENSP00000344937, ENSP00000376079) is a poorly characterized characterized protein. DPY19L3 transcript has been reported as differentially expressed in a large-scale study on multiple myeloma (Publication Number: US20080280779A1). However no data are available at level of protein expression. In the present invention we disclose DPY19L3 protein as associated with tumor and preferably used as a marker for breast tumor, and in general for these cancer types. As described below, an antibody generated towards DPY19L3 protein shows a selective immunoreactivity in histological preparation of breast cancer tissues, which indicates the presence of this protein in these cancer samples. Finally the protein is detected on the surface of tumor cell lines by the specific antibody, suggesting that it can be exploited as target for affinity ligands with therapeutic activity.

Chromosome 14 open reading frame 135 (C14orf135, Pecanex-like protein C14orf135, synonyms: Hepatitis C virus F protein-binding protein 2, HCV F protein-binding protein 2; Gene ID: ENSG00000126773; Transcript IDs: ENST00000317623, ENST00000404681; Protein IDs: ENSP00000317396, ENSP00000385713) is a uncharacterized protein. This protein is mentioned in a patent application on ovarian tumor (Publication number: US2006432604A). In the present invention we report C14orf135 as a protein without previous known association with breast tumor class and preferably used as a marker for breast tumor, and in general for cancers of this type. As described below, an antibody generated towards C14orf135 protein shows a selective immunoreactivity in histological preparation of breast cancer tissues, which indicates the presence of this protein in this cancer samples; moreover this antibody also stains plasma membranes of tumor cells, indicating that c14orf135 protein is localized on the cell surface.

DENN/MADD domain containing 1B (DENND1B; synonyms: DENN domain-containing protein 1B, Protein FAM31B, C1orf218; Gene ID: ENSG00000162701. Transcript IDs: ENST00000294738, ENST00000367396, ENST00000400967, ENST00000235453; Protein IDs: ENSP00000294738, ENSP00000356366, ENSP00000383751, ENSP00000235453) is a poorly characterized protein without previous known association with breast tumors and is preferably used as a marker for breast tumor and in general for these cancer types. As described below, an antibody generated towards DENND1B protein shows a selective immunoreactivity in histological preparation of breast cancer tissues, which indicates the presence of this protein in these cancer samples.

EMI domain-containing protein 1 Precursor (EMID1; synonyms: Emilin and multimerin domain-containing protein 1, Protein Emu1; Gene ID: >OTTHUMG00000030824

| Transcript IDs: | Protein IDs: |
|---|---|
| OTTHUMT00000075712 | OTTHUMP00000028901, |
| ENST00000429226 | ENSP00000403816, |
| ENST00000430127 | ENSP00000399760, |
| ENST00000435427 | ENSP00000402621, |
| ENST00000404820 | ENSP00000384452, |
| ENST00000334018 | ENSP00000335481, |
| ENST00000429415 | ENSP00000409801, |
| ENST00000448676 | ENSP00000413034, |
| ENST00000404755 | ENSP00000385414, |
| ENST00000435194 | ENSP00000417004, |
| ENST00000426629 | ENSP00000403484, |
| ENST00000457925 | ENSP00000405422, |
| ENST00000433143 | ENSP00000408339, |
| ENST00000455501 | ENSP00000413947), |

is a poorly characterized protein. EMID gene is mentioned in a patent application on follicular thyroid carcinoma (Publication number US2006035244 (A1). However, no data are available on the presence of this protein in breast tumor. Therefore, we disclose EMID1 as a protein without previous known association with breast tumors and preferably used as a marker for breast tumor and in general for these cancer types. As described below, an antibody generated towards EMID1 protein shows a selective immunoreactivity in histological preparation of breast cancer tissues, which indicates the presence of this protein in these cancer samples. In particular this antibody stains tumor secretion products indicating that EMID1 protein is specifically released by tumor cells.

Endoplasmic reticulum metallopeptidase 1 (ERMP1, synonyms: FLJ23309, FXNA, KIAA1815; GENE ID: ENSG00000099219; Transcript IDs: ENST00000214893, ENST00000339450, ENST00000381506; Protein IDs: ENSP00000214893, ENSP00000340427, ENSP00000370917) is a transmembrane metallopeptidase, so far described as localized to the endoplasmic reticulum. ERMP1 transcript has been found differentially expressed in the rat ovary at the time of folliculogenesis. A lower level of ERMP1 transcript in the rat ovary resulted in substantial loss of primordial, primary and secondary follicles, and structural disorganization of the ovary, suggesting that is required for normal ovarian histogenesis (9). ERMP1 has been also included in a patent application (Publication number US 2003064439) on novel nucleic acid sequences encoding melanoma associated antigen molecules, however no solid data documented the expression of ERMP1 protein in tumor. Based on this, ERMP1 protein has never been previously associated with tumor. In the present invention, differently with published scientific data, we disclose ERMP1 as a protein associated with tumor, preferably used as a marker for breast tumor, and in general for cancers of this type. As described below, an antibody generated towards ERMP1 protein shows a selective immunoreactivity in histological preparation of breast cancer tissues, which indicates the presence of this protein in this cancer type. In particular our immunoistochemistry analysis indicates that the protein shows plasma membrane localization in tumot cells.

Moreover, localization analysis of ovary tumor cell lines showed that this proteins is exposed on the cell surface and accessible to the binding of specific antibodies. Finally, silencing of ERMP1 gene significantly reduced the invasiveness and proliferation properties of tumor cells lines. Based on the above evidences, ERMP1 is a likely target for the development of anti-cancer therapies being exposed to the action of affinity ligand and being involved in cellular processes relevant for tumor development.

Cysteine-rich secretory protein 3 Precursor (CRISP-3, synonyms: SGP28 protein; GENE ID: ENSG00000096006; Transcript IDs: ENST00000393666, ENST00000371159, ENST00000263045; Protein IDs: ENSP00000377274, ENSP00000360201, ENSP00000263045) is also known as specific granule protein of 28 KDa. In humans, high level of CRISP3 transcript protein has been detected in salivary glands, pancreas and prostate, while low expression was found in other tissues such as epidydimis, ovary, thymus and colon (10). Upregulation of CRISP3 has been shown in malignant prostatic epithelium at RNA and protein level. Strong immunostaining for CRISP3 has been associated with high-grade prostatic-intraepithelial-neoplasia and preserved in prostatic cancer (11). CRISP3 has been proposed as predictor of recurrence after radical prostatectomy for localized prostate cancer (12). While CRISP3 protein has been detected and largely characterized in prostate tumor, no previous data exist on its association with breast tumor. In the present invention we disclose CRISP3 as a marker for breast tumor, and in general for cancers of this type. As described below, an antibody generated towards CRISP3 protein shows a selective immunoreactivity in histological preparation of breast cancer tissues, which indicates the presence of this protein in this cancer type.

Killer cell lectin-like receptor subfamily G member 2 (C-type lectin domain family 15 member B) (KLRG2, synonyms: CLEC15B, FLJ44186; GENE ID: ENSG00000188883; Transcript IDs: ENST00000340940, ENST00000393039; Protein IDs: ENSP00000339356, ENSP00000376759) is a poorly uncharacterized protein. A KLRG2 sequence is included in a patent application on the use of an agent with tumor-inhibiting action of a panel of targets associated with different tumors, whose expression is mainly shown at RNA level (Publication number WO2005030250). However no data are provided documenting the presence of KLRG2 protein in the tumors. Moreover, no experimental evidence is given on the specificity of the proposed anti-tumor agent for KLRG2. Based on these considerations, in the present invention we disclose KLRG2 as a protein without previous known association with tumor class under investigation and preferably used as a marker for breast tumor, and in general for cancers of this type. As described below, an antibody generated towards KLRG2 protein shows a selective immunoreactivity in histological preparation of breast cancer tissues, which indicates the presence of this protein in this cancer type. In particular our immunoistochemistry analysis indicates that the protein shows plasma membrane localization in tumor cells. Moreover, localization analysis of tumor cell lines showed that the proteins is exposed on the cell surface and accessible to the binding of specific antibodies. Finally, silencing of KLRG2 significantly reduced the invasiveness and proliferation properties of breast tumor cells lines. Based on the above evidences, KLRG2 is a likely target for the development of anti-cancer therapies being exposed to the action of affinity ligands and being involved in cellular processes relevant for tumor development.

A further aspect of this invention is a method of screening a tissue sample for malignancy, which comprises determining the presence in said sample of at least one of the above-mentioned tumor markers. This method includes detecting either the marker protein, e.g. by means of labeled monoclonal or polyclonal antibodies that specifically bind to the target protein, or the respective mRNA, e.g. by means of polymerase chain reaction techniques such as RT-PCR. The methods for detecting proteins in a tissue sample are known to one skilled in the art and include immunoradiometric, immunoenzymatic or immunohistochemical techniques, such as radioimmunoassays, immunofluorescent assays or enzyme-linked immunoassays. Other known protein analysis techniques, such as polyacrylamide gel electrophoresis (PAGE), Western blot or Dot blot are suitable as well. Preferably, the detection of the protein marker is carried out with the immune-histochemistry technology, particularly by means of High Through-Put methods that allow the analyses of the antibody immune-reactivity simultaneously on different tissue samples immobilized on a microscope slide. Briefly, each Tissue Micro Array (TMA) slide includes tissue samples suspected of malignancy taken from different patients, and an equal number of normal tissue samples from the same patients as controls. The direct comparison of samples by qualitative or quantitative measurement, e.g. by enzimatic or colorimetric reactions, allows the identification of tumors.

In one embodiment, the invention provides a method of screening a sample of breast tissue for malignancy, which comprises determining the presence in said sample of the C6orf98, C9orf46, FLJ37107, YIPF2, UNQ6126, TRYX3, DPY19L3, SLC39A10, C14orf135, DENND1B, EMID1, ERMP1, CRISP3 and KLRG2 protein tumor marker, variants or isoforms thereof as described above.

A further aspect of the invention is a method *in vitro* for determining the presence of a breast tumor in a subject, which comprises the steps of:
- providing a sample of the tissue suspected of containing tumor cells;
- determining the presence of a tumor marker as above defined, or a combination thereof in said tissue sample by detecting the expression of the marker protein or the presence of the respective mRNA transcript;
wherein the detection of one or more tumor markers in the tissue sample is indicative of the presence of tumor in said subject.

The methods and techniques for carrying out the assay are known to one skilled in the art and are preferably based on immunoreactions for detecting proteins and on PCR methods for the detection of mRNAs. The same methods for detecting proteins or mRNAs from a tissue sample as disclosed above can be applied.

A further aspect of this invention is the use of the tumor markers herein provided as targets for the identification of candidate antitumor agents. Accordingly, the invention provides a method for screening a test compound which comprises contacting the cells expressing a tumor-associated protein selected from: Chromosome 6 open reading frame 98 (C6orf98); Chromosome 9 open reading frame 46 (C9orf46); Putative uncharacterized protein (FLJ37107); Yip1 domain family, member 2 (YIPF2); Uncharacterized protein UNQ6126/PRO20091 (UNQ6126); Trypsin-X3 Precursor (TRYX3); DPY-19-like 3 (DPY19L3); Solute carrier family 39 (zinc transporter), member 10 (SLC39A10); Chromosome 14 open reading frame 135 (C14orf135); DENN/MADD domain containing 1B (DENND1B), EMI domain-containing protein 1 Precursor (EMID1) Endoplasmic reticulum metallopeptidase 1 (ERMP1), Cysteine-rich secretory protein 3 Precursor (CRISP3) and Killer cell lectin-like receptor subfamily G member 2 (KLRG2).
with the test compound, and determining the binding of said compound to said tumor-associated protein. In addition, the ability of the test compound to modulate the activity of each target molecule can be assayed.

A further aspect of the invention is an antibody or a fragment thereof, which is able to specifically recognize and bind to one of the tumor-associated proteins described above. The term "antibody" as used herein refers to all types of immunoglobulins, including IgG, IgM, IgA, IgD and IgE. Such antibodies may include polyclonal, monoclonal, chimeric, single chain, antibodies or fragments such as Fab or scFv. The antibodies may be of various origin, including human, mouse, rat, rabbit and horse, or chimeric antibodies. The production of antibodies is well known in the art. For the production of antibodies in experimental animals, various hosts including goats, rabbits, rats, mice, and others, may be immunized by injection with polypeptides of the present invention or any fragment or oligopeptide or derivative thereof which has immunogenic properties or forms a suitable epitope. Monoclonal antibodies may be produced following the procedures described in Kohler and Milstein, Nature 265:495 (1975) or other techniques known in the art.

The antibodies to the tumor markers of the invention can be used to detect the presence of the marker in histologic preparations or to distinguish tumor cells from normal cells. To that purpose, the antibodies may be labeled with radiocative, fluorescent or enzyme labels.

In addition, the antibodies can be used for treating proliferative diseases by modulating, e.g. inhibiting or abolishing the activity of a target protein according to the invention. Therefore, in a further aspect the invention provides the use of antibodies to a tumor-associated protein selected from: Chromosome 6 open reading frame 98 (C6orf98); Chromosome 9 open reading frame 46 (C9orf46); Putative uncharacterized protein (FLJ37107); Yip1 domain family, member 2 (YIPF2); Uncharacterized protein UNQ6126/PRO20091 (UNQ6126); Trypsin-X3 Precursor (TRYX3); DPY-19-like 3 (DPY19L3); Solute carrier family 39 (zinc transporter), member 10 (SLC39A10); Chromosome 14 open reading frame 135 (C14orf135); DENN/MADD domain containing 1B (DENND1B), EMI domain-containing protein 1 Precursor (EMID1), Endoplasmic reticulum metallopeptidase 1 (ERMP1) Cysteine-rich secretory protein 3 Precursor (CRISP3) and Killer cell lectin-like receptor subfamily G member 2 (KLRG2).
for the preparation of a therapeutic agent for the treatment of proliferative diseases. For use in therapy, the antibodies can be formulated with suitable carriers and excipients, optionally with the addition of adjuvants to enhance their effetcs.

A further aspect of the invention relates to a diagnostic kit containing suitable means for detection, in particular the polypeptides or polynucleotides, antibodies or fragments or derivatives thereof described above, reagents, buffers, solutions and materials needed for setting up and carrying out the immunoassays, nucleic acid hybridization or PCR assays described above. Parts of the kit of the invention can be packaged individually in vials or bottles or in combination in containers or multicontainer units.

### Description of the Figures

**Figure 1****. Analysis of purified C6orf98 recombinant protein**
   Left panel: Comassie staining of purified His-tag C6orf98 fusion protein separated by SDS-PAGE; Right panel: WB on the purified recombinant protein stained with anti-C6orf98 antibody. Arrow marks the protein band of the expected size. Molecular weight markers are reported on the left.
**Figure 2****. Staining of breast tumor TMA with anti-C6orf98 antibodies**
   Examples of TMA of tumor (lower panel) and normal tissue samples (upper panel) stained with anti- C6orf98 antibodies. The antibody-stains specifically tumor cells (in dark gray);
**Figure 3****. Analysis of purified C9orf46 recombinant protein**
   Left panel: Comassie staining of purified His-tag C9orf46 fusion protein separated by SDS-PAGE; Right panel: WB on the C9orf46 protein stained with anti-C9orf46 antibody. Arrow marks the protein band of the expected size. Molecular weight markers are reported on the left.
**Figure 4****. Staining of breast tumor TMA with anti-C9orf46 antibodies**
   Examples of TMA of tumor (lower panel) and normal tissue samples (upper panel) stained with anti-C9orf46 antibodies. The antibody stains specifically tumor cells (in dark gray).
**Figure 5****. Expression of C9orf46 in breast tumor cell lines and tissue homogenates**
   Western blot analysis of C9orf46 expression in total protein extracts from: A) BT549 (line1) and MCF-7 (line2) breast tumor cells (corresponding to 2x10⁵ cells); B) HeLa cells (corresponding to 2x10⁵ cells) transfected with the empty pcDNA3 vector (lane 1) or with the plasmid construct encoding the C9orf46 gene (lane 2); C) Normal (lane 1=Pt#1; lane 2=Pt#2) or cancerous breast tissues from patients (lane 3=Pt#1; lane 4=Pt#2); stained with anti-C9orf46 antibody. Arrow marks the expected C9orf46 band. Molecular weight markers are reported on the left.

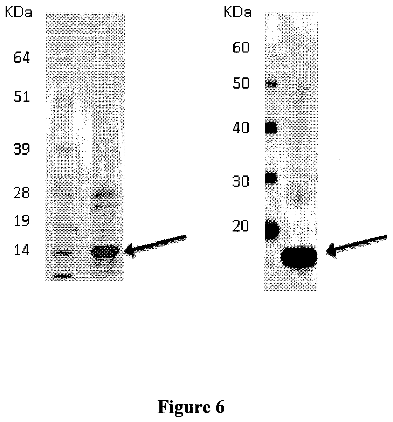

**Figure 6****. Analysis of purified FLJ37107 recombinant protein**
   Left panel: Comassie staining of purified His-tag FLJ37107 fusion protein separated by SDS-PAGE; Right panel: WB on the recombinant FLJ37107 protein stained with anti-FLJ37107 antibody. Arrow marks the protein band of the expected size. Molecular weight markers are reported on the left.

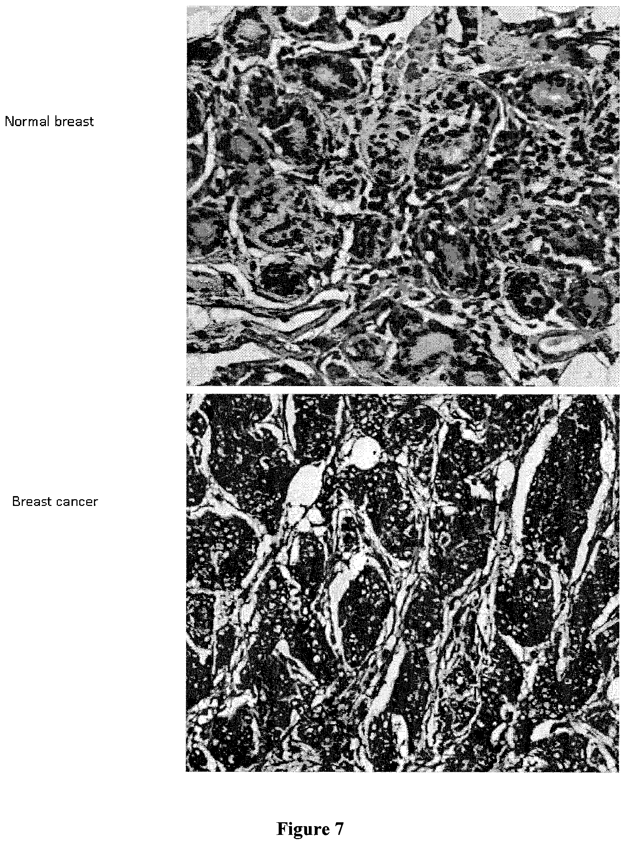

**Figure 7****. Staining of breast tumor TMA with anti-FLJ37107 antibodies**
   Examples of TMA of tumor (lower panel) and normal tissue samples (upper panel) stained with anti- FLJ37107 antibodies. The antibody stains specifically tumor cells (in dark gray).

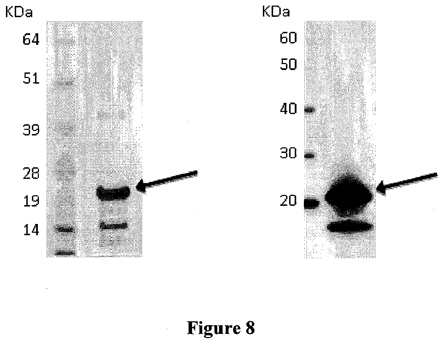

**Figure 8****. Analysis of purified YIPF2 recombinant protein**
   Left panel: Comassie staining of purified His-tag YIPF2 fusion protein separated by SDS-PAGE; Right panel: WB on the purified protein stained with anti-YIPF2 antibody. Arrow marks the protein band of the expected size.
   Molecular weight markers are reported on the left.

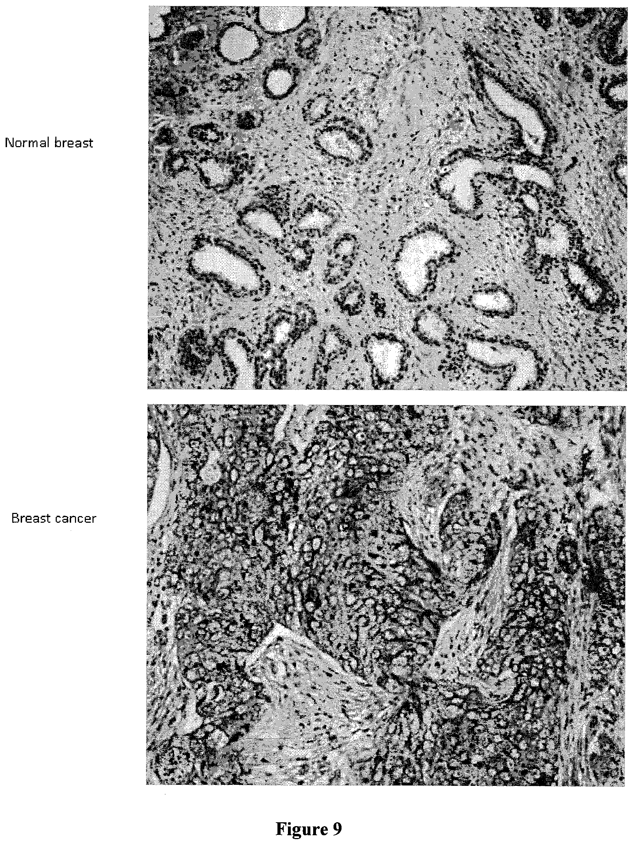

**Figure 9****. Staining of breast tumor TMA with anti-YIPF2 antibodies**
   Examples of TMA of tumor (lower panel) and normal tissue samples (upper panel) stained with anti-YIPF2 antibodies. The antibody stains specifically tumor cells (in dark gray).

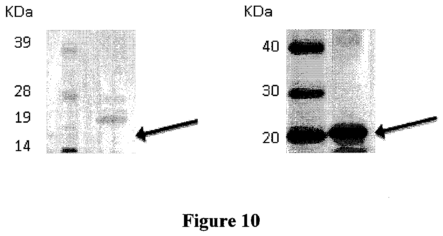

**Figure 10****. Analysis of purified UNQ6126 recombinant protein**
   Left panel: Comassie staining of purified His-tag UNQ6126 fusion protein separated by SDS-PAGE; Right panel: WB on the purified recombinant protein stained with anti- UNQ6126 antibody. Arrow marks the protein band of the expected size. Molecular weight markers are reported on the left.

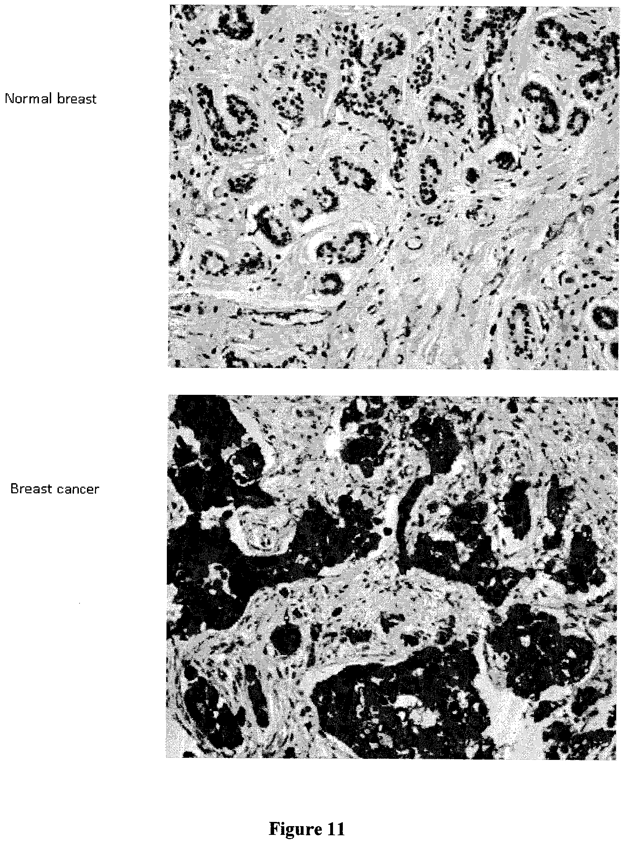

**Figure 11****. Staining of breast tumor TMA with anti-UNQ6126 antibodies**
   Examples of TMA of tumor (lower panel) and normal tissue samples (upper panel) stained with anti- UNQ6126 antibodies. The antibody stains specifically tumor cells (in dark gray).

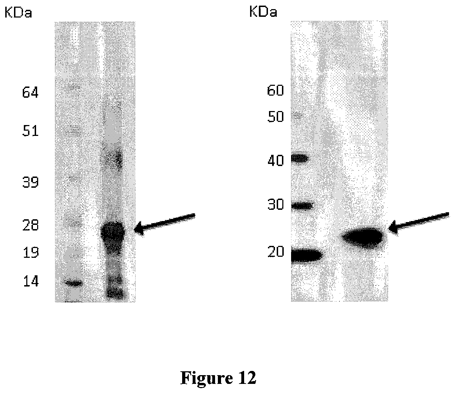

**Figure 12****. Analysis of purified TRYX3 recombinant protein**
   Left panel: Comassie staining of purified His-tag TRYX3 fusion protein separated by SDS-PAGE; Right panel: WB on the purified recombinant protein stained with anti-TRYX3 antibody. Arrow marks the protein band of the expected size. Molecular weight markers are reported on the left.

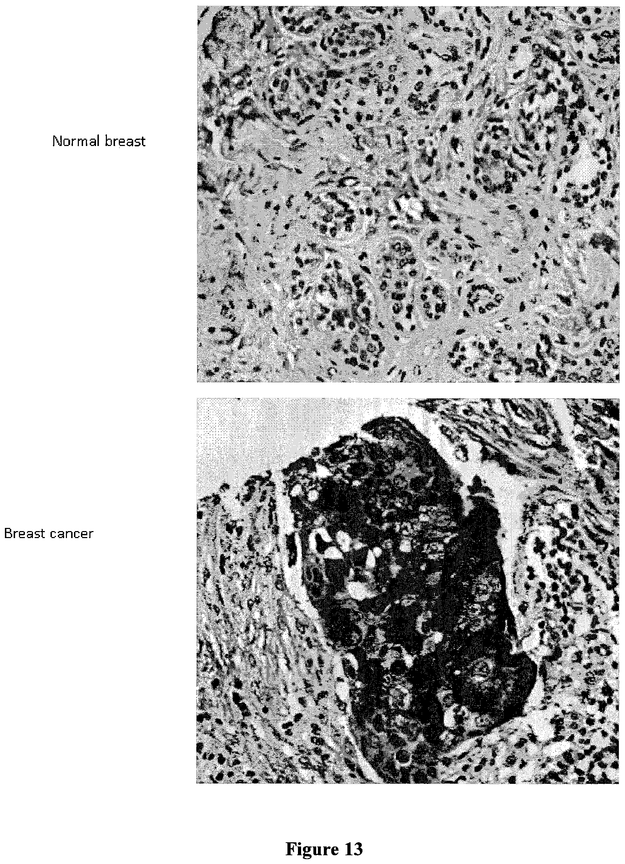

**Figure 13****. Staining of breast tumor TMA with anti-TRYX3 antibodies**
   Examples of TMA of tumor (lower panel) and normal tissue samples (upper panel) stained with anti-TRYX3 antibodies. The antibody stains specifically tumor cells (in dark gray).

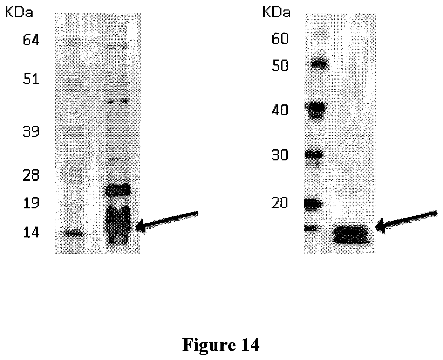

**Figure 14****. Analysis of purified DPY19L3 recombinant protein**
   Left panel: Comassie staining of purified His-tag DPY19L3 fusion protein separated by SDS-PAGE; Right panel: WB on the purified protein stained with anti-DPY19L3 antibody. Arrow marks the protein band of the expected size. Molecular weight markers are reported on the left.

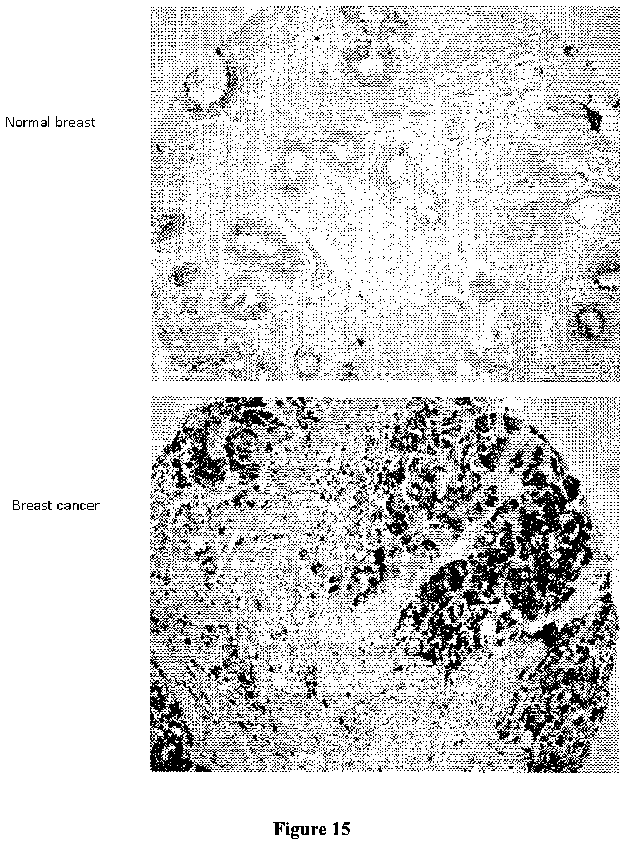

**Figure 15****. Staining of breast tumor TMA with anti-DPY19L3 antibodies**
   Examples of TMA of tumor (lower panel) and normal tissue samples (upper panel) stained with anti-DPY19L3 antibodies. The antibody stains specifically tumor cells (in dark gray).

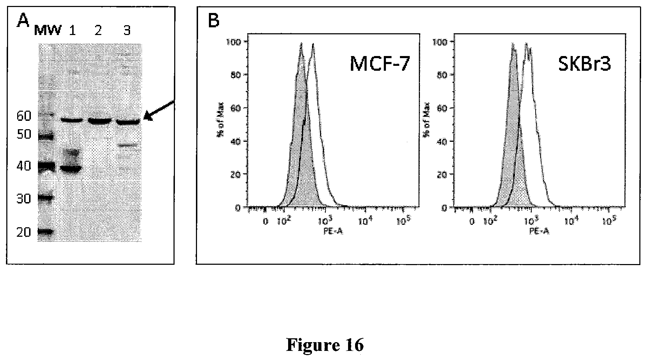

**Figure 16****. Expression and localization of DPY19L3 in tumor cell lines**
   Panel A: Western blot analysis of DPY19L3 expression in total protein extracts separated by SDS-PAGE from: MCF-7 (lane 1), MDA-MB231 (lane 2), SKBR-3 (lane 3), breast derived tumor cells; Arrow marks the protein band of the expected size.
      Molecular weight markers are reported on the left.
   Panel B: Flow cytometry analysis of DPY19L3 cell surface localization in MCF-7 and SKBR-3 cells stained with a negative control antibody (filled curve) or with anti-DPY19L3 antibody (empty curve). X axis, Fluorescence scale; Y axis, Cells (expressed as % relatively to major peaks).

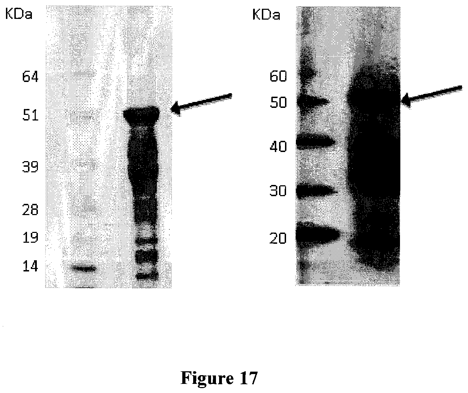

**Figure 17****. Analysis of purified SLC39A10 recombinant protein**
   Left panel: Comassie staining of purified His-tag SLC39A10 protein separated by SDS-PAGE; Right panel: WB on the recombinant protein stained with anti-SLC39A10 antibody. The low molecular weight bands correspond to partially degraded forms of SLC39A10 protein. Molecular weight markers are reported on the left.

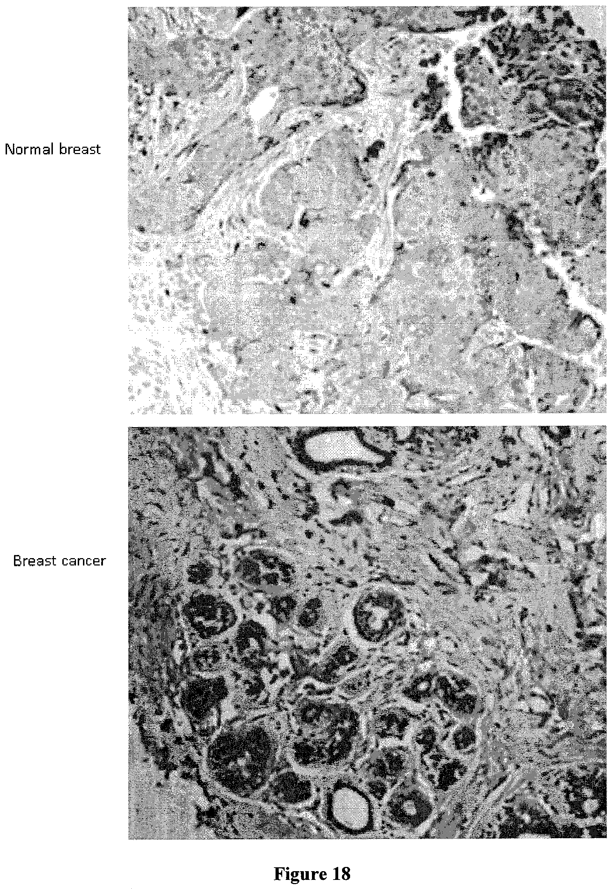

**Figure 18****. Staining of breast tumor TMA with anti-SLC39A10 antibodies**
   Examples of TMA of tumor (lower panel) and normal tissue samples (upper panel) stained with anti-SLC39A10 antibodies. The antibody stains specifically tumor cells (in dark gray).

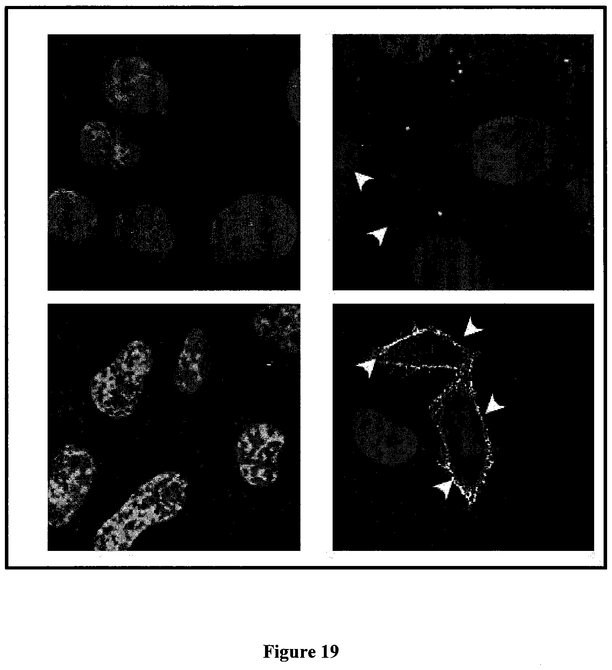

**Figure 19****. Confocal microscopy analysis of expression and localization of SLC39A10**
   HeLa cells transfected with the empty pcDNA3 vector (upper panels) or with the plasmid construct encoding the SLC39A10 gene (lower panels) stained with secondary antibodies (left panels) and with anti-SLC39A10 antibodies (right panels). Arrowheads mark surface specific localization.

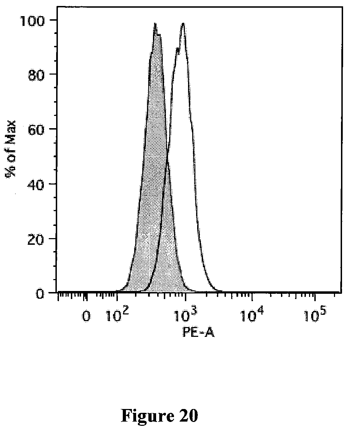

**Figure 20****. Expression and localization of SLC39A10 in breast tumor cells**
   Flow cytometry analysis of SLC39A10 cell surface localization SKBR3 tumor cells stained with a negative control antibody (filled curve or with anti-SLC39A10 antibody (empty curve). X axis, Fluorescence scale; Y axis, Cells (expressed as percentage relatively to major peaks).

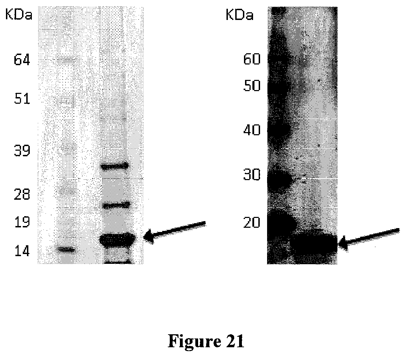

**Figure 21****. Analysis of purified C14orf135 recombinant protein**
   Left panel: Comassie staining of purified His-tag C14orf135 fusion protein espressed in *E. coli* separated by SDS-PAGE; Right panel: WB on the recombinant protein stained with anti-C14orf135 antibody. Arrow marks the protein band of the expected size. Molecular weight markers are reported on the left.

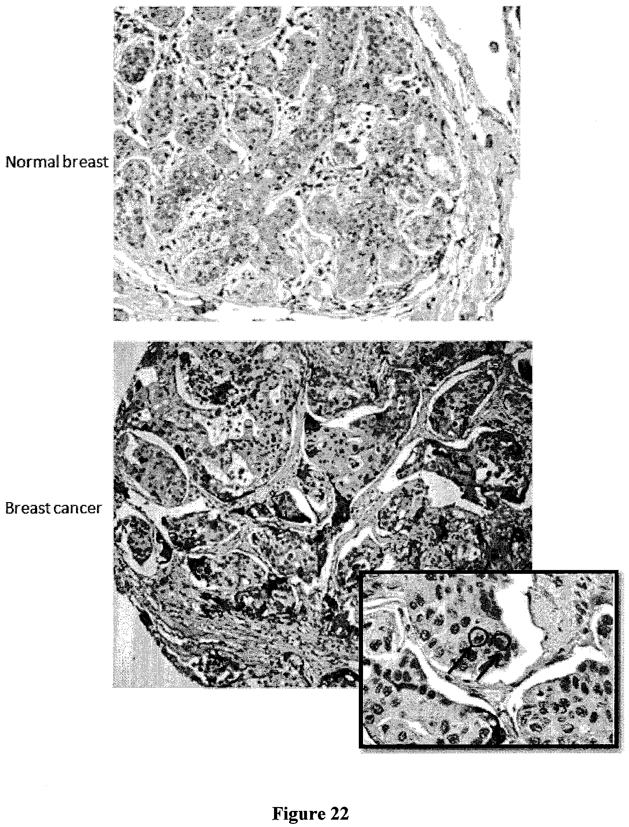

**Figure 22****. Staining of breast tumor TMA with anti-C14orf135 antibodies**
   Examples of TMA of tumor (lower panel) and normal tissue samples (upper panel) stained with anti-C14orf135 antibodies. The antibody stains specifically tumor cells and their secretion products (in dark gray). Moreover antibody stain also accumulated at the plasma membrane of tumor cells (boxed image, markerd by arrows).

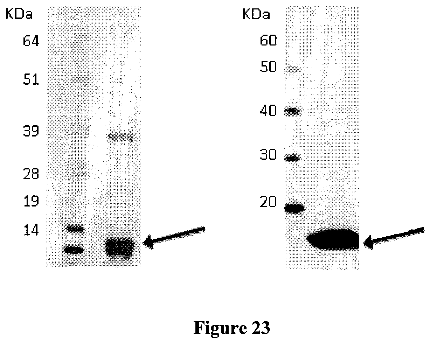

**Figure 23****. Analysis of purified DENND1B recombinant protein**
   Left panel: Comassie staining of purified His-tag DENND1B fusion protein separated by SDS-PAGE; Right panel: WB on the purified DENND1B protein stained with anti-DENND1B antibody. Arrow marks the protein band of the expected size. Molecular weight markers are reported on the left.

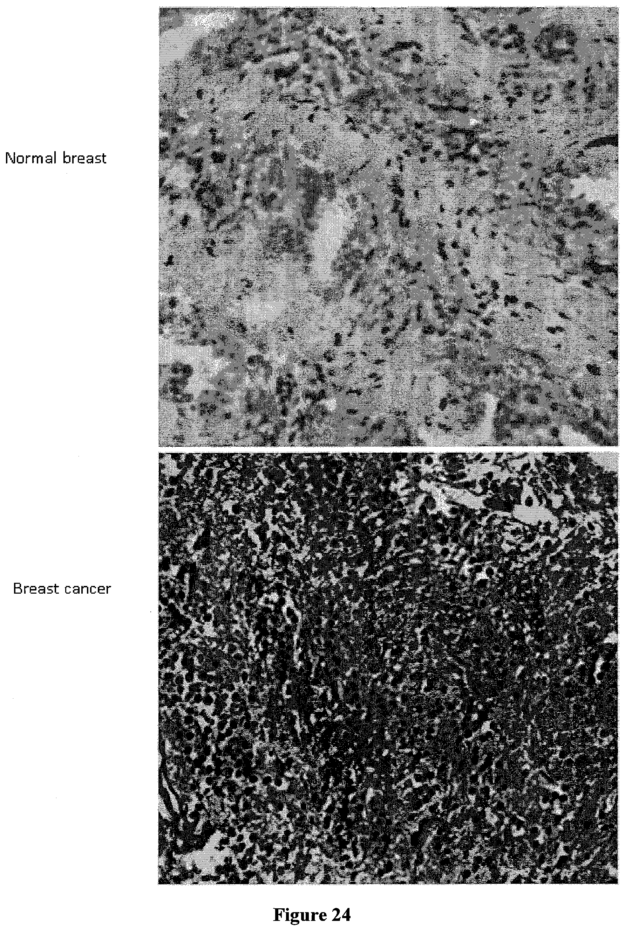

**Figure 24****. Staining of breast tumor TMA with anti-DENND1B antibodies**
   Examples of TMA of tumor (lower panel) and normal tissue samples (upper panel) stained with anti-DENND1B antibodies. The antibody stains specifically tumor cells (in dark gray).

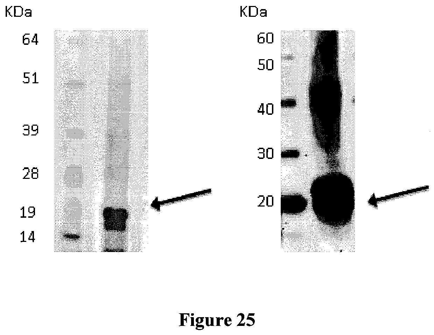

**Figure 25****. Analysis of purified EMID1 recombinant protein**
   Left panel: Comassie staining of purified His-tag EMID1 fusion protein separated by SDS-PAGE; Right panel: WB on the recombinant protein stained with anti- EMID1 antibody. Arrow marks the protein band of the expected size. The high molecular weight bands are consistent with multimers of the protein as defined by Mass spectromic analysis. Molecular weight markers are reported on the left.

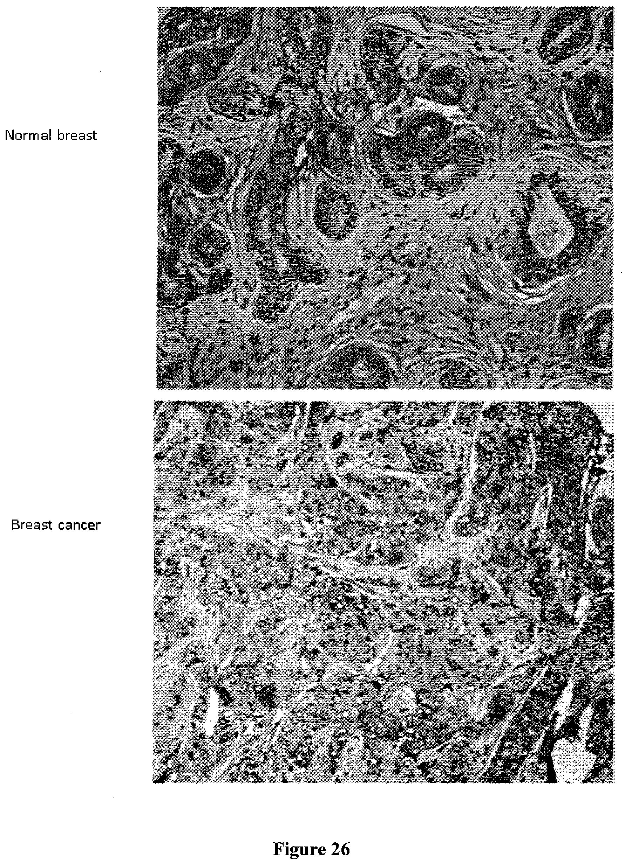

**Figure 26****. Staining of breast tumor TMA with anti-EMID1 antibodies**
   Examples of TMA of tumor (lower panel) and normal tissue samples (upper panel) stained with anti-EMID1 antibodies. The antibody specifically stains secretion products of tumor cells (in dark gray).

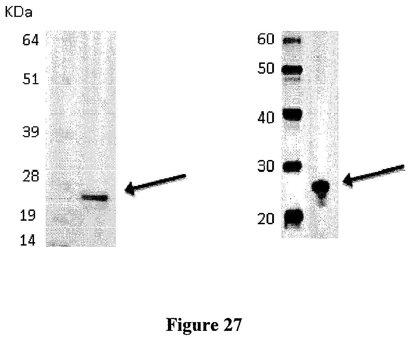

**Figure 27****. Analysis of purified ERMP1 recombinant protein**
   Left panel: Comassie staining of purified His-tag ERMP1 fusion protein separated by SDS-PAGE; Right panel: WB on the recombinant protein stained with anti- ERMP1 antibody. Arrow marks the protein band of the expected size. Molecular weight markers are reported on the left.

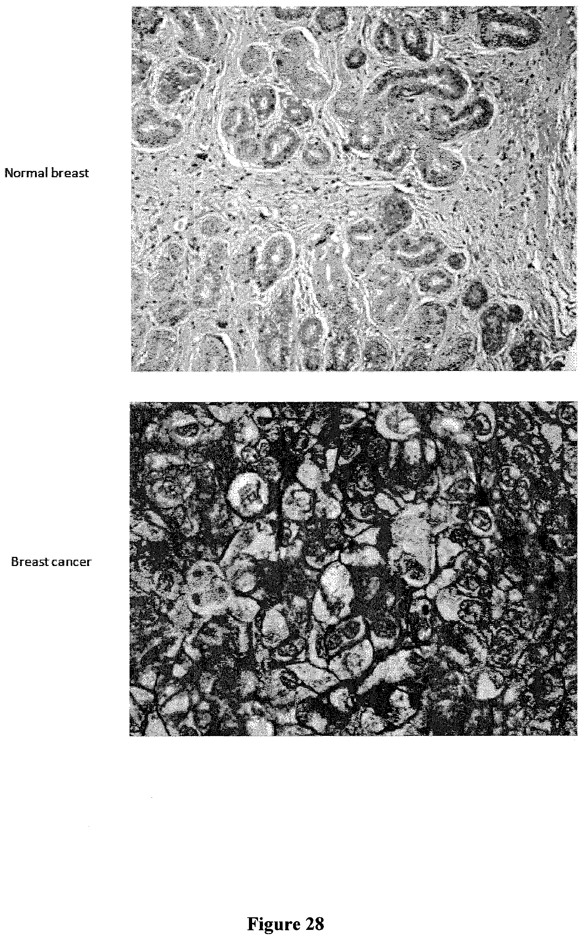

**Figure 28****. Staining of breast tumor TMA with anti-ERMP1 antibodies**
   Examples of TMA of tumor (lower panel) and normal tissue samples (upper panel) stained with anti-ERMP1 antibodies. The antibody stains specifically tumor cells (in dark gray).

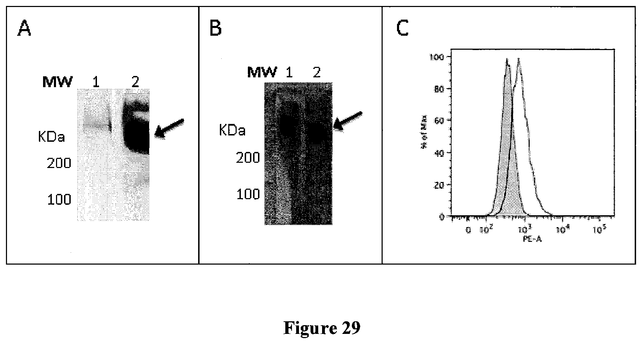

**Figure 29****. Expression and localization of ERMP1 in tumor cell lines**
   **Panel A:** Western blot analysis of ERMP1 expression in total protein extracts separated by SDS-PAGE from HEK-293T cells (corresponding to 1x10⁶ cells) transfected with the empty pcDNA3 vector (lane 1) or with the plasmid construct encoding the ERMP1 gene (lane 2);
   **Panel B:** Western blot analysis of ERMP1 expression in total protein extracts separated by SDS-PAGE from MCF-7 (lane 1) and SKBR-3 (lane 2) tumor cells (corresponding to 2x10⁵ cells). Arrow marks the expected ERMP1 band. Molecular weight markers are reported on the left.
   **Panel C:** Flow cytometry analysis of ERMP1 cell surface localization in SKBR-3 tumor cells stained with a negative control antibody (filled curve or with anti-ERMP1 antibody (empty curve). X axis, Fluorescence scale; Y axis, Cells (expressed as % relatively to major peaks).

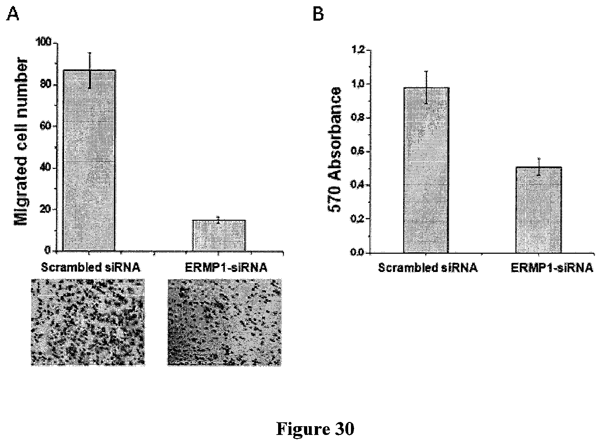

**Figure 30****. ERMP1 confers malignant cell phenotype** - The proliferation and the invasiveness properties of the MCF7 cell line were assessed after transfection with ERMP1-siRNA and a scramble siRNA control using the MTT and the Boyden in vitro invasion assays, respectively.
   **Panel A.** Cell migration/invasiveness measured by the Boyden migration assay. The graph represents the reduced migration/invasiveness of MCF7 treated with the ERMP1-specific siRNA. Small boxes under the columns show the visual counting of the migrated cells.
   **Panel B.** Cell proliferation determined by the MTT incorporation assay. The graph represents the reduced proliferation of the MCF7 tumor cells upon treatment with ERMP1-siRNA, as determined by spectrophotometric reading.

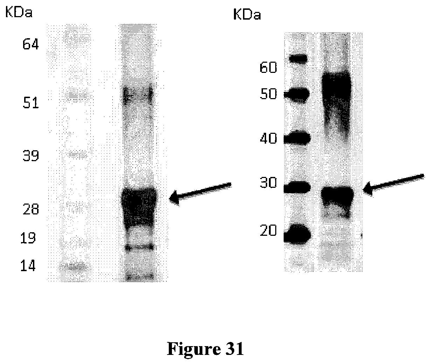

**Figure 31****. Analysis of purified CRISP3 recombinant protein**
   Left panel: Comassie staining of purified His-tag CRISP3 fusion protein separated by SDS-PAGE; Right panel: WB on the purified recombinant CRISP3 protein stained with anti- CRISP3 antibody. Arrow marks the protein band of the expected size. The high molecular weight bands are consistent with protein dimersas defined by Mass spectromic analysis. Molecular weight markers are reported on the left.

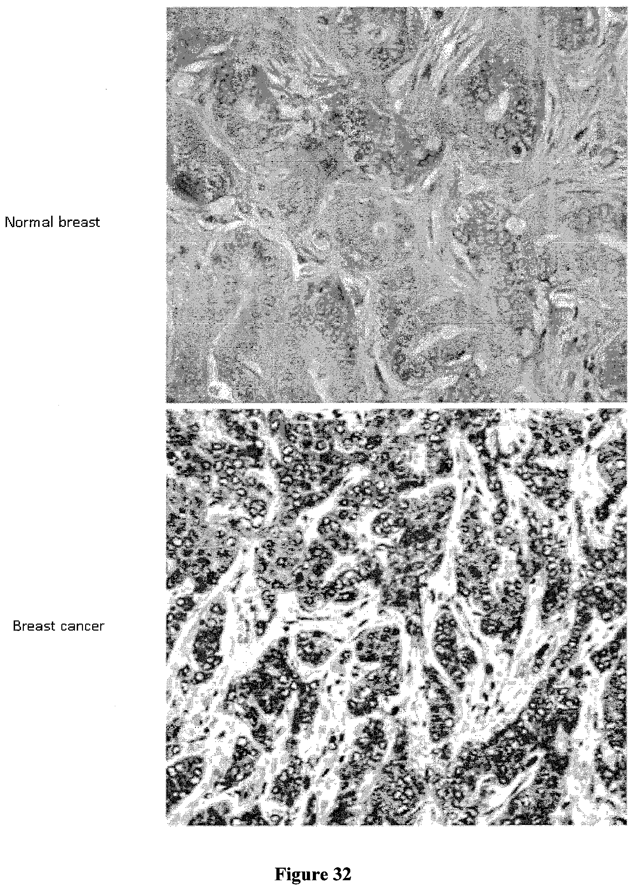

**Figure 32****. Staining of breast tumor TMA with anti-CRISP3 antibodies**
   Examples of TMA of breast tumor (lower panel) and normal tissue samples (upper panel) stained with anti-CRISP3 antibodies. The antibody stains specifically tumor cells (in dark gray).

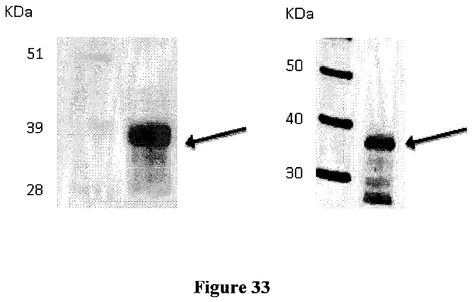

**Figure 33****. Analysis of purified KLRG2 recombinant protein expressed in *E.coli***
   Left panel: Comassie staining of purified His-tag KLRG2 fusion protein espressed in *E. coli* separated by SDS-PAGE; Right panel: WB on the purified recombinant protein stained with anti-KLRG2 antibody. Arrow marks the protein band of the expected size. Molecular weight markers are reported on the left.

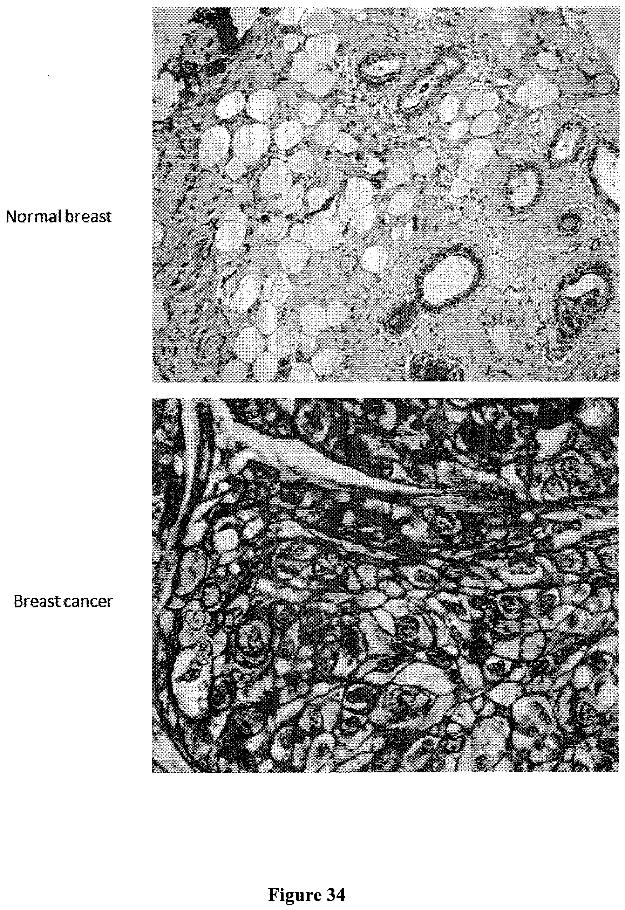

**Figure 34****. Staining of breast tumor TMA with anti-KLRG2 antibodies.** Examples of TMA of tumor (lower panel) and normal tissue samples (upper panel) stained with anti-KLRG2 antibodies. The antibody-stains specifically tumor cells (in dark gray).

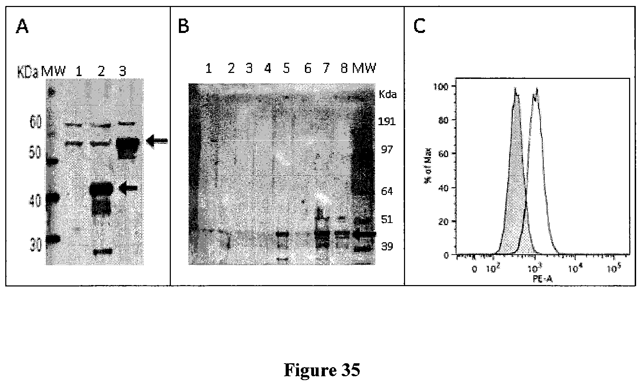

**Figure 35****. Expression and localization of KLRG2 in tumor cell lines**
   **Panel A:** Western blot analysis of KLRG2 expression in total protein extracts separated by SDS-PAGE from HeLa cells (corresponding to 1x10⁶ cells) transfected with the empty pcDNA3 vector (lane 1), with the plasmid construct encoding the isoform 2 of the KLRG2 gene (lane 2); or with the plasmid construct encoding the isoform1 of the KLRG2 gene (lane 3); Arrows mark the expected KLRG2 bands.
   **Panel B:** Western blot analysis of KLRG2 expression in total protein extracts separated by SDS-PAGE from normal breast tissues (1=Pt#1; 2=Pt#2; 3=Pt#3; 4=Pt#4) or from breast cancer tissues 5=Pt#1; 6=Pt#2; 7=Pt#3; 8=Pt#4); stained with anti-KLRG2 antibody. Arrow marks one of the expected KLRG2 band. Molecular weight markers are reported on the right.
   **Panel C:** Flow cytometry analysis of KLRG2 cell surface localization in SKBR-3 cells stained with a negative control antibody (filled curve or with anti-KLRG2 antibody (empty curve). X axis, Fluorescence scale; Y axis, Cells (expressed as % relatively to major peaks).

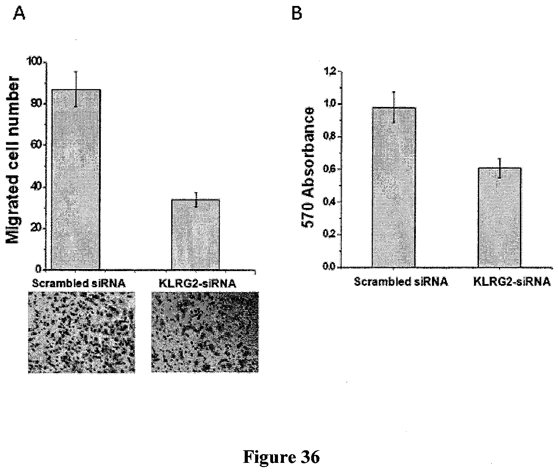

**Figure 36****. KLRG2 confer malignant cell phenotypes**
   The proliferation and the migration/invasiveness phenotypes of MCF7 cell line were assessed after transfection with KLRG2-siRNA and a scramble siRNA control using the MTT and the Boyden in vitro invasion assay, respectively.
   **Panel A.** Cell migration/invasiveness measured by the Boyden migration assay. The graph represents the reduced migration/invasiveness of MCF7 treated with the KLRG2 specific siRNA. Small boxes under the columns show the visual counting of the migrated cells.
   **Panel B.** Cell proliferation determined by the MTT incorporation assay. The graph represents the reduced proliferation of the MCF7 tumor cells upon treatment with KLRG2-siRNA, as determined by spectrophotometric reading.

The following examples further illustrate the invention.

### EXAMPLES

### Example 1. Generation of recombinant human protein antigens and

### antibodies to identify tumor markers

### Methods

The entire coding region or suitable fragments of the genes encoding the target proteins, were designed for cloning and expression using bioinformatic tools with the human genome sequence as template (Lindskog M et al (2005). Where present, the leader sequence for secretion was replaced with the ATG codon to drive the expression of the recombinant proteins in the cytoplasm of E. coli. For cloning, genes were PCR-amplified from templates derived from Mammalian Gene Collection (http://mgc.nci.nih.gov/) clones using specific primers. Clonings were designed so as to fuse a 10 histidine tag sequence at the 5' end, annealed to in house developed vectors, derivatives of vector pSP73 (Promega) adapted for the T4 ligation independent cloning method (Nucleic Acids Res. 1990 October 25; 18(20): 6069-6074) and used to transform *E.coli* NovaBlue cells recipient strain. *E. coli* tranformants were plated onto selective LB plates containing 100 µg/ml ampicillin (LB Amp) and positive E.coli clones were identified by restriction enzyme analysis of purified plasmid followed by DNA sequence analysis. For expression, plasmids were used to transform BL21-(DE3) *E.coli* cells and BL21-(DE3) E. coli cells harbouring the plasmid were inoculated in ZYP-5052 growth medium (Studier, 2005) and grown at 37°C for 24 hours. Afterwards, bacteria were collected by centrifugation, lysed into B-Per Reagent containing 1 mM MgCl2, 100 units DNAse I (Sigma), and 1 mg/ml lysozime (Sigma). After 30 min at room temperature under gentle shaking, the lysate was clarified by centrifugation at 30.000 g for 40 min at 4°C. All proteins were purified from the inclusion bodies by resuspending the pellet coming from lysate centrifugation in 40 mM TRIS-HCl, 1 mM TCEP {Tris(2-carboxyethyl)-phosphine hydrochloride, Pierce} and 6M guanidine hydrochloride, pH 8 and performing an IMAC in denaturing conditions. Briefly, the resuspended material was clarified by centrifugation at 30.000 g for 30 min and the supernatant was loaded on 0.5 ml columns of Ni-activated Chelating Sepharose Fast Flow (Pharmacia). The column was washed with 50 mM TRIS-HCl buffer, 1 mM TCEP, 6M urea, 60 mM imidazole, 0.5M NaCl, pH 8. Recombinant proteins were eluted with the same buffer containing 500 mM imidazole. Proteins were analysed by SDS-Page and their concentration was determined by Bradford assay using the BIORAD reagent (BIORAD) with a bovine serum albumin standard according to the manufacturer's recommendations. The identity of recombinant affinity purified proteins was further confirmed by mass spectrometry (MALDI-TOF), using standard procedures.

To generate antisera, the purified proteins were used to immunize CD1 mice (6 week-old females, Charles River laboratories, 5 mice per group) intraperitoneally, with 3 protein doses of 20 micrograms each, at 2 week-interval. Freund's complete adjuvant was used for the first immunization, while Freund's incomplete adjuvant was used for the two booster doses. Two weeks after the last immunization animals were bled and sera collected from each animal was pooled.

### Results

Gene fragments of the expected size were obtained by PCR from specific clones of the Mammalian Gene Collection or, alternatively, from cDNA generated from pools of total RNA derived from Human testis, Human placenta, Human bone marrow, Human fetal brain, using primers specific for each gene.

For the C6orf98 gene, a fragment corresponding to nucleotides 67 to 396 of the transcript ENST00000409023 and encoding a protein of 110 residues, corresponding to the amino acid region from 22 to 132 of ENSP00000386324 sequence was obtained.

For the C9orf46 gene, a fragment corresponding to nucleotides 439 to 663 of the transcript ENST00000107020 and encoding a protein of 75 residues, corresponding to the amino acid region from 73 to 147 of ENSP00000223864 sequence was obtained.

For the FLJ37107 gene, a fragment corresponding to nucleotides 661-972of the transcript gi|58218993|ref|NM_001010882.1 and encoding a protein of 104 residues, corresponding to the amino acid region from 1 to 104of gi|58218994|ref|NP_001010882.1 sequence was obtained.

For the YIPF2 gene, a fragment corresponding to nucleotides 107 to 478 of the transcript ENST00000393508 and encoding a protein of 124 residues, corresponding to the amino acid region from 1 to 124 of ENSP00000377144 sequence was obtained.

For the UNQ6126 gene, a fragment corresponding to a fragment corresponding to nucleotides 88 to 471 of the transcript gi|169216088|ref|XM_001719570.1| and encoding a protein of 128 residues, and encoding an amino acid region from 30 to 147 of sp|Q6UXV3|YV010 sequence was obtained.

For the TRYX3 gene, a fragment corresponding to nucleotides 230 to 781 of the transcript ENST00000304182 and encoding a protein of 184 residues, corresponding to the amino acid region from 41 to 224 of ENSP00000307206 sequence was obtained.

For the DPY19L3 gene, a fragment corresponding to nucleotides 158 to 463 of the transcript ENST00000392250 and encoding a protein of 102 residues, corresponding to the amino acid region from 1 to 102 of ENSP00000376081 sequence was obtained.

For the SLC39A10 gene, a DNA fragment corresponding to nucleotides 154-1287 of the transcript ENST00000359634 and encoding a protein of 378 residues, corresponding to the amino acid region from 26 to 403 of ENSP00000352656 sequence was obtained.

For the C14orf135 gene, a fragment corresponding to nucleotides 2944 to 3336 of the transcript ENST00000317623 and encoding a protein of 131 residues, corresponding to the amino acid region 413 to 543 of ENSP00000317396 sequence was obtained.

For the DENND1B gene, a fragment corresponding to nucleotides 563 to 1468 of the transcript ENST00000235453 and encoding a protein of 302 residues, corresponding to the amino acid region from 95 to 396 of ENSP00000235453 sequence was obtained.

For the EMID1 gene, a fragment corresponding to nucleotides 203-670 of the transcript OTTHUMT00000075712 and encoding a protein of 156 residues, corresponding to the amino acid region from 33 to 188 of OTTHUMP00000028901 sequence was obtained.

For the ERMP1 gene, a fragment corresponding to nucleotides 55-666 of the transcript ENST00000339450 and encoding a protein of 204 residues, corresponding to the amino acid region from 1 to 204 of ENSP00000340427 sequence was obtained.

For the CRISP3 gene, a fragment corresponding to nucleotides 62-742 of the transcript ENST00000393666 and encoding a protein of 227 residues, corresponding to the amino acid region from 19 to 245 of ENSP0000037727 sequence was obtained.

For the KLRG2 gene, a fragment corresponding to nucleotides 70 to 849 of the transcript ENST00000340940 and encoding a protein of 260 residues, corresponding to the amino acid region from 1 to 260 of ENSP00000339356 sequence was obtained.

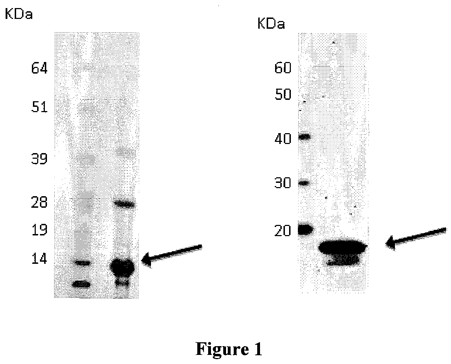

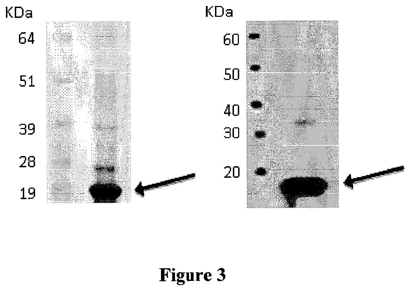

A clone encoding the correct amino acid sequence was identified for each gene/gene-fragment and, upon expression in *E. coli,* a protein of the correct size was produced and subsequently purified using affinity chromatography (Figures 1, 3, 6, 8, 10, 12, 14, 17, 21, 23, 25, 27, 31, 33 left panels). As shown in the figures, in some case SDS-PAGE analysis of affinity-purified recombinant proteins revealed the presence of extra bands, of either higher and/or lower masses. Mass spectrometry analysis confirmed that they corresponded to either aggregates or degradation products of the protein under analysis.

Antibodies generated by immunization specifically recognized their target proteins in Western blot (WB) (Figures 1, 3, 6, 8, 10, 12, 14, 17, 21, 23, 25, 27, 31, 33; right panels).

### Example 2. Tissue profiling by immune-histochemistry

### Methods

The analysis of the antibodies' capability to recognize their target proteins in tumor samples was carried out by Tissue Micro Array (TMA), a miniaturized immuno-histochemistry technology suitable for HTP analysis that allows to analyse the antibody immuno-reactivity simultaneously on different tissue samples immobilized on a microscope slide.

Since the TMAs include both tumor and healthy tissues, the specificity of the antibodies for the tumors can be immediately appreciated. The use of this technology, differently from approaches based on transcription profile, has the important advantage of giving a first hand evaluation on the potential of the markers in clinics. Conversely, since mRNA levels not always correlate with protein levels (approx. 50% correlation), studies based on transcription profile do not provide solid information regarding the expression of protein markers.

A tissue microarray was prepared containing formalin-fixed paraffin-embedded cores of human tissues from patients affected by breast cancer and corresponding normal tissues as controls and analyzed using the specific antibody sample. In total, the TMA design consisted in 10 tumor breast tumor samples and 10 normal tissues from 5 well pedigreed patients (equal to two tumor samples and 2 normal tissues from each patient) to identify promising target molecules differentially expressed in cancer and normal cells. The direct comparison between tumor and normal tissues of each patient allowed the identification of antibodies that stain specifically tumor cells and provided indication of target expression in breast tumor.

To further confirm the association of each protein with breast tumors a tissue microarray was prepared containing 100 formalin-fixed paraffin-embedded cores of human breast tissues from 50 patients (equal to two tissue samples from each patient).

All formalin fixed, paraffin embedded tissues used as donor blocks for TMA production were selected from the archives at the IEO (Istituto Europeo Oncologico, Milan). Corresponding whole tissue sections were examined to confirm diagnosis and tumour classification, and to select representative areas in donor blocks. Normal tissues were defined as microscopically normal (non- neoplastic) and were generally selected from specimens collected from the vicinity of surgically removed tumors. The TMA production was performed essentially as previously described (Kononen J et al (1998) Nature Med. 4:844-847; Kallioniemi OP et al (2001) Hum. MoI. Genet. 10:657-662). Briefly, a hole was made in the recipient TMA block. A cylindrical core tissue sample (1 mm in diameter) from the donor block was acquired and deposited in the recipient TMA block. This was repeated in an automated tissue arrayer "Galileo TMA CK 3500" (BioRep, Milan) until a complete TMA design was produced. TMA recipient blocks were baked at 42 <0>C for 2 h prior to sectioning. The TMA blocks were sectioned with 2-3 mm thicknes using a waterfall microtome (Leica), and placed onto poly-L-lysinated glass slides for immunohistochemical analysis. Automated immunohistochemistry was performed as previously described (Kampf C. et al (2004) Clin. Proteomics 1:285-300). In brief, the glass slides were incubated for 30' min in 60°C, de-paraffinized in xylene (2 x 15 min) using the Bio-Clear solution (Midway. Scientific, Melbourne, Australia), and re-hydrated in graded alcohols. For antigen retrieval, slides were immersed 0.01 M Na-citrate buffer, pH 6.0 at 99°C for 30 min Slides were placed in the Autostainer (R) (DakoCytomation) and endogenous peroxidase was initially blocked with 3% H2O2, for 5 min. Slides were then blocked in Dako Cytomation Wash Buffer containing 5% Bovine serum albumin (BSA) and subsequently incubated with mouse antibodies for 30' (dilution 1:200 in Dako Real ™ dilution buffer). After washing with DakoCytomation wash buffer, slides were incubated with the goat anti-mouse peroxidase conjugated Envision(R) for 30 min each at room temperature (DakoCytomation). Finally, diaminobenzidine (DakoCytomation) was used as chromogen and Harris hematoxylin (Sigma- Aldrich) was used for counterstaining. The slides were mounted with Pertex(R) (Histolab).

The staining results have been evaluated by a trained pathologist at the light microscope, and scored according to both the percentage of immunostained cells and the intensity of staining. The individual values and the combined score (from 0 to 300) were recorded in a custom-tailored database. Digital images of the immunocytochemical findings have been taken at a Leica DM LB light microscope, equipped with a Leica DFC289 color camera.

### Results

A TMA design was obtained, representing tumor tissue samples and normal tissues, derived from patients affected by breast tumor. The results from tissue profiling showed that the antibodies specific for the recombinant proteins (see Example 1) are strongly immunoreactive on breast tumor cancer tissues, while no or poor reactivity was detected in normal tissues, indicating the presence of the target proteins in breast tumors. Based on this finding, the detection of target proteins in tissue samples can be associated with breast tumor. In some cases immunoreactivity accumulated at the cell membrane of tumor cells providing a first-hand indication on the surface localization of the target proteins.

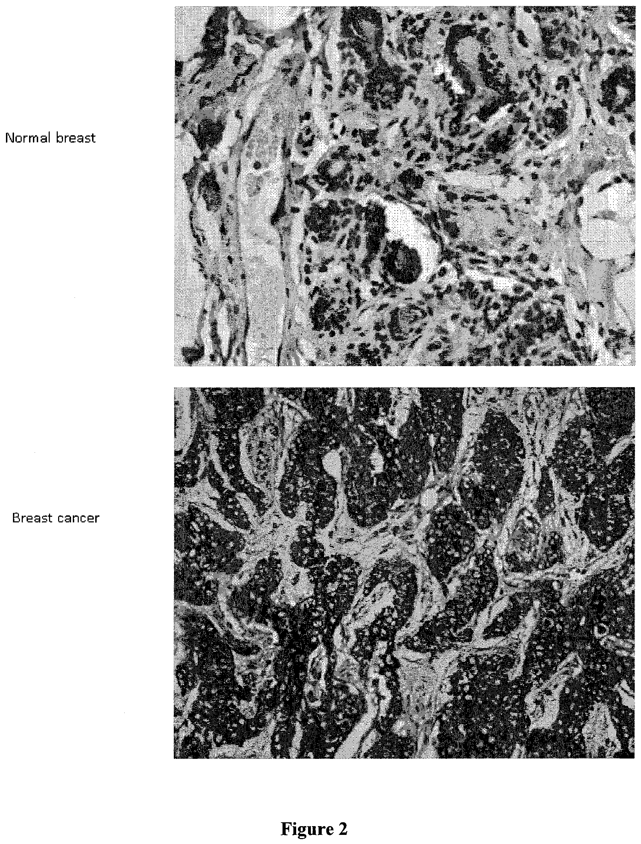

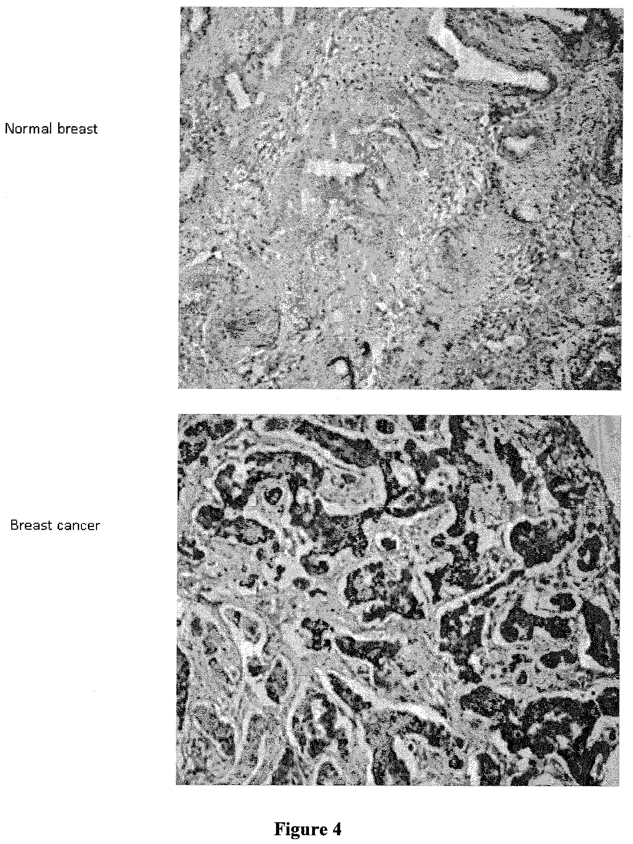

The capability of target-specific antibodies to stain breast tumor tissues is summarized in Table I. Representative examples of microscopic enlargements of tissue samples stained by each antibody are reported in Figures 2; 4; 7; 9; 11; 13; 15; 18; 22; 24; 26; 28; 32, 34).

Table reports the percentage of positive breast tumor tissue samples after staining with the target specific antibodies.

| **Marker name** | **Percentage of Breast tumor tissues showing positive IHC staining** |
|---|---|
| C6orf98 | 80 |
| C9orf46 | 20 |
| FLJ37107 | 60 |
| YIPF2 | 40 |
| UNQ6126 | 82 |
| TRYX3 | 40 |
| DPY19L3 | 83 |
| SLC39A10 | 27 |
| C14orf135 | 20 * |
| DENND1B | 20 |
| EMID1 | 20 * |
| ERMP1 | 45** |
| CRISP3 | 40 |
| KLRG2 | 34** |

| | |
|---|---|
| (*) The antibody stains both breast tumor cells and secretion products indicating that the corresponding proteins are specifically released by tumor cells. (**) The antibody stains the cell membrane of tumor cells | |

### Example 3. Expression and localization of target protein in transfected mammalian cells

### Methods

The specificity of the antibodies for each target proteins was assessed by Western blot analysis on total protein extracts from eukaryotic cells transiently transfected with plasmid constructs containing the complete sequences of the genes encoding the target proteins. Where indicated, expression and localization of target proteins were investigated by confocal microscopy analysis of transfected cells. Examples of this type of experiments are represented for C9orf46 (corresponding to Transcript ID ENST00000223864), KLRG2 (cloned sequences corresponding to Transcripts ENST00000340940 and ENST00000393039, corresponding to two transcript variants), ERMP1 (cloned sequence corresponding to Transcripts ENST00000339450), SLC39A10 (cloned sequence corresponding to Transcript ENST00000359634).

For clonings, cDNA were generated from pools of total RNA derived from Human testis, Human placenta, Human bone marrow, Human fetal brain, in reverse transcription reactions and the entire coding regions were PCR-amplified with specific primers pairs. PCR products were cloned into plasmid pcDNA3 (Invitrogen). HeLa or Hek-293T cells were grown in DMEM-10% FCS supplemented with 1 mM Glutamine were transiently transfected with preparation of the resulting plasmids and with the empty vector as negative control using the Lipofectamine-2000 transfection reagent (Invitrogen). After 48 hours, cells were collected and analysed by Western blot or confocal microscopy. For Western blot, cells were lysed with PBS buffer containing 1% Triton X100 and total cell extracts (corresponding to 2x10⁵ cells) were separated on pre-cast SDS-PAGE gradient gels (NuPage 4-12% Bis-Tris gel, Invitrogen) under reducing conditions, followed by electro-transfer to nitrocellulose membranes (Invitrogen) according to the manufacturer's recommendations. The membranes were blocked in blocking buffer composed of 1x PBS-0.1% Tween 20 (PBST) added with 10% dry milk, for 1 h at room temperature, incubated with the antibody diluted 1:2500 in blocking buffer containing 1% dry milk and washed in PBST-1%. The secondary
HRP-conjugated antibody (goat anti-mouse immunoglobulin/HRP, Perkin Elmer) was diluted 1:5000 in blocking buffer and chemiluminescence detection was carried out using a Chemidoc-IT UVP CCD camera (UVP) and the Western lightning^{Tm} cheminulescence Reagent Plus (Perkin Elmer), according to the manufacturer's protocol.

For confocal microscopy analysis, the cells were plated on glass cover slips and after 48 h were washed with PBS and fixed with 3% *p*-formaldheyde solution in PBS for 20 min at RT. For surface staining, cells were incubated overnight at 4°C with polyclonal antibodies (1:200). The cells were then stained with Alexafluor 488-labeled goat anti-mouse antibodies (Molecular Probes). DAPI (Molecular Probes) was used to visualize nuclei; Live/Dead® red fixable (Molecular Probes) was used to visualize membrane. The cells were mounted with glycerol plastine and observed under a laser-scanning confocal microscope (LeicaSP5).

### Results

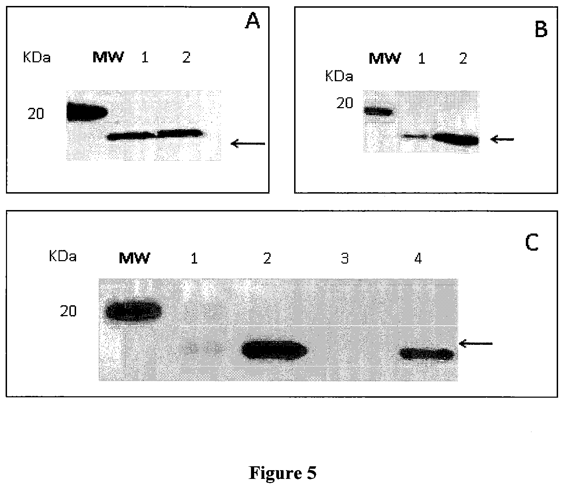

The selected coding sequences for C9orf46, SLC39A10, KLRG2 and ERMP1 were cloned in a eukaryotic expression vector and the derived plasmids were used for transient transfection of HeLa or HEK293T cells. Expression of target proteins Corf46 and KLRG2 was detected by Western blot in total protein extracts from HeLa, while expression of ERMP1 was analysed in trasnfected HEK-293T cells. Overall the data confirmed that the marker-specific antibodies recognized specifically their target proteins. Concerning C9orf46, a band of the expected size was visible in HeLa cells transfected with the C9orf46- expressing plasmid while the same band was either not visible or very faintly detected in HeLa cells transfected with the empty pcDNA3 plasmid (Figure 5B). In the case of KLRG2, specific protein bands of expected size were detected in cells transfected with either of the two plasmids encoding the two annotated KLRG2 variants (Figure 35A). As for cells transfected with ERMP1-encoding plasmid, a band of high molecular mass was specifically detected by the anti-ERMP1 antibody indicating that the protein forms stable aggregates (Figure 29A). Expression of protein SLC3910 was carried by confocal microscopy of transfected cells. The the anti-SLC39A10 specifically detected its target protein expressed by transfected cells, while no staining was visible in cell transfected with the empty pcDNA3 vector untransfected cells. In particular, the antibody mainly stained the surface of transfected cells (Figure 19).

This indicates that this target protein is localized on the extracellular plasma membrane, accessible to the external environment.

### Example 4. Detection of target protein in tumor tissue homogenates

The presence of protein bands corresponding to the marker proteins was also investigated in tissue homogenates of breast tumor biopsies as compared to normal tissues from patients. Homogenates were prepared by mechanic tissue disruption in buffer containing 40 mM TRIS-HCl, 1 mM TCEP {Tris(2-carboxyethyl)-phosphine hydrochloride, Pierce} and 6M guanidine hydrochloride, pH 8. Western blot was performed by separation of the total protein extracts (20 µg/lane) proteins were detected by specific antibodies.

### Results

An example of this type of experiments is represented for protein C9orf46 and KLRG2. Antibodies specific C9orf46 and KLRG2 detected a specific protein band in breast tumor homogenates, while no or very faint bands were detected in normal breast homogenates, confirming the presence of the marker proteins in breast tumor. Results are reported in Figure 5C and Figure 35B.

### Example 5. Expression of target protein in tumor cell lines

Expression of target proteins was also assessed by WB and/or Flow cytometry on total extracts from breast tumor cell lines, including BT549, MCF7, MDA-MB231 and SKBR-3.

In each analysis, cells were cultured in under ATCC recommended conditions, and sub-confluent cell monolayers were detached with PBS-0.5 mM EDTA. For Western blot analysis, cells were lysed by several freeze-thaw passages in PBS-1% Triton. Total protein extracts were loaded on SDS-PAGE (2x10⁵ cells/lane), and subjected to WB with specific antibodies as described above.

For flow cytometry analysis, cells (2x10⁴ per well) were pelletted in 96 U-bottom microplates by centrifugation at 200 x g for 5 min at 4°C and incubated for 1 hour at 4°C with the appropriate dilutions of the marker-specific antibodies. Cells were washed twice in PBS-5% FCS and incubated for 20 min with the appropriate dilution of R-Phycoerythrin (PE)-conjugated secondary antibodies (Jackson Immuno Research, PA, USA) at 4°C. After washing, cells were analysed by a FACS Canto II flow cytometer (Becton Dickinson). Data were analyzed with FlowJo 8.3.3 program.

### Results

Example of the expression analysis is represented for C9orf46, DPY19L3, ERMP1, and SLC39A10.

Western blot analysis of C9orf46 showed that a protein band of the expected sizes was detected in total protein extracts of breast tumor cell lines (BT549, MCF7), confirming its expression in breast tumor cell lines derived from breast tumor (Figure 5A). Concerning ERMP1, both Western blot and flow cytometry analysis are represented. Western blot analysis shows a band of high molecular mass detected in the breast cell lines MCF7 and SKBR-3, showing an electrophoretic pattern similar to that reported in transfected cells (see Example 3). This further confirms the existence of stable aggregates for this protein confirming its expression in cell lines derived from breast tumor (Figure 29B). Flow cytometry analysis indicates that ERMP1 is detected on the surface of the SKBR-3 cell line (Figure 29C). As for DPY19L3, Western blot analysis showed a band of expected size in MCF7, MDA-MB231 and SKBr-3 was detected by the antibody o a panel of tumor cells lines (Figure 16A). Flow cytometry analysis indicates that this protein is detected on the surface of MCF7 and SKBr-2 cell lines (Figure 16B). Finally, expression and localization of SLC39A10 was analysed by flow cytometry. Results show that this protein is detected on the surface of the SKBR-3 cell line by the specific antibody (Figure 20).

### Example 6. Expression of the marker proteins confers malignant cell phenotype

To verify that the proteins included in the present invention can be exploited as targets for therapeutic applications, the effect of marker depletion was evaluated *in vitro* in cellular studies generally used to define the role of newly discovered proteins in tumor development. Marker-specific knockdown and control tumor cell lines were assayed for their proliferation and the migration/invasiveness phenotypes using the MTT and the Boyden in vitro invasion assay, respectively.

### Method

Expression of marker genes were silenced in tumor cell lines by the siRNA technology and the influence of the reduction of marker expression on cell parameters relevant for tumor development was assessed in *in vitro* assays. The expression of marker genes was knocked down in a panel of epithelial tumor cell lines previously shown to express the tumor markers using a panel of marker-specific siRNAs (whose target sequences are reported in the Table II) using the HiPerfect transfection reagent (QIAGEN) following the manufacturer's protocol. As control, cells treated with irrelevant siRNA (scrambled siRNA) were analysed in parallel. At different time points (ranging from 24 to 72 hours) post transfection, the reduction of gene transcription was assessed by quantitative RT-PCR (Q-RT-PCR) on total RNA, by evaluating the relative marker transcript level, using the beta-actin, GAPDH or MAPK genes as internal normalization control. Afterwards, cell proliferation and migration/invasiveness assays were carried out to assess the effect of the reduced marker expression. Cell proliferation was determined using the MTT assay, a colorimetric assay based on the cellular conversion of a tetrazolium salt into a purple colored formazan product. Absorbance of the colored solution can be quantified using a spectrophotometer to provide an estimate of the number of attached living cells. Approximately 5 x 10³ cells/100µl were seeded in 96-well plates in DMEM with 10% FCS to allow cell attachment. After overnight incubation with DMEM without FCS, the cells were treated with 2,5% FBS for 72 hours. Four hours before harvest 15 µL of the MTT dye solution (Promega) were added to each well. After 4-hour incubation at 37°C, the formazan precipitates were solubilized by the addition of 100 µL of solubilization solution (Promega) for 1h at 37°C,. Absorbance at 570 nm was determined on a multiwell plate reader (SpectraMax, Molecular Devices).

Cell migration/invasiveness was tested using the Boyden *in vitro* invasion assay, as compared to control cell lines treated with a scramble siRNA. This assay is based on a chamber of two medium-filled compartments separated by a microporous membrane. Cells are placed in the upper compartment and are allowed to migrate through the pores of the membrane into the lower compartment, in which chemotactic agents are present. After an appropriate incubation time, the membrane between the two compartments is fixed and stained, and the number of cells that have migrated to the lower side of the membrane is determined. For this assay, a transwell system, equipped with 8-µm pore polyvinylpirrolidone-free polycarbonate filters, was used. The upper sides of the porous polycarbonate filters were coated with 50 µg/cm² of reconstituted Matrigel basement membrane and placed into six-well culture dishes containing complete growth medium. Cells (1x10⁴ cells/well) were loaded into the upper compartment in serum-free growth medium. After 16 h of incubation at 37°C, non-invading cells were removed mechanically using cotton swabs, and the microporous membrane was stained with Diff-Quick solution. Chemotaxis was evaluated by counting the cells migrated to the lower surface of the polycarbonate filters (six randomly chosen fields, mean ± SD).

### Results

Examples of this analysis are reported for ERMP1 and KLRG2 in the breast tumor cell line MCF7. Gene silencing experiments with marker-specific siRNA reduced the marker transcripts (approximately 30-40 fold reduction), as determined by Q-RT_PCR. T II reports the sequences targeted by the siRNA molecules. The reduction of the expression of either of the two genes significantly impairs the proliferation and the invasiveness phenotypes of the MCF7 breast tumor cell line (Figures 30 and 36). This indicates that both proteins are involved in tumor development and are therefore likey targets for the development of anti-cancer therapies.

| **Table II** | |
|---|---|
| **NCBI gene** | **siRNA Target Sequence** |
| KLRG2 | CGAGGACAATCTGGATATCAA |
| | CTGGAGCCCTCGAGCAAGAAA |
| ERMP1 | CCCGTGGTTCATCTGATATAA |
| | AAGGACTTTGCTCGGCGTTTA |
| | TACGTGGATGTTTGTAACGTA |
| | CTCGTATTGGCTCAATCATAA |

### References

1) Anderson, L., and Seilhamer, J. (1997). A comparison of selected mRNA and protein abundances in human liver. Electrophoresis 18, 533 - 537;
2) Chen, G., Gharib, T. G., Wang, H., Huang, C. C., Kuick, R., Thomas, D. G., Shedden, K. A., Misek, D. E., Taylor, J. M., Giordano, T. J., Kardia, S. L., Iannettoni, M. D., Yee, J., Hogg, P. J., Orringer, M. B., Hanash, S. M., and Beer, D. G. (2003) Protein profiles associated with survival in lung adenocarcinoma. Proc. Natl. Acad. Sci. U. S. A 100, 13537 - 13542;
3) Ginestier, C., Charafe-Jauffret, E., Bertucci, F., Eisinger, F., Geneix, J., Bechlian, D., Conte, N., Adelaide, J., Toiron, Y., Nguyen, C., Viens, P., Mozziconacci, M. J., Houlgatte, R., Birnbaum, D., and Jacquemier, J. (2002) Distinct and complementary information provided by use of tissue and DNA microarrays in the study of breast tumor markers. Am. J. Pathol. 161, 1223 - 1233;
4) Gygi, S. P., Rochon, Y., Franza, B. R., and Aebersold, R. (1999) Correlation between protein and mRNA abundance in yeast. Mol. Cell. Biol. 19, 1720 - 1730; Nishizuka, S., Charboneau, L., Young, L., Major, S., Reinhold, W. C., Waltham, M., Kouros-Mehr, H., Bussey, K. J., Lee, J. K., Espina, V., Munson, P. J., Petricoin, E., III, Liotta, L. A., and Weinstein, J. N. (2003) Proteomic profiling of the NCI-60 cancer cell lines using new high-density reverse-phase lysate microarrays. Proc. Natl. Acad. Sci. U. S. A 100, 14229 - 14234;
5) Tyers, M., and Mann, M. (2003) From genomics to proteomics. Nature 422, 193 - 197;
6) Kagara N, Tanaka N, Noguchi S, Hirano T. (2007) Zinc and its transporter ZIP10 are involved in invasive behavior of breast cancer cells. Cancer Sci. 98:692 - 697;
7) Kasper G, Weiser AA, Rump A, Sparbier K, Dahl E, Hartmann A, Wild P, Schwidetzky U, Castaños-Vélez E, Lehmann K. (2005) Expression levels of the putative zinc transporter LIV-1 are associated with a better outcome of breast cancer patients. Int J Cancer. 117:961 - 973;
8) Nguyen ST, Hasegawa S, Tsuda H, Tomioka H, Ushijima M, Noda M, Omura K, Miki Y, (2007) Identification of a predictive gene expression signature of cervical lymph node metastasis in oral squamous cell carcinoma., Cancer Sci. 98:740 - 746;
9) Garcia-Rudaz,C., Luna,F., Tapia,V., Kerr,B., Colgin,L., Galimi,F., Dissen,G.A., Rawlings,N.D. and Ojeda,S.R. (2007) Fxna, a novel gene differentially expressed in the rat ovary at the time of folliculogenesis, is required for normal ovarian histogenesis. Development. 134, 945-957;
10) Kratzschmar J, Haendler B, Eberspaecher U, Roosterman D, Donner P, Schleuning WD. (1996) The human cysteine-rich secretory protein (CRISP) family. Primary structure and tissue distribution of CRISP-1, CRISP-2 and CRISP-3. Eur J Biochem. 236:827 - 836
11) Bjartell,A., Johansson,R., Bjork,T., adaleanu,V., Lundwall,A., Lilja,H., Kjeldsen,L. and Udby,L. (2006) Immunohistochemical detection of cysteine-rich secretory protein 3 in tissue and in serum from men with cancer or benign enlargement of the prostate gland, Prostate. 66: 591 - 603;
12) Bjartell,A.S., Al-Ahmadie,H., Serio,A.M., Eastham,J.A., Eggener,S.E., Fine,S.W., Udby,L., Gerald,W.L., Vickers,A.J., Lilja,H., Reuter,V.E. and Scardino,P.T. (2007), Association of cysteine-rich secretory protein 3 and beta-microseminoprotein with outcome after radical prostatectomy. Clin. Cancer Res. 13: 4130-4138.

## Claims

1. The use of KLRG2, SEQ ID NO: 85, SEQ ID NO: 86, or a different isoform having sequence identity of at least 80% to SEQ ID NO: 85 or SEQ ID NO: 86, or a nucleic acid molecule containing a sequence coding for a KLRG2 protein, as a tumor marker for the detection of breast cancer.

2. The use of claim 1, wherein the different isoform has at least 90%, or more preferably at least 95%, sequence identity to SEQ ID NO: 85 or SEQ ID NO: 86.

3. The use of claim 1, wherein said sequence coding for a KLRG2 protein is selected from SEQ ID NO: 87 or SEQ ID NO: 88.

4. A method of screening a tissue sample for malignancy, said method comprising determining the presence in said sample of a tumor marker as defined in any of claims 1 to 3.

5. The method of claim 4, wherein the tissue sample is a sample of breast tissue.

6. The method of claim 4 or 5, wherein the tumor marker is a protein, said method being based on immunoradiometric, immunoenzymatic or immunohistochemical techniques.

7. The method of claim 4 or 5, wherein either:
(i) the tumor marker is a nucleic acid molecule, said method being based on polymerase chain reaction techniques; or
(ii) said method includes detecting the tumor marker mRNA, optionally by means of polymerase chain reaction techniques or RT-PCR.

8. A method *in vitro* for determining the presence of a breast tumor in a subject, which comprises the steps of:
(a) providing a sample of the tissue suspected of containing tumor cells;
(b) determining the presence of a tumor marker as defined in any of claims 1 to 3 in said tissue sample by detecting the expression of the marker protein or the presence of the respective mRNA transcript;
wherein the detection of the tumor marker in the tissue sample is indicative of the presence of tumor in said subject.

9. A method of screening a test compound as an antitumor candidate, which comprises contacting cells expressing a tumor marker protein as defined in claim 1 or claim 2 with the test compound, and determining the binding of said compound to said tumor marker.

10. The method of claim 9, further comprising assaying the ability of the test compound to modulate the activity of the tumor marker protein.

11. An antibody or a fragment thereof which is able to specifically recognize and bind to KLRG2, SEQ ID NO: 85, SEQ ID NO: 86, or a different isoform having sequence identity of at least 80% to SEQ ID NO: 85 or SEQ ID NO: 86, for use in the treatment of proliferative diseases.

12. An antibody or a fragment thereof which is able to specifically recognize and bind to KLRG2, SEQ ID NO: 85, SEQ ID NO: 86, or a different isoform having sequence identity of at least 80% to SEQ ID NO: 85 or SEQ ID NO: 86, for use in the treatment of breast tumor.

13. The antibody or fragment thereof, for use of claim 12 or 13, wherein the different isoform has at least 90%, or more preferably at least 95%, sequence identity to SEQ ID NO: 85 or SEQ ID NO: 86.

14. The antibody or fragment thereof, for use of any of claims 11 to 13, wherein the antibody or fragment treats said proliferative diseases or breast tumor by modulating, preferably by inhibiting or abolishing, the activity of KLRG2, SEQ ID NO:85, SEQ ID NO: 86, or said different isoform.

15. The antibody, for use of any of claims 11 to 14, wherein the antibody is:
(i) monoclonal or polyclonal; and/or
(ii) labelled with a radioactive, fluorescent or enzyme label.

16. A diagnostic kit containing an antibody as defined in any of claims 11 to 15 and/or an oligonucleotide complementary to the nucleic acid molecule defined in claim 1 or claim 3, and optionally reagents, buffers, solutions and materials to carry out an immunoassay or a PCR assay.

17. A siRNA molecule having a sequence complementary to SEQ ID NO:89 or SEQ ID NO:90, for use in tumor-gene silencing, or in the treatment of breast tumor.
